# EUROPEAN PATENT APPLICATION

(11) **EP 4 772 502 A1**
(43) Date of publication of application: **08.07.2026**
(21) Application number: 24858716.4
(22) Date of filing: 30.08.2024
(51) Int. Cl.: C07D 333/04, C07D 409/10, C07D 321/12, A61K 31/381, A61K 31/335, A61P 3/10

(54) **ARYL OR HETEROARYL SUBSTITUTED CARBAMATE COMPOUND AND DERIVATIVE AND USE THEREOF**

(30) Priority: 31.08.2023 CN 202311128222; 14.08.2024 CN 202411118554
(71) Applicant: Shenzhen Zhongge Biological Technology Co., Ltd., Futian District Shenzhen, Guangdong 518017 (CN)
(72) Inventor: ZHAO, Liyu, Shenzhen, Guangdong 518017 (CN); CAO, Bin, Shenzhen, Guangdong 518017 (CN); MA, Nannan, Shenzhen, Guangdong 518017 (CN); CHEN, Zhaoqiang, Shenzhen, Guangdong 518017 (CN); MA, Jin, Shenzhen, Guangdong 518017 (CN); XIE, Dewei, Shenzhen, Guangdong 518017 (CN); YANG, Yuwen, Shenzhen, Guangdong 518017 (CN); DOU, Guosheng, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN); WEN, Shuhao, Shenzhen, Guangdong 518017 (CN)
(74) Representative: Botti & Ferrari S.p.A.
(86) International application number: PCT/CN2024/115748
(87) International publication number: WO 2025/045185

(57) **Abstract**

The present application relates to a compound of formula (I), a pharmaceutical composition comprising the compound, and a use of the pharmaceutical composition for treating and/or preventing diseases, disorders or conditions associated with AMPK kinase.

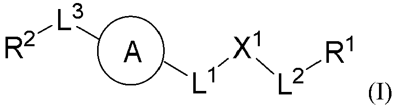

## Description

### Reference to Related Application

The present application claims priority to Chinese patent application CN 202311128222.7 filed on August 31, 2023 and Chinese patent application CN 202411118554.1 filed on August 14, 2024, the contents of which are incorporated herein by reference in their entirety and for all purposes.

### Technical Field

The present application relates to an aryl- or heteroaryl-substituted carbamate compound and a derivative thereof, a pharmaceutical composition comprising the compound or derivative, and use of the pharmaceutical composition for treating and/or preventing a disease, disorder or condition associated with AMPK kinase.

### Background Art

AMPK (adenosine monophosphate (AMP)-activated protein kinase) is a serine/threonine protein kinase, and a heterotrimeric complex consisting of a kinase domain-containing α-subunit and two regulatory subunits (β- and γ-subunits). AMPK is regulated by upstream kinases LKB1 (liver kinase B1) and CAMKK2 (calcium/calmodulin-dependent protein kinase kinase 2). Phosphorylation of Thr174 in the activation loop (T-loop) of its α-subunit (Thr172 in the α2 isoform) leads to a 500- to 1000-fold increase in activity. AMPK acts as a metabolic switch; activation of AMPK can affect lipid, cholesterol, carbohydrate, and amino acid metabolism, as well as mitochondrial function, autophagy, and cell growth.

Type II diabetes, also known as non-insulin-dependent diabetes, is a chronic metabolic disease mainly characterised by hyperglycaemia, insulin deficiency, and insulin resistance. Besides genetic factors, lifestyle and diet greatly influence the incidence of type II diabetes. Currently, the main drugs for treating type II diabetes include biguanides, sulphonylureas, thiazolidinediones, DPP-4 inhibitors, SGLT-2 inhibitors, and GLP-1 analogues. However, all currently used therapeutic drugs have certain side effects, so there is a need to develop hypoglycaemic drugs with novel mechanisms of action to meet the needs of diabetic patients.

AMPK reduces lipid storage by phosphorylating a variety of substrates. These pathways work together to promote fatty acid oxidation while inhibiting the synthesis of fatty acids and cholesterol. AMPK may also indirectly control the rate of fatty acid oxidation by regulating mitochondrial function. In addition to regulating fatty acid metabolism and indirectly affecting carbohydrate metabolism, AMPK also directly regulates carbohydrate metabolism pathways through a variety of mechanisms. Activation of AMPK can promote cellular glucose uptake by enhancing the translocation and expression of GLUT protein. It has been reported that AMPK agonists can significantly reduce blood glucose levels in mice, restoring them to normal; therefore, AMPK agonists have the potential to lower blood glucose (Cell Metabolism 25, 1147-1159, ACS Med. Chem. Lett. 2018, 9, 1, 39-44).

International patent application WO 2013153479 A2 discloses an indolecarboxylic acid compound with AMPK agonist activity: 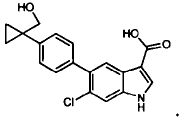 US patent application US 20150203450 A1 discloses an azaindole derivative with AMPK agonist activity: 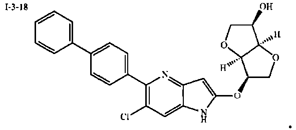 All these compounds possess fused bicyclic heteroaromatic rings. Similarly, WO 2012116145, WO 2014031441, WO 2014031445 and WO 2014139388 disclose indole or benzimidazole derivatives with fused bicyclic heteroaromatic rings, which are reported to have AMPK agonist activity.

Tamura et al. attempted to use compound 11 (a heteroaryl-substituted carbamate compound) as an AMPK agonist, but no AMPK agonist activity was 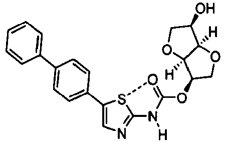 detected: Compound 11 . Tamura et al. pointed out that all existing AMPK agonists possess fused bicyclic heteroaromatic rings, thus concluding that the aromatic bicyclic ring is essential for AMPK agonist activity (Yuusuke Tamura et al., Identification of novel indole derivatives as highly potent AMPK activators with anti-diabetic profiles, Bioorganic & Medicinal Chemistry Letters, Vol. 68, 15 July 2022, 128769).

There is a need to develop novel AMPK agonist compounds for the treatment of diseases, disorders or conditions associated with AMPK kinase.

### Summary of the Invention

The present invention provides a novel AMPK agonist compound for the treatment and/or prevention of diseases, disorders or conditions associated with AMPK kinase. In some embodiments, the compounds of the present invention have an aryl-substituted carbamate structure. The inventors have surprisingly found that such compounds exhibit excellent AMPK agonist activity. The compounds of the present invention have better physicochemical properties (e.g., solubility, physical and/or chemical stability), improved pharmacokinetic properties (e.g., improved bioavailability, improved metabolic stability, and suitable half-life and duration of action), improved safety (lower toxicity (e.g., reduced cardiotoxicity) and/or fewer side effects), less tendency to develop drug resistance and other superior properties.

In one aspect, the present invention provides a compound of formula (I) as defined below: 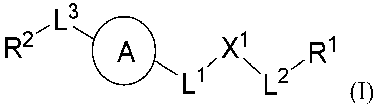 or a stereoisomer, a tautomer, a diastereomer, a racemate, a cis-trans isomer, an isotopically labelled compound (preferably a deuterated compound), an N-oxide, a metabolite, an ester, a prodrug, a crystal form, a hydrate, a solvate or a pharmaceutically acceptable salt thereof.

In another aspect, the present invention provides a pharmaceutical composition, comprising the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, and a pharmaceutically acceptable excipient, carrier or diluent. The pharmaceutical composition is preferably a solid preparation, a liquid preparation or a transdermal preparation.

In another aspect, the present invention provides the use of the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, or the pharmaceutical composition of the present invention in the preparation of a medicament as an AMPK agonist.

In another aspect, the present invention provides the use of the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, or the pharmaceutical composition of the present invention in the preparation of a medicament for treating and/or preventing a disease, disorder or condition associated with AMPK kinase.

In another aspect, the present invention provides the use of the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, or the pharmaceutical composition of the present invention in the preparation of a medicament for treating and/or preventing a disease, disorder or condition that can be treated and/or prevented by activating AMPK.

In another aspect, the present invention provides a method for treating and/or preventing a disease, disorder or condition associated with AMPK kinase, comprising administering to an individual in need thereof a therapeutically effective amount of the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, or the pharmaceutical composition of the present invention.

In another aspect, the present invention provides a method for treating and/or preventing a disease, disorder or condition that can be treated and/or prevented by activating AMPK, comprising administering to an individual in need thereof a therapeutically effective amount of the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to the present invention, or the pharmaceutical composition of the present invention.

In some embodiments, the disease, disorder or condition is preferably selected from type II diabetes, dyslipidaemia, obesity, chronic kidney disease, diabetic nephropathy, acute kidney injury, polycystic kidney disease and alopecia.

### Detailed Description of Embodiments

### Definitions

Unless otherwise defined hereinafter, all technical terms and scientific terms used herein are intended to have the same meanings as commonly understood by those skilled in the art. References to techniques used herein are intended to refer to techniques commonly understood in the art, including those variations of techniques or substitutions of equivalent techniques that are obvious to those skilled in the art. While the following terms are believed to be well understood by those skilled in the art, the following definitions are still set forth to better explain the present invention.

The terms "including", "comprising", "having", "containing" or "involving" and other variations thereof herein are inclusive or open-ended, and do not exclude other unrecited elements or method steps (i.e., these terms also encompass the terms "consisting essentially of" and "consisting of").

As used herein, the term "alkane" means a straight or branched saturated aliphatic hydrocarbon.

As used herein, the term "alkyl" means a straight or branched monovalent saturated aliphatic hydrocarbon, which can be regarded as a group obtained by losing 1 hydrogen atom from an alkane. In some embodiments, the alkyl has 1 to 12, such as 1 to 6 (e.g., 1, 2, 3, 4, 5 or 6) carbon atoms. For example, as used herein, the term "C₁₋₆ alkyl" refers to a straight or branched group having 1 to 6 carbon atoms, including "C₂₋₆ alkyl", "C₂₋₅ alkyl" and "C₁₋₄ alkyl". Examples of "C₁₋₆ alkyl" include methyl, ethyl, n-propyl, isopropyl, n-butyl, isobutyl, sec-butyl, tert-butyl, n-pentyl, and n-hexyl. The term "C₁₋₄ alkyl" refers to alkyl having 1 to 4 carbon atoms (i.e., methyl, ethyl, n-propyl, isopropyl, n-butyl, isobutyl, sec-butyl, or tert-butyl).

As used herein, the term "alkylene" refers to a group obtained by further losing 1 hydrogen atom from the "alkyl" as defined above. In some embodiments, the alkylene has 1 to 12 carbon atoms, preferably has 1, 2, 3, 4, 5, or 6 carbon atoms, such as "C₁₋₆ alkylene", "C₂₋₆ alkylene", "C₂₋₅ alkylene", and "C₁₋₄ alkylene". Examples of "C₁₋₆ alkylene" include methylene, ethylene, n-propylene, isopropylene, n-butylene, isobutylene, sec-butylene, tert-butylene, n-pentylene and n-hexylene. The term "C₁₋₄ alkylene" refers to alkylene having 1 to 4 carbon atoms.

As used herein, the term "alkoxy" refers to -O-alkyl, wherein the alkyl is as defined above.

As used herein, the term "alkenyl" means a straight or branched monovalent aliphatic hydrocarbyl comprising one or more double bonds. In some embodiments, the alkenyl has 2, 3, 4, 5 or 6 carbon atoms ("C₂₋₆ alkenyl", e.g., "C₂₋₄ alkenyl"). The alkenyl is, for example, -CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl, the compound can be present in the form of pure E (entgegen), the form of pure Z (zusammen) or the form of any mixture thereof. The term "alkenylene" is a corresponding divalent group, including, for example, "C₂₋₆ alkenylene", "C₂₋₄ alkenylene", etc., and specific examples thereof include, but are not limited to: -CH=CH-, -CH₂CH=CH-, -C(CH₃)=CH-, butenylene, pentenylene, hexenylene, cyclopentenylene, cyclohexenylene, etc.

As used herein, the term "alkynyl" means a straight or branched monovalent aliphatic hydrocarbyl comprising one or more triple bonds. In some embodiments, the alkynyl has 2, 3, 4, 5 or 6 carbon atoms ("C₂₋₆ alkynyl", e.g., "C₂₋₄ alkynyl"). The alkynyl is, for example, -C≡CH, -CH₂C≡CH, -C≡C-CH₃, -CH₂-C≡C-CH₃, 2-pentynyl, 3-pentynyl, 4-pentynyl, 2-hexynyl, 3-hexynyl, 4-hexynyl, 5-hexynyl, 3-methyl-2-butynyl and 2-methyl-3-pentynyl. The term "alkynylene" is a corresponding divalent group, including, for example, "C₂₋₆ alkynylene", "C₂₋₄ alkynylene", etc., and specific examples thereof include, but are not limited to: - C≡C-, -CH₂C≡C-, -C≡C-CH₂-, -CH₂-C≡C-CH₂-, pentynylene, hexynylene, etc.

As used herein, the term "fused" means that two or more cyclic structures share two adjacent atoms with each other.

As used herein, the terms "carbocyclic ring" and "carbocyclyl" refer to a ring or ring system in which all ring members are C atoms, and the ring or ring system may be saturated ("cycloalkyl"), partially unsaturated (e.g., having one or more double bonds within the ring, i.e., "cycloalkenyl"), or aromatic ("aryl"). The carbocyclic ring has, for example, 3-12 (suitably 3-10, 3-8, 3-7, 3-6, 4-6, or 5-6) ring carbon atoms.

As used herein, the terms "cyclohydrocarbyl", "hydrocarbon ring" and "cyclohydrocarbylene" refer to saturated (i.e., "cycloalkyl" and "cycloalkylene") or partially unsaturated (i.e., having one or more double bonds (i.e., "cycloalkenyl" and "cycloalkenylene") and/or triple bonds within the ring) monocyclic or polycyclic fused hydrocarbon rings having, for example, 3-12 (suitably 3-10, 3-8, 3-7, 3-6, 4-6 or 5-6) ring carbon atoms, including, but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclobutenyl(ene) (ring), cyclopentenyl(ene) (ring), cyclohexenyl(ene) (ring), cycloheptenyl(ene) (ring), cyclooctenyl(ene) (ring), cyclononenyl(ene) (ring), etc. In some embodiments, the cyclohydrocarbyl includes aryl-fused cyclohydrocarbyl, provided that the entire ring system is non-aromatic, such as

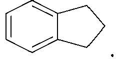

As used herein, the terms "cycloalkyl" and "cycloalkylene" refer to a saturated monocyclic or polycyclic (such as bicyclic) hydrocarbon ring (e.g., a monocyclic ring such as cyclopropyl, cyclobutyl, cyclopentyl, cyclohexyl, cycloheptyl, cyclooctyl, cyclononyl, or a bicyclic ring, including spiro, fused or bridged systems (such as bicyclo[1.1.1]pentyl, bicyclo[2.2.1]heptyl, bicyclo[3.2.1]octyl or bicyclo[5.2.0]nonyl, decalinyl, etc.). The cycloalkyl has 3-15 carbon atoms, suitably 3-12, 3-10, 3-8, 3-7, 3-6, 4-6 or 5-6 carbon atoms. For example, the terms "C₃₋₆ cycloalkyl" and "C₃₋₆ cycloalkylene" refer to a saturated monocyclic or polycyclic (such as bicyclic) hydrocarbon ring having 3 to 6 ring-forming carbon atoms (e.g., cyclopropyl, cyclobutyl, cyclopentyl or cyclohexyl).

As used herein, the terms "cycloalkenyl" and "cycloalkenylene" refer to a monocyclic or polycyclic (such as bicyclic) fused hydrocarbon ring (e.g., a monocyclic ring such as cyclopropenyl, cyclobutenyl, cyclopentenyl, cyclopentadienyl, cyclohexenyl, cyclohexadienyl, cycloheptenyl, cyclooctenyl, cyclononenyl, or a bicyclic ring) having 1 or more double bonds within the ring. The cycloalkenyl and "cycloalkenylene" have 3 to 10 carbon atoms, suitably 3-8, such as 3-7, 3-6, 4-6 or 5-6 carbon atoms.

As used herein, the terms "heterocyclyl", "heterocyclic ring" and "heterocyclylene" refer to saturated (i.e., "heterocycloalkyl" and "heterocycloalkylene") or partially unsaturated (e.g., having one or more double bonds within the ring (i.e., "heterocycloalkenyl" and "heterocycloalkenylene")) monocyclic or bicyclic structures, which have 2, 3, 4, 5, 6, 7, 8, or 9 carbon atoms and one or more (e.g., 1, 2, 3, or 4) heteroatom-containing groups selected from O, S, S(=O), S(=O)₂, and NR' in the ring, wherein R' is a suitable substituent, such as H or alkyl. The heterocyclic ring can be connected to the remainder of the molecule via any of the carbon atoms or a nitrogen atom (if present). In particular, a 3- to 12-membered heterocyclic ring is a group having 3-12 (e.g., 3-10, 3-8, 3-7, 3-6, 4-11, 4-9, 4-7, 4-6, 5-12, 5-6, 6-10, 6-9, 6-8, 7-11, or 8-12) ring atoms in the ring, the ring atoms including carbon atom(s) and heteroatom(s). Examples that can be listed include, but are not limited to, oxiranyl, aziridinyl, azetidinyl, oxetanyl, tetrahydrofuryl, tetrahydrothienyl, dioxolinyl, pyrrolidyl, pyrrolidonyl, oxazolidine, thiazolidinyl, pyrazolidinyl, imidazolidinyl, pyrrolinyl, tetrahydropyranyl, piperidyl, hexahydropyrimidyl, triazinanyl, morpholinyl, dithianyl, thiomorpholinyl, piperazinyl, trithianyl, azocanyl, dihydropyrrolyl, dihydroimidazolyl, azocinyl. The heterocyclyl includes aryl- or heteroaryl-fused heterocyclyl, provided that the entire ring system is non-aromatic, such as

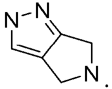

As used herein, the heterocyclic ring described above includes a nitrogen-containing heterocyclic ring, an oxygen-containing heterocyclic ring, and a sulphur-containing heterocyclic ring. For example, the "nitrogen-containing heterocyclic ring" has at least one nitrogen atom, and may optionally have one or more (e.g., one, two, three or four) ring members selected from N, O, C=O, S, S=O and S(=O)₂. The nitrogen-containing heterocyclic ring is connected to the remainder of the molecule via a nitrogen atom. The nitrogen-containing heterocyclic ring is preferably a saturated nitrogen-containing monocyclic ring. In particular, a 3- to 12-membered nitrogen-containing heterocyclic ring is a group having 3-12 ring atoms in the ring, the ring atoms including carbon atom(s) and heteroatom(s) (at least one of which is a nitrogen atom), and includes, but not limited to a three-membered nitrogen-containing heterocyclic ring (such as aziridinyl), a four-membered nitrogen-containing heterocyclic ring (such as azetidinyl), a five-membered nitrogen-containing heterocyclic ring (such as pyrrolyl, pyrrolidyl (pyrrolidine ring), pyrrolinyl, pyrrolidonyl, imidazolyl, imidazolidinyl, imidazolinyl, pyrazolyl, and pyrazolinyl), a six-membered nitrogen-containing heterocyclic ring (such as piperidyl (piperidine ring), morpholinyl, thiomorpholinyl, and piperazinyl), a seven-membered nitrogen-containing heterocyclic ring, etc. The "oxygen-containing heterocyclic ring" has at least one oxygen atom, and may optionally have one or more (e.g., one, two, three or four) ring members selected from N, C=O, S, S=O and S(=O)₂. The oxygen-containing heterocyclic ring is preferably a saturated oxygen-containing monocyclic or bicyclic ring. In particular, a 3- to 12-membered oxygen-containing heterocyclic ring is a group having 3-12 ring atoms in the ring, the ring atoms including carbon atom(s) and heteroatom(s) (at least one of which is an oxygen atom), and includes, but not limited to a three-membered oxygen-containing heterocyclic ring (such as oxiranyl), a four-membered oxygen-containing heterocyclic ring (such as oxetanyl), a five-membered oxygen-containing heterocyclic ring (such as tetrahydrofuryl or dihydrofuryl), a six-membered oxygen-containing heterocyclic ring (such as tetrahydropyranyl or morpholinyl), a 8- to 10-membered fused bicyclic oxygen-containing heterocyclic ring (such as 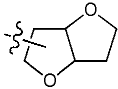 ), etc.

As used herein, the term "aryl" refers to an all-carbon monocyclic or fused polycyclic aromatic group having a conjugated π-electron system. For example, as used herein, the term "C₆₋₁₀ aryl" means an aromatic group containing 6 to 10 carbon atoms, such as phenyl (benzene ring) or naphthyl.

As used herein, the terms "heteroaryl" and "aromatic heterocyclic ring" refer to a monocyclic or polycyclic (e.g., bicyclic or tricyclic) aromatic ring system having 5 to 14 ring atoms, e.g., 5, 6, 7, 8, 9, 10, 11, 12, 13 or 14 ring atoms, in particular having 1, 2, 3, 4, 5, 6, 7, 8, 9, 10, 11, 12 or 13 carbon atoms and 1, 2, 3, 4 or 5 identical or different heteroatoms independently selected from N, O, S and S(O)₂. One or more ring carbon atoms in the heteroaryl may be replaced by C(O). The heteroaryl may be benzo-fused. Examples of heteroaryl include, but are not limited to: pyridyl, pyridonyl, pyrimidyl, pyrimidonyl, pyrazinyl, pyridazinyl, thiazolyl, thienyl, oxazolyl, furyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazolopyridyl, quinolyl, indolyl, pyrrolopyridazinyl, benzofuryl, benzothienyl, indazolyl, benzooxazolyl, benzoisoxazolyl, quinazolinyl, pyrrolopyridyl, pyrazolopyrimidyl, imidazopyridazinyl, pyrazolopyridyl, triazolopyridyl, isoquinolinyl, tetrahydroisoquinolinyl, benzimidazolyl, cinnolinyl, indolizinyl, phthalazinyl, isoindolyl, pteridinyl, purinyl, furazanyl, benzofurazanyl, quinoxalinyl, naphthyridinyl, or furopyridyl.

As used herein, the term "oxo" or "oxo group" is defined as =O.

As used herein, the term "halo" or "halogen" group is defined as including F, Cl, Br, or I.

As used herein, the term "haloalkyl" refers to alkyl substituted with one or more (such as 1 to 3) identical or different halogen atoms, wherein the alkyl is as defined herein. The terms "C₁₋₈ haloalkyl", "C₁₋₆ haloalkyl" and "C₁₋₄ haloalkyl" refer to haloalkyl having 1 to 8 carbon atoms, 1 to 6 carbon atoms and 1 to 4 carbon atoms, respectively, such as -CF₃, -C₂F₅, -CHF₂, -CH₂F, -CH₂CF₃, -CH₂Cl, or -CH₂CH₂CF₃.

As used herein, the term "haloalkenyl" refers to alkenyl substituted with one or more (such as 1 to 3) identical or different halogen atoms, wherein the alkenyl is as defined herein. The terms "C₂₋₈ haloalkenyl", "C₂₋₆ haloalkenyl" and "C₂₋₄ haloalkenyl" refer to haloalkenyl having 2 to 8 carbon atoms, 2 to 6 carbon atoms and 2 to 4 carbon atoms, respectively.

As used herein, the term "haloalkynyl" refers to alkynyl substituted with one or more (such as 1 to 3) identical or different halogen atoms, wherein the alkynyl is as defined herein. The terms "C₂₋₈ haloalkynyl", "C₂₋₆ haloalkynyl" and "C₂₋₄ haloalkynyl" refer to haloalkynyl having 2 to 8 carbon atoms, 2 to 6 carbon atoms and 2-4 carbon atoms, respectively.

The term "substituted" means that one or more (e.g., one, two, three, or four) hydrogens on a specified atom are replaced by selections from the indicated groups, provided that the substitution does not exceed the normal valence of the specified atom under current circumstances; and the substitution results in a stable compound. Combinations of substituents and/or variables are permissible only if such combinations form stable compounds.

If a group is described as "optionally substituted with" or "optionally substituted", the group can be: (1) unsubstituted or (2) substituted. If a carbon of a group is described as being optionally substituted with one or more of the listed substituents, one or more hydrogens on that carbon (to the extent of any hydrogen(s) present) may be replaced individually and/or collectively by independently selected optional substituents. If a nitrogen of a group is described as being optionally substituted with one or more of the listed substituents, one or more hydrogens on that nitrogen (to the extent of any hydrogen(s) present) may each be replaced by independently selected optional substituents.

If a substituent is described as "independently selected from" a group, each substituent is selected independently of the other. Thus, each substituent may be the same as or different from another (other) substituent.

As used herein, the term "one or more" means 1 or more than 1 under reasonable conditions, such as 2, 3, 4, 5, or 10.

Unless otherwise indicated, as used herein, the attachment point of the substituent may be at any suitable position of the substituent.

When the bond of a substituent is shown to pass through a bond connecting two atoms in a ring ("floating bond"), such a substituent may be bonded to any ring-forming atom in the substitutable ring, unless otherwise indicated. In cases where an available ring member is shown to carry a substitutable hydrogen atom, the substitutable hydrogen atom is substantially substituted (i.e., not present) when the floating bond is attached to the available ring member.

The present invention also includes all pharmaceutically acceptable isotopically labelled compounds that are identical to the compounds of the present invention except that one or more atoms are replaced by atoms having the same atomic number but having an atomic mass or mass number different from the atomic mass or mass number prevailing in nature. Examples of isotopes suitable for inclusion in the compound of the present invention include, but are not limited to, isotopes of hydrogen (e.g., deuterium (D,²H), tritium (T,³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g. ¹⁵O, ¹⁷O and ¹⁸O); isotopes of phosphorus (e.g. ³²P); and isotopes of sulphur (e.g., ³⁵S). Certain isotopically labelled compounds of the present invention (such as those incorporating radioactive isotopes) can be used in drug and/or substrate tissue distribution studies (e.g., analysis). The radioactive isotopes tritium (i.e.,³H) and carbon-14 (i.e., ¹⁴C) are particularly useful for this purpose because of their ease of incorporation and ease of detection. Substitution with positron emitting isotopes (e.g., ¹¹C, ¹⁸F, ¹⁵O, and ¹³N) may be used to examine substrate receptor occupancy in positron emission tomography (PET) studies. Isotopically labelled compounds of the present invention can be prepared by methods similar to those described in the accompanying schemes and/or examples and preparations by using appropriate isotopically labelled reagents instead of previously employed non-labelled reagents. Pharmaceutically acceptable solvates of the present invention include those in which the crystallisation solvent may be isotopically substituted, for example, D₂O, acetone-*d₆* or DMSO-*d₆*. In some embodiments, the isotopically labelled compounds of the present invention are deuterated compounds.

The term "stereoisomer" refers to an isomer which is formed due to at least one asymmetric centre and has the same chemical composition but differs in the spatial arrangement of atoms or groups. In compounds having one or more (e.g., 1, 2, 3, or 4) asymmetric centres, racemic mixtures, single enantiomers, diastereomer mixtures, and individual diastereomers can be generated. Specific individual molecules may also exist in geometric isomers (cis/trans). Similarly, the compounds of the present invention may exist as mixtures of two or more structurally distinct forms in rapid equilibrium, commonly referred to as tautomers. Representative examples of tautomers include keto-enol tautomers, phenol-ketone tautomers, nitroso-oxime tautomers, imine-enamine tautomers, etc. It is to be understood that the scope of the present application encompasses all such isomers or mixtures thereof in any ratio (e.g., 60%, 65%, 70%, 75%, 80%, 85%, 90%, 95%, 96%, 97%, 98%, 99%).

"Diastereomers" refer to stereoisomers having two or more chiral centres and whose molecules are not mirror images of each other. Diastereomers have different physical properties, such as melting points, boiling points, spectral properties and reactivity. Mixtures of diastereomers can be separated by high-resolution analytical methods such as electrophoresis and chromatography.

"Enantiomers" refer to two stereoisomers of a compound that are non-superimposable mirror images of each other.

The term "chiral" refers to molecules having the property of non-superimposability of mirror image pairs, while the term "achiral" refers to molecules that are superimposable on their mirror image pairs.

The compounds of the present invention can be prepared in racemic form, or single enantiomers can be prepared by enantioselective synthesis or by resolution.

As used herein, the term "cis-trans isomer" or "geometric isomer" is caused by the fact that double bonds or single bonds of ring-forming carbon atoms cannot rotate freely. The compounds provided herein include all cis, trans, syn, anti, entgegen (E) and zusammen (Z) isomers and their corresponding mixtures.

The solid line (-), solid wedge ( 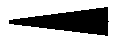 ) or dashed wedge ( 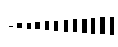 ) can be used herein to depict a chemical bond of the compounds of the present invention. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and dashed wedges are used to define the relative stereochemistry rather than the absolute stereochemistry. Unless otherwise indicated, the compounds of the present invention may exist in forms of stereoisomers, including cis and trans isomers, optical isomers (e.g., R- and S-enantiomers), diastereomers, geometric isomers, rotamers, conformers, atropisomers, and mixtures thereof. The compounds of the present invention may exhibit more than one type of isomerism, and consist of mixtures thereof (such as racemic mixtures and diastereomeric pairs).

It will also be understood that certain compounds of the present invention may be present in free forms for therapeutic use or, where appropriate, in the form of pharmaceutically acceptable derivatives thereof. In the present invention, pharmaceutically acceptable derivatives include, but are not limited to, pharmaceutically acceptable salts, esters, solvates, metabolites or prodrugs that, upon administration thereof to a patient in need thereof, are capable of providing, directly or indirectly, the compound of the present invention or a metabolite or residue thereof. Therefore, when reference is made herein to a "compound of the present invention", it is also intended to encompass the above-mentioned various derivative forms of the compound.

The term "pharmaceutically acceptable" means that the substance or composition must be chemically and/or toxicologically compatible with the other ingredients making up the preparation and/or the mammal to be treated therewith.

Pharmaceutically acceptable salts of the compounds of the present invention include acid addition salts and base addition salts thereof.

Suitable acid addition salts are formed from acids that form pharmaceutically acceptable salts. Examples include aspartate, benzoate, bicarbonate/carbonate, bisulphate/sulphate, fumarate, glucoheptonate, gluconate, glucuronate, hexafluorophosphate, hydrobromide/bromide, hydroiodate/iodide, maleate, malonate, methylsulphate, naphthylate, nicotinate, nitrate, orotate, oxalate, palmitate and other similar salts.

Suitable base addition salts are formed from bases that form pharmaceutically acceptable salts. Examples include aluminium salts, arginine salts, choline salts, diethylamine salts, lysine salts, magnesium salts, meglumine salts, potassium salts and other similar salts.

A review of suitable salts is provided in "Handbook of Pharmaceutical Salts: Properties, Selection, and Use" by Stahl and Wermuth (Wiley-VCH, 2002). Methods for preparing pharmaceutically acceptable salts of the compounds of the present invention are known to those skilled in the art.

As used herein, the term "ester" means an ester derived from the respective compound of the general formula in the present application, including a physiologically hydrolysable ester (which can be hydrolyzed under physiological conditions to release the compound of the present invention in a free acid or alcohol form). The compounds of the present invention may also be esters themselves.

The present invention encompasses all possible crystal forms or polymorphs of the compounds of the present invention, which may be a single polymorph or a mixture of more than one polymorph in any ratio.

The compound of the present invention may exist in the form of a solvate (preferably a hydrate), in which the compound of the present invention comprises a polar solvent as a structural element of the crystal lattice of the compound, in particular, for example, water, methanol or ethanol. The amount of the polar solvent, in particular water, may be present in a stoichiometric or non-stoichiometric ratio.

Those skilled in the art will appreciate that not all nitrogen-containing heterocyclic rings are capable of forming N-oxides, as nitrogen requires an available lone pair of electrons to be oxidized to an oxide; Those skilled in the art will recognise nitrogen-containing heterocyclic rings that are capable of forming N-oxides. Those skilled in the art will also recognise that tertiary amines are capable of forming N-oxides. Synthetic methods for preparing N-oxides of heterocyclic rings and tertiary amines are well known to those skilled in the art and include oxidation of heterocyclic rings and tertiary amines with peroxyacids such as peracetic acid and m-chloroperbenzoic acid (MCPBA), hydrogen peroxide, alkyl hydroperoxides such as tert-butyl hydroperoxide, sodium perborate, and dioxirane such as dimethyldioxirane. These methods for preparing N-oxides have been extensively described and reviewed in the literature, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Also included within the scope of the present invention are metabolites of the compounds of the present invention, i.e. substances formed in vivo upon administration of the compounds of the present invention. Such products can be produced, for example, by oxidation, reduction, hydrolysis, amidation, deamidation, esterification, delipidation, enzymatic hydrolysis, and the like of the compounds being administered. Accordingly, the present invention includes metabolites of the compounds of the present invention, and the metabolites include compounds prepared by a process of contacting the compounds of the present invention with a mammal for a time period sufficient to produce their metabolites.

The present invention further includes within its scope prodrugs of the compounds of the present invention, which are certain derivatives of the compounds of the present invention that may themselves have little or no pharmacological activity but are converted, for example by hydrolytic cleavage, into compounds of the present invention having the desired activity when administered into or onto the body. Typically, such prodrugs will be functional derivatives of the compounds that are readily converted in vivo to the desired therapeutically active compound. Additional information on the use of prodrugs can be found in "Pro-drugs as Novel Delivery Systems", Volume 14, ACS Symposium Series (T. Higuchi and V. Stella) and "Bioreversible Carriers in Drug Design", Pergamon Press, 1987 (edited by E. B. Roche, American Pharmaceutical Association). Prodrugs of the present invention can be prepared, for example, by replacing appropriate functional groups present in the compounds of the present invention with certain moieties known to those skilled in the art as "pro-moieties" (e.g., as described in "Design of Prodrugs," H. Bundgaard (Elsevier, 1985)).

The present invention also encompasses the compounds of the present invention containing protecting groups. In any process of preparing the compounds of the present invention, it may be necessary and/or desirable to protect sensitive or reactive groups on any of the molecules concerned, thereby forming chemically protected forms of the compounds of the present invention. This can be achieved using conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which references are incorporated herein by reference. The protecting groups may be removed at an appropriate subsequent stage using methods known in the art.

As used herein, the term "about" refers to within a range of ± 10%, preferably within a range of ± 5%, more preferably within a range of ± 2% of the stated numerical value.

### Compound

In one aspect, the present invention provides a compound of formula (I): 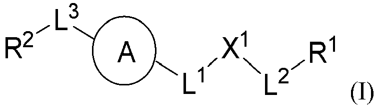 or a stereoisomer, a tautomer, a diastereomer, a racemate, a cis-trans isomer, an isotopically labelled compound (preferably a deuterated compound), an N-oxide, a metabolite, an ester, a prodrug, a crystal form, a hydrate, a solvate or a pharmaceutically acceptable salt thereof, wherein:
X¹ is selected from -C(=O)-, -C(=S)- and -CH₂-;
L¹ is selected from -O-, -S-, -NR^{c}- and -CR^{a}R^{b}-;
L² is selected from -O-, -S-, -NR^{c}-, -CR^{a}R^{b}-, -O-CR^{a}R^{b}- and -S-CR^{a}R^{b}-;
alternatively, -L¹-X¹-L²-, as a whole, is -NH-;
R¹ is selected from C₁₋₆ alkyl, C₃₋₈ cycloalkyl, 4- to 10-membered heterocyclyl, C₆₋₁₀ aryl and 5- to 10-membered heteroaryl, wherein the C₁₋₆ alkyl, C₃₋₈ cycloalkyl, 4- to 10-membered heterocyclyl, C₆₋₁₀ aryl and 5- to 10-membered heteroaryl are optionally substituted with 1, 2 or 3 R^{1a};
R^{1a} is independently selected from halogen, -OH, -CN, an oxo group, -COOR^{c}, -C(=O)NHR^{c}, C₁₋₆ alkyl, C₁₋₆ haloalkyl, C₁₋₆ alkoxy, C₁₋₆ haloalkoxy, -C₁₋₆ alkylene-OH, -OC₁₋₆ alkylene-OH, -C₁₋₆ alkylene-NH₂, -C₁₋₆ alkylene-COOR^{c}, -C₁₋₆ alkylene-C(=O)NHR^{c}, -S(=O)₂-C₁₋₆ alkyl and 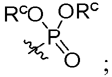
R^{a} and R^{b}, at each occurrence, are each independently selected from H, D, halogen, -OH, -CN, C₁₋₆ alkyl, C₁₋₆ alkoxy, C₁₋₆ haloalkyl, C₁₋₆ haloalkoxy and C₃₋₆ cycloalkyl;
R^{c}, at each occurrence, is independently selected from H, D, C₁₋₆ alkyl, C₁₋₆ haloalkyl and C₃₋₆ cycloalkyl;
ring A 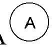 is selected from a benzene ring and a 5- to 6-membered aromatic heterocyclic ring, and the benzene ring and 5- to 6-membered aromatic heterocyclic ring are each optionally substituted with 1, 2 or 3 R^{A}, wherein the R^{A} is independently selected from H, D, halogen, -OH, -NH₂, -CN, C₁₋₆ alkyl, C₂₋₆ alkenyl, C₂₋₆ alkynyl, C₁₋₆ alkoxy, C₁₋₆ haloalkyl, C₂₋₆ haloalkenyl, C₂₋₆ haloalkynyl, C₁₋₆ haloalkoxy, C₃₋₆ cycloalkyl, 3- to 6-membered heterocyclyl, phenyl, 5- to 6-membered heteroaryl, -NH(C₁₋₆ alkyl) and -N(C₁₋₆ alkyl)₂; alternatively, where applicable, two adjacent R^{A}, together with the ring atoms to which they are attached, may form C₄₋₆ carbocyclyl, 4- to 6-membered heterocyclyl or 5- to 6-membered heteroaryl;
L³ is selected from a bond, -O-, -S-, -NR^{L3}-, -C(=O)-NR^{L3}-, -C(=O)-O-, -C₁₋₆ alkylene-O-, -C₁₋₆ alkylene-NR^{L3}-, -C₁₋₆ alkylene-S-, -C₂₋₆ alkenylene- and -C₂₋₆ alkynylene-;
R^{L3} is selected from H, D and C₁₋₆ alkyl;
R² is selected from C₆₋₁₀ aryl, C₃₋₁₀ saturated or partially unsaturated cyclohydrocarbyl, 4- to 10-membered heterocyclyl and 5- to 10-membered heteroaryl, and the C₆₋₁₀ aryl, C₃₋₁₀ saturated or partially unsaturated cyclohydrocarbyl, 4- to 10-membered heterocyclyl and 5- to 10-membered heteroaryl are optionally substituted with 1, 2, 3, 4 or 5 groups each independently selected from R^{2a}, R^{2b}, R^{2c}, R^{2d} and R^{2e};
R^{2a}, R^{2b}, R^{2c}, R^{2d} and R^{2e} are each independently selected from H, halogen, - OH, -CN, C₁₋₆ alkyl, C₁₋₆ haloalkyl, -NH₂, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl, wherein the C₁₋₆ alkyl, C₁₋₆ haloalkyl, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl are each optionally substituted with 1, 2 or more R; and
R is independently selected from: H, halogen, -OH, -CN, -NH₂, an oxo group, -COOH, C₁₋₆ alkyl, C₁₋₆ haloalkyl, -C₁₋₆ alkyl substituted with 1 or 2 OH, C₁₋₆ alkoxy, -C₁₋₆ alkyleneoxy-C₁₋₆ alkoxy, -C(=O)NH-C₁₋₆ alkyl, -C(=O)NH-C₃₋₁₀ cycloalkyl, - C(=O)-C₁₋₆ alkyl, -NHC(=O)-C₁₋₆ alkyl, -N=S(=O)R^{s1}R^{s2}, -NH-S(=O)₂C₁₋₆ alkyl, - S(=O)₂-C₁₋₆ alkyl, -S(=O)₂-4- to 10-membered heterocyclyl, -S(=O)₂NH-C₁₋₆ alkyl, -S(=O)₂NH-C₃₋₁₀ cycloalkyl, -C₃₋₁₀ cycloalkylene-COOH, -C(=O)-4- to 10-membered heterocyclyl, 4- to 10-membered heterocyclyl, C₆₋₁₀ aryl and 5- to 10-membered heteroaryl, wherein the 4- to 10-membered heterocyclyl is optionally substituted with 1, 2 or more substituents independently selected from C₁₋₆ alkyl, - NH₂, =NH and an oxo group, the C₆₋₁₀ aryl and 5- to 10-membered heteroaryl are each optionally substituted with 1, 2 or more substituents independently selected from halogen, -OH, -CN, -NH₂, -C₁₋₆ alkylene-OH, -C₁₋₆ alkylene-CN and -C₁₋₆ alkylene-NH₂, and R^{s1} and R^{s2} are each C₁₋₆ alkyl, alternatively, R^{s1} and R^{s2}, together with the S atom to which they are attached, form 4- to 6-membered heterocycloalkyl; provided that:
   (1) the compound of formula (I) is not the following compound: 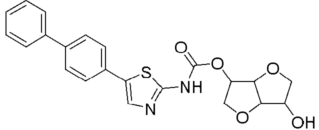 (including 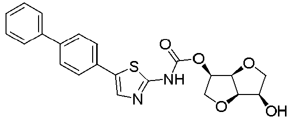 ), or a stereoisomer, a tautomer, a diastereomer, a racemate, a cis-trans isomer, an isotopically labelled compound, an N-oxide, a metabolite, an ester, a prodrug, a crystal form, a hydrate, a solvate or a pharmaceutically acceptable salt thereof; and
   (2) when ring A is a thiazole ring, L³ is -NR^{L3}-, and R² is substituted or unsubstituted phenyl, L¹ is not -CR^{a}R^{b}-;
   (3) when -L¹-X¹-L²-, as a whole, is -NH-, 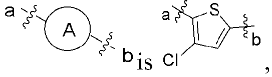 and R² is C₆₋₁₀ aryl, wherein the bond labelled "a" is attached to L³ and the bond labelled "b" is attached to L¹.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein:
X¹ is selected from -C(=O)-, -C(=S)- and -CH₂-;
L¹ is selected from -O-, -S-, -NR^{c}- and -CR^{a}R^{b}-;
L² is selected from -O-, -S-, -NR^{c}-, -CR^{a}R^{b}-, -O-CR^{a}R^{b}- and -S-CR^{a}R^{b}-;
R¹ is selected from C₁₋₆ alkyl, C₃₋₈ cycloalkyl, 4- to 10-membered heterocyclyl, C₆₋₁₀ aryl and 5- to 10-membered heteroaryl, wherein the C₁₋₆ alkyl, C₃₋₈ cycloalkyl, 4- to 10-membered heterocyclyl, C₆₋₁₀ aryl and 5- to 10-membered heteroaryl are optionally substituted with 1, 2 or 3 R^{1a};
R^{1a} is independently selected from halogen, -OH, -CN, an oxo group, -COOR^{c}, -C(=O)NHR^{c}, C₁₋₆ alkyl, C₁₋₆ haloalkyl, C₁₋₆ alkoxy, C₁₋₆ haloalkoxy, -C₁₋₆ alkylene-OH, -C₁₋₆ alkylene-NH₂, -C₁₋₆ alkylene-COOR^{c}, -C₁₋₆ alkylene-C(=O)NHR^{c}, - S(=O)₂-C₁₋₆ alkyl and 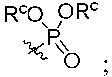
R^{a} and R^{b}, at each occurrence, are each independently selected from H, D, halogen, -OH, -CN, C₁₋₆ alkyl, C₁₋₆ alkoxy, C₁₋₆ haloalkyl, C₁₋₆ haloalkoxy and C₃₋₆ cycloalkyl;
R^{c}, at each occurrence, is independently selected from H, D, C₁₋₆ alkyl, C₁₋₆ haloalkyl and C₃₋₆ cycloalkyl;
ring A 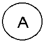 is selected from a benzene ring and a 5- to 6-membered aromatic heterocyclic ring, and the benzene ring and 5- to 6-membered aromatic heterocyclic ring are each optionally substituted with 1, 2 or 3 R^{A}, wherein the R^{A} is independently selected from halogen, -OH, -NH₂, -CN, C₁₋₆ alkyl, C₂₋₆ alkenyl, C₂₋₆ alkynyl, C₁₋₆ alkoxy, C₁₋₆ haloalkyl, C₂₋₆ haloalkenyl, C₂₋₆ haloalkynyl, C₁₋₆ haloalkoxy, C₃₋₆ cycloalkyl, 3- to 6-membered heterocyclyl, phenyl, 5- to 6-membered heteroaryl, -NH(C₁₋₆ alkyl) and -N(C₁₋₆ alkyl)₂; alternatively, where applicable, two adjacent R^{A}, together with the ring atoms to which they are attached, may form C₄₋₆ carbocyclyl, 4- to 6-membered heterocyclyl or 5- to 6-membered heteroaryl;
L³ is selected from a bond, -O-, -S-, -NR^{L3}-, -C(=O)-NR^{L3}-, -C(=O)-O-, -C₁₋₆ alkylene-O-, -C₁₋₆ alkylene-NR^{L3}-, -C₁₋₆ alkylene-S-, -C₁₋₆ alkylene-, -C₂₋₆ alkenylene- and -C₂₋₆ alkynylene-;
R^{L3} is selected from H, D and C₁₋₆ alkyl;
R² is selected from C₆₋₁₀ aryl, C₃₋₁₀ saturated or partially unsaturated cyclohydrocarbyl, 4- to 10-membered heterocyclyl and 5- to 10-membered heteroaryl, and the C₆₋₁₀ aryl, C₃₋₁₀ saturated or partially unsaturated cyclohydrocarbyl, 4- to 10-membered heterocyclyl and 5- to 10-membered heteroaryl are optionally substituted with 1, 2, 3, 4 or 5 groups each independently selected from R^{2a}, R^{2b}, R^{2c}, R^{2d} and R^{2e};
R^{2a}, R^{2b}, R^{2c}, R^{2d} and R^{2e} are each independently selected from H, halogen, - OH, -CN, C₁₋₆ alkyl, C₁₋₆ haloalkyl, -NH₂, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl, wherein the C₁₋₆ alkyl, C₁₋₆ haloalkyl, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl are each optionally substituted with 1, 2 or more R; and
R is independently selected from: H, halogen, -OH, -CN, -NH₂, an oxo group, -COOH, C₁₋₆ alkyl, C₁₋₆ haloalkyl, -C₁₋₆ alkyl substituted with 1 or 2 OH, C₁₋₆ alkoxy, -C₁₋₆ alkyleneoxy-C₁₋₆ alkoxy, -C(=O)NH-C₁₋₆ alkyl, -C(=O)NH-C₃₋₁₀ cycloalkyl, - C(=O)-C₁₋₆ alkyl, -NHC(=O)-C₁₋₆ alkyl, -N=S(=O)R^{s1}R^{s2}, -NH-S(=O)₂C₁₋₆ alkyl, - S(=O)₂-C₁₋₆ alkyl, -S(=O)₂-4- to 10-membered heterocyclyl, -S(=O)₂NH-C₁₋₆ alkyl, -S(=O)₂NH-C₃₋₁₀ cycloalkyl, -C₃₋₁₀ cycloalkylene-COOH, -C(=O)-4- to 10-membered heterocyclyl, 4- to 10-membered heterocyclyl, C₆₋₁₀ aryl and 5- to 10-membered heteroaryl, wherein the 4- to 10-membered heterocyclyl is optionally substituted with 1, 2 or more substituents independently selected from C₁₋₆ alkyl, - NH₂, =NH and an oxo group, the C₆₋₁₀ aryl and 5- to 10-membered heteroaryl are each optionally substituted with 1, 2 or more substituents independently selected from halogen, -OH, -CN, -NH₂, -C₁₋₆ alkylene-OH, -C₁₋₆ alkylene-CN and -C₁₋₆ alkylene-NH₂, and R^{s1} and R^{s2} are each C₁₋₆ alkyl, alternatively, R^{s1} and R^{s2}, together with the S atom to which they are attached, form 4- to 6-membered heterocycloalkyl;
provided that:
   (1) the compound of formula (I) is not the following compound: 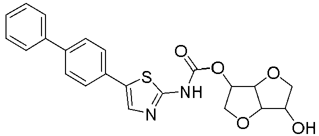 (including 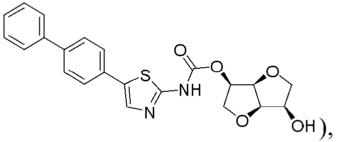 or a stereoisomer, a tautomer, a diastereomer, a racemate, a cis-trans isomer, an isotopically labelled compound, an N-oxide, a metabolite, an ester, a prodrug, a crystal form, a hydrate, a solvate or a pharmaceutically acceptable salt thereof; and
   (2) when ring A is a thiazole ring, L³ is -NR^{L3}-, and R² is substituted or unsubstituted phenyl, L¹ is not -CR^{a}R^{b}-.

In some embodiments, the present invention provides the compound of formula (I), wherein: when ring A is a thiazole ring and L³ is absent, R² is not unsubstituted biphenyl.

In some embodiments, the present invention provides the compound of formula (I), wherein ring A is selected from a benzene ring and a 5- to 6-membered aromatic heterocyclic ring, the benzene ring and 5- to 6-membered aromatic heterocyclic ring are each optionally substituted with 1, 2 or 3 R^{A}, and the R^{A} is independently selected from H, D, F, Cl, Br, -OH, -NH₂, -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, phenyl, -NH(C₁₋₄ alkyl) and -N(C₁₋₄ alkyl)₂; where applicable, two adjacent R^{A}, together with the ring atoms to which they are attached, may form C₅₋₆ carbocyclyl, 5- to 6-membered heterocyclyl or 5-to 6-membered heteroaryl.

In some preferred embodiments, ring A is selected from a benzene ring and a 5- to 6-membered aromatic heterocyclic ring, wherein the benzene ring and 5- to 6-membered aromatic heterocyclic ring are each optionally substituted with 1, 2 or 3 R^{A}, and the R^{A} is independently selected from H, D, F, Cl, Br, -OH, -NH₂, -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, - NH(C₁₋₄ alkyl) and -N(C₁₋₄ alkyl)₂; where applicable, two adjacent R^{A}, together with the ring atoms to which they are attached, may form C₅₋₆ carbocyclyl, 5- to 6-membered heterocyclyl or 5- to 6-membered heteroaryl.

In some more preferred embodiments, ring A is selected from a benzene ring and a 5- to 6-membered aromatic heterocyclic ring, wherein the benzene ring and 5-to 6-membered aromatic heterocyclic ring are each optionally substituted with 1, 2 or 3 R^{A}, and the R^{A} is independently selected from H, D, F, Cl, -OH, -NH₂, -CN, methyl, ethyl, isopropyl, sec-butyl, isobutyl, tert-butyl, vinyl, allyl, ethynyl, propargyl, methoxy, ethoxy, isopropoxy, tert-butoxy, -CF₃, -OCF₃, cyclopropyl, cyclobutyl, oxetanyl, azetidinyl, -NHCH₃ and -N(CH₃)₂; where applicable, two adjacent R^{A}, together with the ring atoms to which they are attached, may form C₅₋₆ carbocyclyl.

In some embodiments, ring A is selected from a benzene ring and a 5- to 6-membered aromatic heterocyclic ring, and the benzene ring and 5- to 6-membered aromatic heterocyclic ring are each optionally substituted with 1, 2 or 3 R^{A}.

In some embodiments, ring A is selected from phenyl, furyl, thienyl, oxazolyl, thiazolyl, pyrrolyl, pyrazolyl, imidazolyl and pyridyl, and the phenyl, furyl, thienyl, oxazolyl, thiazolyl, pyrrolyl, pyrazolyl, imidazolyl and pyridyl are each optionally substituted with 1 or 2 R^{A}.

In some embodiments, ring A is selected from phenyl, thienyl, imidazolyl and thiazolyl, and the phenyl, thienyl, imidazolyl and thiazolyl are each optionally substituted with 1 or 2 R^{A}.

In some embodiments, ring A is thienyl, and the thienyl is optionally substituted with 1 or 2 R^{A}.

In some embodiments, ring A is selected from: 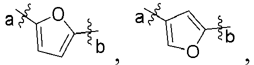 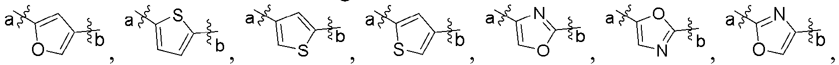 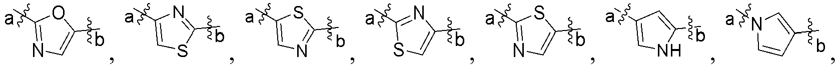 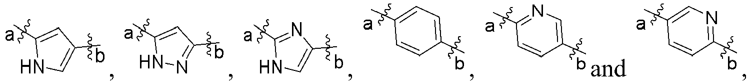 wherein any of the above groups is optionally substituted with 1, 2 or 3 (preferably 1 or 2, more preferably 1) R^{A}, and wherein the bond labelled "a" is attached to L³ and the bond labelled "b" is attached to L¹.

In some more preferred embodiments, ring A is selected from: 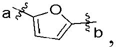 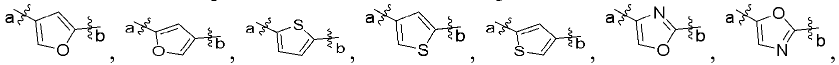 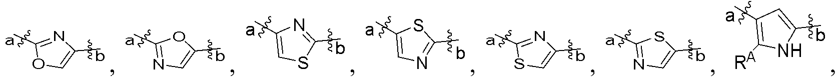 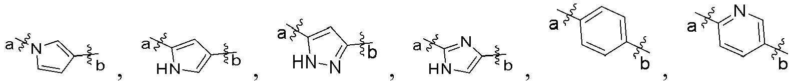 and 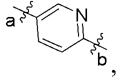 wherein any of the above groups is optionally substituted with 1, 2 or 3 R^{A}, and where applicable, two adjacent R^{A}, together with the ring atoms to which they are attached, may form C₅₋₆ carbocyclyl; and wherein the bond labelled "a" is attached to L³ and the bond labelled "b" is attached to L¹.

In a further more preferred embodiment, ring A is 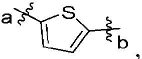 which is optionally substituted with 1 or 2 R^{A}, wherein where applicable, two adjacent R^{A}, together with the ring atoms to which they are attached, may form C₅₋₆ carbocyclyl, thereby forming a group selected from 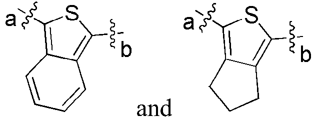 as the ring A; and wherein the bond labelled "a" is attached to L³ and the bond labelled "b" is attached to L¹.

In some embodiments, ring A is selected from: 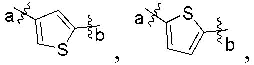 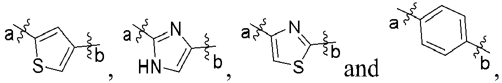 wherein any of the above groups is optionally substituted with 1 or 2 R^{A}.

In some embodiments, ring A is selected from: 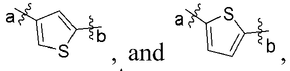 wherein any of the above groups is optionally substituted with 1 R^{A}.

In some embodiments, ring A is 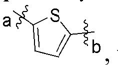 which is optionally substituted with 1 R^{A}.

Preferred embodiments are those compounds of formula (I) wherein the ring A is substituted with 1 or 2 R^{A}.

In a further more preferred embodiment, ring A is selected from:

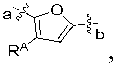

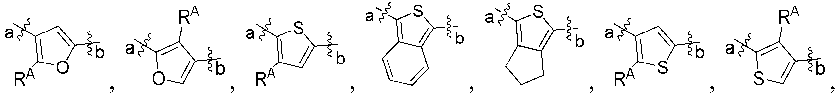

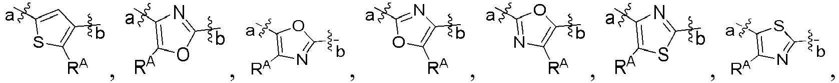

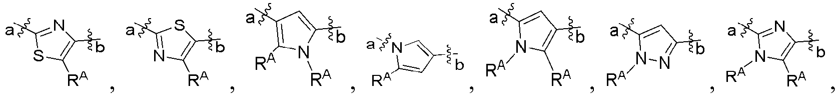

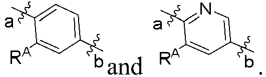

In some embodiments, ring A is selected from:

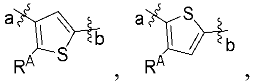

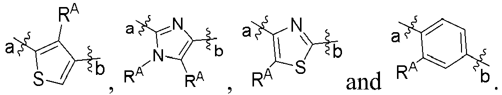

In some embodiments, ring A is selected from:

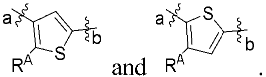

In some embodiments, ring A is 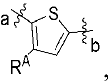 wherein the bond labelled "a" is attached to L³ and the bond labelled "b" is attached to L¹.

In some embodiments, the R^{A} is independently selected from H, D, halogen, - CN, C₁₋₆ alkyl, C₂₋₆ alkenyl, C₂₋₆ alkynyl, C₁₋₆ alkoxy, C₁₋₆ haloalkyl, C₂₋₆ haloalkenyl, C₂₋₆ haloalkynyl, C₁₋₆ haloalkoxy, C₃₋₆ cycloalkyl, 3- to 6-membered heterocyclyl, and phenyl; alternatively, where applicable, two adjacent R^{A}, together with the ring atoms to which they are attached, may form C₅₋₆ carbocyclyl, 5- to 6-membered heterocyclyl or 5- to 6-membered heteroaryl.

In some embodiments, the R^{A} is independently selected from H, D, halogen, - CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, and phenyl; alternatively, where applicable, two adjacent R^{A}, together with the ring atoms to which they are attached, may form C₅₋₆ carbocyclyl.

In some embodiments, the R^{A} is independently selected from H, D, halogen (such as F and Cl), -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, and phenyl.

In some embodiments, the R^{A} is independently selected from H, halogen (such as Cl), C₁₋₄ alkyl, C₃₋₄ cycloalkyl, and phenyl.

In some embodiments, the R^{A} is independently selected from H, halogen (such as Cl), and C₁₋₄ alkyl. In some embodiments, each of the R^{A} is independently selected from F, Cl, Br, -OH, -NH₂, -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, -NH(C₁₋₄ alkyl) and -N(C₁₋₄ alkyl)₂.

In some preferred embodiments, each of the R^{A} is independently selected from F, Cl, -OH, -NH₂, -CN, methyl, ethyl, isopropyl, sec-butyl, isobutyl, tert-butyl, vinyl, allyl, ethynyl, propargyl, methoxy, ethoxy, isopropoxy, tert-butoxy, -CF₃, -OCF₃, cyclopropyl, cyclobutyl, oxetanyl, azetidinyl, -NHCH₃ and -N(CH₃)₂.

In some embodiments, each of the R^{A} is independently selected from H, D, F, Cl, -CN, methyl, ethyl, isopropyl, sec-butyl, isobutyl, tert-butyl, vinyl, allyl, ethynyl, propargyl, methoxy, ethoxy, isopropoxy, tert-butoxy, -CF₃, -OCF₃, cyclopropyl, cyclobutyl, oxetanyl, azetidinyl, and phenyl.

In some more preferred embodiments, each of the R^{A} is independently selected from F, Cl, -CN, methyl, ethyl, isopropyl, tert-butyl, ethynyl, methoxy, ethoxy, - OCF₃, cyclopropyl, cyclobutyl, oxetanyl and azetidinyl.

In some embodiments, each of the R^{A} is independently selected from H, F, Cl, methyl, ethyl, isopropyl, tert-butyl, cyclopropyl, cyclobutyl, and phenyl.

In some embodiments, each of the R^{A} is independently selected from Cl and methyl.

In some embodiments, the ring A is selected from: 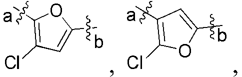 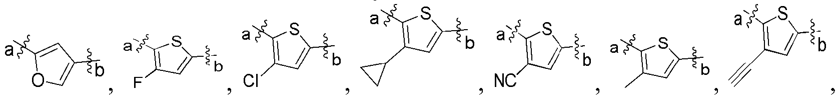 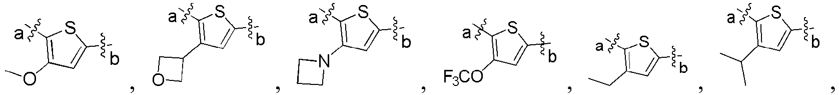 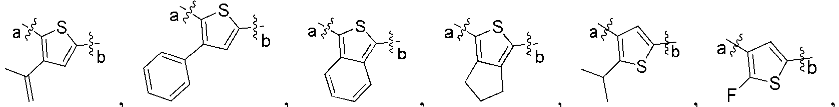 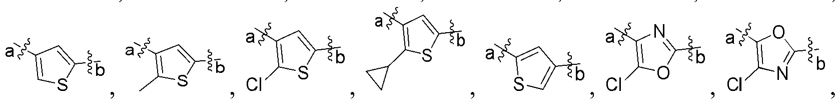 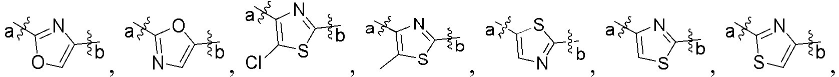 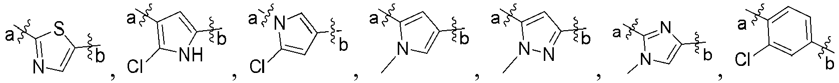 and 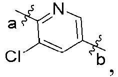 wherein the bond labelled "a" is attached to L³ and the bond labelled "b" is attached to L¹.

In some particularly preferred embodiments, the ring A is selected from: 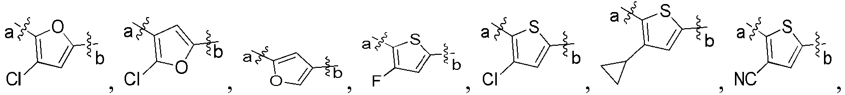 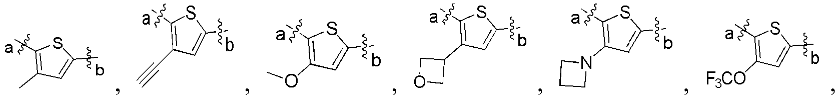 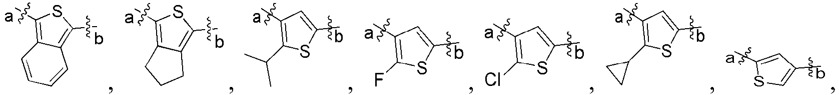 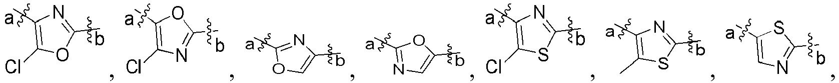 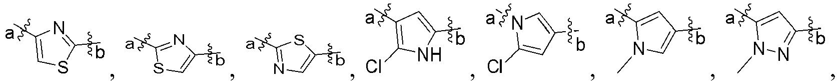 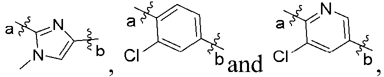 wherein the bond labelled "a" is attached to L³ and the bond labelled "b" is attached to L¹.

In some embodiments, the ring A is selected from:

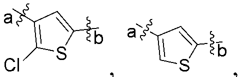

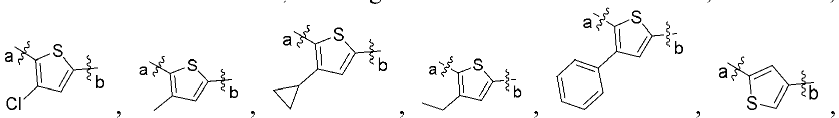

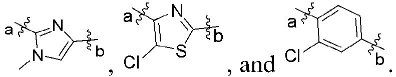

In some embodiments, the ring A is selected from: 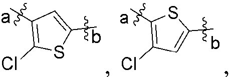 and

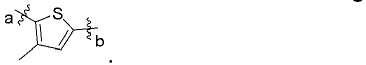

In some embodiments, the ring A is selected from:

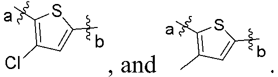

In some embodiments, the ring A is

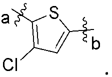

In some embodiments, the present invention provides the compound of formula (I), which has a structure shown in formula (I-1):

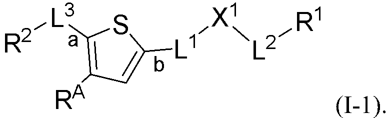

Preferably, moiety 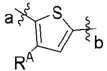 is selected from 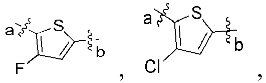 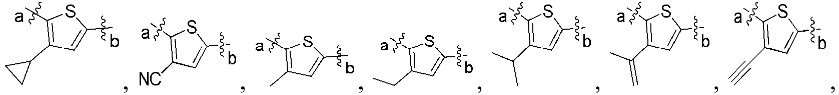 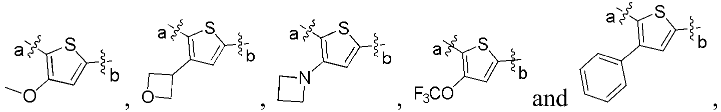 preferably, moiety 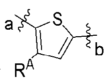 is selected from 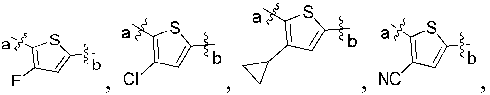 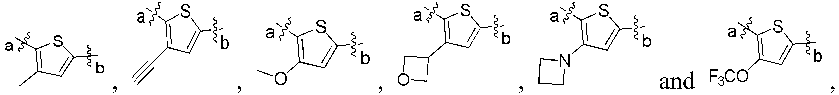 preferably 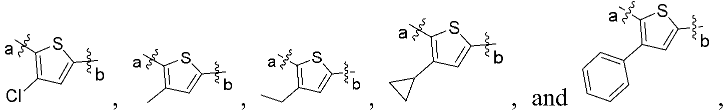 preferably 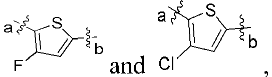 preferably 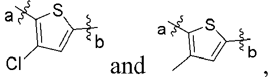 more preferably 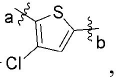 wherein the bond labelled "a" is attached to L³ and the bond labelled "b" is attached to L¹.

In some embodiments, the present invention provides the compound of formula (I), which has a structure shown in formula (I-2):

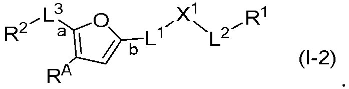

Preferably, moiety 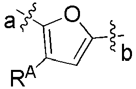 is 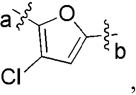 wherein the bond labelled "a" is attached to L³ and the bond labelled "b" is attached to L¹.

In some embodiments, the present invention provides the compound of formula (I), which has a structure shown in formula (I-3'):

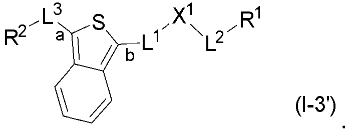

In some embodiments, the present invention provides the compound of formula (I), which has a structure shown in formula (I-4'):

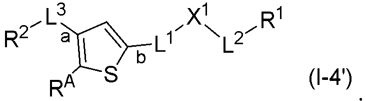

Preferably, moiety 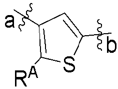 is selected from 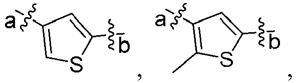 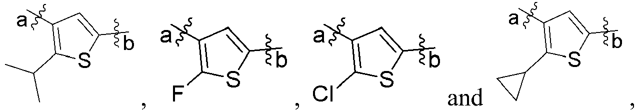 preferably, moiety 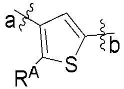 is selected from 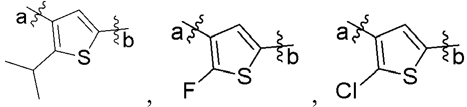 and 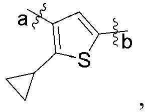 preferably 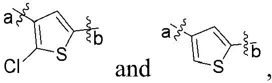 more preferably 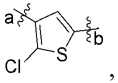 wherein the bond labelled "a" is attached to L³ and the bond labelled "b" is attached to L¹.

In some embodiments, the present invention provides the compound of formula (I), which has a structure shown in formula (I-5'):

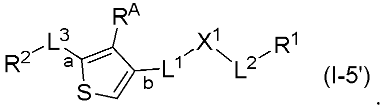

Preferably, 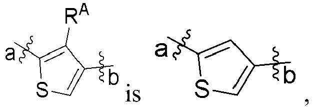 wherein the bond labelled "a" is attached to L³ and the bond labelled "b" is attached to L¹.

In some embodiments, the present invention provides the compound of formula (I), which has a structure shown in formula (I-6'):

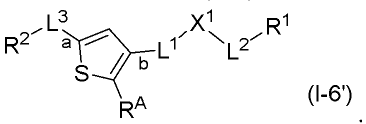

In some embodiments, the present invention provides the compound of formula (I), which has a structure shown in formula (I-7'):

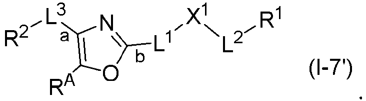

Preferably, moiety 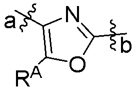 is 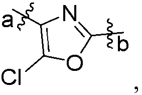 wherein the bond labelled "a" is attached to L³ and the bond labelled "b" is attached to L¹.

In some embodiments, the present invention provides the compound of formula (I), which has a structure shown in formula (I-8):

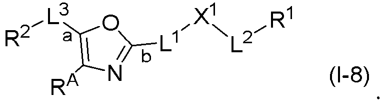

Preferably, moiety 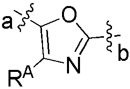 is 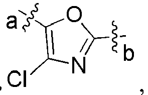 wherein the bond labelled "a" is attached to L³ and the bond labelled "b" is attached to L¹.

In some embodiments, the present invention provides the compound of formula (I), which has a structure shown in formula (I-9):

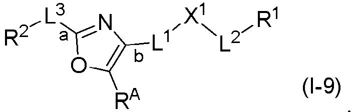

Preferably, moiety 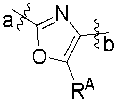 is 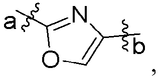 wherein the bond labelled "a" is attached to L³ and the bond labelled "b" is attached to L¹.

In some embodiments, the present invention provides the compound of formula (I), which has a structure shown in formula (I-10):

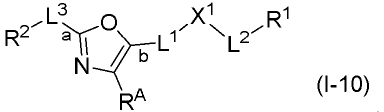

Preferably, moiety 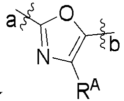 is 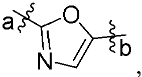 wherein the bond labelled "a" is attached to L³ and the bond labelled "b" is attached to L¹.

In some embodiments, the present invention provides the compound of formula (I), which has a structure shown in formula (I-11):

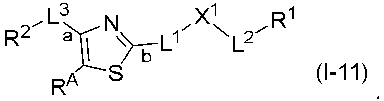

Preferably, moiety 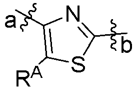 is selected from 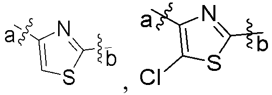 and 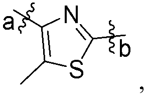 preferably 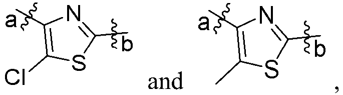 more preferably 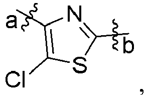 wherein the bond labelled "a" is attached to L³ and the bond labelled "b" is attached to L¹.

In some embodiments, the present invention provides the compound of formula (I), which has a structure shown in formula (I-12):

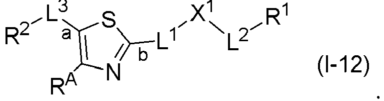

Preferably, moiety 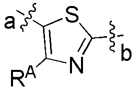 is 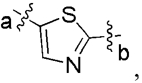 wherein the bond labelled "a" is attached to L³ and the bond labelled "b" is attached to L¹.

In some embodiments, the present invention provides the compound of formula (I), which has a structure shown in formula (1-13):

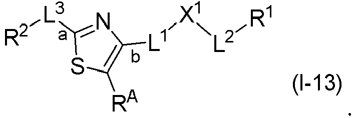

Preferably, moiety 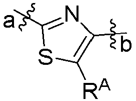 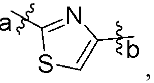 wherein the bond labelled "a" is attached to L³ and the bond labelled "b" is attached to L¹.

In some embodiments, the present invention provides the compound of formula (I), which has a structure shown in formula (1-14):

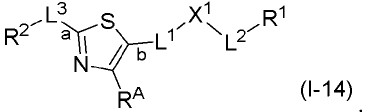

Preferably, moiety 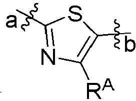 is 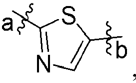 wherein the bond labelled "a" is attached to L³ and the bond labelled "b" is attached to L¹.

In some embodiments, the present invention provides the compound of formula (I), which has a structure shown in formula (I-15):

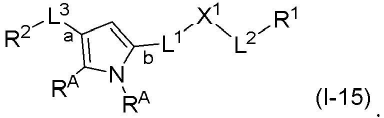

Preferably, moiety 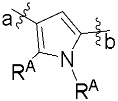 is 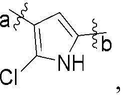 wherein the bond labelled "a" is attached to L³ and the bond labelled "b" is attached to L¹.

In some embodiments, the present invention provides the compound of formula (I), which has a structure shown in formula (1-16):

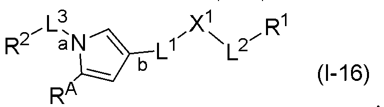

Preferably, moiety 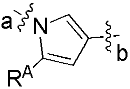 is 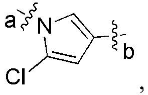 wherein the bond labelled "a" is attached to L³ and the bond labelled "b" is attached to L¹.

In some embodiments, the present invention provides the compound of formula (I), which has a structure shown in formula (I-17):

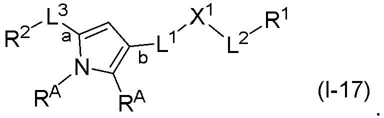

Preferably, moiety 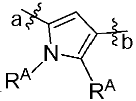 is 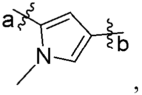 wherein the bond labelled "a" is attached to L³ and the bond labelled "b" is attached to L¹.

In some embodiments, the present invention provides the compound of formula (I), which has a structure shown in formula (I-18):

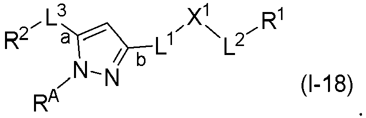

Preferably, moiety 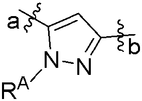 is 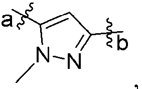 wherein the bond labelled "a" is attached to L³ and the bond labelled "b" is attached to L¹.

In some embodiments, the present invention provides the compound of formula (I), which has a structure shown in formula (I-19):

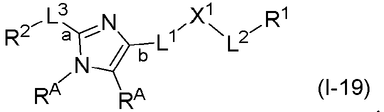

Preferably, moiety 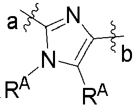 is 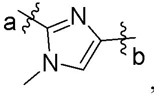 wherein the bond labelled "a" is attached to L³ and the bond labelled "b" is attached to L¹.

In some embodiments, the present invention provides the compound of formula (I), which has a structure shown in formula (I-20):

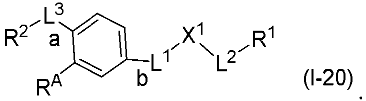

Preferably, moiety 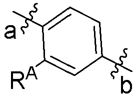 is 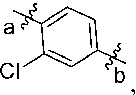 wherein the bond labelled "a" is attached to L³ and the bond labelled "b" is attached to L¹.

In some embodiments, the present invention provides the compound of formula (I), which has a structure shown in formula (I-21):

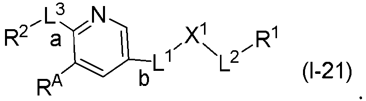

Preferably, moiety 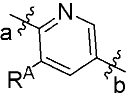 is 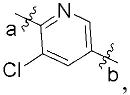 wherein the bond labelled "a" is attached to L³ and the bond labelled "b" is attached to L¹.

In some embodiments, the present invention provides the compound of formula (I), which has a structure shown in formula (I-22):

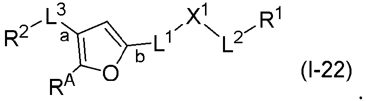

Preferably, moiety 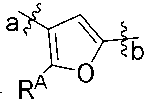 is 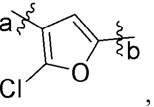 wherein the bond labelled "a" is attached to L³ and the bond labelled "b" is attached to L¹.

In some embodiments, the present invention provides the compound of formula (I), which has a structure shown in formula (I-23'):

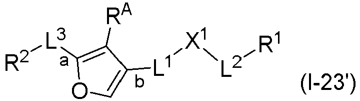

Preferably, moiety 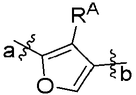 is 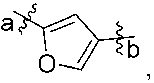 wherein the bond labelled "a" is attached to L³ and the bond labelled "b" is attached to L¹.

In some embodiments, the present invention provides the compound of formula (I), which has a structure shown in formula (I-23):

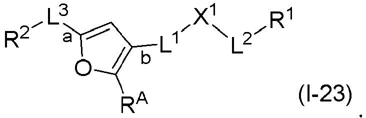

Preferably, moiety 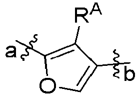 is 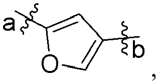 wherein the bond labelled "a" is attached to L³ and the bond labelled "b" is attached to L¹.

In some embodiments, the present invention provides the compounds of the present invention as described above, wherein L³ is selected from a bond, -O-, -S-, - NR^{L3}-, #-C(=O)-NR^{L3}-*, #-C(=O)-O-*, #-C₁₋₆ alkylene-O-*, #-C₁₋₆ alkylene-NR^{L3}-*, #-C₁₋₆ alkylene-S-*, -C₁₋₆ alkylene-, -C₂₋₆ alkenylene- and -C₂₋₆ alkynylene-, and wherein the bond labelled "#" is attached to R² and the bond labelled "*" is attached to ring A.

In some embodiments, L³ is selected from a bond, -O-, -S-, -NR^{L3}-, #-C(=O)-NR^{L3}-*, #-C(=O)-O-*, #-C₁₋₆ alkylene-O-*, #-C₁₋₆ alkylene-NR^{L3}-*, #-C₁₋₆ alkylene-S-*, -C₂₋₆ alkenylene- and -C₂₋₆ alkynylene-, wherein the bond labelled "#" is attached to R² and the bond labelled "*" is attached to ring A.

In some embodiments, L³ is selected from a bond, -O-, -S-, -NR^{L3}-, #-C(=O)-NR^{L3}-*, #-C(=O)-O-*, #-C₁₋₄ alkylene-O-*, #-C₁₋₄ alkylene-NR^{L3}-*, #-C₁₋₄ alkylene-S-*, -C₂₋₄ alkenylene- and -C₂₋₄ alkynylene-, wherein the bond labelled "#" is attached to R² and the bond labelled "*" is attached to ring A.

In some preferred embodiments, L³ is selected from a bond, -O-, -S-, -NR^{L3}-, #-C(=O)-NR^{L3}-*, #-C₁₋₄ alkylene-O-*, #-C₁₋₄ alkylene-NR^{L3}-*, #-C₁₋₄ alkylene-S-*, -C₁₋₄ alkylene-, -C₂₋₄ alkenylene- and -C₂₋₄ alkynylene-, wherein the bond labelled "#" is attached to R² and the bond labelled "*" is attached to ring A.

In some embodiments, L³ is selected from a bond, -S-, -O-, -NR^{L3}-, #-C(=O)-NR^{L3}-*, #-C(=O)-O-*, #-(CH₂)n-O-*, #-(CH₂)ₙ-NR^{L3}-*, #-(CH₂)ₙ-S-*, vinylene, propenylene, ethynylene and propynylene, wherein the bond labelled "#" is attached to R² and the bond labelled "*" is attached to ring A, and n is selected from 1 and 2.

In some more preferred embodiments, L³ is selected from a bond, -S-, -O-, - NR^{L3}-, #-C(=O)-NR^{L3}-*, #-(CH₂)ₙ-O-*, #-(CH₂)ₙ-NR^{L3}-*, #-(CH₂)ₙ-S-*, vinylene, propenylene, ethynylene and propynylene, wherein the bond labelled "#" is attached to R² and the bond labelled "*" is attached to ring A, and n is selected from 1 and 2.

In some embodiments, L³ is selected from a bond, -O-, -S-, -NR^{L3}-, #-C(=O)-NR^{L3}-*, #-C(=O)-O-*, #-C₁₋₄ alkylene-NR^{L3}-* and -C₂₋₄ alkynylene-, wherein the bond labelled "#" is attached to R² and the bond labelled "*" is attached to ring A. In some embodiments, L³ is selected from a bond or -C₂₋₄ alkynylene- (such as ethynylene and propynylene).

In some embodiments, L³ is selected from a bond or ethynylene.

In some embodiments, R^{L3} is selected from H, D and C₁₋₄ alkyl.

In some preferred embodiments, R^{L3} is selected from H, D, -CH₃, -CH₂CH₃ and -CH(CH₃)₂.

In some more preferred embodiments, R^{L3} is H.

In some more preferred embodiments, L³ is selected from a bond, -O-, -S-, - NH-, #-C(=O)-NH-*, #-CH₂-NH-*, #-CH₂-O-*, #-CH₂-S-* and -C≡C-, wherein the bond labelled "#" is attached to R² and the bond labelled "*" is attached to ring A.

In some more preferred embodiments, L³ is selected from a bond, -O-, -S-, - NH-, #-C(=O)-NH-*, #-CH₂-NH-* and -C≡C-, wherein the bond labelled "#" is attached to R² and the bond labelled "*" is attached to ring A.

In a further more preferred embodiment, L³ is a bond.

In some embodiments, the present invention provides the compounds of the present invention as described above, wherein R² is selected from C₃₋₆ cycloalkyl, phenyl, 5- to 6-membered heteroaryl, 9-membered heteroaryl, 9- to 10-membered heterocyclyl and C₉₋₁₀ saturated or partially unsaturated cyclohydrocarbyl, and the C₃₋₆ cycloalkyl, phenyl, 5- to 6-membered heteroaryl, 9-membered heteroaryl, 9- to 10-membered heterocyclyl and C₉₋₁₀ saturated or partially unsaturated cyclohydrocarbyl are optionally substituted with 1, 2, 3, 4, or 5 groups each independently selected from R^{2a}, R^{2b}, R^{2c}, R^{2d} and R^{2e}.

In some embodiments, R² is selected from C₃₋₆ cycloalkyl, phenyl, 5- to 6-membered heteroaryl, 9-membered heteroaryl, 9- to 10-membered heterocyclyl and C₉₋₁₀ partially unsaturated cyclohydrocarbyl, and the C₃₋₆ cycloalkyl, phenyl, 5- to 6-membered heteroaryl, 9-membered heteroaryl, 9- to 10-membered heterocyclyl and C₉₋₁₀ partially unsaturated cyclohydrocarbyl are optionally substituted with 1, 2, 3, 4, or 5 groups each independently selected from R^{2a}, R^{2b}, R^{2c}, R^{2d} and R^{2e}.

In some embodiments, R² is selected from C₃₋₆ cycloalkyl, phenyl, 5- to 6-membered heteroaryl, and 9-membered heteroaryl, and the C₃₋₆ cycloalkyl, phenyl, 5- to 6-membered heteroaryl, and 9-membered heteroaryl are optionally substituted with 1, 2, 3, 4, or 5 groups each independently selected from R^{2a}, R^{2b}, R^{2c}, R^{2d} and R^{2e}.

In some embodiments, R² is selected from C₃₋₆ cycloalkyl, phenyl, and 9-membered heteroaryl, and the C₃₋₆ cycloalkyl, phenyl, and 9-membered heteroaryl are optionally substituted with 1, 2, 3, 4, or 5 groups each independently selected from R^{2a}, R^{2b}, R^{2c}, R^{2d} and R^{2e}.

In some embodiments, R² is selected from phenyl and 9-membered heteroaryl, and the phenyl and 9-membered heteroaryl are optionally substituted with 1, 2, 3, 4, or 5 groups each independently selected from R^{2a}, R^{2b}, R^{2c}, R^{2d} and R^{2e}.

In some preferred embodiments, R² is selected from cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl, and the cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e}.

In some more preferred embodiments, R² is selected from cyclopropyl, cyclobutyl, phenyl, furyl, thienyl, thiazolyl, oxazolyl, pyridyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl, and the cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e}.

In some embodiments, R² is selected from cyclopropyl, cyclobutyl, phenyl, thienyl, pyridyl, indolyl, indolinyl, benzofuryl, 2,3-dihydrobenzofuryl, 2,3-dihydroindenyl and 1,2,3,4-tetrahydronaphthyl, and the cyclopropyl, cyclobutyl, phenyl, thienyl, pyridyl, indolyl, indolinyl, benzofuryl, 2,3-dihydrobenzofuryl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e}

In some embodiments, R² is selected from cyclopropyl, cyclobutyl, phenyl, and indolyl, and the cyclopropyl, cyclobutyl, phenyl and indolyl are each optionally substituted with 1, 2, 3, 4, or 5 groups each independently selected from R^{2a}, R^{2b} R^{2c}, R^{2d} and R^{2e}.

In some embodiments, R² is selected from phenyl and indolyl, and the phenyl and indolyl are each optionally substituted with 1, 2, 3, 4, or 5 groups each independently selected from R^{2a}, R^{2b}, R^{2c} R^{2d} and R^{2e}.

In some more preferred embodiments, R² is selected from:

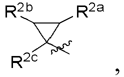

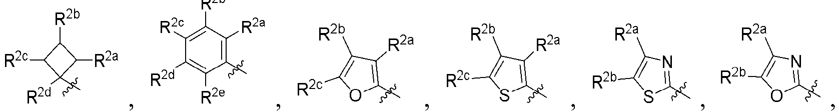

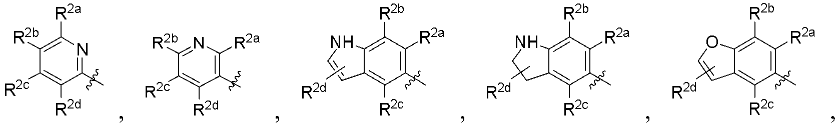

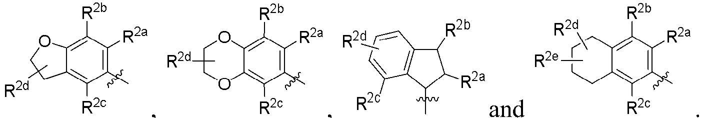

In some embodiments, R² is selected from

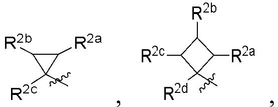

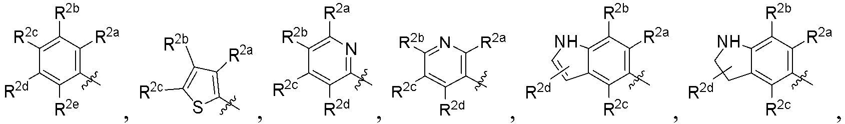

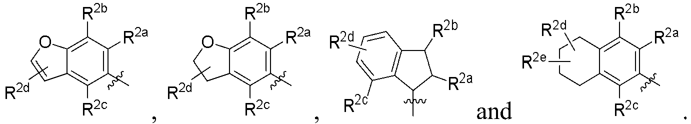

In some embodiments, R² is selected from

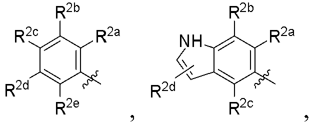

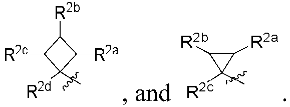

In some embodiments, R² is selected from

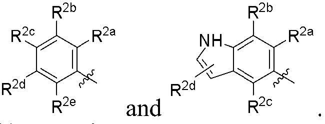

In some embodiments, R^{a}, R^{2b}, R^{2c}, R^{2d} and R^{2e} are each independently selected from H, halogen, -OH, -CN, C₁₋₄ alkyl, C₁₋₄ haloalkyl, -NH₂, -NHC₁₋₄ alkyl, -N(C₁₋₄ alkyl)₂, C₁₋₄ alkoxy, C₁₋₄ haloalkoxy, C₃₋₆ cycloalkyl, C₃₋₆ cycloalkenyl, 4-to 6-membered monocyclic heterocyclyl, 8- to 10-membered bicyclic heterocyclyl, phenyl and 5- to 6-membered heteroaryl, wherein the C₁₋₄ alkyl, C₁₋₄ haloalkyl, - NHC₁₋₄ alkyl, -N(C₁₋₄ alkyl)₂, C₁₋₄ alkoxy, C₁₋₄ haloalkoxy, C₃₋₆ cycloalkyl, C₃₋₆ cycloalkenyl, 4- to 6-membered monocyclic heterocyclyl, 8- to 10-membered bicyclic heterocyclyl, phenyl and 5- to 6-membered heteroaryl are each optionally substituted with 1, 2 or 3 R.

In some embodiments, R^{2a}, R^{2b}, R^{2c}, R^{2d} and R^{2e} are each independently selected from H, -OH, C₁₋₄ alkyl, C₁₋₄ alkoxy, C₃₋₆ cycloalkyl, 4- to 6-membered monocyclic heterocyclyl, phenyl and 5- to 6-membered heteroaryl, and the C₁₋₄ alkyl, C₁₋₄ alkoxy, C₃₋₆ cycloalkyl, 4- to 6-membered monocyclic heterocyclyl, phenyl and 5- to 6-membered heteroaryl are each optionally substituted with 1, 2 or 3 R.

In some preferred embodiments, R^{2a}, R^{2b}, R^{2c}, R^{2d} and R^{2e} are each independently selected from H, F, Cl, Br, -OH, -CN, methyl, ethyl, propyl, butyl, halomethyl, haloethyl, halopropyl, halobutyl, -NH₂, -NH-methyl, -NH-ethyl, -NH-propyl, -NH-butyl, -N(CH₃)₂, -N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, oxetanyl, azetidinyl, tetrahydrofuryl, pyrrolidyl, tetrahydropyranyl, piperidyl, morpholinyl, dihydrotriazolyl, 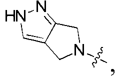 phenyl, imidazolyl, pyrazolyl, thienyl, furyl, pyrrolyl, thiazolyl, thiadiazolyl, oxazolyl, oxadiazolyl, triazolyl, tetrazolyl, pyridyl, pyrimidyl and pyrazinyl, and the methyl, ethyl, propyl, butyl, halomethyl, haloethyl, halopropyl, halobutyl, -NH₂, -NH-methyl, -NH-ethyl, -NH-propyl, -NH-butyl, - N(CH₃)₂, -N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, oxetanyl, azetidinyl, tetrahydrofuryl, pyrrolidyl, tetrahydropyranyl, piperidyl, morpholinyl, dihydrotriazolyl, 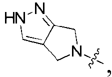 phenyl, imidazolyl, pyrazolyl, thienyl, furyl, pyrrolyl, thiazolyl, thiadiazolyl, oxazolyl, oxadiazolyl, triazolyl, tetrazolyl, pyridyl, pyrimidyl and pyrazinyl are each optionally substituted with 1, 2 or 3 R.

In some embodiments, R^{2a}, R^{2b}, R^{2c}, R^{2d} and R^{2e} are each independently selected from H, F, -OH, -CH₃, -CH₂CH₃, -CH₂CH₂CH₃, -CH₂CH₂CH₂CH₃, - CH(CH₃)₂, -C(CH₃)₃, -N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, piperidyl, morpholinyl, tetrahydrofuryl, pyrrolidyl, tetrahydropyranyl, 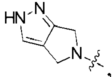 phenyl and pyridyl, and the -CH₃, -CH₂CH₃, -CH₂CH₂CH₃, -CH₂CH₂CH₂CH₃, -CH(CH₃)₂, - C(CH₃)₃, -N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, piperidyl, morpholinyl, tetrahydrofuryl, pyrrolidyl, tetrahydropyranyl, 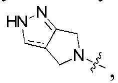 phenyl and pyridyl are each optionally substituted with 1, 2 or 3 R.

In some more preferred embodiments, R^{2a}, R^{2b}, R^{2c}, R^{2d} and R^{2e} are each independently selected from H, F, -OH, -CH₃, -CH₂CH₃, -CH₂CH₂CH₃, -CH(CH₃)₂, -C(CH₃)₃, -N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, 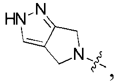 phenyl and pyridyl, and the -CH₃, -CH₂CH₃, -CH₂CH₂CH₃, -CH(CH₃)₂, -C(CH₃)₃, -N(CH₃)₂, -OCH₃, - OCH₂CH₃, -OCH₂CH₂CH₃, -OCH(CH₃)₂, -OCF₃, cyclopropyl, cyclobutyl, cyclohexenyl, azetidinyl, oxetanyl, morpholinyl, tetrahydrofuryl, pyrrolidyl, tetrahydropyranyl, 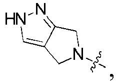 phenyl and pyridyl are each optionally substituted with 1, 2 or 3 R.

In some embodiments, R^{2a}, R^{2b}, R^{2c}, R^{2d} and R^{2e} are each independently selected from H, -OH, -CH₃, -CH₂CH₃, -CH₂CH₂CH₃, -OCH₃, -OCH₂CH₃, - OCH₂CH₂CH₃, cyclopropyl, cyclobutyl, phenyl, pyridyl, pyrrolidyl, morpholinyl, tetrahydropyranyl, and piperidyl, and the -CH₃, -CH₂CH₃, -CH₂CH₂CH₃, -OCH₃, - OCH₂CH₃, -OCH₂CH₂CH₃, cyclopropyl, cyclobutyl, phenyl, pyridyl, pyrrolidyl, morpholinyl, tetrahydropyranyl and piperidyl are each optionally substituted with 1, 2 or 3 R.

In some embodiments, R is a group selected from the following (1) to (3):
(1) H, halogen, -OH, -CN, -NH₂, an oxo group, -COOH, C₁₋₄ alkyl, C₁₋₄ haloalkyl, -C₁₋₄ alkyl substituted with 1 or 2 OH, C₁₋₄ alkoxy, -C₁₋₄ alkyleneoxy-C₁₋₄ alkoxy, -C(=O)NH-C₁₋₄ alkyl, -C(=O)NH-C₃₋₆ cycloalkyl, -C(=O)-C₁₋₄ alkyl, - NHC(=O)-C₁₋₄ alkyl, -N=S(=O)R^{s1}R^{s2}, -NH-S(=O)₂C₁₋₄ alkyl, -S(=O)₂-C₁₋₄ alkyl, - S(=O)₂-4- to 6-membered heterocyclyl, -S(=O)₂NH-C₁₋₄ alkyl, -S(=O)₂NH-C₃₋₆ cycloalkyl, -C₃₋₆ cycloalkylene-COOH and -C(=O)-4- to 6-membered heterocyclyl, wherein R^{s1} and R^{s2} are each C₁₋₄ alkyl, alternatively, R^{s1} and R^{s2}, together with the S atom to which they are attached, form 4- to 6-membered heterocycloalkyl;
(2) 4- to 6-membered heterocyclyl, wherein the 4- to 6-membered heterocyclyl is optionally substituted with 1, 2 or more substituents independently selected from C₁₋₄ alkyl, -NH₂, =NH and an oxo group; and
(3) phenyl and 5- to 6-membered heteroaryl, wherein the phenyl and 5- to 6-membered heteroaryl are each optionally substituted with 1, 2 or more substituents independently selected from halogen, -OH, -CN, -NH₂, -C₁₋₄ alkylene-OH, -C₁₋₄ alkylene-CN and -C₁₋₄ alkylene-NH₂.

In some embodiments, R is selected from H, -NH₂, -OH, -C₁₋₄ alkyl substituted with one OH, C₁₋₄ alkyl, -S(=O)₂-C₁₋₄ alkyl, and 4- to 6-membered heterocyclyl.

In some preferred embodiments, R is a group selected from the following (1) to (3):
(1) H, halogen, -OH, -CN, -NH₂, an oxo group, -COOH, methyl, ethyl, propyl, butyl, halomethyl, haloethyl, halopropyl, halobutyl, -CH₂OH, -ethylene-OH, 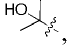 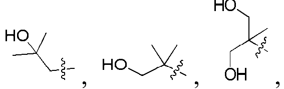 methoxy, ethoxy, propoxy, butoxy, -methyleneoxy-methoxy, -methyleneoxy-ethoxy, -methyleneoxy-propoxy, -ethyleneoxy-methoxy, - ethyleneoxy-ethoxy, -ethyleneoxy-propoxy, -C(=O)-NH-methyl, -C(=O)-NH-ethyl, -C(=O)-NH-propyl, -C(=O)-NH-butyl, -N=S(=O)(methyl)₂, - N=S(=O)(methyl)(ethyl), -N=S(=O)(methyl)(propyl), -N=S(=O)(methyl)(butyl), - N=S(=O)(ethyl)₂, -N=S(=O)(ethyl)(propyl), -(NH)ₚ-W-methyl, -(NH)ₚ-W-ethyl, - (NH)ₚ-W-propyl, -(NH)ₚ-W-butyl, -W-NH-cyclopropyl, -W-oxetanyl, -W-azetidinyl, -W-tetrahydrofuryl, -W-pyrrolidyl, -W-tetrahydropyranyl, -W-piperidyl, -W-morpholinyl, -cyclopropylene-COOH and 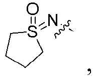 wherein each W is independently C(=O) or S(=O)₂, and each p is independently 0 or 1;
(2) oxetanyl, azetidinyl, tetrahydrofuryl, pyrrolidyl, tetrahydropyranyl, piperidyl, morpholinyl, dihydropyrazolyl, dihydroimidazolyl and dihydrotriazolyl, wherein the oxetanyl, azetidinyl, tetrahydrofuryl, pyrrolidyl, tetrahydropyranyl, piperidyl, morpholinyl, dihydropyrazolyl, dihydroimidazolyl and dihydrotriazolyl are each optionally substituted with 1, 2 or more substituents independently selected from methyl, ethyl, propyl, tert-butyl, -NH₂, =NH and an oxo group; and
(3) phenyl, thienyl, furyl, pyrrolyl, pyrazolyl, thiazolyl, thiadiazolyl, oxazolyl, oxadiazolyl, triazolyl, tetrazolyl, pyridyl, pyrimidyl and pyrazinyl, wherein the phenyl, thienyl, furyl, pyrrolyl, pyrazolyl, thiazolyl, thiadiazolyl, oxazolyl, oxadiazolyl, triazolyl, tetrazolyl, pyridyl, pyrimidyl and pyrazinyl are each optionally substituted with 1, 2 or more substituents independently selected from halogen, -OH, -CN, -NH₂, -C₁₋₄ alkylene-OH, -C₁₋₄ alkylene-CN and -C₁₋₄ alkylene-NH₂.

In some preferred embodiments, R is a group selected from the following (1), (2') and (3):
wherein (1) and (3) are as described above;
(2') oxetanyl, azetidinyl, tetrahydrofuryl, pyrrolidyl, tetrahydropyranyl, piperidyl, morpholinyl, thiomorpholinyl, dihydropyrazolyl, dihydroimidazolyl and dihydrotriazolyl, wherein the oxetanyl, azetidinyl, tetrahydrofuryl, pyrrolidyl, tetrahydropyranyl, piperidyl, morpholinyl, thiomorpholinyl, dihydropyrazolyl, dihydroimidazolyl and dihydrotriazolyl are each optionally substituted with 1, 2 or more substituents independently selected from methyl, ethyl, propyl, tert-butyl, - NH₂, =NH and an oxo group.

In some more preferred embodiments, R is selected from: H, F, -OH, -CN, - NH₂, an oxo group, -COOH, -CH₃, 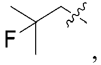 -CH₂OH, 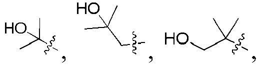 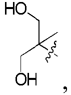 -OCH₃, -OCH₂CH₂OCH₃, -C(=O)CH₃, -C(=O)NHCH₃, -NHC(=O)CH₃, - N=S(=O)(CH₃)₂, 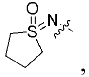 -NH-S(=O)₂CH₃, -S(=O)₂CH₃,

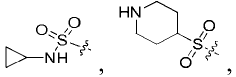

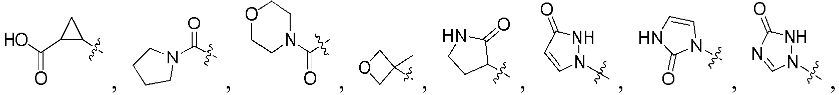

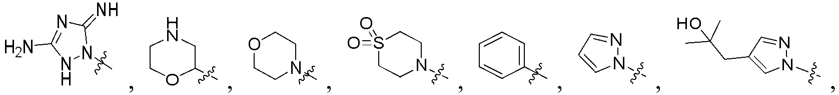

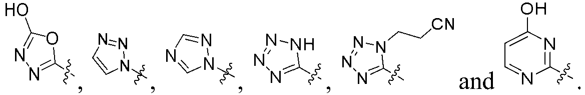

In some embodiments, R is H, -NH₂, -OH, -CH₂OH, -CH₃, -S(=O)₂CH₃, and

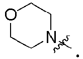

In some embodiments, R is H, -OH, -CH₂OH, -CH₃, -S(=O)₂CH₃, and

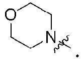

In a further more preferred embodiment, R is H.

In some embodiments, R² is selected from:

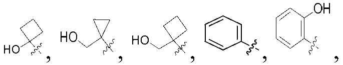

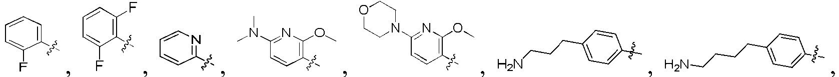

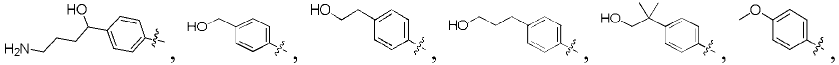

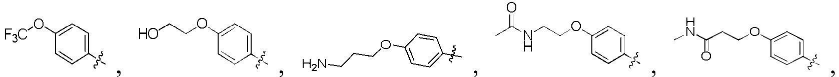

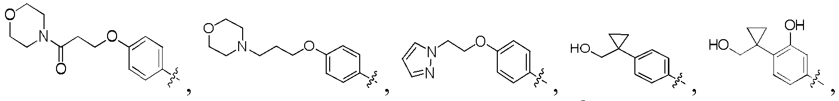

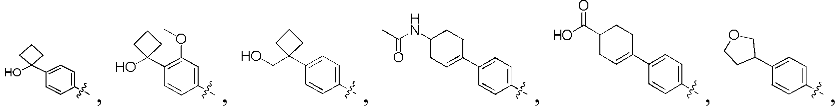

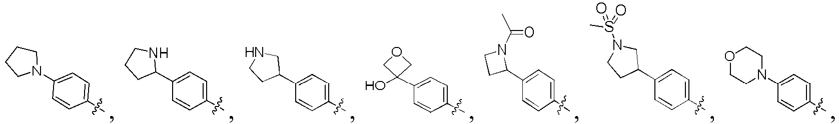

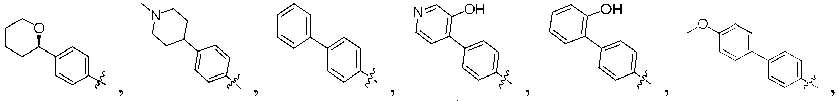

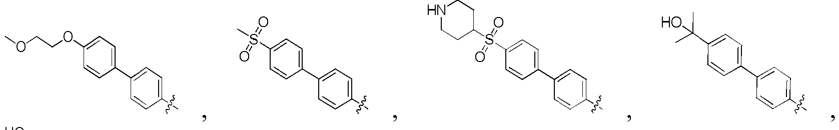

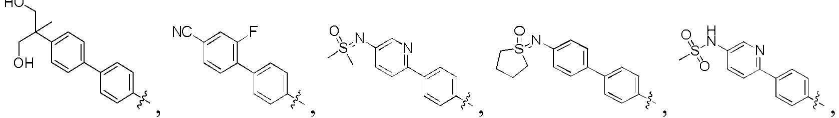

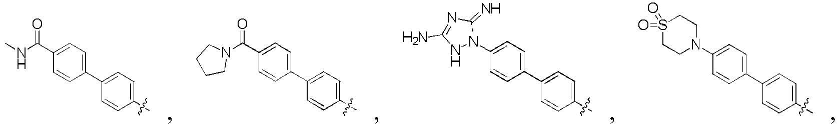

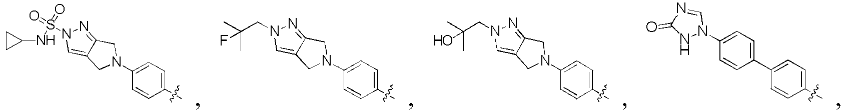

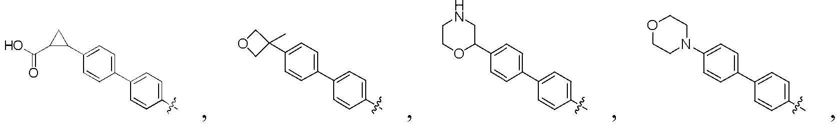

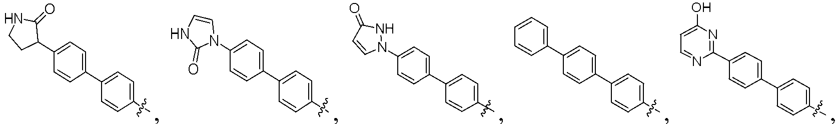

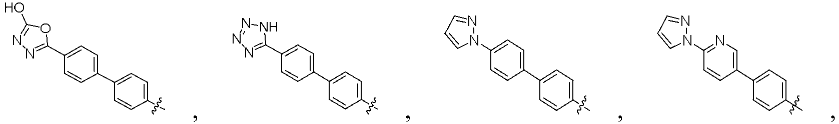

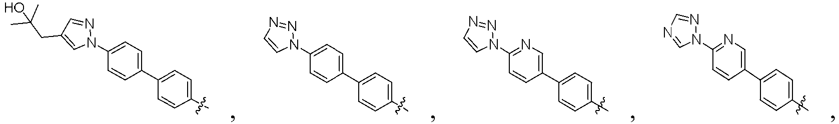

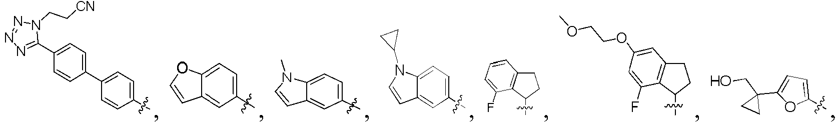

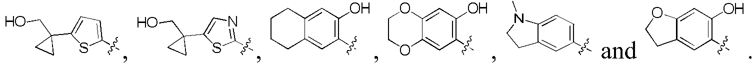

In some particularly preferred embodiments, R² is selected from:

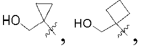

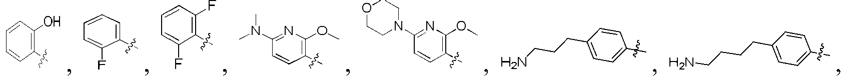

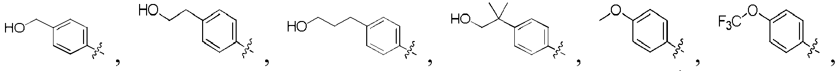

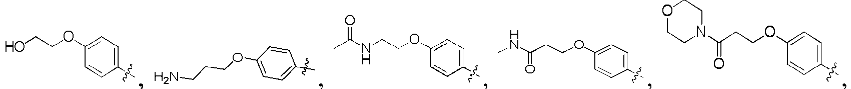

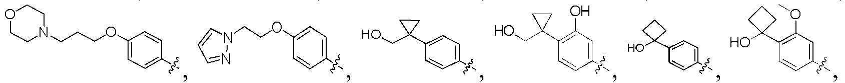

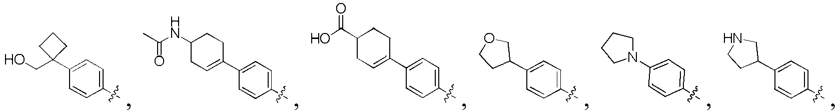

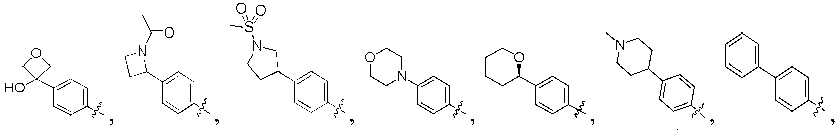

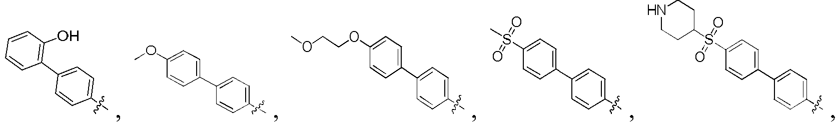

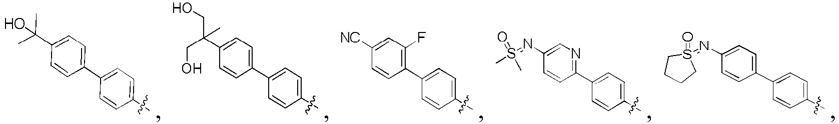

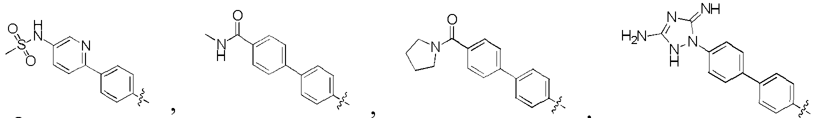

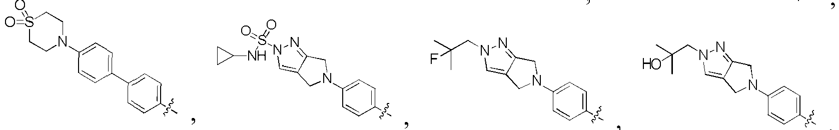

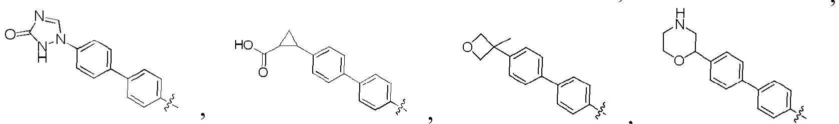

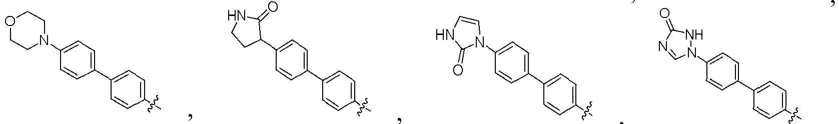

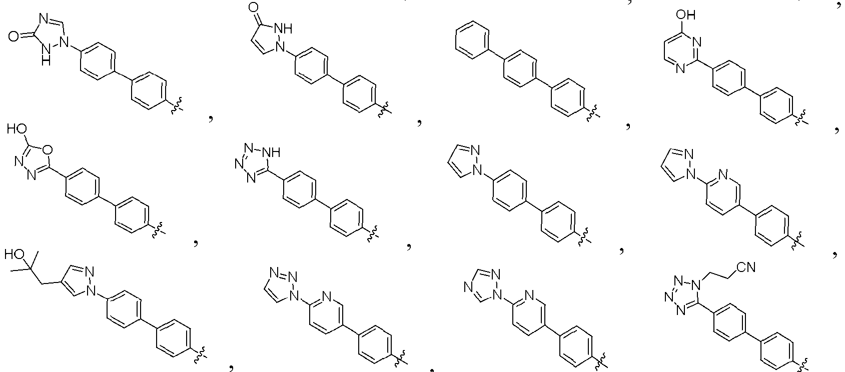

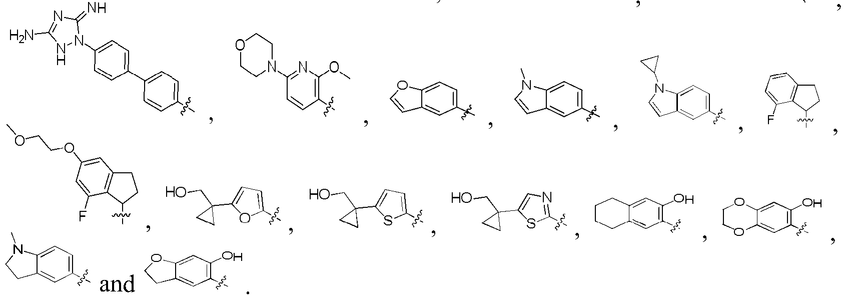

In some embodiments, R² is selected from

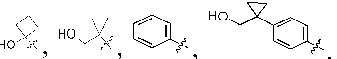

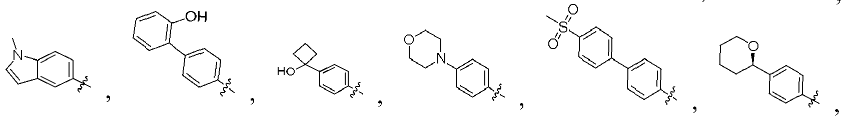

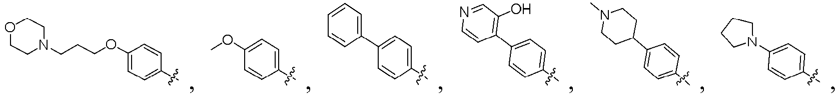

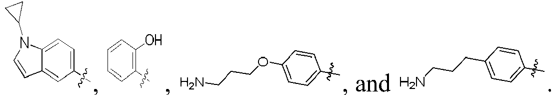

In some embodiments, R² is selected from

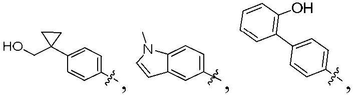

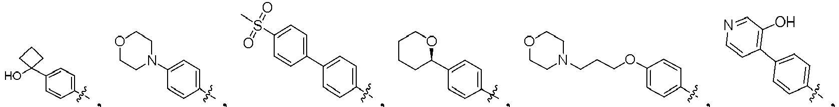

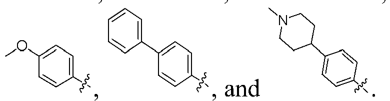

In some embodiments, the compounds of the present invention have structures shown in formulas (II-1) to (II-9): 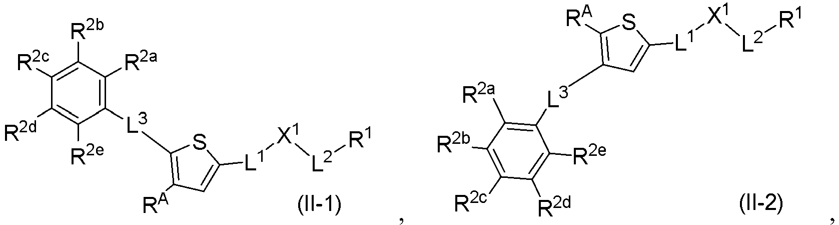 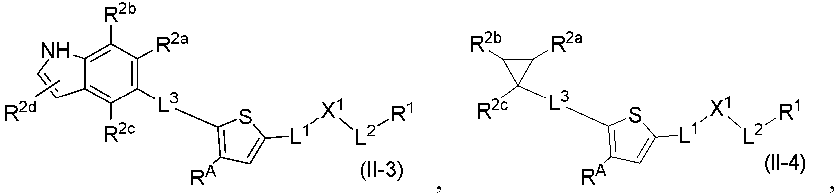 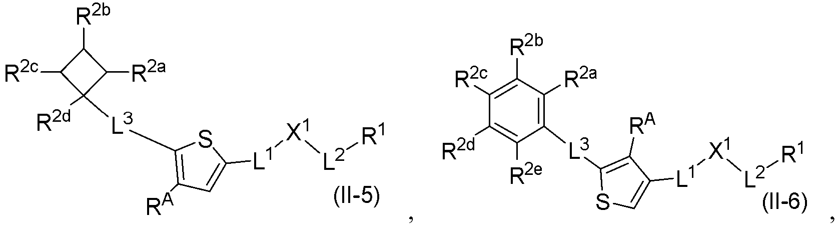 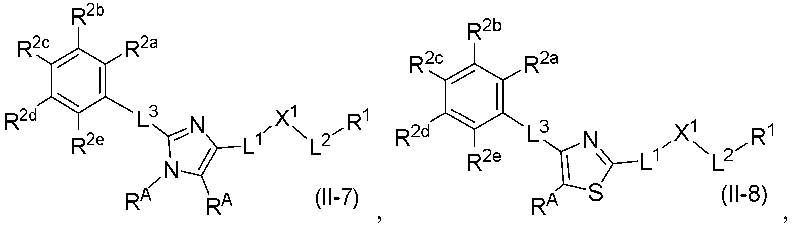 and

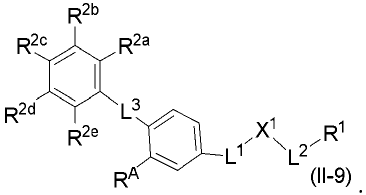

In some embodiments, the compound provided by the present invention has a structure shown in formula (II-1).

In some embodiments, the compound provided by the present invention has a structure shown in formula (II-2).

In some embodiments, the compound provided by the present invention has a structure shown in formula (II-3).

In some embodiments, the compound provided by the present invention has a structure shown in formula (II-4).

In some embodiments, the compound provided by the present invention has a structure shown in formula (II-5).

In some embodiments, the compound provided by the present invention has a structure shown in formula (II-6).

In some embodiments, the compound provided by the present invention has a structure shown in formula (II-7).

In some embodiments, the compound provided by the present invention has a structure shown in formula (II-8).

In some embodiments, the compound provided by the present invention has a structure shown in formula (II-9).

In some embodiments, the compounds provided by the present invention have structures shown in formulas (I-24) to (I-33):

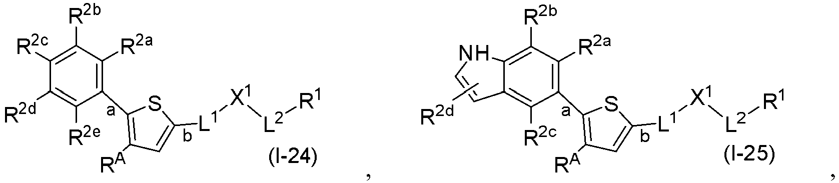

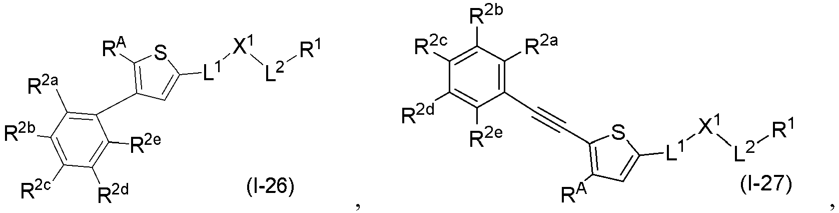

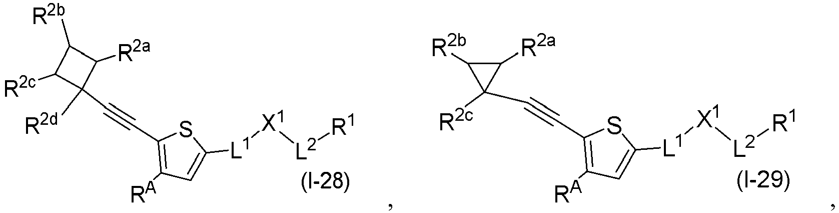

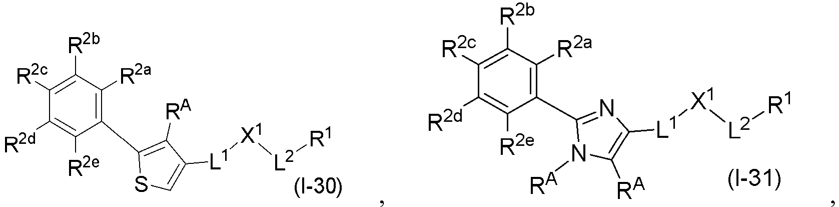

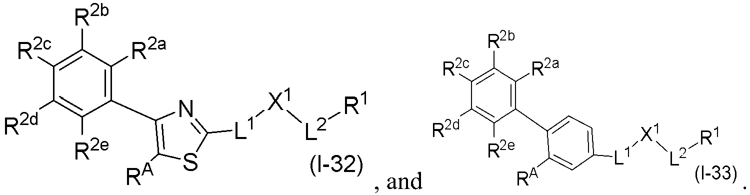

In some embodiments, the compound provided by the present invention has a structure shown in formula (I-24).

In some embodiments, the compound provided by the present invention has a structure shown in formula (I-25).

In some embodiments, the compound provided by the present invention has a structure shown in formula (I-26).

In some embodiments, the compound provided by the present invention has a structure shown in formula (I-27).

In some embodiments, the compound provided by the present invention has a structure shown in formula (I-28).

In some embodiments, the compound provided by the present invention has a structure shown in formula (I-29).

In some embodiments, the compound provided by the present invention has a structure shown in formula (I-30).

In some embodiments, the compound provided by the present invention has a structure shown in formula (I-31).

In some embodiments, the compound provided by the present invention has a structure shown in formula (I-32).

In some embodiments, the compound provided by the present invention has a structure shown in formula (I-33).

In some such embodiments, X¹ is -C(=O)-. In some other embodiments, X¹ is -C(=S)-. In some other embodiments, X¹ is -CH₂-.

In some such embodiments, X¹ is selected from -C(=O)- and -C(=S)-; preferably, X¹ is -C(=O)-.

In some embodiments, the present invention provides the compounds of the present invention as described above, wherein R^{a} and R^{b}, at each occurrence, are each independently selected from H, D, F, Cl, Br, -OH, -CN, C₁₋₄ alkyl, C₁₋₄ alkoxy, C₁₋₄ haloalkyl, C₁₋₄ haloalkoxy and C₃₋₄ cycloalkyl.

In some preferred embodiments, R^{a} and R^{b}, at each occurrence, are each independently H.

In some embodiments, the present invention provides the compounds of the present invention as described above, wherein R^{c}, at each occurrence, is independently selected from H, D, C₁₋₄ alkyl, C₁₋₄ haloalkyl and C₃₋₄ cycloalkyl.

In some preferred embodiments, R^{c}, at each occurrence, is independently H and C₁₋₄ alkyl, preferably H, methyl, ethyl, n-propyl, isopropyl, n-butyl, sec-butyl, isobutyl and tert-butyl, more preferably H and methyl.

In some embodiments, the present invention provides the compounds of the present invention as described above, wherein L¹ is selected from -O-, -S-, -NR^{c}- and -CR^{a}R^{b}-.

In some embodiments, the present invention provides the compounds of the present invention as described above, wherein L¹ is selected from -O-, -NR^{c}- and - CR^{a}R^{b}-.

In some embodiments, L¹ is -NR^{c}-.

In some preferred embodiments, L¹ is selected from -O-, -S-, -NH, -N(CH₃)- and -CH₂-.

In some embodiments, L¹ is -NH-.

In some embodiments, the present invention provides the compounds of the present invention as described above, wherein L² is selected from -O-, -S-, -NR^{c}-, - CR^{a}R^{b}-, #-O-CR^{a}R^{b}-* and #-S-CR^{a}R^{b}-*, and wherein the bond labelled "#" is attached to X¹ and the bond labelled "*" is attached to R¹.

In some embodiments, L² is selected from -O- or -CR^{a}R^{b}-.

In some embodiments, L² is -O-.

In some preferred embodiments, L² is selected from -O-, -S-, -NH-, -N(CH₃)-, -CH₂-, #-O-CH₂-* and #-S-CH₂-*, wherein the bond labelled "#" is attached to X¹ and the bond labelled "*" is attached to R¹.

In some more preferred embodiments, L² is selected from -O-, -S-, -NH-, - N(CH₃)-, -CH₂- and #-O-CH₂-*, wherein the bond labelled "#" is attached to X¹ and the bond labelled "*" is attached to R¹.

In some embodiments, L² is selected from -O- or -CH₂-.

In some embodiments, the present invention provides the compounds of the present invention as described above, wherein moiety -L¹-X¹-L²- is selected from: 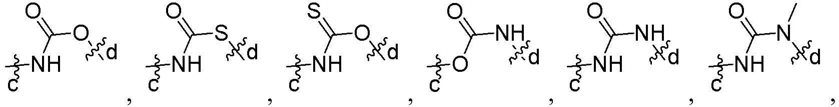 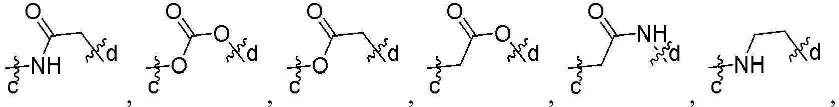 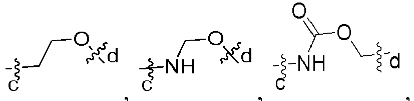 and -NH-, wherein the bond labelled "c" is attached to ring A and the bond labelled "d" is attached to R¹.

In some embodiments, moiety -L¹-X¹-L²- is selected from

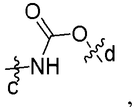

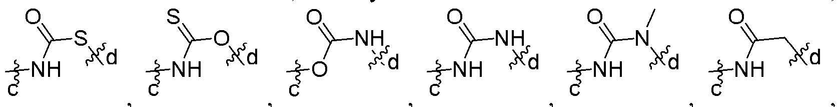

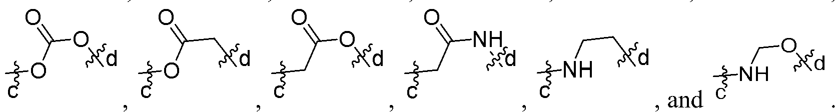

In some embodiments, moiety -L¹-X¹-L²- is selected from 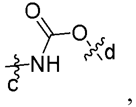 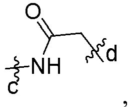 and -NH-.

In some embodiments, moiety -L¹-X¹-L²- is

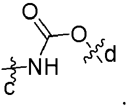

In some embodiments, the present invention provides the compound of formula (I), which has a structure shown in formula (III'): 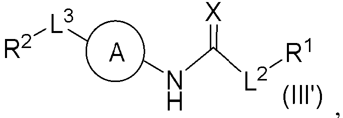 wherein X is selected from O and S; preferably, X is O.

In some embodiments, the present invention provides the compound of formula (I), which has a structure shown in formula (III):

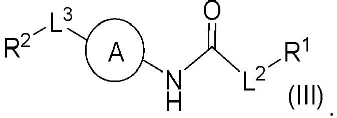

In some embodiments, the present invention provides the compound of formula (I), which has a structure shown in formula (III-1):

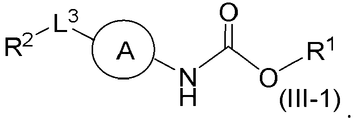

In some embodiments, the compounds provided by the present invention have structures shown in formulas (III-A) to (III-F):

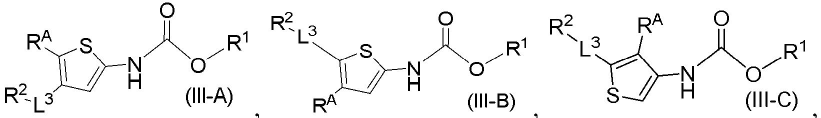

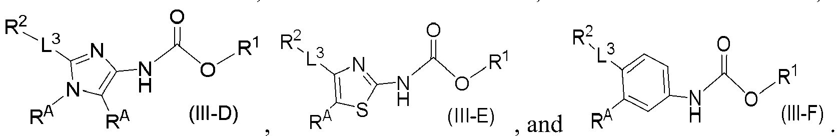

In some embodiments, the compound provided by the present invention has a structure shown in formula (III-A).

In some embodiments, the compound provided by the present invention has a structure shown in formula (III-B).

In some embodiments, the compound provided by the present invention has a structure shown in formula (III-C).

In some embodiments, the compound provided by the present invention has a structure shown in formula (III-D).

In some embodiments, the compound provided by the present invention has a structure shown in formula (III-E).

In some embodiments, the compound provided by the present invention has a structure shown in formula (III-F).

In some embodiments, the present invention provides the compound of formula (I), which has a structure shown in formula (III-2) or (III-3):

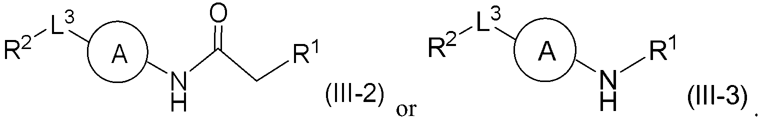

In some embodiments, the compound of formula (I) of the present invention has a structure shown in formula (III-G) or (III-H):

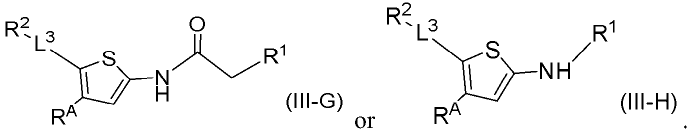

In some embodiments, the compound of formula (I) of the present invention has a structure shown in formula (III-I) or (III-J):

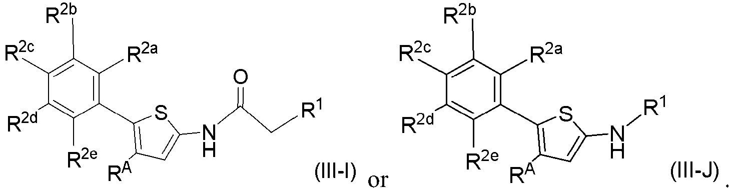

In some embodiments, the present invention provides the compounds of the present invention as described above, wherein R¹ is selected from C₁₋₄ alkyl, C₃₋₆ cycloalkyl, 4- to 6-membered monocyclic heterocyclyl, 8- to 10-membered fused bicyclic heterocyclyl, phenyl and 5- to 6-membered heteroaryl, wherein the C₁₋₄ alkyl, C₃₋₆ cycloalkyl, 4- to 6-membered monocyclic heterocyclyl, 8- to 10-membered fused bicyclic heterocyclyl, phenyl and 5- to 6-membered heteroaryl are optionally substituted with 1, 2 or 3 R^{1a}.

In some preferred embodiments, R¹ is selected from C₁₋₄ alkyl, C₃₋₆ cycloalkyl, 4- to 6-membered monocyclic nitrogen-containing heterocyclyl, 4- to 6-membered monocyclic oxygen-containing heterocyclyl, 8- to 10-membered fused bicyclic oxygen-containing heterocyclyl, phenyl and 5- to 6-membered heteroaryl, wherein the C₁₋₄ alkyl, C₃₋₆ cycloalkyl, 4- to 6-membered monocyclic nitrogen-containing heterocyclyl, 4- to 6-membered monocyclic oxygen-containing heterocyclyl, 8- to 10-membered fused bicyclic oxygen-containing heterocyclyl, phenyl and 5- to 6-membered heteroaryl are optionally substituted with 1, 2 or 3 R^{1a}.

In some embodiments, R¹ is selected from C₁₋₄ alkyl, C₃₋₆ cycloalkyl, 8- to 10-membered fused bicyclic oxygen-containing heterocyclyl, phenyl and 5- to 6-membered heteroaryl, wherein any of the above groups is optionally substituted with 1, 2 or 3 R^{1a}.

In some embodiments, R¹ is selected from C₁₋₄ alkyl, C₃₋₆ cycloalkyl, and 8- to 10-membered fused bicyclic oxygen-containing heterocyclyl, wherein any of the above groups is optionally substituted with 1, 2 or 3 R^{1a}.

In some embodiments, R¹ is selected from C₃₋₆ cycloalkyl and 8- to 10-membered fused bicyclic oxygen-containing heterocyclyl, wherein any of the above groups is optionally substituted with 1, 2 or 3 R^{1a}.

In some embodiments, R¹ is selected from methyl, ethyl, n-propyl, isopropyl, sec-butyl, isobutyl, tert-butyl, cyclopropyl, cyclobutyl, cyclopentyl, cyclohexyl, oxetanyl, tetrahydrofuryl, tetrahydropyranyl, thietanyl, tetrahydrothienyl, tetrahydrothiopyranyl, azetidinyl, pyrrolidyl, piperidyl, oxadiazolyl, thiadiazolyl, pyrimidyl, phenyl and 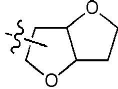 (preferably 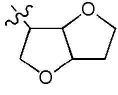 ), wherein any of the above groups is optionally substituted with 1, 2 or 3 R^{1a}.

In some more preferred embodiments, R¹ is selected from methyl, ethyl, n-propyl, isopropyl, sec-butyl, isobutyl, tert-butyl, cyclopropyl, cyclobutyl, cyclopentyl, cyclohexyl, oxetanyl, tetrahydrofuryl, tetrahydropyranyl, thietanyl, tetrahydrothienyl, tetrahydrothiopyranyl, azetidinyl, pyrrolidyl, piperidyl, phenyl and 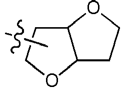 (preferably 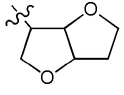 ), wherein any of the above groups is optionally substituted with 1, 2 or 3 R^{1a}.

In some embodiments, R¹ is selected from n-propyl, cyclopentyl, cyclohexyl, oxadiazolyl, thiadiazolyl, pyrimidyl, phenyl and 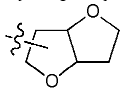 (preferably 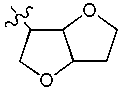 ), wherein any of the above groups is optionally substituted with 1, 2 or 3 R^{1a}.

In some embodiments, R¹ is selected from n-propyl, cyclopentyl, cyclohexyl and 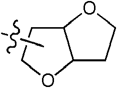 (preferably 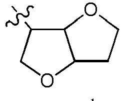 ), wherein any of the above groups is optionally substituted with 1, 2 or 3 R^{1a}.

In some embodiments, R¹ is selected from cyclopentyl and 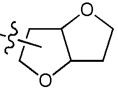 (preferably 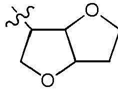 ), wherein any of the above groups is optionally substituted with 1, 2 or 3 R^{1a}.

In some embodiments, R¹ is 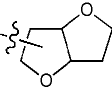 (preferably 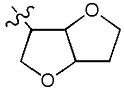 ), which is optionally substituted with 1, 2 or 3 R^{1a}.

In some embodiments, R¹ is a group selected from the following (1) to (2):
(1) methyl, ethyl and n-propyl, and the methyl, ethyl and n-propyl are each optionally substituted with 1, 2 or 3 R^{1a};
(2) 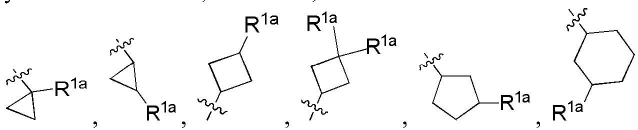 (preferably 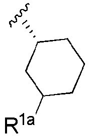 ), 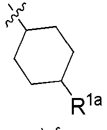 (preferably 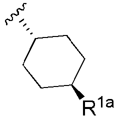 ), 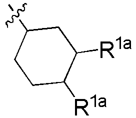 (preferably 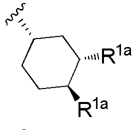 ), 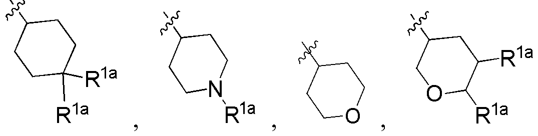 (preferably 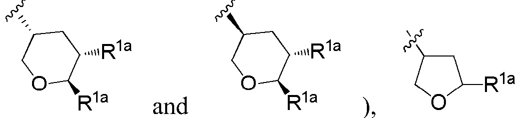 (preferably 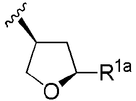 ), 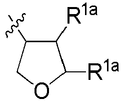 (preferably 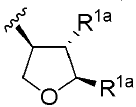 ), 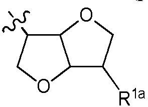 (preferably 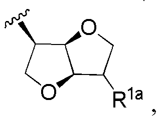 more preferably 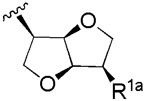 ), 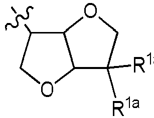 a (preferably 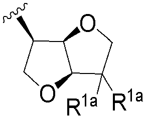 ),

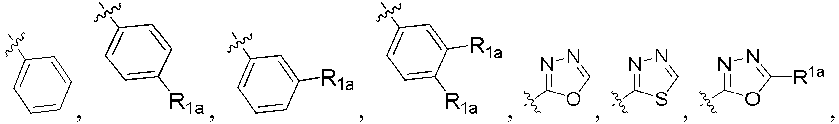

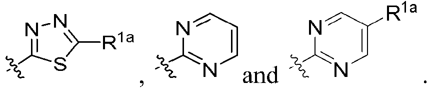

In some more preferred embodiments, R¹ is a group selected from the following (1) to (2):
(1) methyl, ethyl and n-propyl, and the methyl, ethyl and n-propyl are each optionally substituted with 1, 2 or 3 R^{1a};
(2) 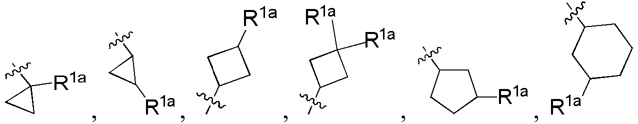 (preferably 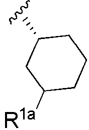 ), 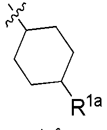 (preferably 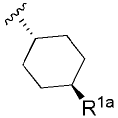 ), 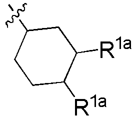 (preferably 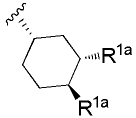 ), 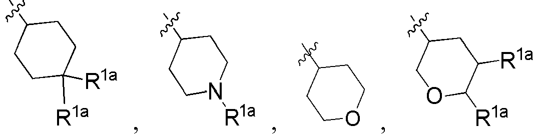 (preferably 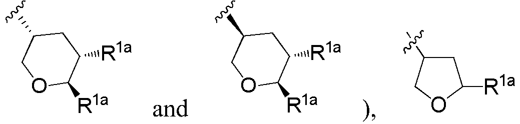 (preferably 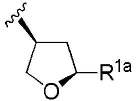 ), 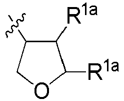 (preferably 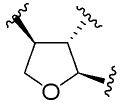 ), 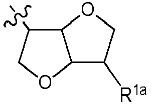 (preferably 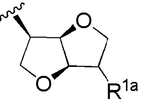 or 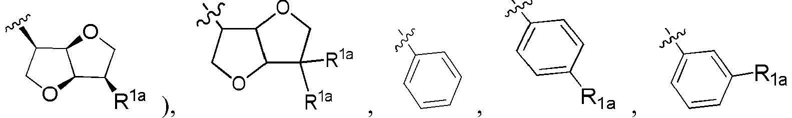 and

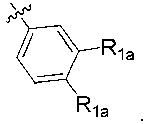

In some embodiments, R¹ is selected from: 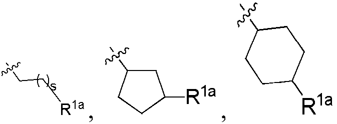 (preferably 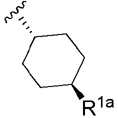 ), and 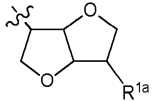 (preferably 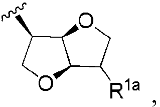 more preferably 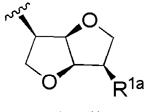 ), wherein s is selected from 0, 1 or 2.

In some embodiments, s is 2.

In some embodiments, R¹ is selected from: 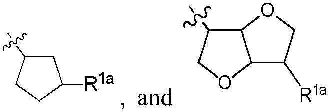 (preferably 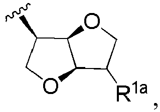 more preferably 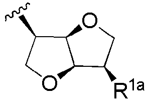 ).

In some embodiments, R¹ is 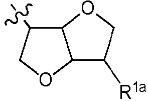 (preferably 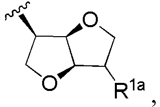 more preferably 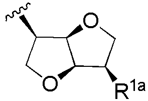 ).

In some embodiments, each R^{1a} is independently selected from halogen, -OH, -CN, an oxo group, -COOR^{c}, C₁₋₄ alkyl, C₁₋₄ haloalkyl, C₁₋₄ alkoxy, C₁₋₄ haloalkoxy, -C₁₋₄ alkylene-OH, -OC₁₋₄ alkylene-OH, -C₁₋₄ alkylene-COOH, -S(=O)₂-C₁₋₄ alkyl and

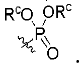

In some embodiments, each R^{1a} is independently selected from halogen, -OH, -CN, an oxo group, -COOH, C₁₋₄ alkyl, C₁₋₄ haloalkyl, C₁₋₄ alkoxy, C₁₋₄ haloalkoxy, -C₁₋₄ alkylene-OH, -C₁₋₄ alkylene-COOH, -S(=O)₂-C₁₋₄ alkyl and

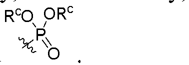

In some preferred embodiments, each R^{1a} is independently selected from halogen, -OH, an oxo group, -COOH, C₁₋₄ alkyl, -C₁₋₄ alkylene-OH, -C₁₋₄ alkylene-COOH, -S(=O)₂-C₁₋₄ alkyl and

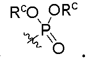

In some more preferred embodiments, each R^{1a} is independently selected from F, Cl, Br, -OH, an oxo group, -COOH, methyl, ethyl, isopropyl, tert-butyl, -CH₂-OH, -CH₂CH₂-OH, -C(CH₃)₂-OH, -CH₂-COOH, -CH₂CH₂-COOH, -C(CH₃)₂-COOH, - S(=O)₂-CH₃ and

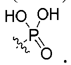

In some embodiments, each R^{1a} is independently selected from -OH, -COOR^{c}, and -C₁₋₄ alkylene-OH.

In some embodiments, each R^{1a} is independently selected from -OH and -C₁₋₂ alkylene-OH.

In some embodiments, each R^{1a} is independently selected from Cl, -OH, -CN, an oxo group, -COOH, -COOEt, methyl, ethyl, -CH₂-OH, -CH₂CH₂-OH, - OCH₂CH₂-OH, -C(CH₃)₂-OH, -CH₂-COOH, -CH₂CH₂-COOH, -C(CH₃)₂-COOH, - S(=O)₂-CH₃ and

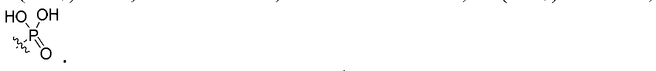

In some more preferred embodiments, each R^{1a} is independently selected from Cl, -OH, an oxo group, -COOH, methyl, ethyl, -CH₂-OH, -CH₂CH₂-OH, -C(CH₃)₂-OH, -CH₂-COOH, -CH₂CH₂-COOH, -C(CH₃)₂-COOH, -S(=O)₂-CH₃ and

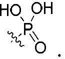

In some embodiments, each R^{1a} is independently selected from -OH, -CH₂-OH, -COOH, and -COOEt.

In some embodiments, each R^{1a} is independently selected from -OH, -CH₂-OH, and -COOH.

In some embodiments, each R^{1a} is independently selected from -OH and -CH₂-OH.

In some embodiments, each R^{1a} is independently -OH.

In some embodiments, R¹ is selected from:

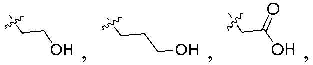

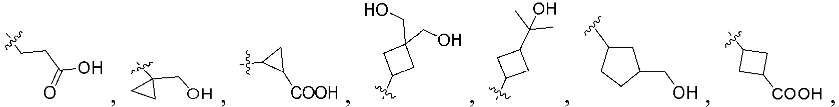

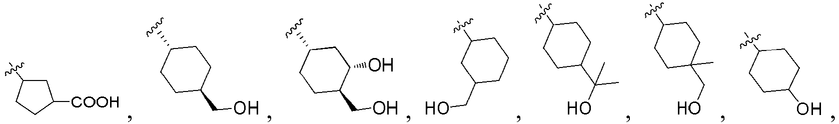

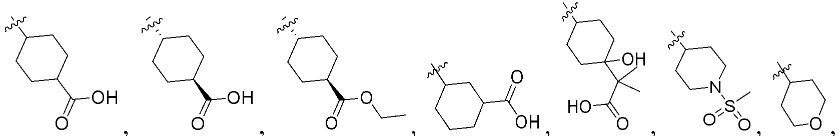

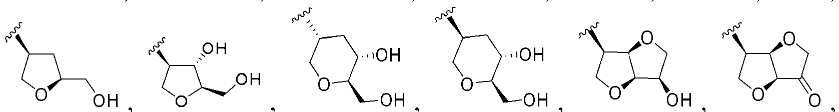

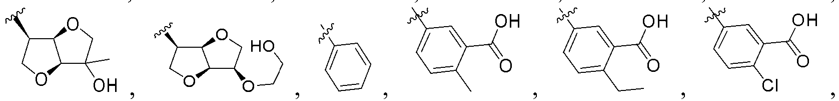

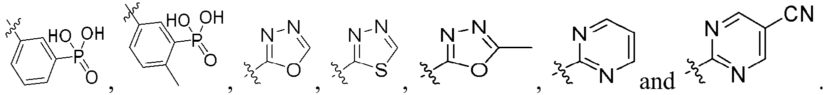

In some particularly preferred embodiments, R¹ is selected from: 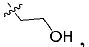 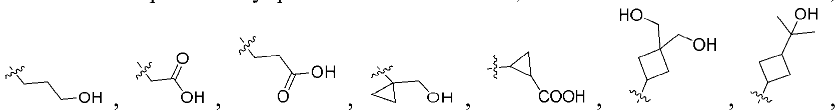 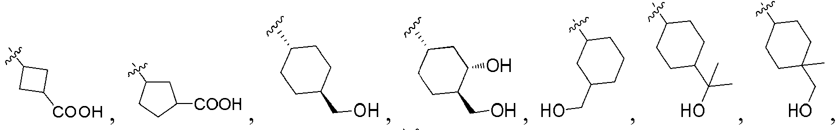 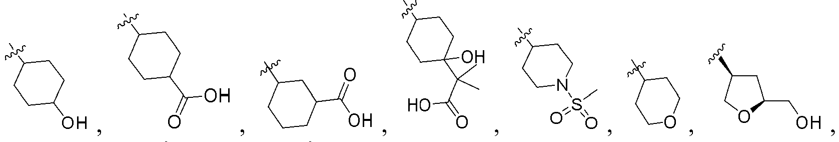 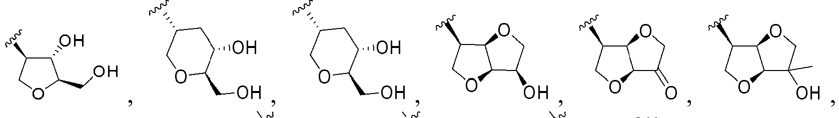 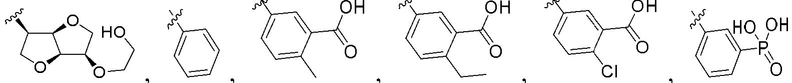 and

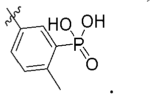

In some embodiments, R¹ is selected from:

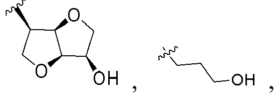

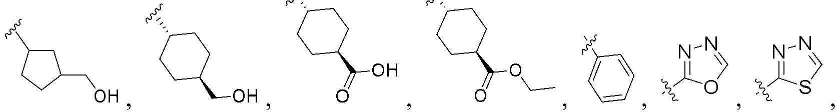

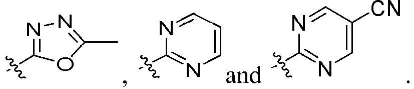

In some embodiments, R¹ is selected from:

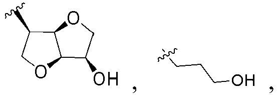

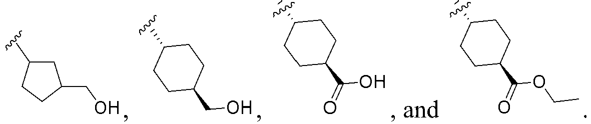

In some embodiments, R¹ is selected from:

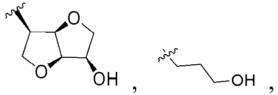

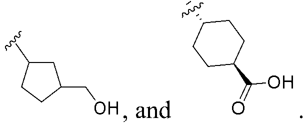

In some embodiments, R¹ is selected from:

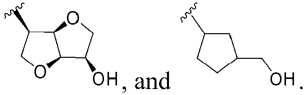

In some embodiments, R¹ is

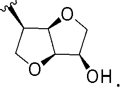

In some embodiments, moiety -L¹-X¹-L²-R¹ is selected from: 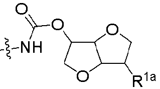 (preferably 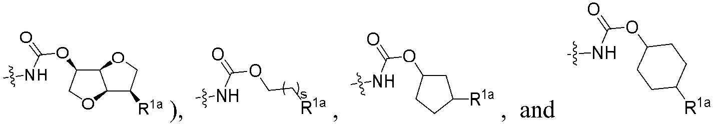 (preferably

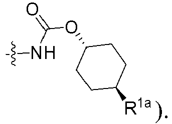

In some embodiments, moiety -L¹-X¹-L²-R¹ is selected from: 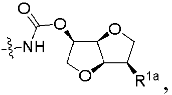 and

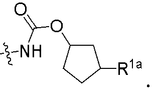

In some embodiments, moiety -L¹-X¹-L²-R¹ is

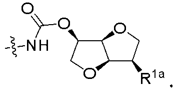

In some embodiments, moiety -L¹-X¹-L²-R¹ is selected from:

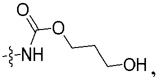

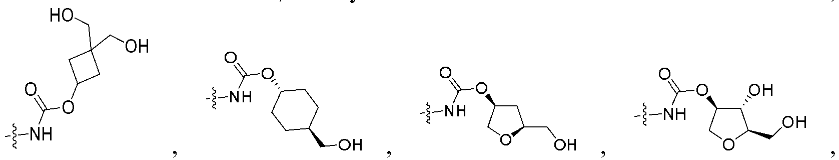

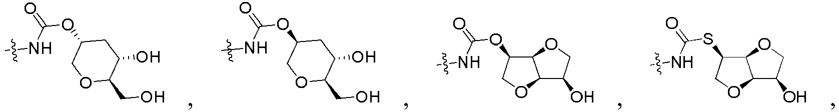

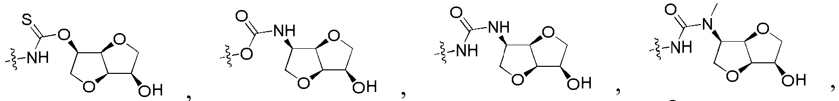

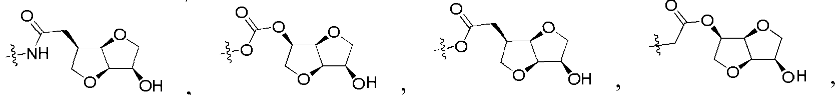

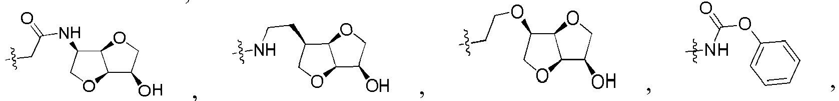

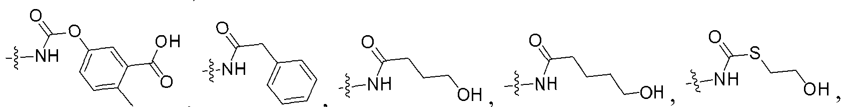

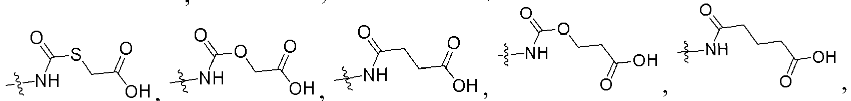

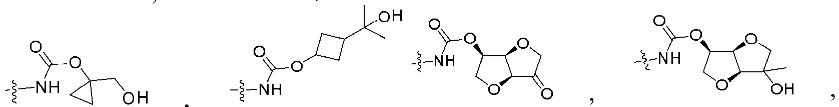

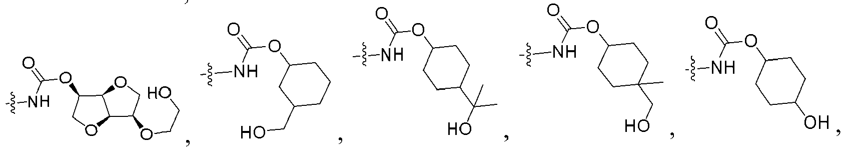

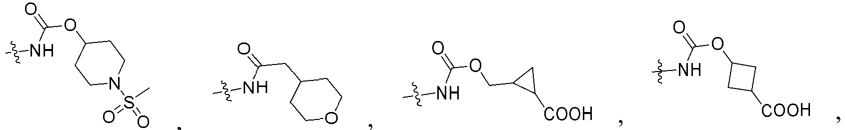

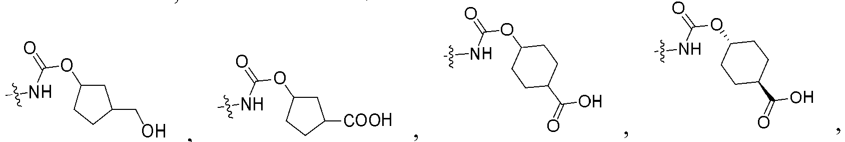

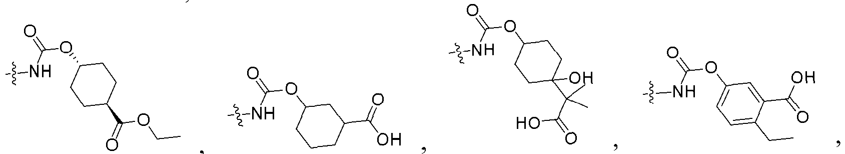

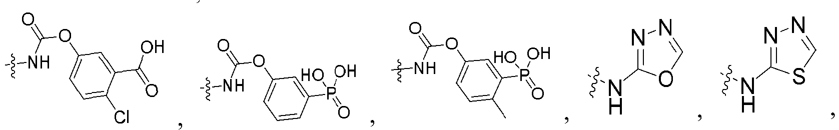

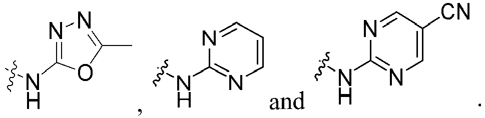

In a further preferred embodiment, moiety -L¹-X¹-L²-R¹ is selected from:

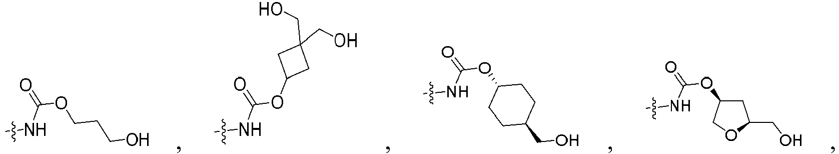

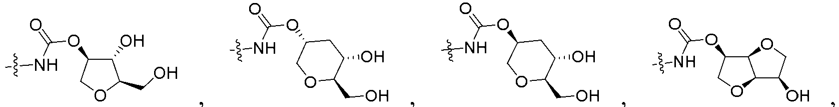

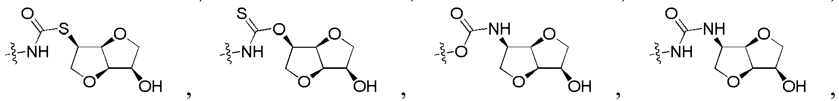

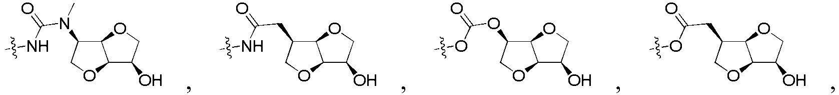

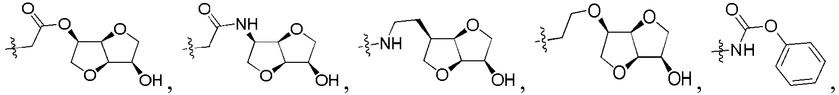

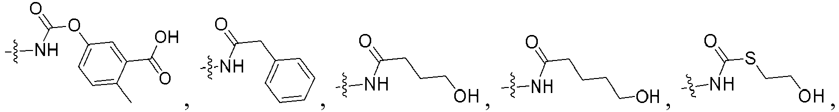

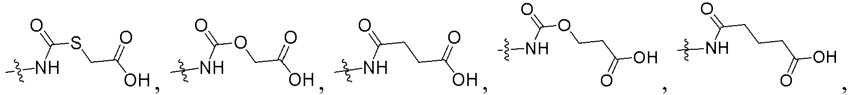

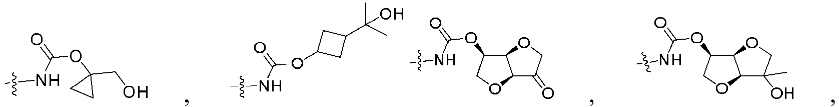

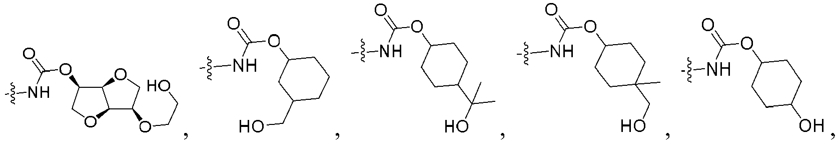

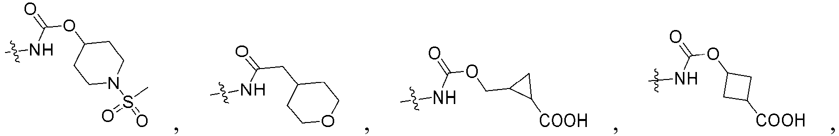

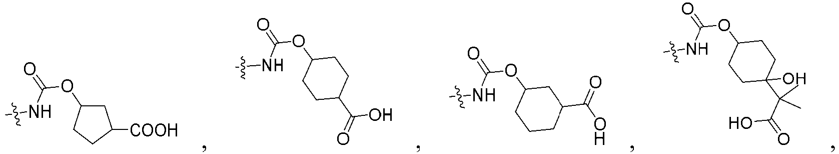

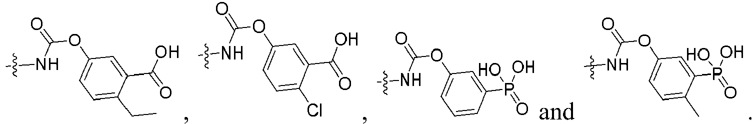

In some embodiments, moiety -L¹-X¹-L²-R¹ is selected from:

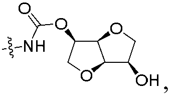

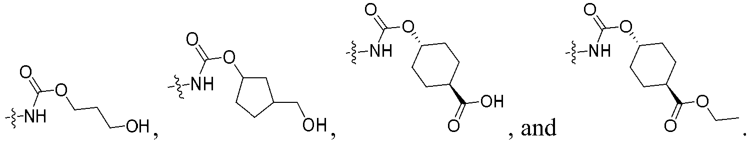

In some embodiments, moiety -L¹-X¹-L²-R¹ is selected from:

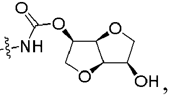

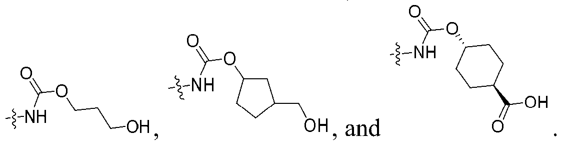

In some embodiments, moiety -L¹-X¹-L²-R¹ is selected from: 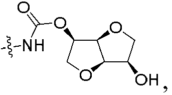 and

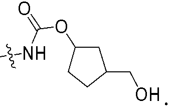

In some embodiments, moiety -L¹-X¹-L²-R¹ is

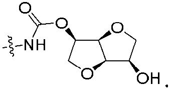

In some embodiments, the present invention provides the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound has a structure shown in any of formulas (IV-1) to (IV-4): 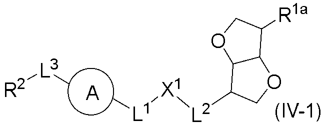 (preferably 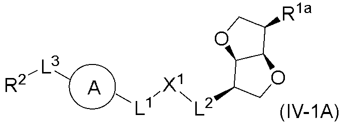 ), 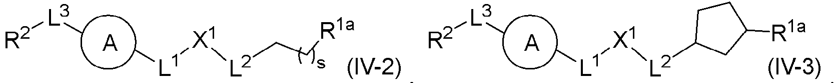 or 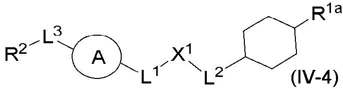 (preferably

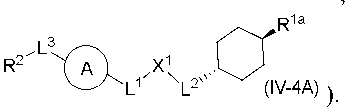

In some embodiments, the present invention provides the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound has a structure shown in any of formulas (IV-5) to (IV-8): 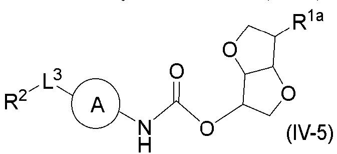 (preferably 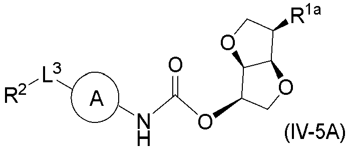 ), 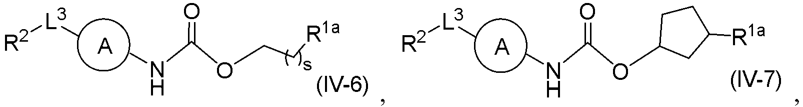 or 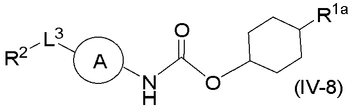 (preferably 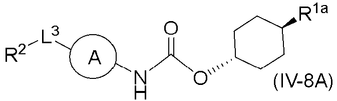 ).

In some embodiments, the present invention provides the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound has a structure shown in any of formulas (V-1) to (V-9): 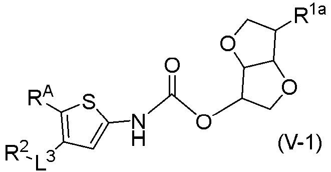 (preferably 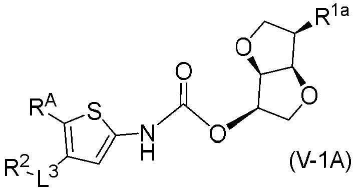 ), 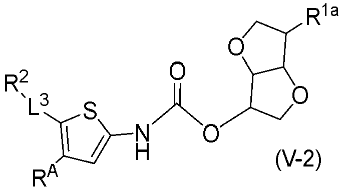 (preferably 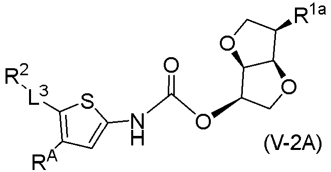 ), 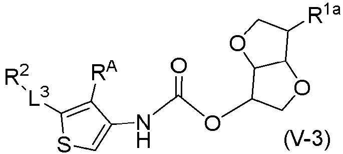 (preferably 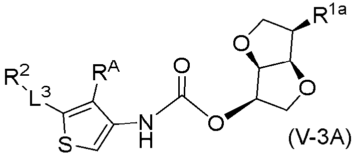 ), 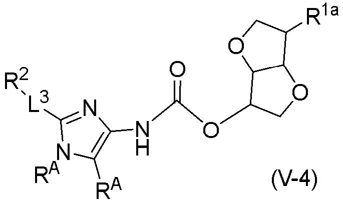 (preferably 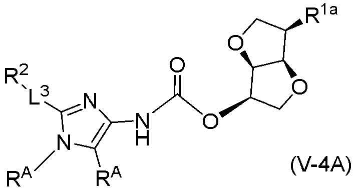 ), 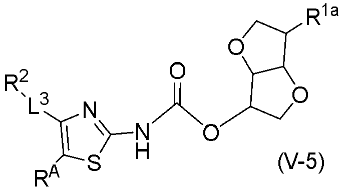 (preferably 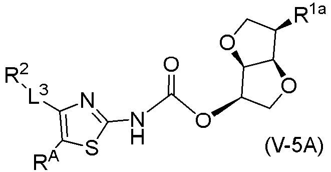 ), 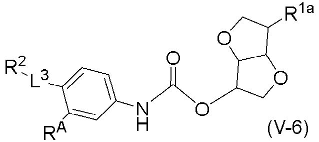 (preferably 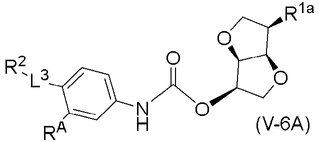 ), 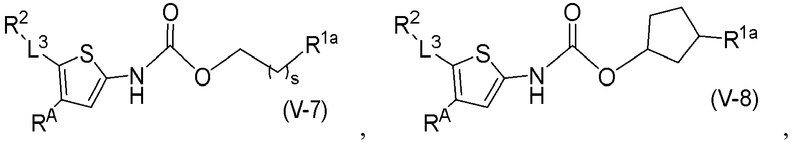 or 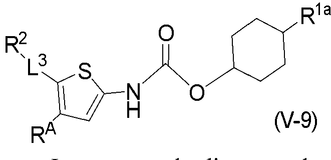 (preferably 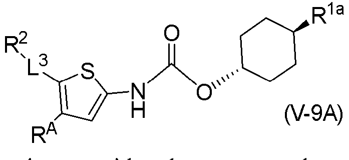 ).

In some embodiments, the present invention provides the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound has a structure shown in any of formulas (VI-1) to (VI-14): 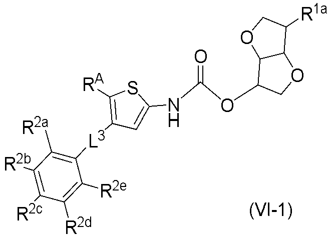 (preferably 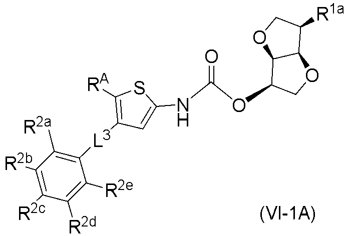 ), 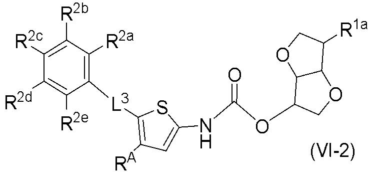 (preferably 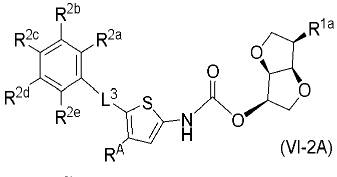 ), 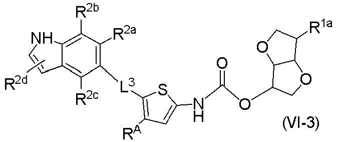 (preferably 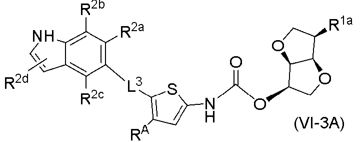 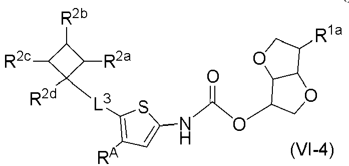 (preferably 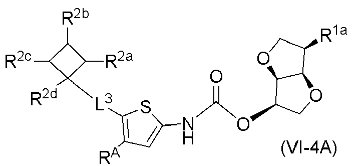 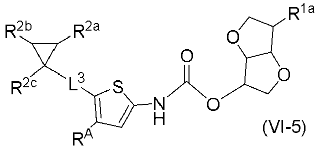 (preferably 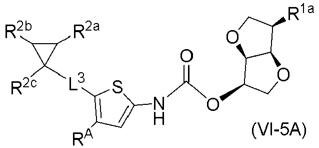 ), 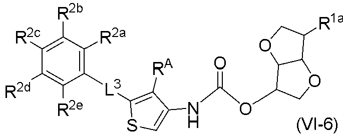 (preferably 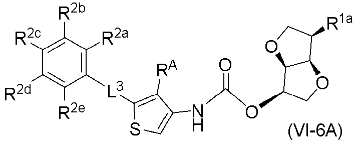 ), 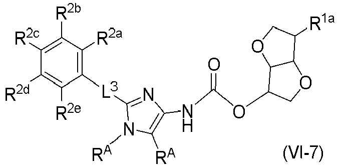 (preferably 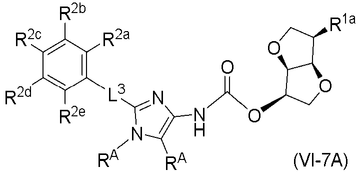 ), 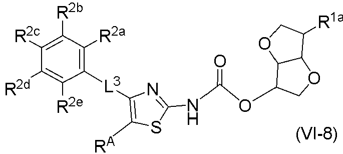 (preferably 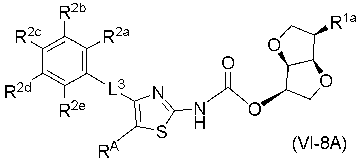 ), 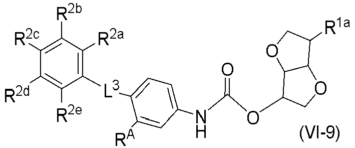 (preferably 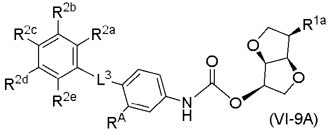 ), 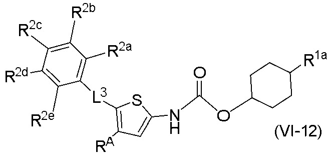 (preferably 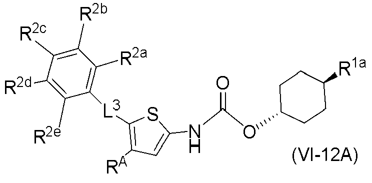 ), 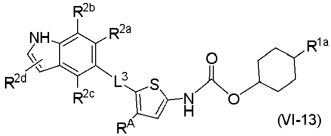 (preferably 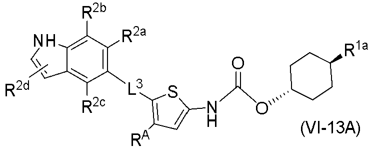 ), or

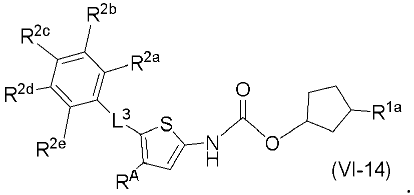

In some embodiments, the present invention provides the compound of formula (I), which has a structure selected from one of the following formulas: 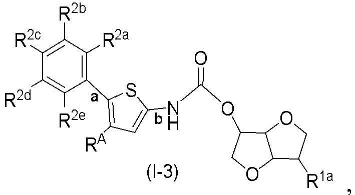 preferably 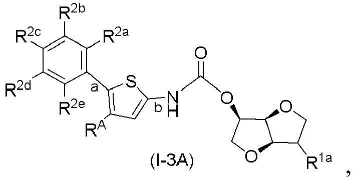 more preferably

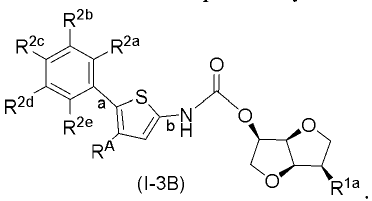

In some embodiments, the present invention provides the compound of formula (I), which has a structure selected from one of the following formulas: 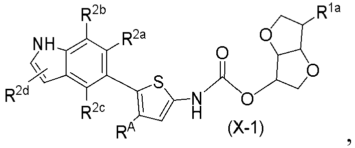 preferably 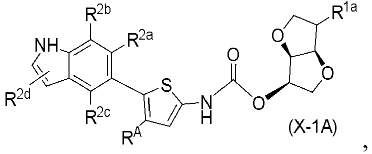 more preferably

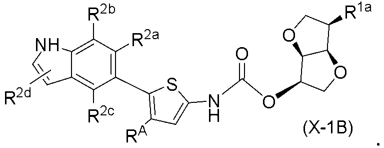

In some embodiments, the present invention provides the compound of formula (I), which has a structure selected from one of the following formulas: 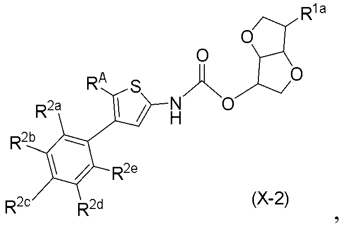 preferably 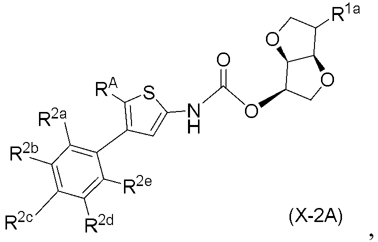 more preferably

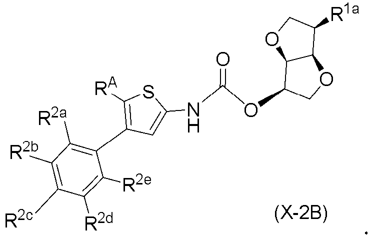

In some embodiments, the present invention provides the compound of formula (I), which has a structure of the following formula:

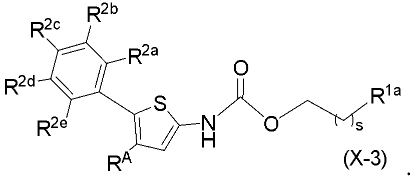

In some embodiments, the present invention provides the compound of formula (I), which has a structure of the following formula:

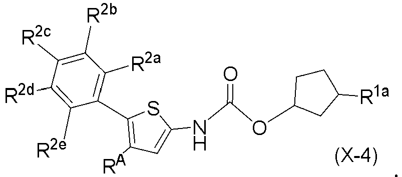

In some embodiments, the present invention provides the compound of formula (I), which has a structure selected from one of the following formulas: 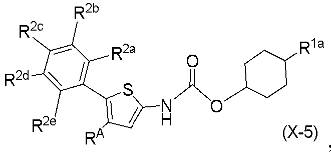 , preferably

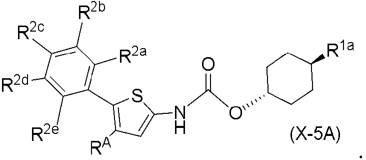

In some embodiments, the present invention provides the compound of formula (I), which has a structure selected from one of the following formulas: 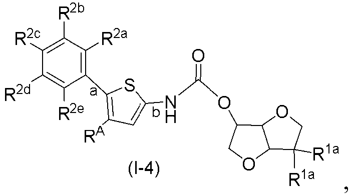 preferably

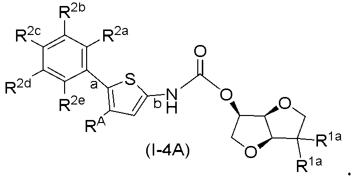

In some embodiments, the present invention provides the compound of formula (I), which has a structure selected from one of the following formulas: 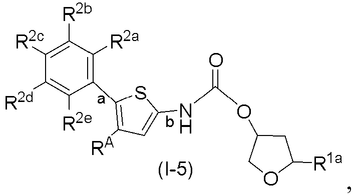 preferably

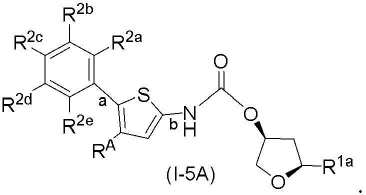

In some embodiments, the present invention provides the compound of formula (I), which has a structure selected from one of the following formulas: 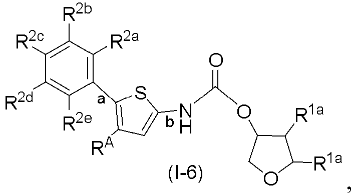 preferably

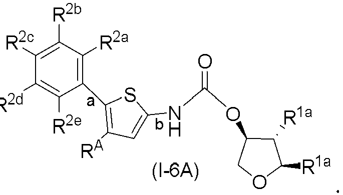

In some embodiments, the present invention provides the compound of formula (I), which has a structure selected from one of the following formulas: 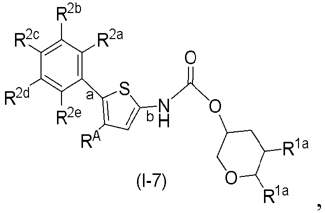 preferably 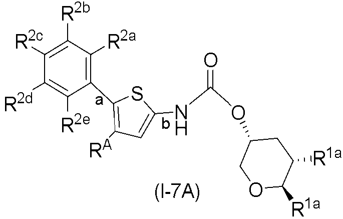 and

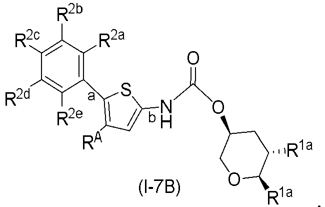

The present invention encompasses compounds obtained by combining any of the embodiments.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from: 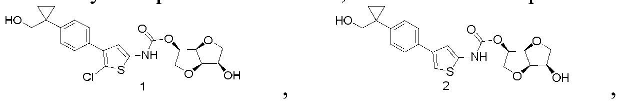 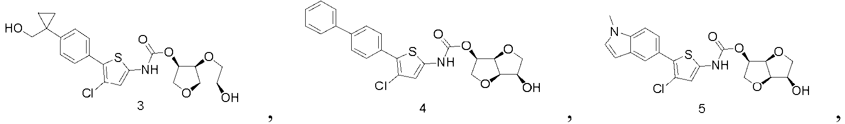 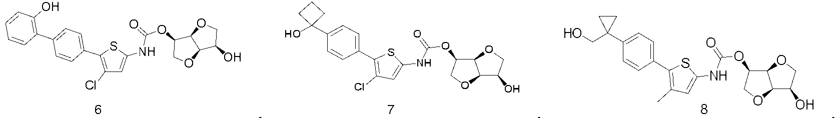 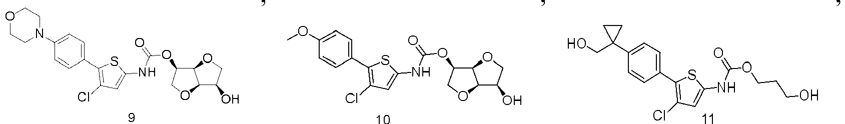 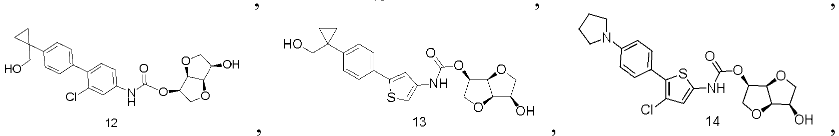 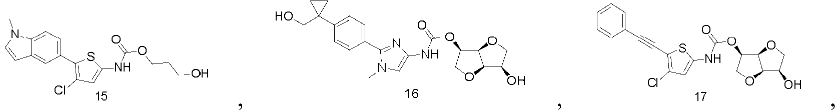 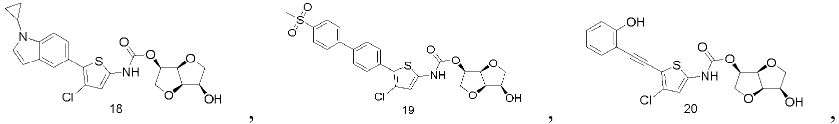 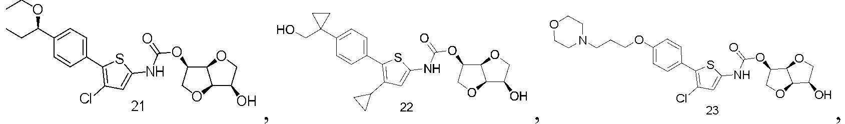 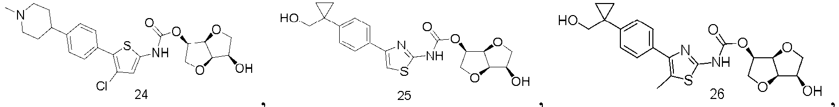 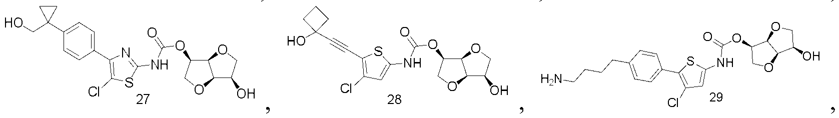 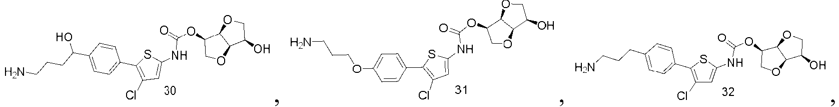 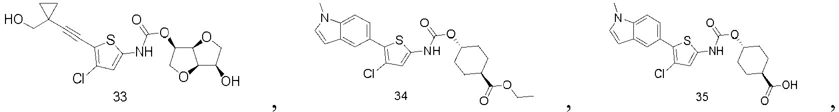 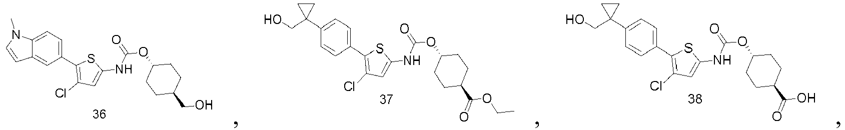 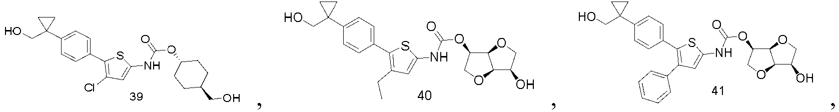 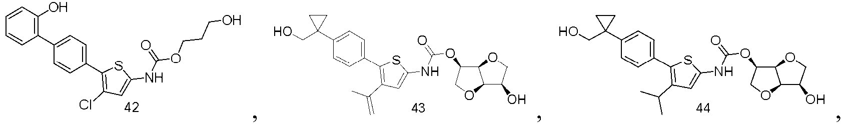 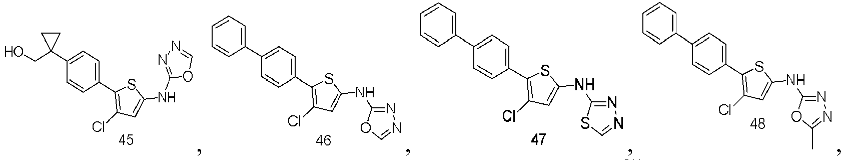 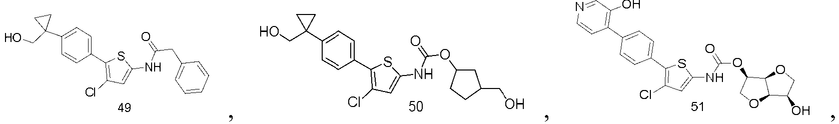 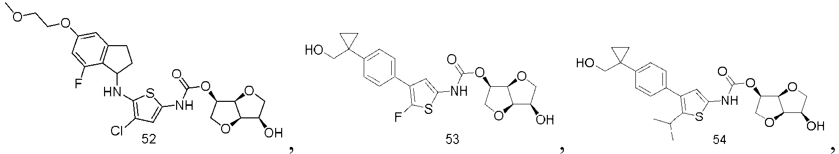 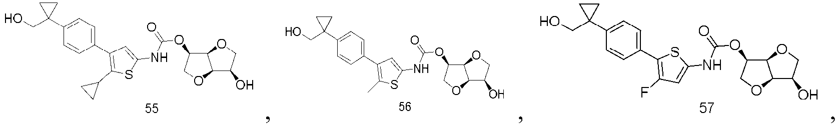 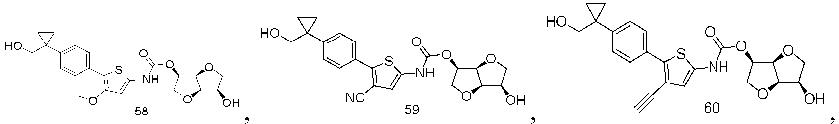 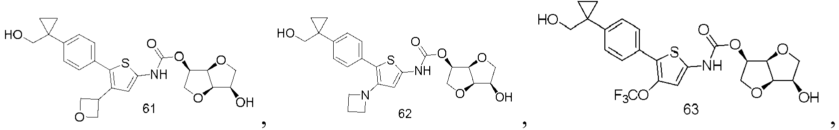 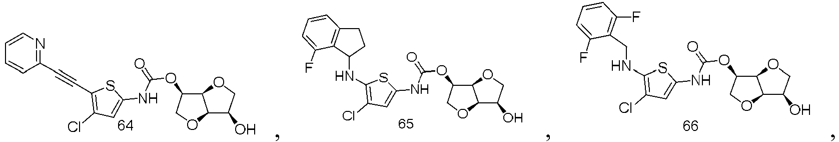 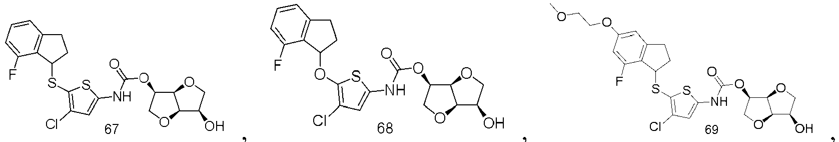 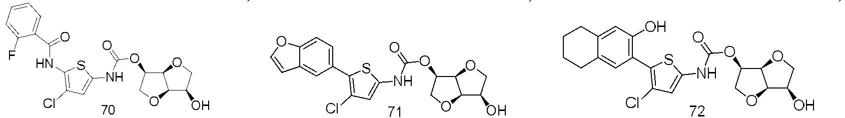 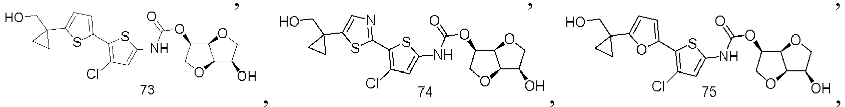 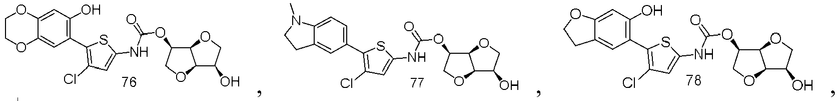 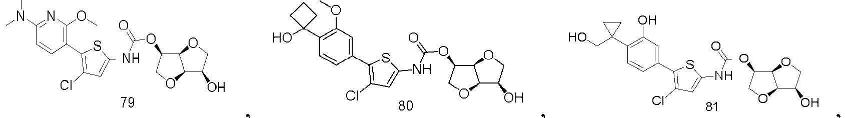 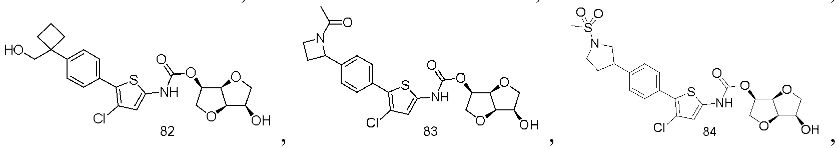 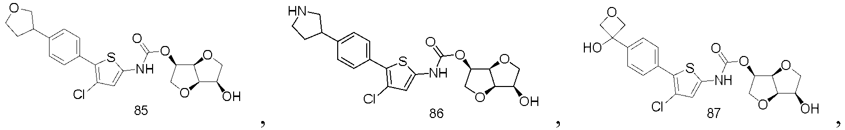 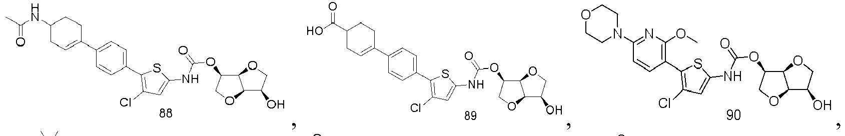 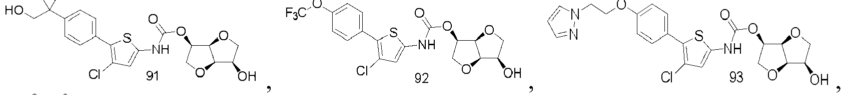 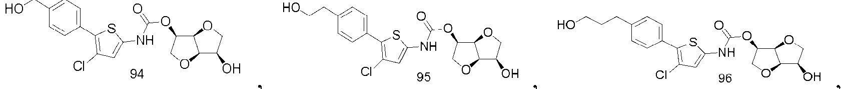 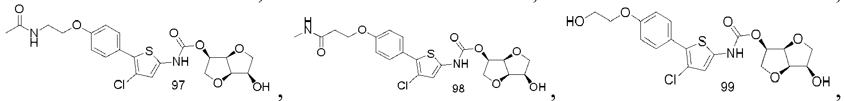 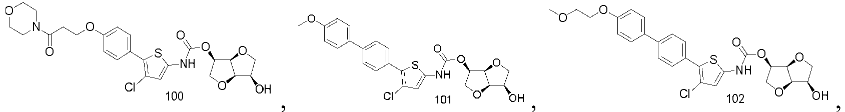 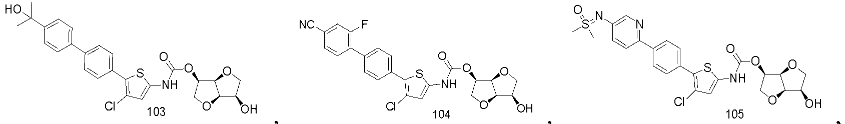 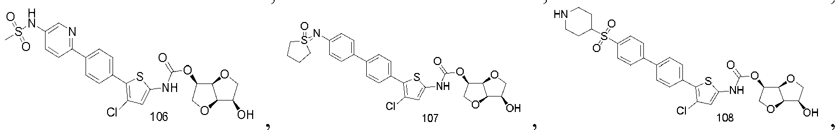 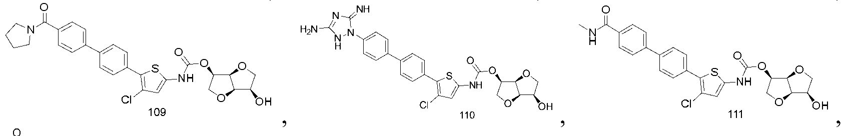 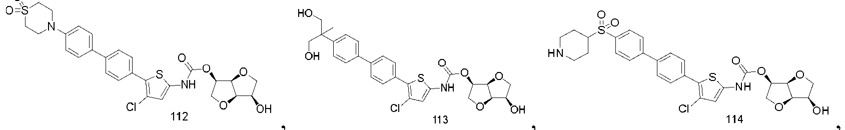 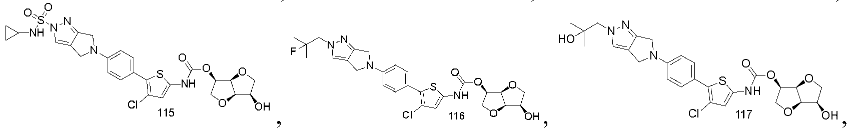 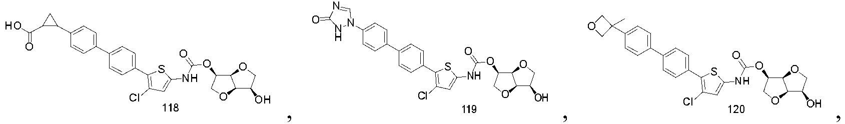 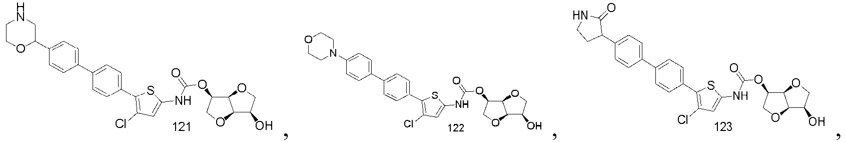 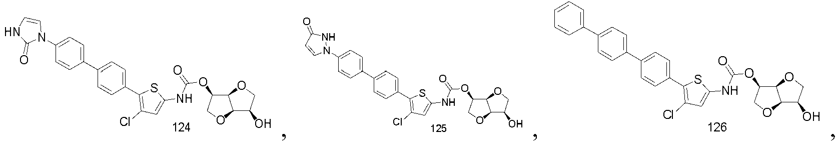 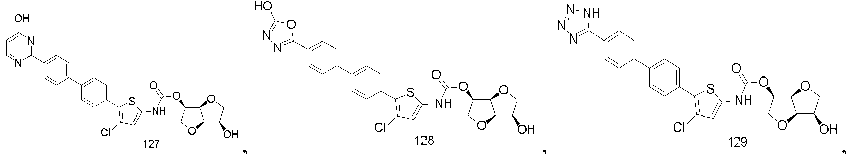 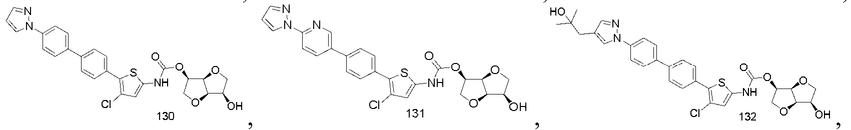 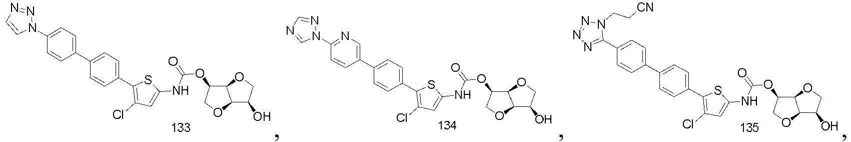 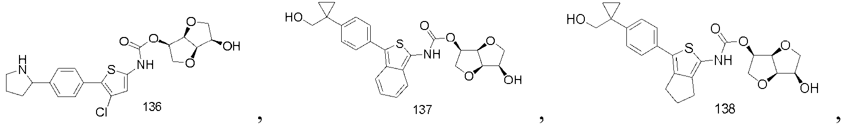 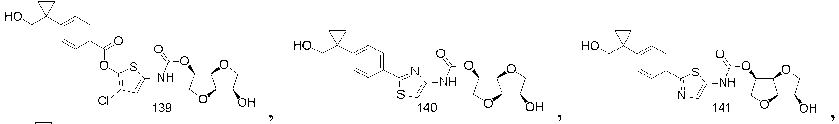 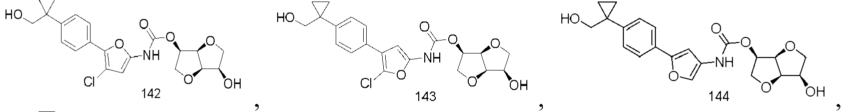 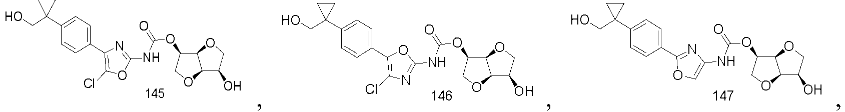 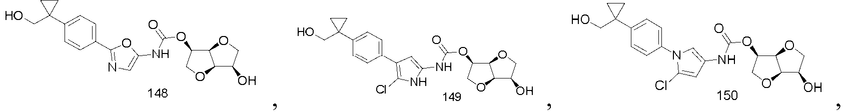 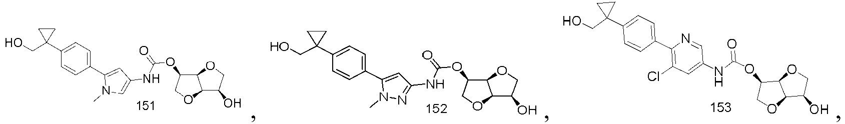 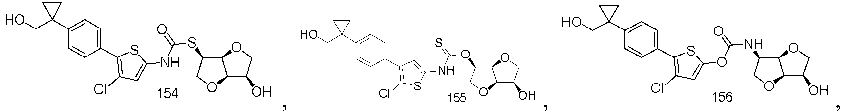 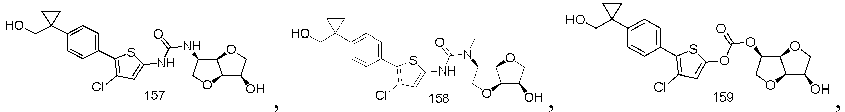 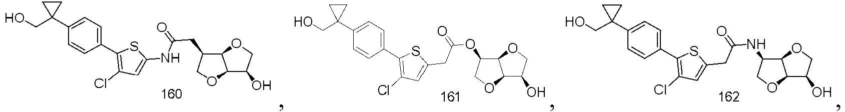 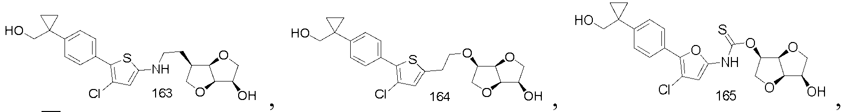 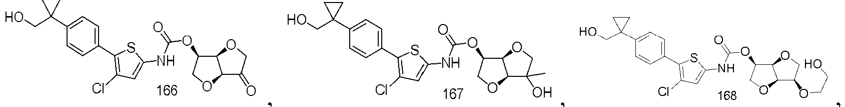 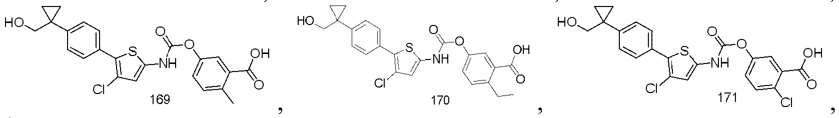 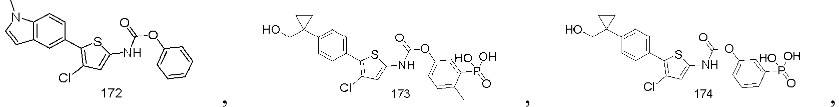 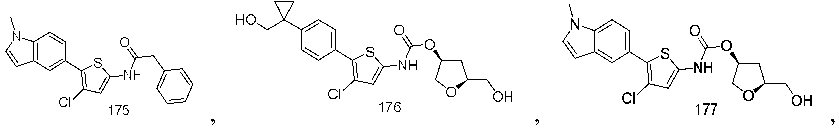 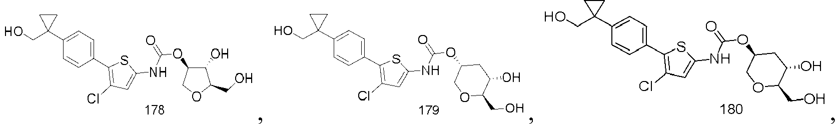 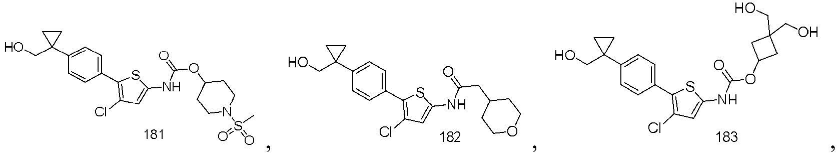 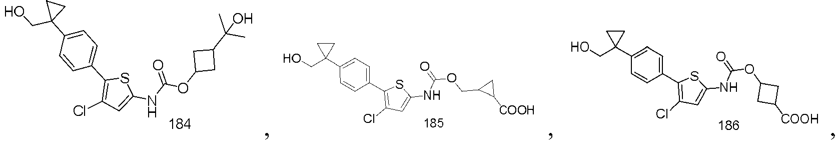 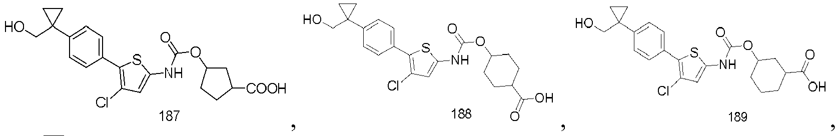 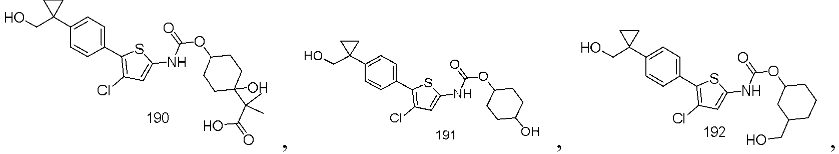 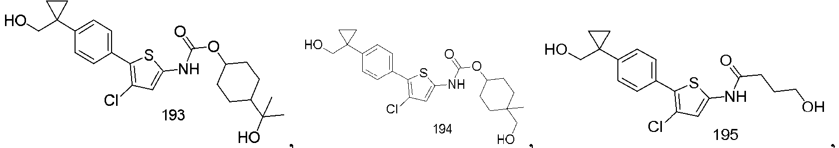 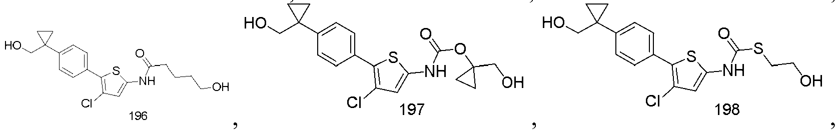 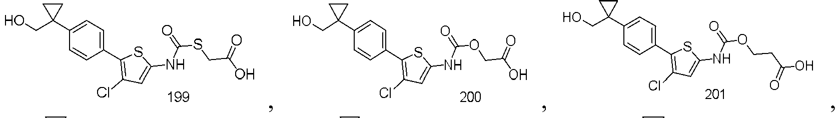 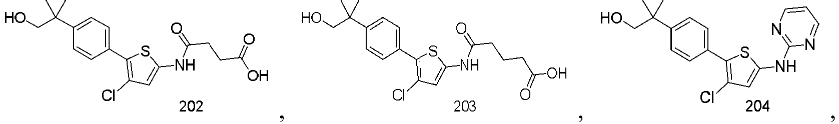 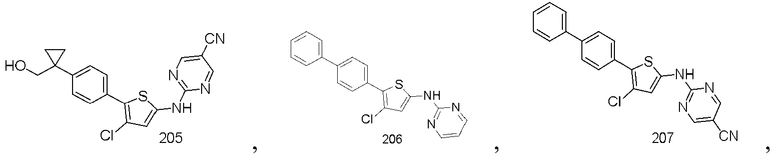 and

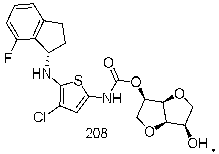

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from Table 1 below:

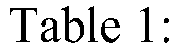

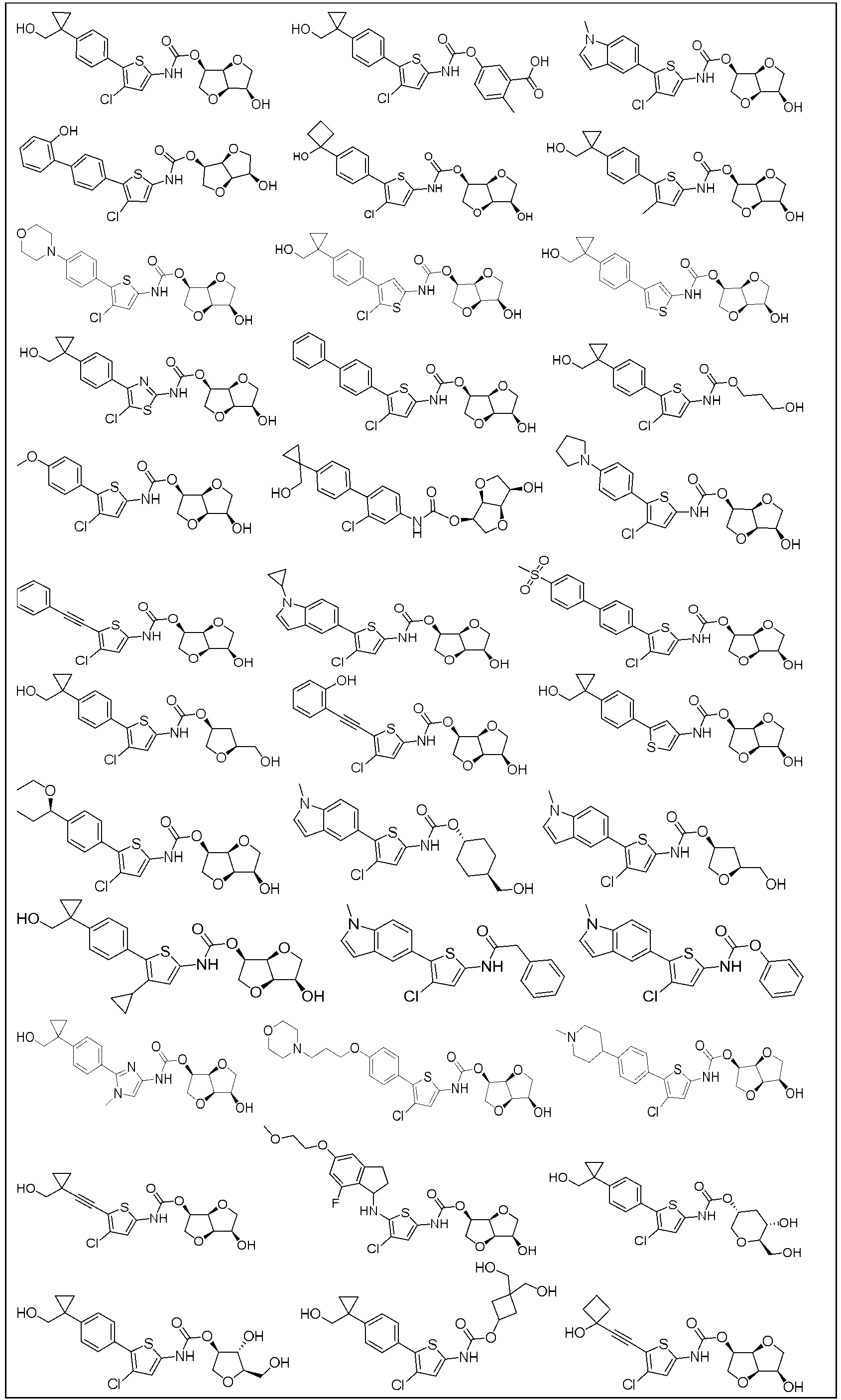

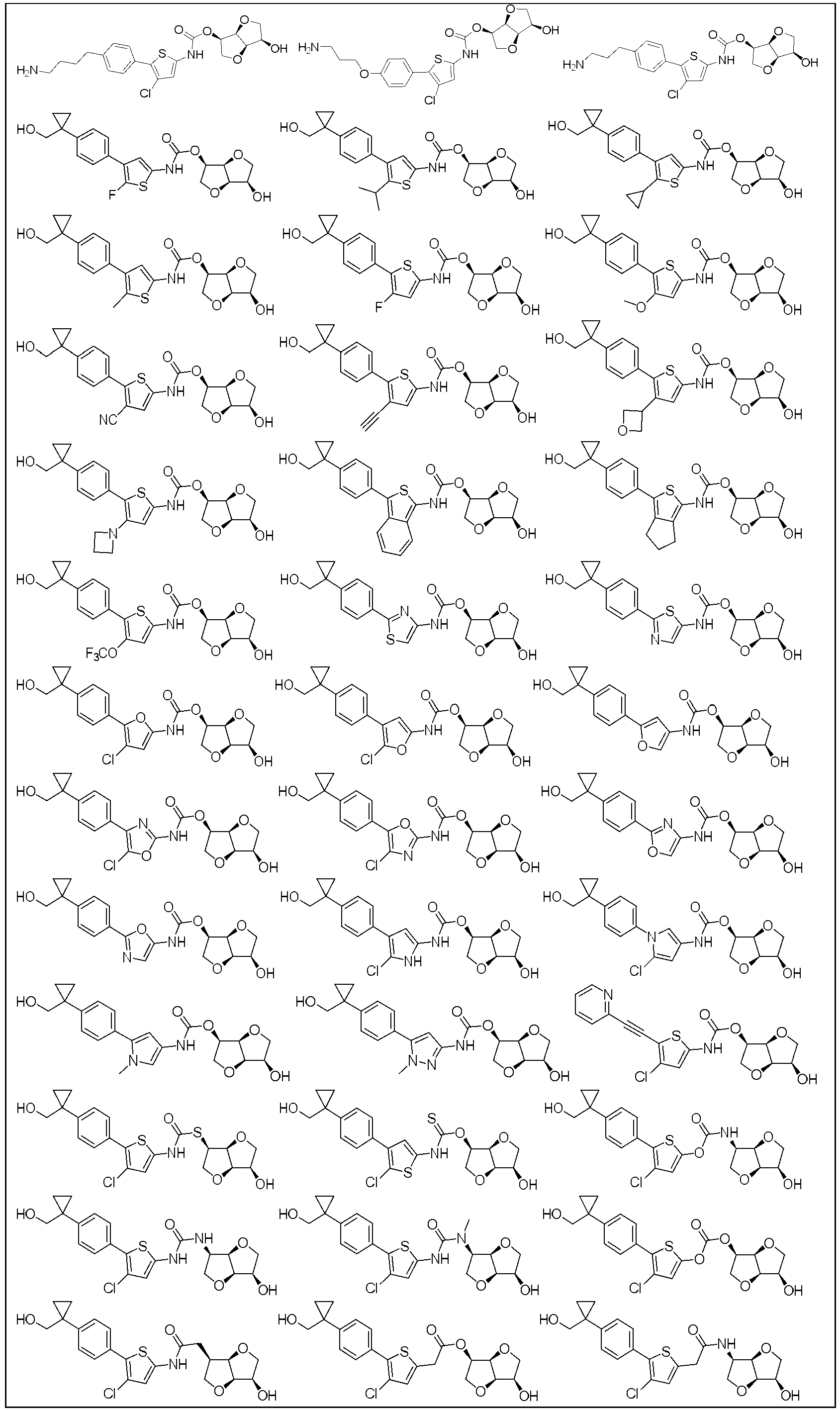

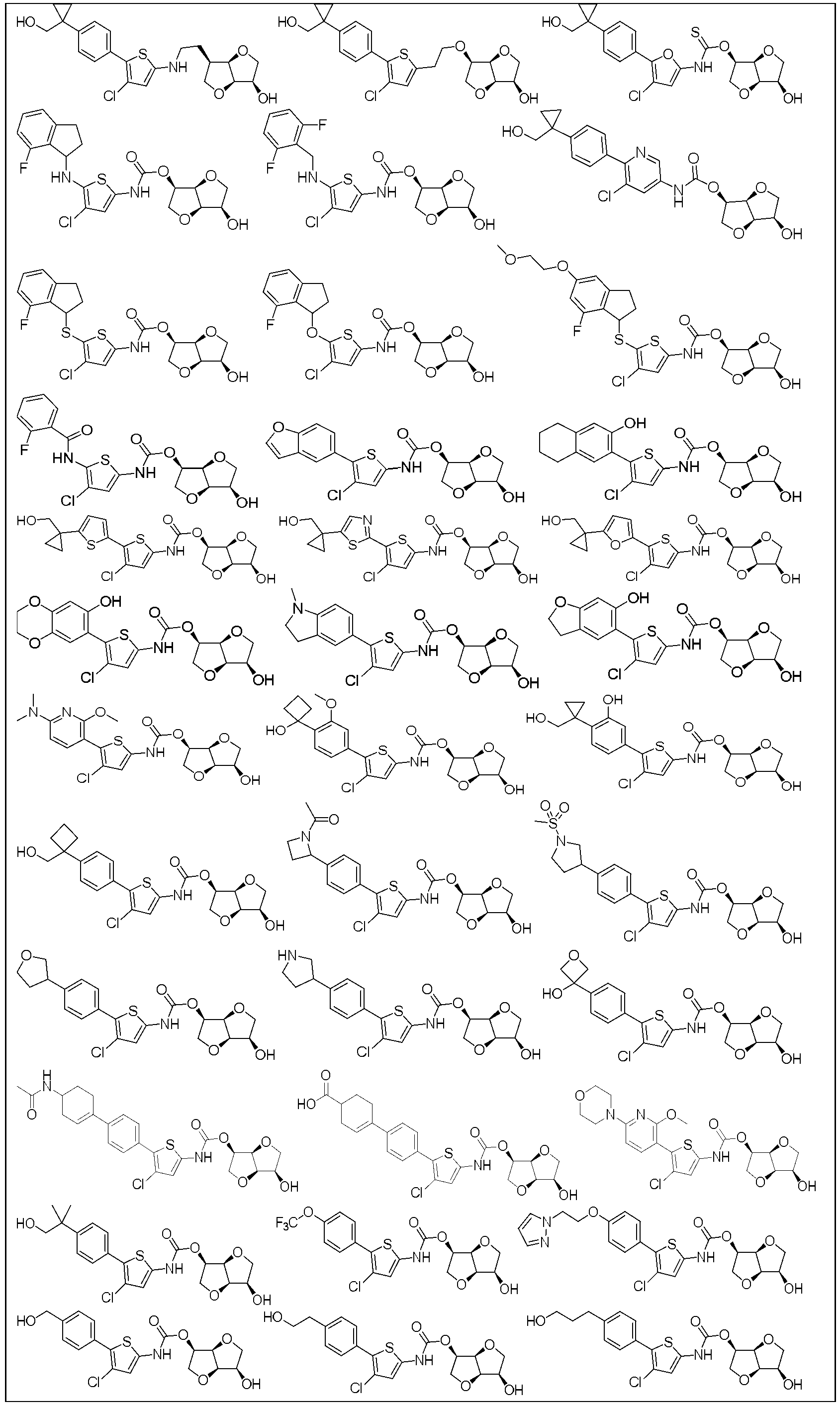

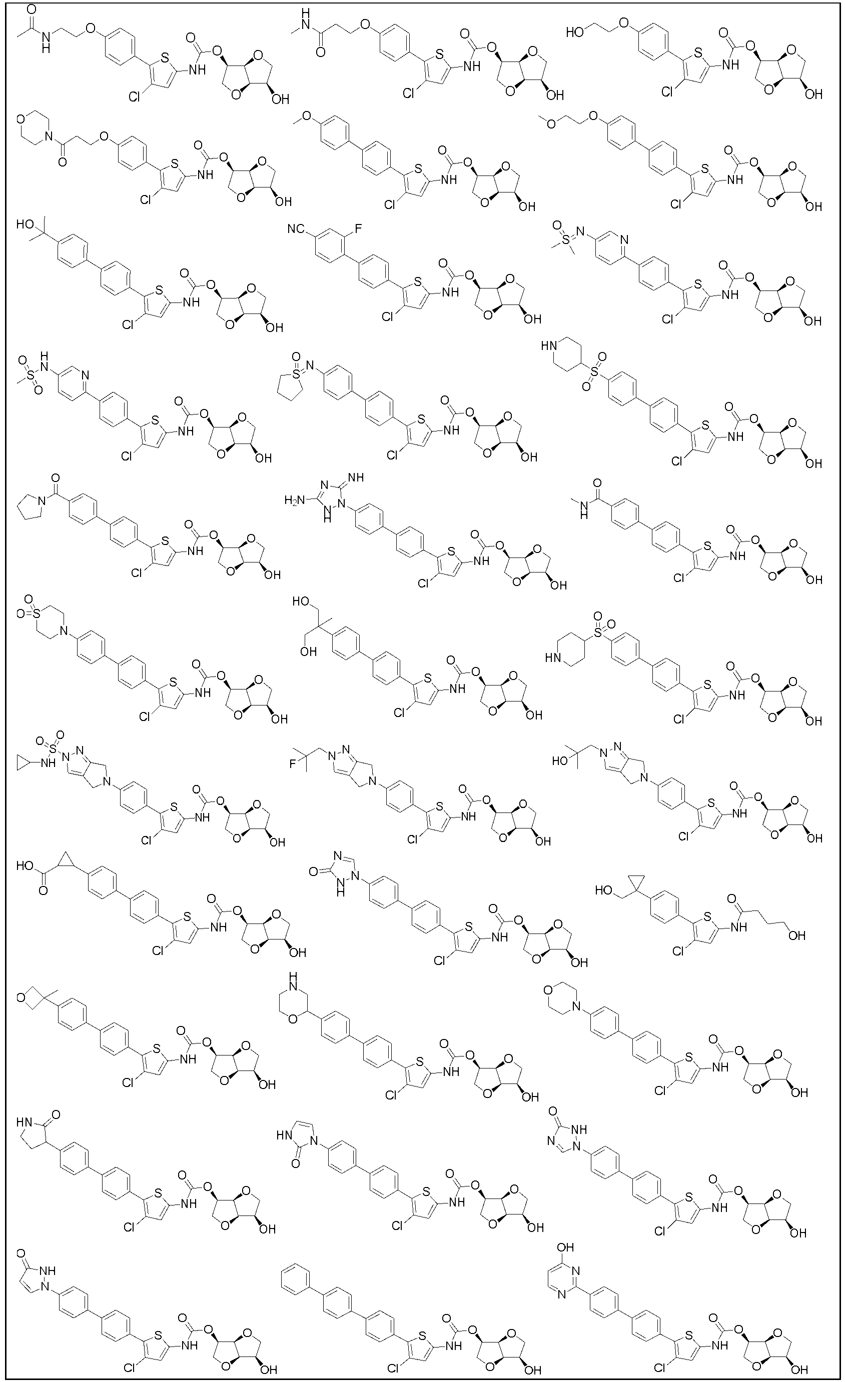

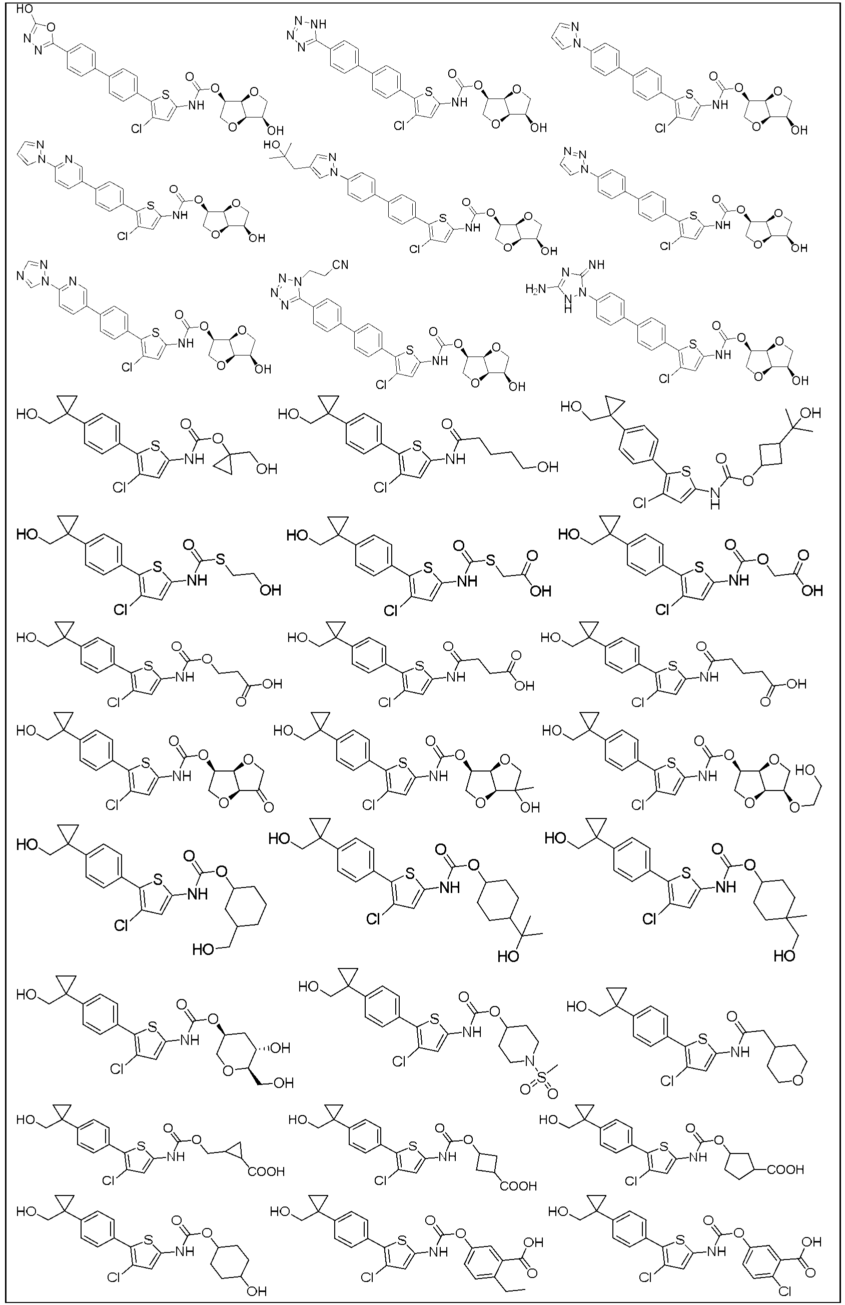

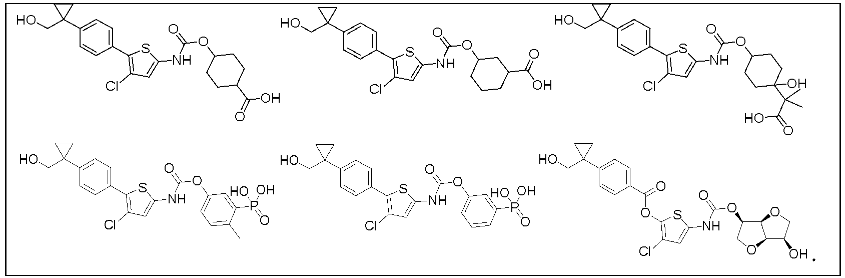

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 1 to compound 11, compound 13 to compound 15, compound 17 to compound 24, compound 28 to compound 44, compound 50 to compound 139, compound 142 to compound 203, and compound 208.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 1 to compound 10, compound 13, compound 14, compound 17 to compound 24, compound 28 to compound 33, compound 40, compound 41, compound 43, compound 44, compound 51 to compound 136, compound 139, and compound 208.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 11, compound 15, and compound 42.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 195, compound 196, and compound 198.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 199, compound 200, compound 201, compound 202, and compound 203.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 137 and compound 138.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 142, compound 143, and compound 144.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 145, compound 146, compound 147, and compound 148.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 149, compound 150, and compound 151.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is compound 152.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is compound 153.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 154 to compound 165.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 166 to compound 168.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 34 to compound 39, compound 50, compound 183 to compound 194, and compound 197.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 169 to compound 175.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 176 to compound 180.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 181 and compound 182.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 1, compound 3 to compound 11, compound 19, compound 21, compound 23, compound 24, compound 38, compound 50, and compound 51.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 3, compound 5 to compound 9, compound 19, compound 21, compound 23, compound 50 and compound 51.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 1, compound 2, compound 53, compound 54, compound 55, and compound 56.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 3 to compound 10, compound 14, compound 18, compound 19, compound 21 to compound 24, compound 40, compound 41, compound 43, compound 44, compound 51, compound 57 to compound 63, compound 80 to compound 82, compound 85 to compound 87, compound 91, compound 92 to compound 96, compound 99, compound 101, compound 103, compound 104, and compound 136.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 34, compound 35, compound 37, compound 38, compound 187, compound 188, compound 189, and compound 190.

In some embodiments, the present invention provides the compound of formula (I), or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof, wherein the compound is selected from compound 36, compound 39, compound 50, and compound 191 to compound 194.

### Preparation method of compounds

The compounds according to the present invention can be synthesised by one of the following synthetic routes as needed.

### Synthetic route 1

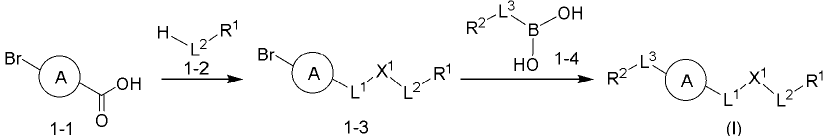

In this route, compound 1-1 undergoes Curtius rearrangement with compound 1-2 to afford intermediate 1-3, and the intermediate 1-3 undergoes Suzuki coupling reaction with compound 1-4 to afford the compound of formula (I); ring A, L¹, L², L³, X¹, R¹ and R² are each as defined above.

### Synthetic route 2

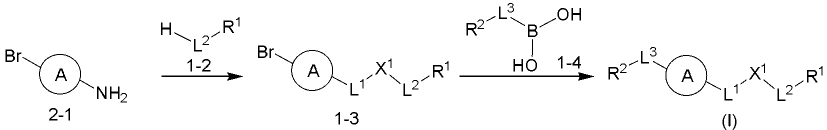

In this route, compound 2-1, compound 1-2 and triphosgene undergo a condensation reaction to afford intermediate 1-3, and the intermediate 1-3 undergoes Suzuki coupling reaction with compound 1-4 to afford the compound of formula (I); ring A, L¹, L², L³, X¹, R¹ and R² are each as defined above.

### Synthetic route 3

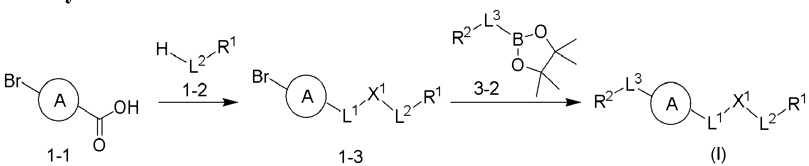

In this route, compound 1-1 undergoes Curtius rearrangement reaction with compound 1-2 to afford intermediate 1-3, and the intermediate 1-3 undergoes Suzuki coupling reaction with compound 3-2 to afford the compound of formula (I); ring A, L¹, L², L³, X¹, R¹ and R² are each as defined above.

### Synthetic route 4

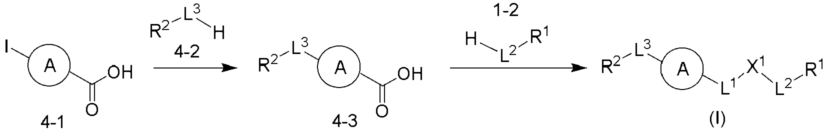

In this route, compound 4-1 undergoes Sonogashira coupling reaction with compound 4-2 to afford intermediate 4-3, and the intermediate 4-3 undergoes Curtius rearrangement reaction with compound 1-2 to afford the compound of formula (I); ring A, L¹, L², L³, X¹, R¹ and R² are each as defined above.

### Pharmaceutical composition and use

In another aspect, the present invention provides a pharmaceutical composition, comprising a therapeutically effective amount of the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, and one or more pharmaceutically acceptable carriers. The pharmaceutical composition is preferably a solid preparation, a liquid preparation or a transdermal preparation.

In another aspect, the present invention provides the use of the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention in the preparation of a medicament.

In another aspect, the present invention provides a method for activating AMPK kinase, comprising contacting the AMPK kinase with the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above. In some embodiments, the method is performed in vitro or ex vivo. In some other embodiments, the method is performed in vivo.

In another aspect, the present invention provides the use of the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention in the preparation of a medicament as an AMPK agonist.

In some embodiments, the compound of the present invention, the pharmaceutical composition of the present invention, or the medicament is used to treat and/or prevent a disease, disorder or condition associated with AMPK kinase.

In some embodiments, the compound of the present invention, the pharmaceutical composition of the present invention, or the medicament is used to treat and/or prevent a disease, disorder or condition that can be treated and/or prevented by activating AMPK.

In another aspect, the present invention further provides a method for treating and/or preventing a disease, disorder or condition associated with AMPK kinase, comprising administering to an individual in need thereof an effective amount of the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention.

In another aspect, the present invention further provides a method for treating and/or preventing a disease, disorder or condition that can be treated and/or prevented by activating AMPK, comprising administering to an individual in need thereof a therapeutically effective amount of the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention.

In yet another aspect, the present invention provides the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention, for use in treating and/or preventing a disease, disorder or condition associated with AMPK kinase.

In yet another aspect, the present invention provides the compound of the present invention, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof as described above, or the pharmaceutical composition of the present invention, for use in treating and/or preventing a disease, disorder or condition that can be treated and/or prevented by activating AMPK.

In some embodiments, the disease, disorder or condition is selected from type II diabetes, dyslipidaemia, obesity, chronic kidney disease, diabetic nephropathy, acute kidney injury, polycystic kidney disease and alopecia. In some embodiments, the disease, disorder or condition is alopecia.

In the present invention, "pharmaceutically acceptable carrier" refers to a diluent, adjuvant, excipient or vehicle that is administered with a therapeutic agent and is suitable for contacting the tissues of humans and/or other animals without excessive toxicity, irritation, allergic reaction or other problems or complications corresponding to a reasonable benefit/risk ratio within the scope of reasonable medical judgment.

Unless otherwise specified, as used herein, the term "treating" means reversing, alleviating or inhibiting the disorders or conditions to which such term applies, or the progression of one or more symptoms of such disorders or conditions, or preventing such disorders or conditions, or one or more symptoms of such disorders or conditions.

As used herein, "individual" includes human or non-human animals. Exemplary human individuals include human individuals suffering from diseases (e.g., diseases described herein) (referred to as patients) or normal individuals. In the present invention, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

In some other embodiments, the pharmaceutical composition of the present invention may further comprise one or more additional therapeutic or prophylactic agents.

### Example

Embodiments of the present invention will be described in detail below with reference to examples; however, those skilled in the art will understand that the following examples are only used to illustrate the present invention and should not be regarded as limiting the scope of the present invention. If no specific conditions are specified in the examples, they are carried out according to conventional conditions or conditions recommended by the manufacturer. If manufacturers of reagents or instruments used are not specified, they are conventional products commercially available.

NMR was measured using a Bruker Avance III 400 nuclear magnetic spectrometer, and the chemical shift (δ) was given in units of 10⁻⁶(ppm). The solvent was deuterated methanol (CD₃OD), deuterated chloroform (CDCl₃) or hexadeuterated dimethyl sulphoxide (DMSO-d6), and the internal standard was tetramethylsilane (TMS).

MS was measured using an Agilent (ESI) mass spectrometer (Agilent 1260, Agilent 6125B).

Thin layer chromatography silica gel plate (aluminium plate (20 cm x 20 cm x 1 mm) produced by Meck, or GF 254 produced in Yantai) was used for thin layer chromatography separation and purification.

Biotage Initiator + (400 W, RT ~ 300°C) microwave reactor was used for reaction under microwave.

TLC or LCMS was commonly used for reaction monitoring, and the commonly used developing solvent systems were: dichloromethane/methanol, n-hexane/ethyl acetate, and petroleum ether/ethyl acetate, and the volume ratio of the solvents was adjusted according to the polarity of the compound or adjusted by adding triethylamine, etc.

The silica gel used in column chromatography was generally 100 - 200 mesh silica gel. Commonly used eluent systems were: dichloromethane/methanol and petroleum ether/ethyl acetate, and the volume ratio of the solvents was adjusted according to the polarity of the compound or adjusted by adding a small amount of triethylamine.

The reagents and solvents of the present invention were purchased from Aldrich Chemical Company, Energy Chemical, J&K Scientific, Bide Pharmatech Ltd., PharmaBlock Sciences (Nanjing), Inc. and Shanghai Titan Scientific Co., Ltd.

### Synthesis examples

### Example 1: Synthesis of compound 1

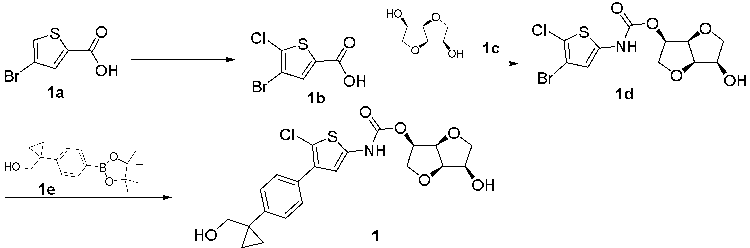

**Step 1:** At room temperature, compound **1a** (4.00 g, 19.32 mmol), *N,N-*dimethylformamide (30 mL) and N-chlorosuccinimide (3.87 g, 28.98 mmol) were sequentially added to a 50 mL reaction flask. After purging with nitrogen 3 times, the mixture was stirred and reacted at 50°C for 16 hours. After the reaction was completed, the reaction solution was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford intermediate **1b**. LC-MS: m/z: 240.8 (M + H)⁺.

**Step 2:** At room temperature, intermediate **1b** (200 mg, 0.82 mmol), compound **1c** (605 mg, 4.12 mmol), dioxane (10 mL), diphenyl phosphorazidate (274 mg, 1.00 mmol) and triethylamine (335 mg, 3.32 mmol) were sequentially added to a 250 mL reaction flask. After purging with nitrogen 3 times, the mixture was stirred and reacted at 90°C for 16 hours. After the reaction was completed, the reaction solution was concentrated under reduced pressure to afford a residue, and the resulting residue was separated and purified by silica gel column chromatography to afford intermediate **1d**. LC-MS: m/z: 384.0 (M+H)⁺.

**Step 3:** Under nitrogen atmosphere, intermediate **1d** (55 mg, 0.14 mmol), compound **1e** (32.95 mg, 0.17 mmol), [1,1'-bis(diphenylphosphino)ferrocene]palladium dichloride-dichloromethane complex (11.68 mg, 0.01 mmol), potassium carbonate (59.29 mg, 0.43 mmol), dioxane (2 mL) and water (0.4 mL) were added to a 8 mL microwave tube. The reaction was carried out at 100°C under microwave conditions for two hours until completion. Subsequently, the reaction solution was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **1.** LC-MS: m/z: 452.2 (M+H)⁺. ¹H NMR (400 MHz, DMSO-*d*₆) δ 11.14 (s, 1H), 7.42 (d, *J =* 8.3 Hz, 2H), 7.37 (d, *J =* 8.4 Hz, 2H), 6.52 (s, 1H), 5.08 (q, *J =* 5.9 Hz, 1H), 4.94 (d, *J =* 6.3 Hz, 1H), 4.70 (s, 1H), 4.64 (t, *J =* 5.1 Hz, 1H), 4.30 (t, *J* = 4.9 Hz, 1H), 4.12 (s, 1H), 3.96 (dd, *J* = 9.5*,* 6.2 Hz, 1H), 3.78 (dt, *J =* 15.3, 8.5 Hz, 2H), 3.55 (d, *J* = 4.1 Hz, 2H), 3.40 (d, *J =* 8.6 Hz, 1H), 0.85 (t, *J =* 2.9 Hz, 2H), 0.79-0.73 (m, 2H).

### Example 2: Synthesis of compound 2

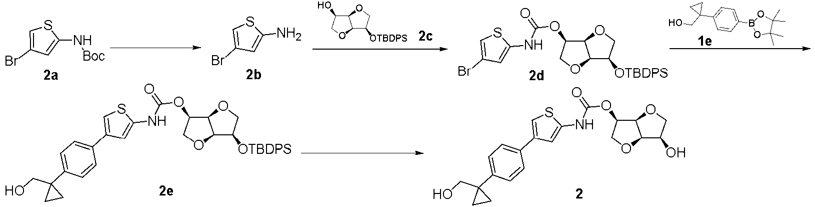

**Step 1:** To a 25 mL single-necked flask were added compound **2a** (450 mg, 1.62 mmol), hydrogen chloride dioxane solution (3 mL) and dioxane (9 mL). The reaction was carried out at room temperature for 3 hours until completion. The reaction solution was concentrated under reduced pressure to afford intermediate **2b.** LC-MS: m/z: 178.0 (M+H)⁺.

**Step 2:** To a 25 mL single-necked flask were sequentially added intermediate **2b** (288 mg, 1.62 mmol), compound **2c** (1.24 mg, 3.24 mmol), triethylamine (982.0 mg, 9.71 mmol), dichloromethane (10 mL) and triphosgene (479.97 mg, 1.62 mmol). The flask was purged with nitrogen, and the reaction was carried out at room temperature overnight until completion. The reaction solution was poured into water (50 mL), and the mixture was extracted with ethyl acetate (7 mL x 3). The organic phase was washed with saturated aqueous sodium chloride solution, dried over anhydrous sodium sulphate, and filtered to afford the filtrate, which was concentrated under reduced pressure to afford a crude product. The crude product was separated and purified by silica gel column chromatography to afford intermediate **2d.** LC-MS: m/z: 610.2 (M + Na)⁺. ¹H NMR (400 MHz, DMSO-*d*₆) δ 11.08 (s, 1H), 7.70-7.66 (m, 2H), 7.63-7.59 (m, 2H), 7.49-7.42 (m, 6H), 6.93 (d, *J =* 1.9 Hz, 1H), 6.45 (d, *J* = 1.9 Hz, 1H), 5.06 (q, *J* = 5.7 Hz, 1H), 4.56 (t, *J =* 5.1 Hz, 1H), 4.18 (q, *J* = 6.5, 4.9 Hz, 2H), 3.99-3.93 (m, 1H), 3.90-3.84 (m, 1H), 3.69 (t, *J* = 7.3 Hz, 1H), 3.53 (t, *J* = 8.0 Hz, 1H), 1.02 (s, 9H).

**Step 3:** Under nitrogen atmosphere, intermediate **2d** (60 mg, 0.1 mmol), compound **1e** (55.90 mg, 0.20 mmol), [1,1'-bis(diphenylphosphino)ferrocene]palladium dichloride-dichloromethane complex (24.97 mg, 0.03 mmol), potassium carbonate (70.44 mg, 0.51 mmol), dioxane (5 mL) and water (1 mL) were added to a 25 mL single-necked flask. The reaction was carried out at 90°C for 16 hours until completion. Subsequently, the reaction system was concentrated under reduced pressure to afford a crude product, and the crude product was separated and purified by silica gel column chromatography to afford intermediate **2e.** LC-MS: m/z: 656.4 (M+H)⁺.

**Step 4:** To a 25 mL single-necked flask were added intermediate **2e** (30 mg, 0.05 mmol), a solution of tetrabutylammonium fluoride in tetrahydrofuran (90 uL, 0.09 mmol) and tetrahydrofuran (2 mL). The flask was purged with nitrogen, and the reaction was carried out at room temperature overnight until completion. The reaction solution was concentrated under reduced pressure to afford a crude product. The crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **2.** LC-MS: m/z: 418.1 (M+H)⁺, ¹H NMR (400 MHz, DMSO-*d*₆) δ 7.49 (d, *J* = 8.2 Hz, 2H), 7.31 (d, *J =* 8.0 Hz, 2H), 7.19 (s, 1H), 6.89 (s, 1H), 5.08 (q, *J* = 5.9 Hz, 1H), 4.97 (d, *J =* 6.4 Hz, 1H), 4.70 (t, *J* = 5.5 Hz, 1H), 4.64 (t, *J =* 5.2 Hz, 1H), 4.31 (t, *J =* 5.0 Hz, 1H), 4.15-4.10 (m, 1H), 4.00-3.96 (m, 1H), 3.82-3.75 (m, 3H), 3.54 (d, *J* = 5.5 Hz, 2H), 0.86-0.73 (m, 4H).

### Example 3: Synthesis of compound 3

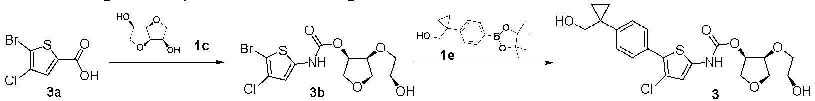

**Step 1:** At room temperature, compound **3a** (1000 mg, 4.14 mmol), compound **1c** (3026 mg, 20.71 mmol), dioxane (80 mL), diphenyl phosphorazidate (1368 mg, 4.97 mmol) and triethylamine (629 mg, 6.21 mmol) were sequentially added to a 250 mL reaction flask. After purging with nitrogen 3 times, the mixture was stirred and reacted at 90°C for 16 hours. After the reaction was completed, the reaction solution was concentrated in vacuo, and the resulting residue was purified by silica gel column chromatography to afford intermediate **3b.** LC-MS: m/z: 384.0 (M+H)⁺. ¹H NMR (400 MHz, DMSO-*d₆*) δ 11.37 (s, 1H), 6.41 (s, 1H), 5.08 (q, *J* = 5.8 Hz, 1H), 4.63 (t, *J* = 5.1 Hz, 1H), 4.29 (t, *J = 4.9* Hz, 1H), 4.15-4.08 (m, 1H), 3.98-3.92 (m, 1H), 3.82-3.72 (m, 2H), 3.38 (t, *J* = 8.5 Hz, 1H).

**Step 2:** At room temperature, intermediate **3b** (80 mg, 0.21 mmol), compound **1e** (68.43 mg, 0.25 mmol),[1,1'-bis(diphenylphosphino)ferrocene]palladium dichloride-dichloromethane complex (16.99 mg, 0.02 mmol), potassium carbonate (86.23 mg, 0.62 mmol), dioxane (3 mL) and water (0.60 mL) were added to a 10 mL microwave tube. The mixture was subjected to a microwave reaction at 100°C under nitrogen atmosphere for two hours. After the reaction was completed, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 3. LC-MS: m/z: 452.1 (M+H)⁺, ¹H NMR (400 MHz, DMSO-*d*₆) δ 8.58 (s, 1H), 7.48 (d, *J* = 8.4 Hz, 2H), 7.36 (d, *J =* 8.4 Hz, 2H), 6.49 (s, 1H), 5.08 (q, *J* = 5.9 Hz, 1H), 4.94 (d, *J* = 6.4 Hz, 1H), 4.70 (t, *J* = 5.7 Hz, 1H), 4.64 (t, *J* = 5.1 Hz, 1H), 4.30 (t, *J =* 4.9 Hz, 1H), 4.17-4.08 (m, 1H), 3.97 (dd, *J* = 9.5, 6.2 Hz, 1H), 3.83-3.71 (m, 2H), 3.55 (d, *J =* 5.7 Hz, 2H), 3.39 (t, *J =* 8.5 Hz, 1H), 0.91-0.81 (m, 2H), 0.81 - 0.72 (m, 2H).

### Example 4: Synthesis of compound 4

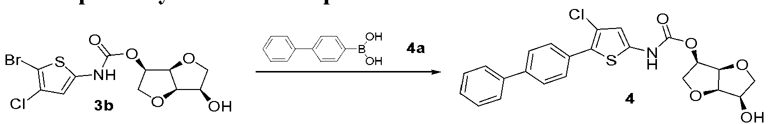

Under nitrogen atmosphere, intermediate **3b** (100 mg, 0.26 mmol), compound **4a** (61.78 mg, 0.31 mmol), [1,1'-bis(diphenylphosphino)ferrocene]palladium dichloride-dichloromethane complex (19.02 mg, 0.03 mmol), potassium carbonate (89.83 mg, 0.65 mmol), dioxane (2 mL) and water (0.4 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **4.** LC-MS: m/z: 458.1 (M+H)⁺, ¹H NMR (400 MHz, CDCl₃) δ 7.73 (d, *J =* 8.4 Hz, 2H), 7.68-7.55 (m, 4H), 7.51-7.38 (m, 3H), 7.36 (t, *J* = 7.3 Hz, 1H), 6.53 (s, 1H), 5.25 (q, *J* = 6.2 Hz, 1H), 4.74 (t, *J* = 5.1 Hz, 1H), 4.56 (t, *J =* 5.2 Hz, 1H), 4.35 (q, *J* = 6.0 Hz, 1H), 4.21 (dd, *J =* 9.4, 6.4 Hz, 1H), 4.01 (dd, *J* = 9.3, 6.0 Hz, 1H), 3.90 (t, *J =* 8.2 Hz, 1H), 3.75-3.58 (m, 1H).

### Example 5: Synthesis of compound 5

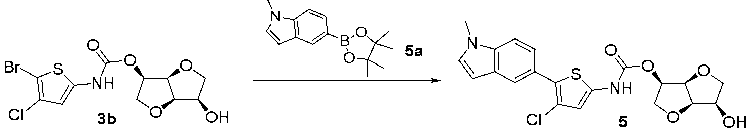

Under nitrogen atmosphere, intermediate **3b** (100 mg, 0.26 mmol), compound **5a** (80.23 mg, 0.31 mmol), [1,1'-bis(diphenylphosphino)ferrocene]palladium dichloride-dichloromethane complex (19.02 mg, 0.03 mmol), potassium carbonate (89.83 mg, 0.65 mmol), dioxane (2 mL) and water (0.4 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **5.** LC-MS: m/z: 435.2 (M+H)⁺, ¹H NMR (400 MHz, DMSO-*d6*) δ 11.08 (s, 1H), 7.74 (d, *J* = 1.8 Hz, 1H), 7.51 (d, *J =* 8.6 Hz, 1H), 7.38 (d, *J* = 3.1 Hz, 1H), 7.34 (dd, *J* = 8.5, 1.8 Hz, 1H), 6.62-6.37 (m, 2H), 5.08 (q, *J =* 6.0 Hz, 1H), 4.94 (d, *J =* 6.5 Hz, 1H), 4.64 (t, *J* = 5.1 Hz, 1H), 4.30 (t, *J* = 4.9 Hz, 1H), 4.19-4.06 (m, 1H), 3.98 (dd, *J =* 9.4, 6.2 Hz, 1H), 3.90-3.65 (m, 5H), 3.40 (t, *J* = 8.5 Hz, 1H).

### Example 6: Synthesis of compound 6

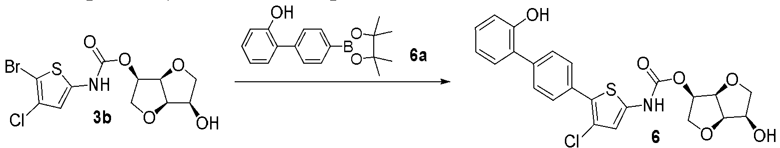

Under nitrogen atmosphere, intermediate **3b** (100 mg, 0.26 mmol), compound **6a** (77.0 mg, 0.26 mmol), [1,1'-bis(diphenylphosphino)ferrocene]palladium dichloride-dichloromethane complex (21 mg, 0.03 mmol), potassium carbonate (89.83 mg, 0.65 mmol), dioxane (2 mL) and water (0.5 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **6.** LC-MS: m/z: 474.0 (M+H)⁺, ¹H NMR (400 MHz, DMSO-d6) δ 11.20 (s, 1H), 9.66 (s, 1H), 7.62 (d, *J* = 1.5 Hz, 4H), 7.30 (dd, *J* = 7.6, 1.7 Hz, 1H), 7.20-7.15 (m, 1H), 6.96 (d, *J =* 8.0 Hz, 1H), 6.89 (t, *J =* 7.5 Hz, 1H), 6.53 (s, 1H), 5.12-5.07(m, 1H), 4.95 (s, 1H), 4.65 (t, *J =* 5.1 Hz, 1H), 4.30 (t, *J* = 4.9 Hz, 1H), 4.13 (s, 1H), 3.99-3.96 (m, 1H), 3.83-3.75 (m, 2H), 3.40 (t, *J =* 8.5 Hz,, 1H).

### Example 7: Synthesis of compound 7

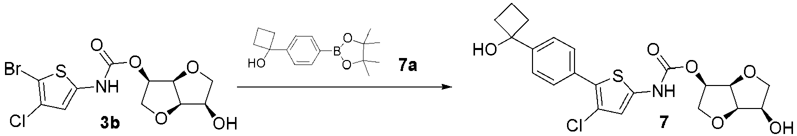

At room temperature, intermediate **3b** (100 mg, 0.26 mmol), compound **7a** (85.54 mg, 0.31 mmol),[1,1'-bis(diphenylphosphino)ferrocene]palladium dichloride-dichloromethane complex (21.23 mg, 0.03 mmol), potassium carbonate (107.79 mg, 0.78 mmol), dioxane (3.00 mL) and water (0.60 mL) were added to a 10 mL microwave tube. The mixture was subjected to a microwave reaction at 100°C under nitrogen atmosphere for two hours. After the reaction was completed, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 7. LC-MS: m/z: 434.2 (M + H -18)⁺, ¹H NMR (400 MHz, DMSO-*d*₆) δ 11.15 (s, 1H), 7.55 (s, 4H), 6.51 (s, 1H), 5.54 (s, 1H), 5.08 (q, *J* = 6.0 Hz, 1H), 4.94 (d, *J* = 6.4 Hz, 1H), 4.64 (t, *J* = 5.1 Hz, 1H), 4.30 (t, *J* = 4.9 Hz, 1H), 4.17 - 4.09 (m, 1H), 3.97 (dd, *J =* 9.5, 6.2 Hz, 1H), 3.83 - 3.74 (m, 2H), 3.39 (t, *J* = 8.5 Hz, 1H), 2.44 - 2.36 (m, 2H), 2.32 - 2.23 (m, 2H), 1.98 - 1.87 (m, 1H), 1.72 - 1.60 (m, 1H).

### Example 8: Synthesis of compound 8

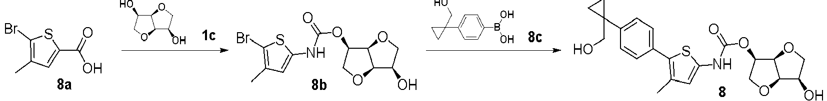

**Step 1:** To a 25 mL single-necked flask were added compound **8a** (1 g, 4.52 mmol), compound **1c** (3.31 g, 22.62 mmol), diphenyl phosphorazidate (1.49 mg, 5.43 mmol), triethylamine (0.92 g, 9.05 mmol) and dioxane (20 mL). The flask was purged with nitrogen, and the reaction was carried out at 90°C for fifteen hours until completion. The reaction solution was poured into water (200 mL), and the mixture was extracted with ethyl acetate (15 mL x 3). The organic phase was washed with saturated aqueous sodium chloride solution, dried over anhydrous sodium sulphate, filtered, and concentrated to afford a crude product. The crude product was separated and purified by silica gel column chromatography to afford intermediate **8b.** LC-MS: m/z: 364.0 (M+H)⁺.

**Step 2:** Under nitrogen atmosphere, intermediate **8b** (100 mg, 0.27 mmol), compound **8c** (88.83 mg, 0.32 mmol), [1,1'-bis(diphenylphosphino)ferrocene]palladium dichloride-dichloromethane complex (22.42 mg, 0.03 mmol), potassium carbonate (94.86 mg, 0.69 mmol), dioxane (2 mL) and water (0.4 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **8.** LC-MS: m/z: 432.2 (M+H)⁺. ¹H NMR (400 MHz, DMSO-*d6*) δ 7.33-7.28 (m, 4H), 6.42 (s, 1H), 5.07-5.02 (m, 1H), 4.97-4.84 (m, 1H), 4.71-4.64 (m, 1H), 4.62 (t, *J =* 5.2 Hz, 1H), 4.30 (t, *J =* 4.8 Hz, 1H), 4.14-4.09 (m, 1H), 3.99-3.95 (m, 1H), 3.81-3.71 (m, 2H), 3.54 (s, 2H), 3.39 (t, *J* = 8.4 Hz, 1H), 2.17 (s, 3H), 8.54-8.29 (m, 2H), 0.76-0.73 (m, 2H).

### Example 9: Synthesis of compound 9

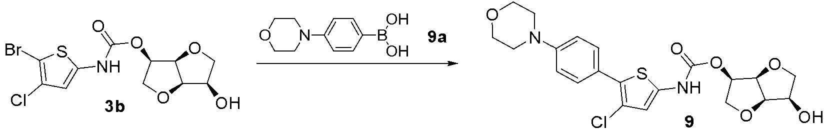

Under nitrogen atmosphere, intermediate **3b** (100 mg, 0.26 mmol), compound **9a** (64.6 mg, 0.31 mmol), [1,1'-bis(diphenylphosphino)ferrocene]palladium dichloride-dichloromethane complex (19.02 mg, 0.03 mmol), potassium carbonate (89.83 mg, 0.65 mmol), dioxane (2 mL) and water (0.4 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **9.** LC-MS: m/z: 467.2 (M+H)⁺. 1H NMR (400 MHz, CDCl₃) δ 7.55 (d, J = 8.4 Hz, 1H), 7.33 (s, 1H), 6.94 (d, J = 8.6 Hz, 1H), 6.49 (s, 1H), 5.23 (dd, J = 12.0, 6.4 Hz, 1H), 4.73 (t, J = 5.0 Hz, 1H), 4.55 (t, J = 5.1 Hz, 1H), 4.34 (d, J = 6.0 Hz, 1H), 4.24 - 4.14 (m, 1H), 4.01 (dd, J = 9.3, 6.0 Hz, 1H), 3.88 (s, 5H), 3.72 - 3.62 (m, 1H), 3.22 (s, 4H).

### Example 10: Synthesis of compound 10

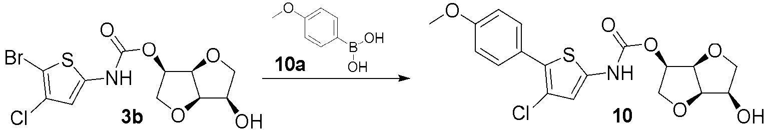

At room temperature, intermediate **3b** (100 mg, 0.26 mmol), compound **10a** (47.41 mg, 0.31 mmol), [1,1-bis(diphenylphosphino)ferrocene]palladium dichloride-dichloromethane complex (21.23 mg, 0.03 mmol), potassium carbonate (107.79 mg, 0.78 mmol), dioxane (3.00 mL) and water (0.60 mL) were added to a 10 mL microwave tube. The mixture was subjected to a microwave reaction at 100°C under nitrogen atmosphere for two hours. After the reaction was completed, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **10.** LC-MS: m/z: 412.0 (M+H)⁺. ¹H NMR (400 MHz, DMSO-*d*₆) δ 11.13 (s, 1H), 7.50 (d, *J* = 8.8 Hz, 2H), 7.02 (d, *J* = 8.8 Hz, 2H), 6.48 (s, 1H), 5.08 (q, *J* = 6.0 Hz, 1H), 4.94 (d, *J =* 6.4 Hz, 1H), 4.63 (t, *J* = 5.1 Hz, 1H), 4.30 (t, *J =* 4.9 Hz, 1H), 4.16 - 4.08 (m, 1H), 3.97 (dd, *J* = 9.4, 6.2 Hz, 1H), 3.82 - 3.73 (m, 5H), 3.39 (t, *J =* 8.5 Hz, 1H).

### Example 11: Synthesis of compound 11

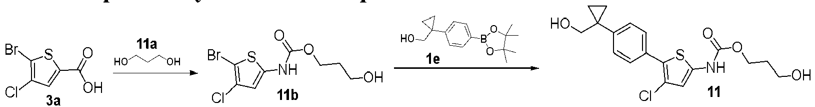

**Step 1:** At room temperature, compound **3a** (500 mg, 2.07 mmol), compound **11a** (788 mg, 10.35 mmol), dioxane (20 mL), diphenyl phosphorazidate (684 mg, 2.48 mmol) and triethylamine (314 mg, 3.11 mmol) were sequentially added to a 250 mL reaction flask. After purging with nitrogen 3 times, the mixture was stirred and reacted at 90°C for 16 hours. After the reaction was completed, the reaction solution was concentrated in vacuo, and the resulting residue was purified by silica gel column chromatography to afford intermediate **11b.** LC-MS: m/z: 313.9 (M+H)⁺. ¹H NMR (400 MHz, DMSO-*d*₆) δ 11.16 (s, 1H), 6.39 (s, 1H), 4.56 (t, *J =* 5.1 Hz, 1H), 4.19 (t, *J =* 6.5 Hz, 2H), 3.53-3.46 (m, 2H), 1.81-1.73 (m, 2H).

**Step 2:** At room temperature, intermediate **11b** (100 mg, 0.32 mmol), compound **1e** (104.59 mg, 0.38 mmol),[1,1'-bis(diphenylphosphino)ferrocene]palladium dichloride-dichloromethane complex (25.96 mg, 0.03 mmol), potassium carbonate (131.79 mg, 0.95 mmol), dioxane (3 mL) and water (0.60 mL) were added to a 10 mL microwave tube. The mixture was subjected to a microwave reaction at 100°C under nitrogen atmosphere for two hours. After the reaction was completed, the reaction solution was filtered, and the crude filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **11** (61.60 mg, 0.16 mmol). LC-MS: m/z: 382.1 (M+H)⁺. ¹H NMR (400 MHz, DMSO-*d*₆) δ 10.96 (s, 1H), 7.48 (d, *J =* 8.2 Hz, 2H), 7.36 (d, *J =* 8.1 Hz, 2H), 6.48 (s, 1H), 4.70 (t, *J =* 5.6 Hz, 1H), 4.56 (t, *J* = 5.1 Hz, 1H), 4.19 (t, *J =* 6.5 Hz, 2H), 3.55 (d, *J =* 5.7 Hz, 2H), 3.50 (q, *J =* 5.9 Hz, 2H), 1.82-1.73 (m, 2H), 0.85 (q, *J =* 4.2, 3.6 Hz, 2H), 0.76 (q, *J* = 4.6, 4.2 Hz, 2H).

### Example 12: Synthesis of compound 12

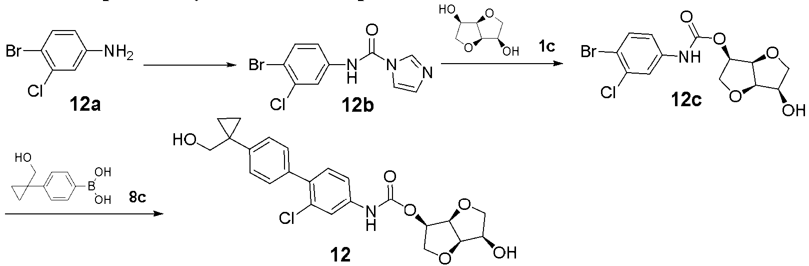

**Step 1:** At room temperature, compound **12a** (200 mg, 0.90 mmol) dissolved in super-dry tetrahydrofuran (3.0 mL) was added to a 25 mL single-necked flask, and then *N,N*'-carbonyldiimidazole (290 mg, 1.80 mmol) was added. The reaction was carried out at 60°C under nitrogen atmosphere for 1 hour until completion. The reaction solution containing intermediate 12b was directly used in the next reaction without purification.

**Step 2:** At room temperature, compound **1c** (200 mg, 1.35 mmol) and *N,N-*diisopropylethylamine (0.60 mL, 3.60 mmol) were added to the aforementioned reaction system containing intermediate 12b. The reaction was carried out at 60°C under nitrogen atmosphere for 1 hour until completion. After the reaction was completed, the reaction solution was concentrated, and the crude product was separated and purified by silica gel column chromatography to afford intermediate **12c.** LC-MS: m/z: 378.0 (M + H)⁺.

**Step 3:** At room temperature, intermediate **12c** (55 mg, 0.15 mmol), compound **8c** (133.47 mg, 0.17 mmol), [1,1'-bis(diphenylphosphino)ferrocene]palladium dichloride (10.63 mg, 0.01 mmol), potassium carbonate (40.15 mg, 0.29 mmol), dioxane (3.0 mL) and water (0.6 mL) were sequentially added to a 25 mL single-necked flask. After purging with nitrogen three times, the reaction solution was reacted at 90°C for 2 hours. After the reaction was completed, the reaction solution was concentrated, and the crude residue was separated and purified by silica gel column chromatography to afford a crude product, which was then separated and purified by high performance liquid chromatography (mobile phase: A: H₂O B: ACN, chromatographic column: Waters-SunFire-C₁₈-10 µm-19*250 mm) to finally afford compound **12.** LC-MS: m/z: 446.2 (M+H)⁺, ¹H NMR (400 MHz, DMSO-*d*₆) δ 10.09 (s, 1H), 7.73 (d, *J* = 2.2 Hz, 1H), 7.45 (dd, *J =* 8.5, 2.2 Hz, 1H), 7.36 (d, *J* = 8.5 Hz, 2H), 7.31 (d, *J* = 8.4 Hz, 3H), 5.07 (q, *J* = 6.2 Hz, 1H), 4.93 (d, *J* = 6.4 Hz, 1H), 4.70 (t, *J =* 5.6 Hz, 1H), 4.64 (t, *J =* 5.0 Hz, 1H), 4.32 (t, *J =* 4.8 Hz, 1H), 4.14 (m, 1H), 3.99 (dd, *J* = 9.2, 6.3 Hz, 1H), 3.81 (dd, *J =* 8.1, 6.8 Hz, 1H), 3.75 (dd, *J =* 9.2, 6.5 Hz, 1H), 3.56 (d, *J* = 5.7 Hz, 2H), 3.42 (t, *J* = 8.5 Hz, 1H), 0.91-0.83 (m, 2H), 0.80-0.71 (m, 2H).

### Example 13: Synthesis of compound 13

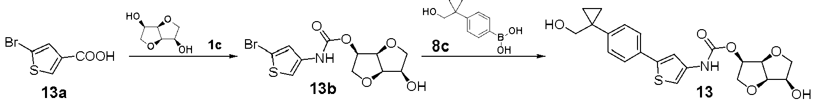

**Step 1:** At room temperature, compound **13a** (200 mg, 0.97 mmol) dissolved in dioxane (8.0 mL) was added to a 50 mL single-necked flask, and then triethylamine (0.54 mL, 3.86 mmol), diphenyl phosphorazidate (531.68 mg, 1.93 mmol) and compound **1c** (564.68 mg, 3.86 mmol) were sequentially added. After purging with nitrogen three times, the reaction solution was reacted at 90°C overnight. After the reaction was completed, the reaction solution was poured into water (70 mL), and the mixture was extracted with ethyl acetate (7 mL × 3). The organic phase was dried over anhydrous sodium sulphate, filtered, and concentrated to afford a crude product, and the crude product was separated and purified by silica gel column chromatography to afford intermediate **13b.** LC-MS: m/z: 352.0 (M+H)⁺.

**Step 2:** At room temperature, intermediate **13b** (60 mg, 0.17 mmol), compound **8c** (39.48 mg, 0.21 mmol), [1,1'-bis(diphenylphosphino)ferrocene]palladium dichloride (12.54 mg, 0.02 mmol), potassium carbonate (47.36 mg, 0.34 mmol), dioxane (3.0 mL) and water (0.6 mL) were sequentially added to a 25 mL single-necked flask. After purging with nitrogen three times, the reaction solution was reacted at 90°C for 2 hours. After the reaction was completed, the reaction solution was concentrated to afford a crude product, and the crude product was separated and purified by silica gel column chromatography to afford compound **13.** LC-MS: m/z: 418.2 (M+H)⁺. ¹H NMR (400 MHz, DMSO-*d*₆) δ 10.13 (s, 1H), 7.50-7.41 (m, 2H), 7.37-7.30 (m, 2H), 7.28 (d, *J* = 1.5 Hz, 1H), 7.14 (s, 1H), 5.05 (q, *J* = 6.2 Hz, 1H), 4.92 (d, *J =* 6.4 Hz, 1H), 4.69 (t, *J =* 5.7 Hz, 1H), 4.63 (t, *J* = 5.1 Hz, 1H), 4.31 (t, *J* = 4.8 Hz, 1H), 4.13 (m, 1H), 3.99 (dd, *J =* 9.2, 6.4 Hz, 1H), 3.80 (dd, *J* = 8.1, 6.8 Hz, 1H), 3.72 (dd, *J* = 9.2, 6.6 Hz, 1H), 3.54 (d, *J =* 5.6 Hz, 2H), 3.41 (t, *J* = 8.5 Hz, 1H), 0.89-0.82 (m, 2H), 0.78-0.70 (m, 2H).

### Example 14: Synthesis of compound 14

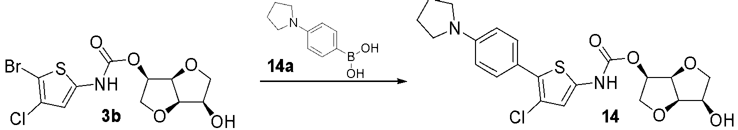

Under nitrogen atmosphere, intermediate **3b** (100 mg, 0.26 mmol), compound **14a** (54.64 mg, 0.29 mmol), [1,1'-bis(diphenylphosphino)ferrocene]palladium dichloride-dichloromethane complex (19.02 mg, 0.03 mmol), potassium carbonate (89.83 mg, 0.65 mmol), dioxane (2 mL) and water (0.4 mL) were added to a 8 mL microwave tube. The reaction was carried out at 100°C under microwave conditions for two hours until completion. Subsequently, the reaction system was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **14.** LC-MS: m/z: 451.2 (M+H)⁺. ¹H NMR (400 MHz, DMSO-*d6*) δ 11.02 (s, 1H), 7.37 (d, *J* = 8.4 Hz, 2H), 6.58 (d, *J* = 8.8 Hz, 2H), 6.44 (s, 1H), 5.08-5.04 (m, 1H), 4.93 (d, *J* = 6.4 Hz, 1H), 4.63 (t, *J* = 4.8 Hz, 1H), 4.30 (t, *J* = 4.8 Hz, 1H), 4.15-4.08 (m, 1H), 3.98-3.94 (m, 1H), 3.81-3.72 (m, 2H), 3.39 (t, *J* = 8.4 Hz, 1H), 3.26-3.23 (m, 4H), 1.97-1.93 (m, 4H).

### Example 15: Synthesis of compound 15

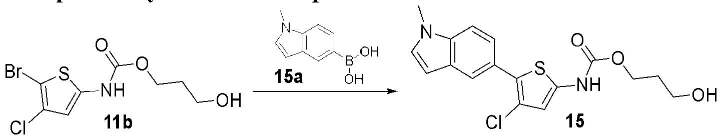

At room temperature, intermediate **11b** (100 mg, 0.32 mmol), compound **15a** (66.75 mg, 0.38 mmol),[1,1'-bis(diphenylphosphino)ferrocene]palladium dichloride-dichloromethane complex (25.96 mg, 0.03 mmol), potassium carbonate (131.79 mg, 0.95 mmol), dioxane (3 mL) and water (0.60 mL) were added to a 10 mL microwave tube. The mixture was subjected to a microwave reaction at 100°C under nitrogen atmosphere for two hours. After the reaction was completed, the reaction solution was filtered, and the crude filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound 15. LC-MS: m/z: 364.9 (M+H)⁺. ¹H NMR (400 MHz, DMSO-*d*₆) δ 8.01 (s, 1H), 7.74 (s, 1H), 7.50 (d, *J* = 8.6 Hz, 1H), 7.40 -7.28 (m, 2H), 6.48 (s, 2H), 4.57 (s, 1H), 4.19 (d, *J* = 6.8 Hz, 2H), 3.81 (s, 3H), 3.51 (s, 2H), 1.83-1.69 (m, 2H).

### Example 16: Synthesis of compound 16

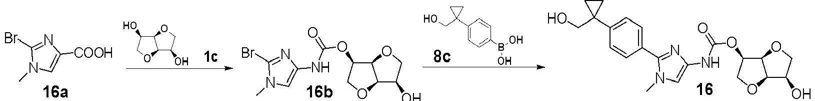

**Step 1:** At room temperature, compound **16a** (200 mg, 0.98 mmol) dissolved in dioxane (10.0 mL) was added to a 50 mL single-necked flask, and then compound **1c** (570.27 mg, 3.90 mmol), diphenyl phosphorazidate (1073.90 mg,3.90 mmol) and triethylamine (0.54 mL, 3.90 mmol) were added. The flask was evacuated and backfilled with nitrogen, and the reaction was carried out at 90°C under nitrogen atmosphere overnight until completion. After the reaction was completed, the reaction solution was poured into water (20 mL). The mixture was extracted with ethyl acetate (5 mL × 3), dried over anhydrous sodium sulphate, filtered, and concentrated to afford a crude product, and the crude product was separated and purified by silica gel column chromatography to afford intermediate **16b.** LC-MS: m/z: 350.1 (M+H)⁺.

**Step 2:** At room temperature, intermediate **16b** (130 mg, 0.4 mmol), compound **8c** (115.82 mg, 0.6 mmol), [1,1'-bis(diphenylphosphino)ferrocene]palladium dichloride (58.85 mg, 0.08 mmol), potassium carbonate (111.15 mg, 0.80 mmol), dioxane (8.0 mL) and water (1.6 mL) were added to a 50 mL single-necked flask. The flask was evacuated and backfilled with nitrogen, and the reaction was carried out at 90°C under nitrogen atmosphere for 2 hours until completion. After the reaction was completed, the reaction solution was concentrated to afford a crude residue, and the crude residue was separated and purified by silica gel column chromatography to afford a crude product, which was then separated and purified by high performance liquid chromatography (mobile phase: A: 0.05% TFA/H₂O B: ACN, chromatographic column: Waters-SunFire-C₁₈-10 µm-19*250 mm) to finally afford compound **16.** LC-MS: m/z: 416.2 (M+H)⁺. ¹H NMR (400 MHz, DMSO-*d*₆) δ 9.75 (s, 1H), 8.76 (s, 1H), 7.47-7.40 (m, 4H), 4.98 (d, *J* = 5.2 Hz, 1H), 4.63-4.55 (m, 1H), 4.26 (s, 2H), 4.10 (s, 2H), 3.91 (s, 2H), 3.75 (s, 2H), 3.70 (s, 3H), 3.58 (s, 2H), 0.90 (dd, *J* = 6.4, 4.8 Hz, 2H), 0.80 (dd, *J* = 6.4, 4.8 Hz, 2H).

### Example 17: Synthesis of compound 17

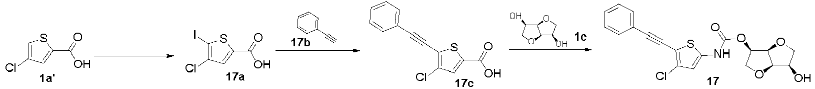

**Step 1:** At room temperature, compound **la'** (3 g, 18.45 mmol), [bis(trifluoroacetoxy)iodo]benzene (4.30 g, 9.96 mmol), iodine (2.34 g, 9.22 mmol) and carbon tetrachloride (50 mL) were added to a 100 mL single-necked flask. The reaction was carried out at room temperature for five hours until completion. The reaction solution was poured into water (50 ml), and the mixture was extracted with ethyl acetate (10 ml x 3). The organic phase was dried over anhydrous sodium sulphate, and filtered to afford the filtrate, which was concentrated under reduced pressure to afford a crude product, and the crude product was slurried with petroleum ether/dichloromethane (V : V=10 : 1) three times to afford intermediate **17a.** LC-MS: m/z: 288.8 (M+H)⁺. ¹H NMR (400 MHz, DMSO-*d6*) δ 7.58 (s, 1H).

**Step 2:** To a 25 mL single-necked flask were added intermediate **17a** (300 mg, 1.04 mmol), compound **17b** (127.46 mg, 1.25 mmol), bis(triphenylphosphine)palladium dichloride (72.99 mg, 0.10 mmol), cuprous iodide (39.61 mg, 0.21 mmol), N,N-diisopropylethylamine (201.62 mg, 1.56 mmol) and tetrahydrofuran (10 mL). The reaction was carried out at room temperature for 16 hours until completion. The reaction solution was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford intermediate **17c.** LC-MS: m/z: 263.0 (M+H)⁺. ¹H NMR (400 MHz, DMSO-*d*₆) δ 7.66 (s, 1H), 7.62-7.59 (m, 2H), 7.50-7.45 (m, 3H).

**Step 3:** To a 25 mL single-necked flask were sequentially added intermediate 17c (200 mg, 0.76 mmol), compound **1c** (556.28 mg, 3.81 mmol), dioxane (15 mL), triethylamine (115.55 mg, 1.14 mmol) and diphenyl phosphorazidate (251.41 mg, 0.91 mmol). The flask was purged with nitrogen, and the reaction was carried out at 90°C for 16 hours until completion. The reaction solution was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **17.** LC-MS: m/z: 406.0 (M+H)⁺. ¹H NMR (400 MHz, DMSO-*d*₆) δ 11.52 (s, 1H), 7.55-7.50 (m, 2H), 7.45-7.40 (m, 3H), 6.49 (s, 1H), 5.11 (q, *J =* 5.8 Hz, 1H), 4.95 (d, *J =* 6.4 Hz, 1H), 4.65 (t, *J* = 5.1 Hz, 1H), 4.29 (t, *J* = 4.9 Hz, 1H), 4.16-4.08 (m, 1H), 3.96 (dd, *J =* 9.5, 6.1 Hz, 1H), 3.83-3.75 (m, 2H), 3.38 (t, *J =* 8.5 Hz, 1H).

### Example 18: Synthesis of compound 18

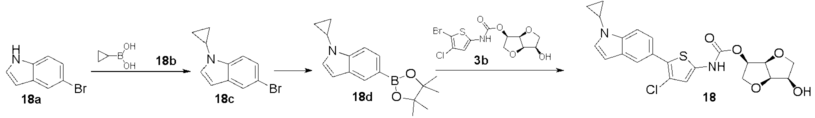

**Step 1:** At room temperature, compound **18a** (1500 mg, 7.65 mmol), compound **18b** (1314 mg, 15.30 mmol), 2,2'-bipyridine (1195 mg, 7.65 mmol), sodium carbonate (1622 mg, 15.30 mmol), anhydrous copper acetate (1390 mg, 7.65 mmol) and 1,2-dichloroethane (20 mL) were added to a 50 mL single-necked flask. The flask was purged with nitrogen three times, and the reaction was carried out at 70°C for 16 hours until completion. The reaction solution was poured into water (100 ml), and the mixture was extracted with ethyl acetate (10 ml × 3). The organic phase was dried over anhydrous sodium sulphate, and concentrated under reduced pressure, and the resulting crude product was separated and purified by silica gel column chromatography to afford intermediate **18c.** LC-MS: m/z: 236.0 (M+H)⁺. ¹H NMR (400 MHz, DMSO-*d*₆) δ 7.73 (d, *J* = 1.9 Hz, 1H), 7.51 (dt, *J* = 8.7, 0.7 Hz, 1H), 7.37 (d, *J* = 3.2 Hz, 1H), 7.28 (dd, *J* = 8.6, 2.0 Hz, 1H), 6.38 (dd, *J* = 3.1, 0.8 Hz, 1H), 3.43 (tt, *J =* 7.2, 3.7 Hz, 1H), 1.09-1.02 (m, 2H), 0.96-0.90 (m, 2H).

**Step 2:** At room temperature, intermediate **18c** (1500 mg, 6.35 mmol), potassium acetate (1870 mg, 19.06 mmol), [1,1'-bis(diphenylphosphino)ferrocene]palladium dichloride-dichloromethane complex (519 mg, 0.64 mmol), bis(pinacolato)diboron (1936 mg, 7.62 mmol) and dioxane (20 mL) were added to a 25 mL single-necked flask. The reaction was carried out at 80°C for 16 hours until completion. The reaction solution was poured into water (100 ml), and the mixture was extracted with ethyl acetate (10 ml × 3). The organic phase was dried over anhydrous sodium sulphate, and concentrated under reduced pressure, and the resulting crude product was separated and purified by silica gel column chromatography to afford intermediate **18d.** LC-MS: m/z: 284.2 (M+H)⁺. ¹H NMR (400 MHz, DMSO-*d*₆) δ 7.94 (s, 1H), 7.56-7.47 (m, 2H), 7.31 (d, *J* = 3.2 Hz, 1H), 6.43 (d, *J* = 3.1 Hz, 1H), 3.46-3.39 (m, 1H), 1.30 (s, 12H), 1.09-1.02 (m, 2H), 0.96 - 0.90 (m, 2H).

**Step 3:** Under nitrogen atmosphere, intermediate **18d** (100 mg, 0.26 mmol), intermediate **3b** (88.35 mg, 0.31 mmol), [1,1'-bis(diphenylphosphino)ferrocene]palladium dichloride-dichloromethane complex (21.23 mg, 0.03 mmol), potassium carbonate (107.79 mg, 0.78 mmol), dioxane (3 mL) and water (0.6 mL) were added to a 8 mL microwave tube. The reaction was carried out at 100°C under microwave conditions for 2 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **18.** LC-MS: m/z: 461.1 (M+H)⁺. ¹H NMR (400 MHz, DMSO-*d*₆) δ 11.10 (s, 1H), 7.73 (s, 1H), 7.62 (d, *J* = 8.5 Hz, 1H), 7.40-7.34 (m, 2H), 6.50 (s, 1H), 6.45 (d, *J =* 3.2 Hz, 1H), 5.08 (q, *J* = 6.0 Hz, 1H), 4.94 (d, *J =* 6.4 Hz, 1H), 4.64 (t, *J =* 5.1 Hz, 1H), 4.30 (t, *J =* 4.9 Hz, 1H), 4.17-4.09 (m, 1H), 3.97 (dd, *J* = 9.4, 6.2 Hz, 1H), 3.85-3.71 (m, 2H), 3.49-3.43 (m, 1H), 3.40 (t, *J =* 8.5 Hz, 1H), 1.10-1.04 (m, 2H), 0.99-0.93 (m, 2H).

### Example 19: Synthesis of compound 19

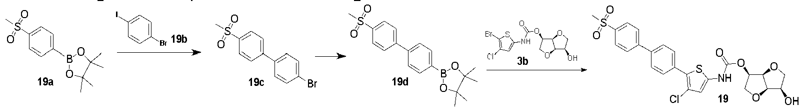

**Step 1:** Under nitrogen atmosphere, compound **19a** (1.1 g, 3.89 mmol), compound **19b** (1 g, 3.53 mmol), palladium acetate (80 mg, 0.35 mmol), potassium phosphate (2.25 g, 10.6 mmol), dioxane (20 mL) and water (4 mL) were added to a 100 mL single-necked flask. The reaction was carried out at 100°C for 15 hours until completion. After the reaction was completed, the reaction system was cooled and filtered, the filtrate was concentrated under reduced pressure to afford a crude product, and the crude product was purified by silica gel column chromatography to afford intermediate **19c.** ¹H NMR (400 MHz, CDCl₃) δ 8.06-7.98 (m, 2H), 7.78-7.70 (m, 2H), 7.67-7.58 (m, 2H), 7.48 (d, *J =* 8.5 Hz, 2H), 3.09 (s, 3H).

**Step 2:** Under nitrogen atmosphere, intermediate **19c** (200 mg, 0.64 mmol), bis(pinacolato)diboron (195.8 mg, 0.77 mmol), [1,1'-bis(diphenylphosphino)ferrocene]palladium dichloride (47.03 mg, 0.06 mmol), potassium acetate (126.15 mg, 1.29 mmol) and dioxane (2 mL) were added to a 15 mL single-necked flask. The mixture was heated to 90°C and reacted for 15 hours until completion. After the reaction was completed, the reaction system was cooled and filtered, the filtrate was concentrated under reduced pressure to afford a crude product, and the crude product was separated and purified by silica gel column chromatography to afford intermediate **19d.** ¹H NMR (400 MHz, CDCl₃) δ 8.04-7.97 (m, 2H), 7.96-7.88 (m, 2H), 7.82-7.77 (m, 2H), 7.66-7.59 (m, 2H), 3.09 (s, 3H), 1.37 (s, 12H).

**Step 3:** Under nitrogen atmosphere, intermediate **19d** (71.72 mg, 0.20 mmol), intermediate **3b** (70 mg, 0.18 mmol), [1,1'-bis(diphenylphosphino)ferrocene]palladium dichloride (13.32 mg, 0.02 mmol), potassium carbonate (62.88 mg, 0.45 mmol), dioxane (2 mL) and water (0.4 mL) were added to a 8 mL microwave tube. The mixture was heated to 100°C and reacted for 2 hours until completion. After the reaction was completed, the reaction solution was cooled and poured into water (10 mL), and the mixture was extracted with ethyl acetate (2 mL x 3). The organic phase was washed with saturated aqueous sodium chloride solution (5 mL), dried over anhydrous sodium sulphate, and then concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **19.** LC-MS: m/z: 536.0 (M+H)⁺. ¹H NMR (400 MHz, DMSO-*d6*) δ 11.29 (s, 1H), 8.03-7.98 (m, 4H), 7.87-7.85 (m, 2H), 7.76-7.74 (m, 2H), 6.55 (s, 1H), 5.10 (q, *J* = 5.9 Hz, 1H), 4.96 (s, 1H), 4.65 (t, *J* = 5.1 Hz, 1H), 4.31 (t, *J =* 4.9 Hz, 1H), 4.13 (m, 1H), 3.98 (dd, *J =* 9.5, 6.2 Hz, 1H), 3.79 (m, 2H), 3.41 (m 1H), 3.27 (s, 3H).

### Example 20: Synthesis of compound 20

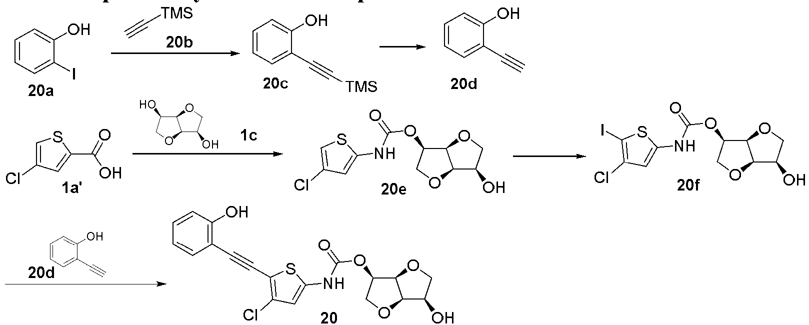

**Step 1:** Under nitrogen atmosphere at room temperature, compound **20a** (2.2 g, 10.00 mmol), compound **20b** (1.71 mL, 12.00 mmol), cuprous iodide (0.04 g, 0.20 mmol), bis(triphenylphosphine)palladium dichloride (0.07 g, 0.10 mmol) and tetrahydrofuran (30 mL) were added to a 100 mL single-necked flask, and triethylamine (12 mL) was added under stirring. The reaction was carried out at room temperature for 16 hours until completion. Subsequently, the reaction solution was filtered, and the filtrate was concentrated under reduced pressure to afford intermediate **20c.** LC-MS: m/z: 191.1 (M+H)⁺.

**Step 2:** At room temperature, intermediate **20c** (2.5 g, 13.14 mmol) and tetrahydrofuran (15 mL) were added to a 100 mL single-necked flask, and tetrabutylammonium fluoride (11 mL, 1.0 M) was added under stirring. The reaction was carried out at room temperature for three hours until completion. Subsequently, the reaction solution was filtered, and the filtrate was concentrated under reduced pressure to afford a crude product, which was separated and purified by silica gel column chromatography to afford intermediate **20d.** ¹H NMR (400 MHz, DMSO-*d6*) δ 9.94 (s, 1H), 7.30 (dd, *J* = 7.6, 1.7 Hz, 1H), 7.22-7.15 (m, 1H), 6.87 (dd, *J* = 8.2, 1.2 Hz, 1H), 6.79-6.74 (m, 1H), 4.12 (s, 1H).

**Step 3:** Under nitrogen atmosphere, compound **la'** (1 g, 6.15 mmol), compound **1c** (4.49 g, 30.75 mmol), diphenyl phosphorazidate (2.03 g, 7.38 mmol), triethylamine (0.93 g, 9.22 mmol) and dioxane (60 mL) were added to a 100 mL single-necked flask. The reaction was carried out at 90°C for sixteen hours until completion. Subsequently, the reaction solution was filtered, and the filtrate was concentrated under reduced pressure to afford a crude product, which was separated and purified by silica gel column chromatography to afford intermediate **20e.** LC-MS: m/z: 306.0 (M+H)⁺. ¹H NMR (400 MHz, DMSO-*d6*) δ 11.07 (s, 1H), 6.94 (d, *J =* 1.9 Hz, 1H), 6.46 (d, *J* = 1.9 Hz, 1H), 5.06 (q, *J =* 6.0 Hz, 1H), 4.93 (d, *J* = 6.5 Hz, 1H), 4.62 (t, *J =* 5.1 Hz, 1H), 4.29 (t, *J* = 4.9 Hz, 1H), 4.15-4.10 (m, 1H), 3.98-3.94 (m, 1H), 3.81-3.72 (m, 2H), 3.38 (t, *J =* 8.5 Hz, 1H).

**Step 4:** At room temperature, intermediate **20e** (1.11 g, 3.63 mmol), silver nitrate (0.74 g, 4.36 mmol), iodine (1.01 g, 3.99 mmol), dimethyl sulphoxide (5 mL) and acetonitrile (20 mL) were added to a 100 mL single-necked flask. The reaction was carried out at room temperature for two hours until completion. The reaction solution was poured into saturated sodium thiosulphate solution (100 ml), and the mixture was extracted with ethyl acetate (10 ml x 3) three times. The organic phase was dried over anhydrous sodium sulphate, and filtered to afford the filtrate, which was concentrated under reduced pressure to afford a crude product, and the crude product was slurried with petroleum ether/dichloromethane (V : V=5 : 1) three times to afford intermediate **20f.** LC-MS: m/z: 432.0 (M+H)⁺.

**Step 5:** Under nitrogen atmosphere at room temperature, intermediate **20f** (100 mg, 0.23 mmol), intermediate **20d** (41.06 mg, 0.35 mmol), cuprous iodide (8.82 mg, 0.05 mmol), bis(triphenylphosphine)palladium dichloride (16.26 mg, 0.02 mmol) and tetrahydrofuran (4 mL) were added to a 25 mL single-necked flask, and triethylamine (1 mL) was added under stirring. The reaction was carried out at room temperature for sixteen hours until completion. Subsequently, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **20.** LC-MS: m/z: 421.8 (M+H)⁺. ¹H NMR (400 MHz, DMSO-*d6*) δ 11.43 (s, 1H), 7.70-7.63 (m, 1H), 7.60 (d, *J =* 8.0 Hz, 1H), 7.36-7.24 (m, 3H), 6.59 (s, 1H), 5.12 (q, *J =* 5.9 Hz, 1H), 4.96 (d, *J* = 6.4 Hz, 1H), 4.66 (t, *J =* 5.2 Hz, 1H), 4.31 (t, *J =* 4.9 Hz, 1H), 4.19-4.08 (m, 1H), 4.00-3.96 (m, 1H), 3.85-3.73 (m, 2H), 3.41 (m, 1H).

### Example 21: Synthesis of compound 21

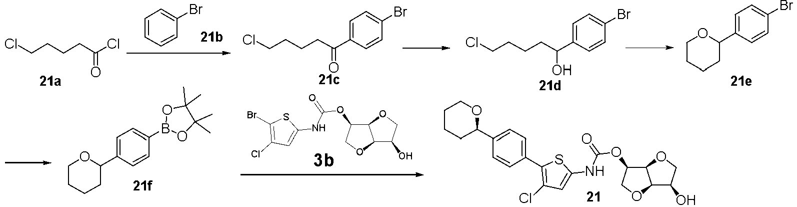

**Step 1:** Under nitrogen atmosphere at 0°C, compound **21a** (3.9 g, 25.16 mmol) and compound **21b** (10 mL) were added to a 100 mL single-necked flask, and aluminium trichloride (3.35 g, 25.13 mmol) was added under stirring. The reaction was carried out at room temperature for 24 hours until completion. The reaction solution was poured into ice water (100 ml), and the mixture was extracted with dichloromethane (20 ml × 3). The organic phase was dried over anhydrous sodium sulphate, filtered, and concentrated to afford intermediate **21c.** LC-MS: m/z: 274.8 (M+H)⁺. ¹H NMR (400 MHz, DMSO-*d6*) δ 7.95-7.87 (m, 2H), 7.75-7.68 (m, 2H), 3.68 (t, *J=* 6.3 Hz, 2H), 3.05 (t, *J=* 6.8 Hz, 2H), 1.87-1.70 (m, 4H).

**Step 2:** At 0°C, intermediate **21c** (3 g, 10.95 mmol) and methanol (30 mL) were added to a 250 mL single-necked flask, and then sodium borohydride (624 mg, 16.42 mmol) was added slowly. The mixture was warmed to room temperature and reacted for two hours until completion. Subsequently, the reaction system was concentrated under reduced pressure to afford a crude product, and the crude product was separated and purified by silica gel column chromatography to afford intermediate **21d.** LC-MS: m/z: 277.1 (M+H)⁺. ¹H NMR (400 MHz, DMSO-*d6*) δ 7.55-7.45 (m, 2H), 7.34-7.25 (m, 2H), 5.28 (d, *J* = 4.5 Hz, 1H), 4.52 (dt, *J =* 7.1, 5.0 Hz, 1H), 3.59 (t, *J =* 6.6 Hz, 2H), 1.78-1.53 (m, 4H), 1.50-1.26 (m, 2H).

**Step 3:** At room temperature, intermediate **21d** (100 mg, 0.36 mmol), tetrahydrofuran (0.3 mL) and tert-butanol (3 mL) were added to a 25 mL single-necked flask, followed by potassium tert-butoxide (121.04 mg, 1.08 mmol). The reaction was carried out at room temperature for four hours until completion. The reaction solution was poured into water (30 ml), and the mixture was extracted with ethyl acetate (5 ml × 3). The organic phase was dried over anhydrous sodium sulphate, filtered, and concentrated to afford intermediate **21e.** LC-MS: m/z: 241.0 (M+H)⁺. ¹H NMR (400 MHz, DMSO-*d6*) δ 7.54-7.46 (m, 2H), 7.34-7.24 (m, 2H), 4.31-4.28 (m, 1H), 4.04-3.99 (m, 1H), 3.57-3.46 (m, 1H), 1.90-1.73 (m, 2H), 1.71-1.58 (m, 1H), 1.59-1.50 (m, 2H), 1.42-1.30 (m, 1H).

**Step 4:** At room temperature, intermediate **21e** (200 mg, 0.83 mmol), bis(pinacolato)diboron (252.92 mg, 1.00 mmol), [1,1'-bis(diphenylphosphino)ferrocene]palladium dichloride-dichloromethane complex (60.73 mg, 0.08 mmol), potassium acetate (244.37 mg, 2.49 mmol) and dioxane (5 mL) were added to a 25 mL single-necked flask. The reaction was carried out at 80°C for sixteen hours until completion. Subsequently, the reaction solution was filtered to afford intermediate **21f** crude product, which was directly used in the next reaction. LC-MS: m/z: 289.2 (M+H)⁺.

**Step 5:** Under nitrogen atmosphere, intermediate **21f** (89.91 mg, 0.31 mmol), intermediate **3b** (100 mg, 0.26 mmol), [1,1'-bis(diphenylphosphino)ferrocene]palladium dichloride-dichloromethane complex (21.23 mg, 0.03 mmol), potassium carbonate (89.83 mg, 0.65 mmol), dioxane (3 mL) and water (0.75 mL) were added to a 8 mL microwave tube. The reaction was carried out at 100°C under microwave conditions for two hours until completion. Subsequently, the reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **21.** LC-MS: m/z: 465.9 (M+H)⁺. ¹H NMR (400 MHz, DMSO-*d6*) δ 11.17 (s, 1H), 7.57-7.50 (m, 2H), 7.39 (d, *J* = 8.3 Hz, 2H), 6.50 (s, 1H), 5.08 (q, *J* = 6.0 Hz, 1H), 4.94 (d, *J* = 6.4 Hz, 1H), 4.64 (t, *J =* 5.1 Hz, 1H), 4.37-4.28 (m, 2H), 4.16-4.09 (m, 1H), 4.05-3.93 (m, 2H), 3.84-3.72 (m, 2H), 3.57-3.49 (m, 1H), 3.39 (t, *J =* 8.5 Hz, 1H), 1.84 (t, *J =* 14.9 Hz, 2H), 1.65 (s, 1H), 1.55 (s, 2H), 1.47-1.37 (m, 1H).

### Example 22: Synthesis of compound 22

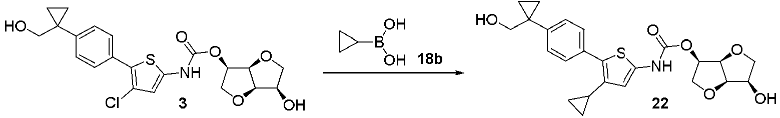

Under nitrogen atmosphere, compound **3** (70 mg, 0.15 mmol), compound **18b** (39.92 mg, 0.46 mmol), palladium acetate (10.43 mg, 0.05 mmol), 2-dicyclohexylphosphino-2',6'-dimethoxybiphenyl (25.44 mg, 0.06 mmol), potassium phosphate (65.76 mg, 0.31 mmol), dioxane (1 mL) and toluene (1 mL) were added to a 10 mL microwave tube. The reaction was carried out at 100°C for 2 hours until completion. Subsequently, the reaction system was concentrated under reduced pressure to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **22.** LC-MS: m/z: 458.2 (M+H)⁺. ¹H NMR (400 MHz, DMSO-*d*₆) δ 10.75 (s, 1H), 7.40 (d, *J* = 8.4 Hz, 2H), 7.34 (d, *J* = 8.4 Hz, 2H), 6.14 (s, 1H), 5.04 (q, *J =* 6.1 Hz, 1H), 4.92 (d, *J* = 6.5 Hz, 1H), 4.68 (t, *J* = 5.6 Hz, 1H), 4.62 (t, *J =* 5.1 Hz, 1H), 4.29 (t, *J* = 4.9 Hz, 1H), 4.17-4.06 (m, 1H), 3.96 (dd, *J* = 9.3, 6.3 Hz, 1H), 3.78 (t, *J =* 6.0 Hz, 1H), 3.73 (dd, *J =* 9.4, 6.2 Hz, 1H), 3.55 (d, *J* = 5.7 Hz, 2H), 3.39 (m, 1H), 1.95-1.89 (m, 1H), 0.91-0.81 (m, 4H), 0.78-0.71 (m, 2H), 0.59-0.49 (m, 2H).

### Example 23: Synthesis of compound 23

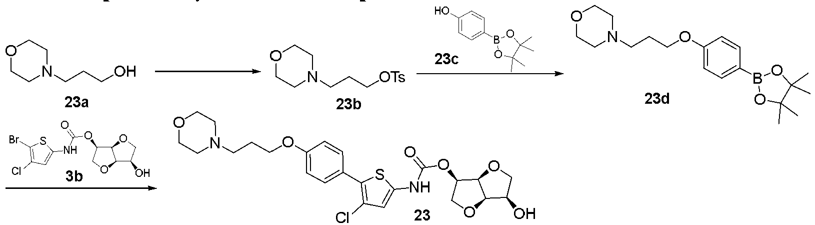

**Step 1:** At 0°C, compound **23a** (1 g, 6.89 mmol), p-toluenesulphonyl chloride (1.445 g, 7.58 mmol) and dichloromethane (25 mL) were added to a 100 mL single-necked flask, and triethylamine (1.39 g, 13.78 mmol) was added under stirring. The reaction was carried out at room temperature for 16 hours until completion. The reaction solution was poured into ice water (200 ml), and the mixture was extracted with dichloromethane (20 ml × 3). The organic phase was dried over anhydrous sodium sulphate, filtered, and concentrated to afford intermediate **23b.** LC-MS: m/z: 300.0 (M+H)⁺. ¹H NMR (400 MHz, DMSO-*d6*) δ 7.51-7.44 (m, 2H), 7.11 (d, *J* = 7.9 Hz, 2H), 4.28-4.23 (m, 2H), 3.77 (d, *J* = 4.7 Hz, 2H), 3.67 (t, *J* = 6.5 Hz, 1H), 3.58 (s, 1H), 3.51 (d, *J* = 5.0 Hz, 2H), 3.09 (q, *J=* 7.8 Hz, 2H), 2.47-2.37 (m, 2H), 2.29 (s, 3H), 1.18 (d, *J=* 7.3 Hz, 2H).

**Step 2:** At room temperature, intermediate **23b** (161.67 mg, 0.54 mmol), compound **23c** (100 mg, 0.45 mmol), potassium carbonate (186.57 mg, 1.35 mmol) and N,N-dimethylformamide (3 mL) were added to a 50 mL single-necked flask. The mixture was warmed to 70°C and reacted for five hours until completion. The reaction solution was poured into water (30 ml), and the mixture was extracted with ethyl acetate (5 ml). The organic phase was washed with saturated aqueous sodium chloride solution (10 mL × 3), dried over anhydrous sodium sulphate, filtered, and concentrated to afford a crude product, and the crude product was separated and purified by silica gel column chromatography to afford intermediate **23d.** LC-MS: m/z: 348.2 (M+H)⁺. ¹H NMR (400 MHz, DMSO-*d6*) δ 7.63-7.55 (m, 2H), 6.95-6.88 (m, 2H), 4.03 (t, *J* = 6.4 Hz, 2H), 3.58-3.55 (m, 4H), 2.41-2.32 (m, 6H), 1.90-1.85 (m, 2H), 1.27 (s, 12H).

**Step 3:** Under nitrogen atmosphere, intermediate **23d** (91 mg, 0.26 mmol), intermediate **3b** (100.79 mg, 0.26 mmol), [1,1'-bis(diphenylphosphino)ferrocene]palladium dichloride-dichloromethane complex (21.40 mg, 0.03 mmol), potassium carbonate (90.54 mg, 0.66 mmol), dioxane (3 mL) and water (0.75 mL) were added to a 8 mL microwave tube. The reaction was carried out at 100°C under microwave conditions for two hours until completion. Subsequently, the reaction solution was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **23.** LC-MS: m/z: 525.2 (M+H)⁺. ¹H NMR (400 MHz, DMSO-*d6*) δ 11.13 (s, 1H), 7.52-7.44 (m, 2H), 7.05-6.96 (m, 2H), 6.48 (s, 1H), 5.08 (q, *J =* 6.0 Hz, 1H), 4.63 (t, *J =* 5.1 Hz, 1H), 4.30 (t, *J* = 4.9 Hz, 1H), 4.16-4.08 (m, 1H), 4.04 (t, *J* = 6.4 Hz, 2H), 3.99-3.95 (m, 1H), 3.82-3.73 (m, 2H), 3.57 (t, *J* = 4.6 Hz, 4H), 3.40 (d, *J* = 8.5 Hz, 2H), 2.45-2.33 (m, 6H), 1.92-1.85 (m, 2H).

### Example 24: Synthesis of compound 24

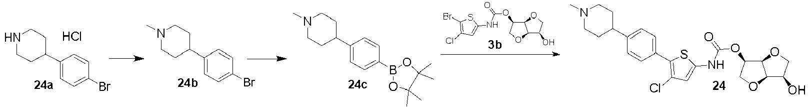

**Step 1:** At 25°C, compound **24a** (1 g, 3.62 mmol), paraformaldehyde (1.47 g, 18.08 mmol), tetrahydrofuran (10 mL) and methanol (10 mL) were added to a 100 mL three-necked flask. Subsequently, sodium triacetoxyborohydride (1.53 g, 7.23 mmol) was added to the reaction system in three portions. The reaction was carried out for 15 hours until completion. The reaction solution was filtered, and the filtrate was concentrated under reduced pressure to afford intermediate **24b.** LC-MS: m/z: 254.0 (M+H)⁺.

**Step 2:** Under nitrogen atmosphere, intermediate **24b** (400 mg, 1.57 mmol), bis(pinacolato)diboron (479.6 mg, 1.89 mmol), [1,1'-bis(diphenylphosphino)ferrocene]palladium dichloride (115.15 mg, 0.16 mmol), potassium acetate (308.9 mg, 3.15 mmol) and dioxane (4 mL) were added to a 15 mL single-necked flask. The mixture was heated to 90°C and reacted for 15 hours until completion. After the reaction was completed, the reaction system was cooled and filtered, the filtrate was concentrated under reduced pressure to afford a crude product, and the crude product was separated and purified by silica gel column chromatography to afford intermediate **24c.** ¹H NMR (400 MHz, CDCl₃) δ 7.75 (d, *J* = 8.1 Hz, 2H), 7.24 (d, *J* = 8.0 Hz, 2H), 3.04 (d, *J =* 11.2 Hz, 2H), 2.51 (td, *J =* 10.9, 5.0 Hz, 1H), 2.37 (s, 3H), 2.13 (td, *J =* 11.2, 3.8 Hz, 2H), 1.93 - 1.76 (m, 4H), 1.33 (s, 12H).

**Step 3:** Under nitrogen atmosphere, intermediate **24c** (43.08 mg, 0.14 mmol), intermediate **3b** (50 mg, 0.13 mmol), [1,1'-bis(diphenylphosphino)ferrocene]palladium dichloride (9.51 mg, 0.01 mmol), potassium carbonate (44.91 mg, 0.32 mmol), dioxane (2 mL) and water (0.4 mL) were added to a 8 mL microwave tube. The mixture was heated to 100°C and reacted for 2 hours until completion. After the reaction was completed, the reaction solution was cooled and poured into water (20 mL), and the mixture was extracted with ethyl acetate (3 mL x 3). The organic phase was washed with saturated aqueous sodium chloride solution (5 mL), dried over anhydrous sodium sulphate, and then concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford compound **24.** LC-MS: m/z: 479.2 (M+H)⁺. ¹H NMR (400 MHz, DMSO-*d6*) δ 11.18 (s, 1H), 8.23 (s, 1H), 7.54-7.45 (m, 2H), 7.32 (d, *J* = 8.4 Hz, 2H), 6.49 (s, 1H), 5.07 (m, 1H), 4.92 (s, 1H), 4.63 (t, *J* = 5.1 Hz, 1H), 4.30 (t, *J =* 4.9 Hz, 1H), 4.15-4.09 (m, 1H), 3.96 (dd, *J =* 9.5, 6.2 Hz, 1H), 3.82-3.74 (m, 2H), 3.41-3.97 (m, 4H), 2.89 (d, *J* = 10.9 Hz, 2H), 2.22 (s, 3H), 2.05-1.95 (m, 2H), 1.80-1.62 (m, 4H).

### Example 25: Synthesis of compound 25

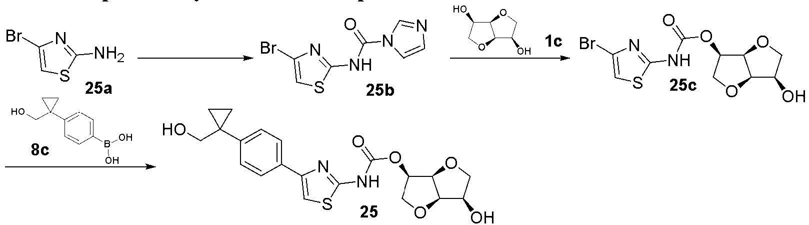

**Step 1:** At room temperature, compound 25a (1000 mg, 5.59 mmol) dissolved in super-dry tetrahydrofuran (15.0 mL) was added to a 25 mL single-necked flask, and then *N,N'*-carbonyldiimidazole (1811.43 mg, 11.17 mmol) was added. The reaction was carried out at 80°C under nitrogen atmosphere for 1 hour until completion. After the reaction was completed, the reaction solution containing intermediate 25b was directly used in the next reaction without treatment.

**Step 2:** At room temperature, compound **1c** (1225.38 mg, 8.38 mmol) and *N,N-*diisopropylethylamine (3.71 mL, 22.36 mmol) were added to the aforementioned reaction solution containing intermediate 25b. The reaction was carried out at 80°C under nitrogen atmosphere for 1 hour until completion. After the reaction was completed, the reaction solution was concentrated to afford a crude product, and the crude product was separated and purified by silica gel column chromatography to afford intermediate **25c.** LC-MS: m/z: 351.0 (M+H)⁺. ¹H NMR (400 MHz, DMSO-*d*₆) δ 12.18 (s, 1H), 7.27 (s, 1H), 5.11 (q, *J* = 5.6 Hz, 1H), 4.95 (d, *J* = 6.4 Hz, 1H), 4.70-4.62 (m, 1H), 4.27 (t, *J* = 4.8 Hz, 1H), 4.15-4.04 (m, 1H), 3.92 (dd, *J* = 9.8, 5.9 Hz, 1H), 3.81 (dd, *J* = 9.8*,* 5.2 Hz, 1H), 3.76 (dd, *J* = 7.9, 7.0 Hz, 1H), 3.42-3.36 (m, 1H).

**Step 3:** At room temperature, intermediate **25c** (80 mg, 0.23 mmol), compound **8c** (65.62 mg, 0.34 mmol), [1,1'-bis(diphenylphosphino)ferrocene]palladium dichloride (33.34 mg, 0.05 mmol), potassium carbonate (62.97 mg, 0.46 mmol), dioxane (10.0 mL) and water (2.0 mL) were sequentially added to a 25 mL single-necked flask. After purging with nitrogen three times, the reaction solution was reacted at 90°C for 2 hours. After the reaction was completed, the reaction solution was concentrated to afford a crude product, which was then separated and purified by high performance liquid chromatography (mobile phase: A: H₂O B: ACN, chromatographic column: Waters-SunFire-C₁₈-10 µm-19*250 mm) to finally afford compound **25.** LC-MS: m/z: 419.2 (M+H)⁺. ¹H NMR (400 MHz, DMSO-*d*₆) δ 11.99 (s, 1H), 7.76 (d, *J =* 8.4 Hz, 2H), 7.52 (s, 1H), 7.33 (d, *J =* 8.4 Hz, 2H), 5.11 (q, *J =* 5.6 Hz, 1H), 4.94 (d, *J* = 6.8 Hz, 1H), 4.69-4.66 (m, 2H), 4.28 (t, *J* = 5.2 Hz, 1H), 4.10 (m, 1H), 3.94 (dd, *J* = 9.6, 6.0 Hz, 1H), 3.86-3.70 (m, 2H), 3.55 (d, *J =* 5.7 Hz, 2H), 3.42 (t, *J* = 8.6 Hz, 1H), 0.89-0.82 (m, 2H), 0.79-0.71 (m, 2H).

### Example 26: Synthesis of compound 26

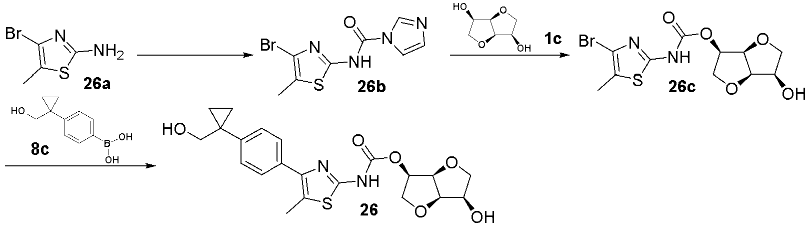

**Step 1:** At room temperature, compound **26a** (1000 mg, 5.18 mmol) dissolved in super-dry tetrahydrofuran (15.0 mL) was added to a 25 mL single-necked flask, and then N,N'-carbonyldiimidazole (1679.79 mg, 10.36 mmol) was added. The reaction was carried out at 80°C under nitrogen atmosphere for 1 hour until completion. After the reaction was completed, the reaction solution containing intermediate 26b was directly used in the next reaction without treatment.

**Step 2:** At room temperature, compound **1c** (1135.51 mg, 7.77 mmol) and *N,N-*diisopropylethylamine (0.86 mL, 5.18 mmol) were added to the aforementioned reaction system containing intermediate 26b. The reaction was carried out at 80°C under nitrogen atmosphere for 1 hour until completion. After the reaction was completed, the reaction solution was concentrated to afford a crude product, and the crude product was separated and purified by silica gel column chromatography to afford intermediate **26c.** LC-MS: m/z: 367.0 (M+H)⁺. ¹H NMR (400 MHz, DMSO-*d*₆) δ 12.00 (s, 1H), 5.10 (q, *J =* 5.6 Hz, 1H), 4.94 (d, *J =* 6.4 Hz, 1H), 4.68-4.61 (m, 1H), 4.27 (t, *J =* 4.8 Hz, 1H), 4.14-4.04 (m, 1H), 3.92 (dd, *J =* 10.0, 6.0 Hz, 1H), 3.82-3.74 (m, 2H), 3.45-3.35 (m, 1H), 2.24 (s, 3H).

**Step 3:** At room temperature, intermediate **26c** (90 mg, 0.25 mmol), compound **8c** (70.98 mg, 0.37 mmol), [1,1'-bis(diphenylphosphino)ferrocene]palladium dichloride (36.06 mg, 0.05 mmol), potassium carbonate (68.12 mg, 0.49 mmol), dioxane (10.0 mL) and water (2.0 mL) were sequentially added to a 25 mL single-necked flask. After purging with nitrogen three times, the reaction solution was reacted at 90°C for 2 hours. After the reaction was completed, the reaction solution was concentrated, and the crude product was separated and purified by high performance liquid chromatography (mobile phase: A: H₂O B: ACN, chromatographic column: Waters-SunFire-C₁₈-10 µm-19*250 mm) to finally afford compound **26.** LC-MS: m/z: 433.2 (M+H)⁺. ¹H NMR (400 MHz, DMSO-*d*₆) δ 11.77 (s, 1H), 7.58-7.45 (m, 2H), 7.40-7.28 (m, 2H), 5.10 (q, *J =* 5.6 Hz, 1H), 4.93 (d, *J* = 6.4 Hz, 1H), 4.70-4.65 (m, 2H), 4.28 (t, *J =* 5.0 Hz, 1H), 4.13-4.06 (m, 1H), 3.93 (dd, *J =* 9.6, 6.0 Hz, 1H), 3.82-3.75 (m, 2H), 3.56 (d, *J =* 5.8 Hz, 2H), 3.41 (dd, *J* = 9.2, 8.0 Hz, 1H), 2.43 (s, 3H), 0.89-0.83 (m, 2H), 0.80-0.71 (m, 2H).

### Example 27: Synthesis of compound 27

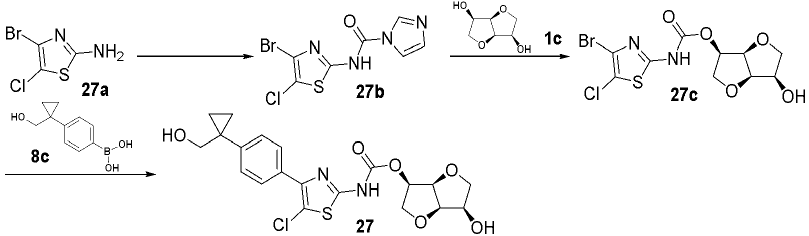

**Step 1:** At room temperature, compound **27a** (870 mg, 4.08 mmol) dissolved in super-dry tetrahydrofuran (12.0 mL) was added to a 25 mL single-necked flask, and then *N,N*'-carbonyldiimidazole (1321.63 mg, 8.15 mmol) was added. The reaction was carried out at 80°C under nitrogen atmosphere for 1 hour until completion. After the reaction was completed, the reaction solution containing intermediate 27b was directly used in the next reaction without treatment.

**Step 2:** At room temperature, compound **1c** (894.38 mg, 6.12 mmol) and *N,N-*diisopropylethylamine (2.70 mL, 16.32 mmol) were added to the aforementioned reaction solution containing intermediate 27b. The reaction was carried out at 80°C under nitrogen atmosphere for 1 hour until completion. After the reaction was completed, the reaction solution was concentrated to afford a crude product, and the crude product was separated and purified by silica gel column chromatography to afford intermediate **27c.** LC-MS: m/z: 385.0 (M+H)⁺.

**Step 3:** At room temperature, intermediate **27c** (73 mg, 0.19 mmol), compound **8c** (54.53 mg, 0.28 mmol), [1,1'-bis(diphenylphosphino)ferrocene]palladium dichloride (27.70 mg, 0.20 mmol), potassium carbonate (52.33 mg, 2.00 mmol), dioxane (8.0 mL) and water (1.6 mL) were sequentially added to a 25 mL single-necked flask. After purging with nitrogen three times, the reaction solution was reacted at 90°C for 2 hours. After the reaction was completed, the reaction solution was concentrated to afford a crude residue, and the crude residue was separated and purified by silica gel column chromatography to afford a crude product, which was then separated and purified by high performance liquid chromatography (mobile phase: A: H₂O B: ACN, chromatographic column: Waters-SunFire-C₁₈-10 µm-19*250 mm) to finally afford compound **27.** LC-MS: m/z: 453.2 (M+H)⁺. ¹H NMR (400 MHz, DMSO-*d6*) δ 12.25 (s, 1H), 7.76 (d, *J =* 8.4 Hz, 2H), 7.39 (d, *J* = 8.4 Hz, 2H), 5.13 (q, *J =* 5.6 Hz, 1H), 4.95 (d, *J =* 6.5 Hz, 1H), 4.71 (t, *J =* 5.6 Hz, 1H), 4.69 - 4.65 (m, 1H), 4.27 (t, *J* = 4.9 Hz, 1H), 4.10 (dt, *J =* 11.8, 6.6 Hz, 1H), 3.92 (dd, *J* = 9.8, 5.8 Hz, 1H), 3.86 - 3.74 (m, 2H), 3.57 (d, *J =* 5.6 Hz, 2H), 3.40 (t, *J =* 8.6 Hz, 1H), 0.90 - 0.84 (m, 2H), 0.81 - 0.75 (m, 2H).

### Example 28: Synthesis of compound 28

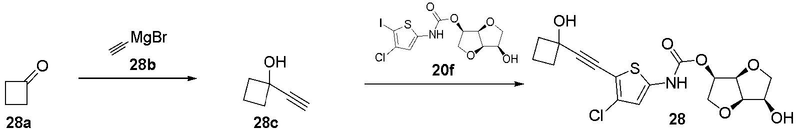

**Step 1:** Under nitrogen atmosphere, tetrahydrofuran (10 mL) and compound **28a** (2.0 g, 28.53 mmol) were added to a 100 mL three-necked flask, and compound **28b** (68 mL 0.5 mol/L) was added at 0°C. Subsequently, the mixture was warmed to 25°C and reacted for 2 hours until completion. The reaction solution was poured into ice water (30 ml), and the mixture was extracted with ethyl acetate (3 × 30 ml) three times. The organic phase was dried over anhydrous sodium sulphate, and filtered, and the filtrate was concentrated under reduced pressure to afford crude intermediate 28c. LC-MS: m/z: 97.1(M+1)⁺.

**Step 2:** Under nitrogen atmosphere, tetrahydrofuran (4 mL), intermediate **28c** (66.81 mg, 0.70 mmol), intermediate **20f** (100 mg, 0.23 mmol), cuprous iodide (8.82 mg, 0.05 mmol) and bis(triphenylphosphine)palladium dichloride (16.26 mg, 0.02 mmol) were added to a 50 mL single-necked flask, and triethylamine (1 mL) was added under stirring. The reaction was carried out at 25°C for 16 h until completion. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **28.** LC-MS: m/z: 400.0(M+1)⁺. ¹H NMR (400 MHz, DMSO-*d*₆) δ 6.41 (s, 1H), 5.93 (s, 1H), 5.08 (q, *J =* 5.9 Hz, 1H), 4.94 (d, *J =* 6.4 Hz, 1H), 4.63 (t, *J =* 5.1 Hz, 1H), 4.29 (t, *J* = 4.9 Hz, 1H), 4.15-4.08 (m, 1H), 3.97-3.93 (m, 1H), 3.81-3.73 (m, 2H), 3.40-3.33 (m, 2H), 2.37-2.30 (m, 2H), 2.23-2.14 (m, 2H), 1.80-1.73 (m, 2H).

### Example 29: Synthesis of hydrochloride of compound 29

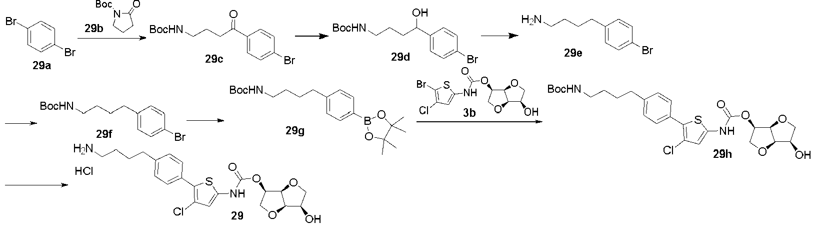

**Step 1:** Under nitrogen atmosphere, tetrahydrofuran (20 mL), compound **29a** (1.09 mL, 8.48 mmol) and n-butyllithium (3.39 mL, 8.48 mmol) were added to a 100 mL three-necked flask. The mixture was reacted at -70°C for 0.5 hours, and then a solution of compound **29b** (1.57 g, 8.48 mmol) in tetrahydrofuran (5 mL) was slowly added to the aforementioned reaction solution. The reaction was carried out at -70°C for 2 hours until completion. Saturated ammonium chloride solution (20 mL) was slowly added to the aforementioned solution, and the reaction solution was extracted with ethyl acetate. The organic phase was dried over anhydrous sodium sulphate, and filtered, the filtrate was concentrated under reduced pressure to afford a crude product, and the crude product was separated and purified by silica gel column chromatography to afford intermediate **29c.** LC-MS: m/z: 364.0(M+Na)⁺. ¹H NMR (400 MHz, DMSO-*d*₆) δ 7.81 (d, *J =* 4.0 Hz, 2H), 7.61 (d, *J =* 4.0 Hz, 2H), 4.62 (brs, 1H), 3.22 (t, *J =* 4.0 Hz, 2H), 2.98 (d, *J =* 8.0 Hz, 2H), 1.94-1.91 (m, 2H), 1.42 (s, 9H).

**Step 2:** At 0°C, methanol (20 mL), intermediate **29c** (1 g, 2.92 mmol) and sodium borohydride (0.55 g, 14.61 mmol) were added to a 100 mL three-necked flask. The mixture was reacted at 25°C for 15 hours, and then the aforementioned reaction solution was quenched with saturated ammonium chloride (100 mL). Water was added, and the mixture was extracted with methyl tert-butyl ether (100 mL × 3). The organic phase was dried over anhydrous sodium sulphate, and filtered, and the filtrate was concentrated under reduced pressure to afford intermediate **29d.** LC-MS: m/z: 366.0(M+Na)⁺.

**Step 3:** Under nitrogen atmosphere, intermediate **29d** (0.50 g, 1.45 mmol), dichloromethane (10 mL) and triethylsilane (1.65 g, 14.61 mmol) were added to a 100 mL three-necked flask. At 0°C, boron trifluoride diethyl etherate (0.50 g, 11.62 mmol) was added to the aforementioned solution. The mixture was reacted at 25°C for 15 hours, and then the aforementioned reaction solution was washed with saturated sodium bicarbonate solution (20 mL). The organic phase was concentrated under reduced pressure to afford a residue, the residue was dissolved in ethyl acetate (30 mL), and the mixture was washed with water twice. The organic phase was dried over anhydrous sodium sulphate, and filtered, and the filtrate was concentrated under reduced pressure to afford intermediate 29e. LC-MS: m/z: 228.0(M+H)⁺.

**Step 4:** To a 100 mL three-necked flask were added intermediate **29e** (0.33 g, 1.45 mmol), tetrahydrofuran (10 mL) and di-tert-butyl dicarbonate (1.58 g, 7.23 mmol). The mixture was reacted at 25°C for 5 hours, and then the aforementioned reaction solution was added to water (50 mL). The mixture was extracted with ethyl acetate (10 mL × 3), and the organic phase was dried over anhydrous sodium sulphate, and filtered. The filtrate was concentrated under reduced pressure to afford a crude product, and the crude product was purified by column chromatography to afford intermediate **29f.** LC-MS: m/z: 272.0(M-55)⁺.

**Step 5:** Under nitrogen atmosphere, intermediate **29f** (1.4 g, 1.25 mmol), potassium acetate (0.31 g, 3.12 mmol), dioxane (14 mL), [1,1'-bis(diphenylphosphino)ferrocene]palladium dichloride (0.09 g, 0.12 mmol) and bis(pinacolato)diboron (0.49 g, 1.87 mmol) were added to a 100 mL three-necked flask. The mixture was reacted at 90°C for 15 hours, and then the aforementioned reaction solution was added to water (100 mL). The mixture was extracted with ethyl acetate (30 mL x 3), and the organic phase was dried over anhydrous sodium sulphate, and filtered. The filtrate was concentrated under reduced pressure to afford a crude product, and the crude product was purified by column chromatography to afford intermediate **29g.** LC-MS: m/z: 398.2 (M+23)⁺.

**Step 6:** Under nitrogen atmosphere, intermediate **29g** (120 mg, 0.31 mmol), intermediate **3b** (117.10 mg, 0.31 mmol), potassium carbonate (107.79 mg, 0.78 mmol), dioxane (2 mL), water (0.4 mL) and [1,1'-bis(diphenylphosphino)ferrocene]palladium dichloride (22.83 mg, 0.03 mmol) were added to a 100 mL three-necked flask. The mixture was reacted at 100°C for 2 hours, and then the aforementioned reaction solution was added to water (100 mL). The mixture was extracted with ethyl acetate (30 mL x 3), and the organic phase was dried over anhydrous sodium sulphate, and filtered. The filtrate was concentrated under reduced pressure to afford a crude product, and the crude product was purified by column chromatography to afford intermediate **29h.** LC-MS: m/z: 575.2 (M+23)⁺.

**Step 7:** To a 100 mL three-necked flask were added intermediate **29h** (100 mg, 0.18 mmol), dichloromethane (2.5 mL) and trifluoroacetic acid (0.5 mL, 6.71 mmol). The mixture was reacted at 25°C for 1 hour, and then the aforementioned reaction solution was concentrated to afford a crude product, and the crude product was separated and purified by high performance liquid chromatography (mobile phase: A: 0.05% HCl/H₂O; B: ACN, column: Waters-SunFire-C₁₈-10 µm-19*250 mm) to afford hydrochloride of compound **29.** LC-MS: m/z: 453.2 (M+H)⁺. ¹H NMR (400 MHz, DMSO-*d*₆) δ 11.20 (s, 1H), 7.76 (s, 3H), 7.51 (d, *J =* 8.0 Hz, 2H), 7.29 (d, *J =* 8.0 Hz, 2H), 6.50 (s, 1H), 5.10-5.05 (m, 1H), 4.97 (d, *J =* 4.0 Hz, 1H), 4.64-4.62 (m, 1H), 4.31-4.30 (m, 1H), 4.14-4.12 (m, 1H), 4.11-3.94 (m, 1H), 3.80-3.74 (m, 2H), 3.41-3.39 (m, 1H), 2.78 (t, *J =* 4.0 Hz, 2H), 2.62 (t, *J =* 4.0 Hz, 2H),1.66-1.53 (m, 4H).

### Example 30: Synthesis of compound 30

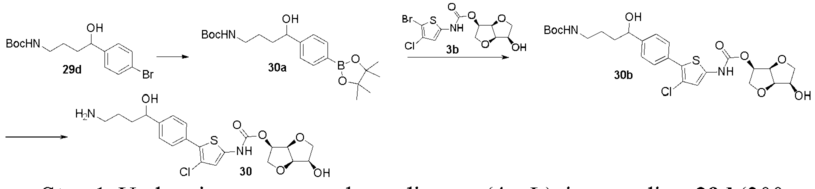

**Step 1:** Under nitrogen atmosphere, dioxane (4 mL), intermediate **29d** (300 mg, 0.87 mmol), potassium acetate (256 mg, 2.61 mmol), [1,1'-bis(diphenylphosphino)ferrocene]palladium dichloride (63 mg, 0.09 mmol) and bis(pinacolato)diboron (442 mg, 1.74 mmol) were added to a 100 mL three-necked flask. The mixture was reacted at 100°C for 15 hours, and then the aforementioned reaction solution was added to water (100 mL). The resulting mixture was extracted with ethyl acetate (30 mL × 3). The organic phase was dried over anhydrous sodium sulphate, and filtered, and the filtrate was concentrated under reduced pressure to afford crude intermediate **30a.** LC-MS: m/z: 414.2(M+Na)⁺.

**Step 2:** Under nitrogen atmosphere, dioxane (2 mL), intermediate **30a** (100 mg, 0.26 mmol), potassium carbonate (89.83 mg, 0.65 mmol), water (0.4 mL), [1,1'-bis(diphenylphosphino)ferrocene]palladium dichloride (19.02 mg, 0.03 mmol) and intermediate **3b** (132.26 mg, 0.34 mmol) were added to a 100 mL three-necked flask. The mixture was reacted at 100°C for 2 hours, and then the aforementioned reaction solution was added to water (100 mL). The resulting mixture was extracted with ethyl acetate (30 mL × 3). The organic phase was dried over anhydrous sodium sulphate, and filtered, the filtrate was concentrated under reduced pressure to afford a crude product, and the crude product was purified by column chromatography to afford intermediate **30b.** LC-MS: m/z: 591.2(M+Na)⁺.

**Step 3:** To a 100 mL three-necked flask were added intermediate **30b** (45 mg, 0.08 mmol), dichloromethane (2.5 mL) and trifluoroacetic acid (0.5 mL, 6.71 mmol). The mixture was reacted at 25°C for 2 hours, and then the aforementioned reaction solution was concentrated to afford a crude product, and the crude product was separated and purified by high performance liquid chromatography (mobile phase: A: 10 mM NH₄HCO₃/H₂O B: ACN, column: Waters-Xbridge-C₁₈-10 µm-19*250 mm) to afford compound **30.** LC-MS: m/z: 451.2 (M-OH)⁺. ¹H NMR (400 MHz, DMSO-*d*₆) δ 7.53-7.49 (m, 2H), 7.44-7.24 (m, 2H), 6.49 (s, 1H), 5.10-5.06 (m, 1H), 4.94 (d, *J =* 8.0 Hz, 1H), 4.63 (t, *J =* 8.0 Hz, 1H), 4.30 (d, *J =* 8.0 Hz, 1H), 4.14-4.10 (m, 2H), 4.07-3.95 (m, 1H), 3.82-3.74 (m, 2H), 3.39-3.32 (m, 1H), 3.04-2.89 (m, 2H), 2.13-2.12 (m, 1H), 1.81-1.73 (m, 2H), 1.52-1.49 (m, 1H).

### Example 31: Synthesis of hydrochloride of compound 31

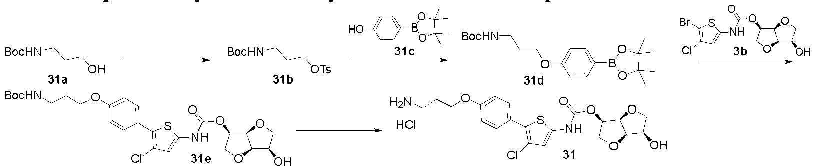

**Step 1:** Under nitrogen atmosphere, dichloromethane (15 mL), compound **31a** (1 g, 5.71 mmol), 4-dimethylaminopyridine (0.35 g, 2.85 mmol), p-toluenesulphonyl chloride (2.18 g, 11.41 mmol) and triethylamine (1.73 g, 17.13 mmol) were added to a 100 mL three-necked flask. The mixture was reacted at 25°C for 15 hours, and then the aforementioned solution was added to water (100 mL). The resulting mixture was extracted with ethyl acetate. The organic phase was dried over anhydrous sodium sulphate, and filtered, the filtrate was concentrated under reduced pressure to afford a crude product, and the crude product was purified by column chromatography to afford intermediate **31b.** LC-MS: m/z: 352.2(M+Na)⁺.

**Step 2:** To a 100 mL three-necked flask were added N,N- dimethylformamide (4 mL), intermediate **31b** (200 mg, 0.61 mmol) and compound **31c** (160.34 mg, 0.73 mmol). The mixture was reacted at 70°C for 15 hours, and then the aforementioned reaction solution was added to water (100 mL). The resulting mixture was extracted with ethyl acetate (30 mL × 3). The organic phase was dried over anhydrous sodium sulphate, and filtered, the filtrate was concentrated under reduced pressure to afford a crude product, and the crude product was separated and purified by column chromatography to afford intermediate **31d.** LC-MS: m/z: 400.2 (M+Na)⁺. ¹H NMR (400 MHz, CDCl₃) δ 7.76-7.72 (m, 2H), 6.90-6.86 (m, 2H), 4.74 (s, 1H), 4.04 (d, *J* = 8.0 Hz, 2H), 3.33-3.30 (m, 2H), 1.99-1.95 (m, 2H), 1.44 (s, 9H),1.33 (s, 12H).

**Step 3:** Under nitrogen atmosphere, intermediate **31d** (100 mg, 0.26 mmol), potassium carbonate (89.83 mg, 0.65 mmol), dioxane (2 mL), water (0.4 mL), [1,1'-bis(diphenylphosphino)ferrocene]palladium dichloride (19.02 g, 0.03 mmol) and intermediate **3b** (98.09 mg, 0.26 mmol) were added to a 100 mL three-necked flask. The mixture was reacted at 100°C for 2 hours, and then the aforementioned reaction solution was added to water (100 mL). The resulting mixture was extracted with ethyl acetate (30 mL × 3). The organic phase was dried over anhydrous sodium sulphate, and filtered, the filtrate was concentrated under reduced pressure to afford a crude product, and the crude product was purified by silica gel column chromatography to afford intermediate **31e.** LC-MS: m/z: 577.2 (M+Na)⁺.

**Step 4:** To a 100 mL three-necked flask were added dichloromethane (2.5 mL), intermediate **31e** (80 mg, 0.14 mmol) and trifluoroacetic acid (0.5 mL, 6.71 mmol). The mixture was reacted at 25°C for 2 hours, and then the aforementioned reaction solution was concentrated under reduced pressure to afford a crude product, and the crude product was separated and purified by high performance liquid chromatography (mobile phase: A: 0.05% HCl/H₂O; B: ACN, column: Waters-SunFire-C18-10 µm-19*250 mm) to afford hydrochloride of compound **31.** LC-MS: m/z: 455.2 (M+H)⁺. ¹H NMR (400 MHz, DMSO-*d*₆) δ 11.10 (s, 1H), 8.00 (s, 3H), 7.52-7.49 (m, 2H), 7.04-7.02 (m, 2H), 6.49 (s, 1H), 5.09-4.97 (m, 2H), 4.63 (d, *J =* 4.0 Hz, 1H), 4.29 (d, *J =* 8.0 Hz, 1H), 4.14-4.09 (m, 3H), 3.98-3.94 (m, 1H), 3.81-3.73 (m, 2H), 3.37 (s, 1H), 2.98-2.93 (m, 2H), 2.07-2.00 (m, 2H).

### Example 32: Synthesis of formate of compound 32

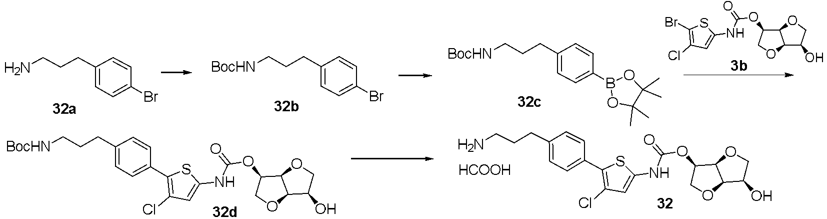

**Step 1:** Under nitrogen atmosphere, tetrahydrofuran (2 mL), compound **32a** (200 mg, 0.93 mmol) and di-tert-butyl dicarbonate (244 mg, 1.12 mmol) were added to a 100 mL three-necked flask. The mixture was reacted at 25°C for 15 hours, and then the aforementioned solution was concentrated under reduced pressure to afford intermediate **32b.** LC-MS: m/z: 336.0(M+Na)⁺. ¹H NMR (400 MHz, CDCl₃) δ 7.41-7.37 (m, 2H), 7.07-7.03 (m, 2H), 4.51 (s, 1H), 3.13 (t, *J =* 8.0 Hz, 2H), 2.59 (t, *J =* 8.0 Hz, 2H), 1.82-1.74 (m, 2H)1.44 (s, 9H).

**Step 2:** Under nitrogen atmosphere, dioxane (10 mL), intermediate **32b** (270 mg, 0.86 mmol), potassium acetate (168.66 mg, 1.72 mmol), [1,1'-bis(diphenylphosphino)ferrocene]palladium dichloride (63 mg, 0.09 mmol) and bis(pinacolato)diboron (240 mg, 0.95 mmol) were added to a 30 mL three-necked flask. The mixture was reacted at 90°C for 15 hours, and then the aforementioned reaction solution was added to water (100 mL). The resulting mixture was extracted with ethyl acetate (30 mL × 3). The organic phase was dried over anhydrous sodium sulphate, and filtered, the filtrate was concentrated under reduced pressure to afford a crude product, and the crude product was separated and purified by silica gel column chromatography to afford intermediate **32c** as a colourless oil. LC-MS: m/z: 384.2(M+Na)⁺. ¹H NMR (400 MHz, CDCl₃) δ 7.71 (d, *J =* 8.0 Hz, 2H), 7.19 (d, *J =* 8.0 Hz, 2H),4.50 (s, 1H), 3.14 (s, 2H), 2.65 (t, *J =* 8.0 Hz, 2H), 1.84-1.79 (m, 2H), 1.44 (s, 9H), 1.33 (s, 12H).

**Step 3:** Under nitrogen atmosphere, dioxane (6 mL), water (1.2 mL), intermediate **32c** (90 mg, 0.23 mmol), potassium carbonate (0.84 mg, 0.58 mmol), [1,1'-bis(diphenylphosphino)ferrocene]palladium dichloride (17 mg, 0.02 mmol) and intermediate **3b** (84.54 mg, 0.23 mmol) were added to a 100 mL three-necked flask. The mixture was reacted at 100°C for 2 hours, and then the aforementioned reaction solution was filtered, and the filtrate was subjected to rotary evaporation to dryness to afford intermediate **32d.**

**Step 4:** To a 100 mL three-necked flask were added dichloromethane (2.5 mL), intermediate **32d** (126.13 mg, 0.23 mmol) and trifluoroacetic acid (0.5 mL, 6.71 mmol). The mixture was reacted at 25°C for 2 hours, and then the aforementioned reaction solution was concentrated under reduced pressure to afford a crude product, and the crude product was separated and purified by high performance liquid chromatography (mobile phase: A: 0.1% FA/H₂O B: ACN, chromatographic column: Waters-CORTECS-C18-2.7 µm-4.6*30 mm) to afford formate of compound **32.** LC-MS: m/z: 439.2 (M+H)⁺. ¹H NMR (400 MHz, DMSO-*d*₆) δ 8.39 (s, 1H), 7.52 (d, *J* = 8.0 Hz, 2H), 7.29 (d, *J =* 8.0 Hz, 2H), 6.50 (s, 1H), 5.10-5.06 (m, 1H), 4.98 (s, 1H), 4.63 (d, *J =* 8.0 Hz, 1H), 4.30 (d, *J =* 8.0 Hz, 1H), 4.15-4.11 (m, 3H), 3.99-3.95 (m, 1H), 3.82-3.74 (m, 2H), 3.39 (s, 1H), 2.75-2.50 (m, 4H), 1.84-1.79 (m, 2H).

### Example 33: Synthesis of compound 33

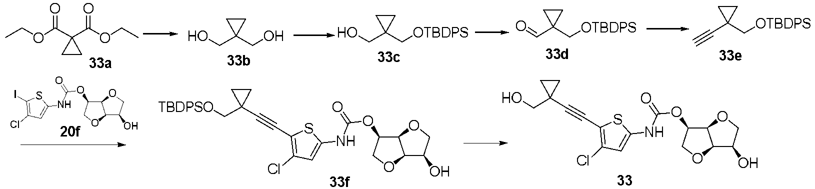

**Step 1:** Under nitrogen atmosphere, tetrahydrofuran (40 mL) and compound 33a (5.0 g, 26.85 mmol) were added to a 250 mL three-necked flask, and lithium aluminium hydride (67.13 mL, 1.0 mol/L) was added at 0°C under stirring. Subsequently, the mixture was warmed to 25°C and reacted for 2 hours until completion. To the reaction solution was added a small amount of Na₂SO₄·10 H₂O, and the mixture was stirred and reacted until no more bubbling was observed. The reaction solution was filtered, dried over anhydrous sodium sulphate, and filtered again. The filtrate was concentrated under reduced pressure to afford intermediate **33b.** LC-MS: m/z: 103.0(M+1)⁺.

**Step 2:** Under nitrogen atmosphere, tetrahydrofuran (80 mL) and intermediate **33b** (2.0 g, 19.58 mmol) were added to a 250 mL three-necked flask, and sodium hydride (0.47 g, 19.58 mmol) was added at 0°C under stirring. The mixture was stirred and reacted for 30 minutes, and then tert-butyldiphenylchlorosilane (5.36 g, 19.58 mmol) was added. Subsequently, the mixture was warmed to room temperature and reacted for 2 hours until completion. The reaction solution was slowly poured into ice water, and the mixture was extracted with ethyl acetate. The organic phase was dried over anhydrous sodium sulphate, and filtered, the filtrate was concentrated under reduced pressure to afford a crude product, and the crude product was separated and purified by silica gel column chromatography to afford intermediate **33c.** LC-MS: m/z: 323.2(M-17)⁺. ¹H NMR (400 MHz, DMSO-*d*₆) δ 7.64-7.58 (m, 4H), 7.48-7.40 (m, 6H), 4.44 (t, *J =* 5.5 Hz, 1H), 3.60 (s, 2H), 3.41 (d, *J =* 5.5 Hz, 2H), 1.00 (s, 9H), 0.41-0.34 (m, 2H), 0.34-0.28 (m, 2H).

**Step 3:** Intermediate **33c** (1.5 g, 4.40 mmol) and dichloromethane (30 mL) were added to a 100 mL single-necked flask, and Dess-Martin periodinane (1.87 g, 4.4 mmol) was added at 0°C under stirring. Subsequently, the mixture was warmed to 25°C and reacted for 2 hours until completion. The reaction solution was filtered, water (50 mL) was added to the filtrate, and the mixture was extracted with dichloromethane (30 mL × 3) three times. The organic phase was dried over anhydrous sodium sulphate, and filtered, the filtrate was concentrated under reduced pressure to afford a crude product, and the crude product was separated and purified by silica gel column chromatography to afford intermediate **33d.** LC-MS: m/z: 361.2(M+23)⁺.

**Step 4:** Intermediate **33d** (1.33 g, 3.93 mmol), carbon tetrabromide (2.61 g, 7.86 mmol) and dichloromethane (10 mL) were added to a 100 mL single-necked flask, and triphenylphosphine (4.12 g, 15.72 mmol) was added at 0°C under stirring. The mixture was stirred and reacted for 2 hours until completion. The reaction solution was poured into water (50 mL), and the mixture was extracted with dichloromethane (30 mL × 3). The organic phase was dried over anhydrous sodium sulphate, and filtered, and the filtrate was concentrated under reduced pressure to afford a crude product. The crude product was separated and purified by silica gel column chromatography to afford an intermediate, and then the intermediate was dissolved in tetrahydrofuran (15 mL). n-Butyllithium (2.36 mL, 2.5 mol/L) was added at -78°C, and the mixture was stirred and reacted for 1 hour until completion. The reaction solution was poured into water (50 mL), and the mixture was extracted with ethyl acetate (30 mL × 3). The organic phase was dried over anhydrous sodium sulphate, and filtered, the filtrate was concentrated under reduced pressure to afford a crude product, and the crude product was separated and purified by silica gel column chromatography to afford intermediate **33e.** LC-MS: m/z: 335.1(M+H)⁺.

**Step 5:** Under nitrogen atmosphere, tetrahydrofuran (6 mL), intermediate **33e** (174.38 mg, 0.52 mmol), intermediate **20f** (150 mg, 0.35 mmol), cuprous iodide (13.24 mg, 0.07 mmol) and bis(triphenylphosphine)palladium dichloride (24.39 mg, 0.03 mmol) were added to a 50 mL single-necked flask, and triethylamine (1.5 mL) was added under stirring. The reaction was carried out at 25°C for 16 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford intermediate **33f.** LC-MS: m/z: 638.2(M+1)⁺.

**Step 6:** Under nitrogen atmosphere, intermediate **33f** (50 mg, 0.08 mmol) and tetrahydrofuran (2 mL) were added to a 25 mL single-necked flask, and tetrabutylammonium fluoride (0.2 mL,1.0 mol/L) was added under stirring. The reaction was carried out at 25°C for 3 hours until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound 33. LC-MS: m/z: 399.9(M+1)⁺. ¹H NMR (400 MHz, DMSO) δ 11.17 (s, 1H), 6.36 (s, 1H), 5.07 (q, *J* = 5.9 Hz, 1H), 4.95 (t, *J =* 6.4 Hz, 2H), 4.62 (t, *J =* 5.1 Hz, 1H), 4.28 (t, *J =* 4.9 Hz, 1H), 4.10 (q, *J =* 7.0 Hz, 1H), 3.96-3.92 (m, 1H), 3.81-3.72 (m, 2H), 3.41 (s, 3H), 0.88 (d, *J* = 3.4 Hz, 4H).

### Example 34: Synthesis of compound 34

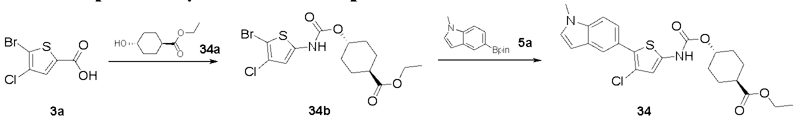

**Step 1:** At room temperature, dioxane (20 mL), compound **3a** (500 mg, 2.07 mmol), compound **34a** (1782 mg, 10.35 mmol), diphenyl phosphorazidate (684 mg, 2.48 mmol) and triethylamine (314 mg, 3.10 mmol) were sequentially added to a 50 mL reaction flask. After purging with nitrogen 3 times, the mixture was stirred and reacted at 90°C for 16 hours. After the reaction was completed, the reaction solution was concentrated under reduced pressure, and the resulting residue was purified by silica gel column chromatography to afford intermediate **34b.** LC-MS: m/z: 410.0(M+H)⁺. ¹H NMR (400 MHz, DMSO-*d*₆) δ 11.16 (s, 1H), 6.38 (s, 1H), 4.68-4.56 (m, 1H), 4.06 (q, *J =* 7.1 Hz, 2H), 2.38-2.29 (m, 1H), 2.04-1.91 (m, 4H), 1.52-1.37 (m, 4H), 1.18 (t, *J =* 7.1 Hz, 3H).

**Step 2:** Under nitrogen atmosphere, dioxane (8 mL), water (1.6 mL), intermediate **34b** (500 mg, 1.22 mmol), compound **5a** (376 mg, 1.46 mmol), [1,1'-bis(diphenylphosphino)ferrocene]palladium dichloride (89 mg, 0.12 mmol) and potassium carbonate (505 mg, 3.65 mmol) were added to a 20 mL microwave tube. The reaction was carried out at 100°C under microwave conditions for 2 hours until completion. Subsequently, the solid was filtered, the reaction system was concentrated under reduced pressure, and the resulting crude product was separated and purified by column chromatography (petroleum ether/ethyl acetate) to afford compound **34.** LC-MS: m/z: 460.9(M+H)⁺. ¹H NMR (400 MHz, DMSO-*d*₆) δ 10.85 (s, 1H), 7.73 (d, *J =* 2.1 Hz, 1H), 7.50 (d, *J =* 8.6 Hz, 1H), 7.38 (d, *J =* 3.1 Hz, 1H), 7.33 (dd, *J =* 8.6, 1.8 Hz, 1H), 6.48 (d, *J =* 2.3 Hz, 1H), 6.47 (s, 1H), 4.67-4.56 (m, 1H), 4.06 (q, *J =* 7.1 Hz, 2H), 3.81 (s, 3H), 2.39-2.30 (m, 1H), 2.06-1.91 (m, 4H), 1.54-1.38 (m, 4H), 1.18 (t, *J =* 7.1 Hz, 3H).

### Example 35: Synthesis of compound 35

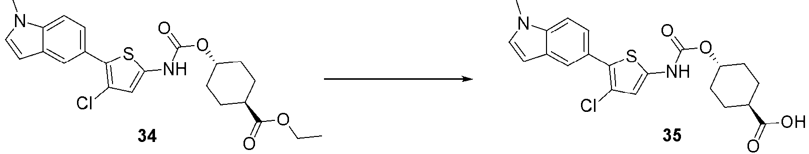

Under nitrogen atmosphere, compound 34 (100 mg, 0.22 mmol), methanol (2 mL) and 20% aqueous potassium hydroxide solution (2 mL) were added to a 10 mL reaction flask. The reaction was carried out at 50°C for 16 hours until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound 35. LC-MS: m/z: 433.2(M+H)⁺. ¹H NMR (400 MHz, DMSO-*d*₆) δ 12.18 (s, 1H), 10.88 (s, 1H), 7.73 (d, *J* = 1.8 Hz, 1H), 7.50 (d, *J=* 8.6 Hz, 1H), 7.38 (d, *J* = 3.1 Hz, 1H), 7.33 (dd, *J =* 8.5, 1.8 Hz, 1H), 6.48 (d, *J =* 6.5 Hz, 2H), 4.66-4.55 (m, 1H), 3.81 (s, 3H), 2.28-2.19 (m, 1H), 2.06-1.91 (m, 4H), 1.51-1.36 (m, 4H).

### Example 36: Synthesis of compound 36

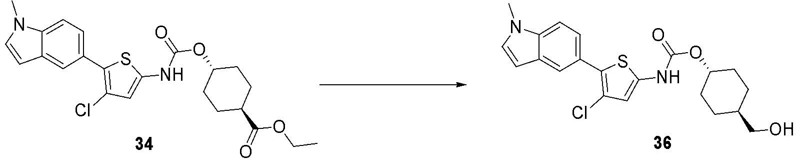

Under nitrogen atmosphere, compound **34** (130 mg, 0.28 mmol) and tetrahydrofuran (3 mL) were added to a 10 mL reaction flask. At 0°C, a solution of lithium aluminium hydride in tetrahydrofuran (1 N, 0.4 mL) was added dropwise to the reaction flask, and then the reaction was carried out at 25°C for 16 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **36.** LC-MS: m/z: 418.9(M+H)⁺. ¹H NMR (400 MHz, DMSO-*d*₆) δ 10.86 (s, 1H), 7.73 (d, *J =* 1.7 Hz, 1H), 7.50 (d, *J =* 8.6 Hz, 1H), 7.38 (d, *J =* 3.1 Hz, 1H), 7.33 (dd, *J =* 8.5, 1.7 Hz, 1H), 6.48 (dd, *J =* 3.1, 0.8 Hz, 1H), 6.46 (s, 1H), 4.60-4.52 (m, 1H), 4.44 (t, *J =* 5.3 Hz, 1H), 3.81 (s, 3H), 3.23 (t, *J =* 5.7 Hz, 2H), 2.05-1.99 (m, 2H), 1.82-1.76 (m, 2H), 1.39-1.30 (m, 3H), 1.06-0.97 (m, 2H).

### Example 37: Synthesis of compound 37

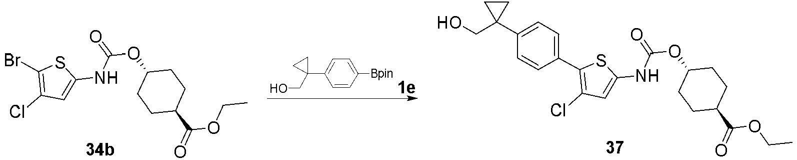

Under nitrogen atmosphere, dioxane (12 mL), water (2.4 mL), intermediate **34b** (600 mg, 1.46 mmol), compound **1e** (481 mg, 1.75 mmol), [1,1'-bis(diphenylphosphino)ferrocene]palladium dichloride (107 mg, 0.15 mmol) and potassium carbonate (606 mg, 4.38 mmol) were added to a 20 mL microwave tube. The reaction was carried out at 100°C under microwave conditions for 2 hours until completion. Subsequently, the solid was filtered, the reaction solution was concentrated under reduced pressure, and the resulting crude product was separated and purified by column chromatography (petroleum ether/ethyl acetate) to afford compound 37. LC-MS: m/z: 478.1(M+H)⁺. ¹H NMR (400 MHz, DMSO-*d*₆) δ 10.95 (s, 1H), 7.47 (d, *J =* 8.4 Hz, 2H), 7.36 (d, *J =* 8.4 Hz, 2H), 6.46 (s, 1H), 4.70 (t, *J =* 5.7 Hz, 1H), 4.66-4.56 (m, 1H), 4.06 (q, *J =* 7.1 Hz, 2H), 3.55 (d, *J =* 5.6 Hz, 2H), 2.40-2.29 (m, 1H), 2.06-1.88 (m, 4H), 1.54-1.37 (m, 4H), 1.18 (t, *J =* 7.1 Hz, 3H), 0.88-0.81 (m, 2H), 0.81-0.74 (m, 2H).

### Example 38: Synthesis of compound 38

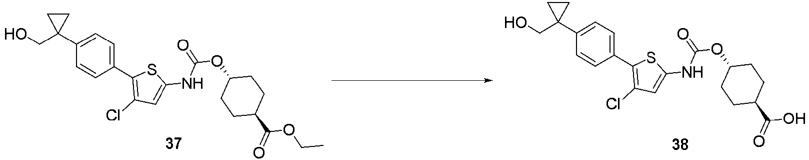

Under nitrogen atmosphere, compound **37** (80 mg, 0.17 mmol), methanol (2 mL) and 20% aqueous potassium hydroxide solution (2 mL) were added to a 10 mL reaction flask. The reaction was carried out at 50°C for 16 hours until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **38.** LC-MS: m/z: 450.2(M+H)⁺. ¹H NMR (400 MHz, DMSO-*d*₆) δ 10.97 (s, 1H), 7.47 (d, *J =* 8.4 Hz, 2H), 7.36 (d, *J =* 8.4 Hz, 2H), 6.46 (s, 1H), 4.69 (s, 1H), 4.63-4.55 (m, 1H), 3.55 (s, 2H), 2.22 (s, 1H), 2.04-1.91 (m, 4H), 1.51-1.36 (m, 4H), 0.87-0.81 (m, 2H), 0.81-0.74 (m, 2H).

### Example 39: Synthesis of compound 39

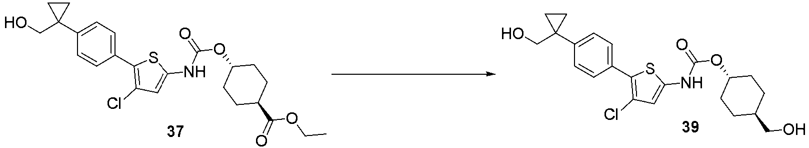

Under nitrogen atmosphere, compound **37** (130 mg, 0.27 mmol) and tetrahydrofuran (4 mL) were added to a 10 mL reaction flask. At 0°C, a solution of lithium aluminium hydride in tetrahydrofuran (1 N, 0.4 mL) was added dropwise to the reaction flask, and then the reaction was carried out at 25°C for 16 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **39.** LC-MS: m/z: 435.9(M+H)⁺. ¹H NMR (400 MHz, DMSO-*d*₆) δ 10.94 (s, 1H), 7.47 (d, *J =* 8.5 Hz, 2H), 7.36 (d, *J =* 8.4 Hz, 2H), 6.46 (s, 1H), 4.70 (t, *J* = 5.6 Hz, 1H), 4.60-4.52 (m, 1H), 4.44 (t, *J =* 5.3 Hz, 1H), 3.55 (d, *J* = 5.7 Hz, 2H), 3.22 (t, *J* = 5.8 Hz, 2H), 2.02 (d, *J =* 11.5 Hz, 2H), 1.79 (d, *J* = 13.2 Hz, 2H), 1.40-1.28 (m, 3H), 1.08-0.96 (m, 2H), 0.89-0.81 (m, 2H), 0.81-0.73 (m, 2H).

### Example 40: Synthesis of compound 40

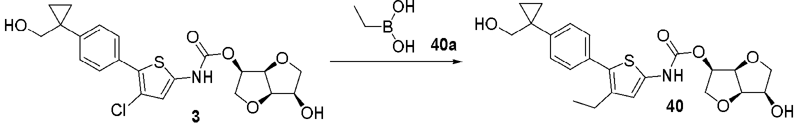

At room temperature, toluene (1.5 mL), compound **3** (100 mg, 0.22 mmol), compound **40a** (164 mg, 2.21 mmol), palladium acetate (5 mg, 0.02 mmol), 2-dicyclohexylphosphino-2',6'-dimethoxybiphenyl (9 mg, 0.02 mmol), potassium phosphate (141 mg, 0.66 mmol) and dioxane (1.5 mL) were added to a 10 mL microwave tube. The reaction was carried out at 100°C under microwave conditions for 2 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **40.** LC-MS: m/z: 446.2.0(M+H)⁺. ¹H NMR (400 MHz, DMSO-*d*₆) δ 10.79 (s, 1H), 7.33 (d, *J =* 8.4 Hz, 2H), 7.26 (d, *J =* 8.3 Hz, 2H), 6.49 (s, 1H), 5.05 (q, *J =* 6.1 Hz, 1H), 4.92 (d, *J =* 6.5 Hz, 1H), 4.68 (t, *J =* 5.7 Hz, 1H), 4.62 (t, *J =* 5.1 Hz, 1H), 4.30 (t, *J =* 4.8 Hz, 1H), 4.17-4.07 (m, 1H), 4.01-3.93 (m, 1H), 3.82-3.71 (m, 2H), 3.54 (d, *J =* 5.7 Hz, 2H), 3.40 (t, *J =* 8.5 Hz, 1H), 2.57-2.51 (m, 2H), 1.12 (t, *J =* 7.5 Hz, 3H), 0.87-0.80 (m, 2H), 0.80-0.73 (m, 2H).

### Example 41: Synthesis of compound 41

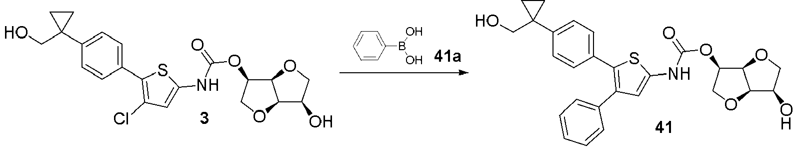

At room temperature, dioxane (1.5 mL), toluene (1.5 mL), compound **3** (100 mg, 0.22 mmol), compound **41a** (54 mg, 0.44 mmol), palladium acetate (10 mg, 0.04 mmol), 2-dicyclohexylphosphino-2',6'-dimethoxybiphenyl (18 mg, 0.04 mmol) and potassium phosphate (141 mg, 0.66 mmol) were added to a 10 mL microwave tube. The reaction was carried out at 100°C under microwave conditions for 2 hours until completion. Subsequently, the solid was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **41.** LC-MS: m/z: 494.2.0(M+H)⁺. ¹H NMR (400 MHz, DMSO-*d*₆) δ 10.97 (s, 1H), 7.33-7.07 (m, 9H), 6.61 (s, 1H), 5.09 (q, *J =* 6.0 Hz, 1H), 4.94 (d, *J* = 6.4 Hz, 1H), 4.72-4.61 (m, 2H), 4.31 (d, *J =* 5.1 Hz, 1H), 4.18-4.09 (m, 1H), 4.03-3.94 (m, 1H), 3.85-3.73 (m, 2H), 3.52 (d, *J =* 5.6 Hz, 2H), 3.42 (t, *J =* 8.5 Hz, 1H), 0.82 (s, 2H), 0.72 (s, 2H).

### Example 42: Synthesis of compound 42

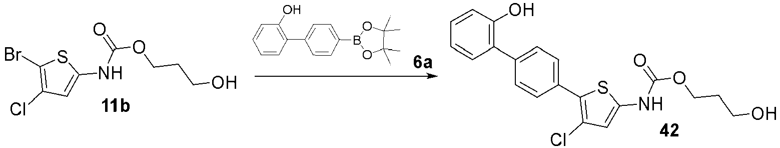

At room temperature, dioxane (30 mL), water (6 mL), intermediate **11b** (1000 mg, 3.18 mmol), compound **6a** (1224 mg, 4.13 mmol), [1,1'-bis(diphenylphosphino)ferrocene]palladium dichloride-dichloromethane complex (260 mg, 0.32 mmol) and potassium carbonate (1318 mg, 9.54 mmol) were added to a 100 mL round-bottom flask. The flask was purged with nitrogen 3 times, and the reaction was carried out at 90°C for 16 hours until completion. The reaction solution was poured into water (100 ml), and the mixture was extracted with ethyl acetate (30 ml × 3). The organic phase was dried over anhydrous sodium sulphate, and filtered, and the filtrate was concentrated under reduced pressure. The resulting residue was purified by column chromatography (dichloromethane/methanol) to afford a crude product, which was separated and purified by high performance liquid chromatography (mobile phase: A: 0.1% FA/H₂O B: ACN, chromatographic column: Waters-CORTECS-C18-2.7 µm-4.6*30 mm) to afford compound **42.** LC-MS: m/z: 403.9(M+H)⁺. ¹H NMR (400 MHz, DMSO-*d*₆) δ 11.03 (s, 1H), 9.69 (s, 1H), 7.65-7.60 (m, 4H), 7.30 (dd, *J =* 7.6, 1.7 Hz, 1H), 7.20-7.15 (m, 1H), 6.96 (dd, *J =* 8.1, 1.2 Hz, 1H), 6.89 (td, *J =* 7.4, 1.2 Hz, 1H), 6.52 (s, 1H), 4.59 (s, 1H), 4.21 (t, *J* = 6.5 Hz, 2H), 3.51 (t, *J =* 6.2 Hz, 2H), 1.85 - 1.74 (m, 2H).

### Example 43: Synthesis of compound 43

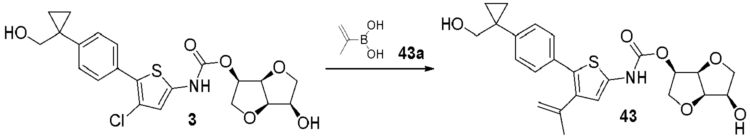

At room temperature, water (0.5 mL), toluene (1.5 mL), dioxane (1.5 mL), compound **3** (140 mg, 0.31 mmol), compound **43a** (133 mg, 1.55 mmol), palladium acetate (14 mg, 0.06 mmol), tricyclohexylphosphine (35 mg, 0.12 mmol) and caesium carbonate (303 mg, 0.93 mmol) were added to a 10 mL microwave tube. The reaction was carried out at 110°C under microwave conditions for 2 hours until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **43.** LC-MS: m/z: 458.0(M+H)⁺. ¹H NMR (400 MHz, DMSO-*d*₆) δ 10.85 (s, 1H), 7.28 (s, 4H), 6.47 (s, 1H), 5.10-5.03 (m, 2H), 4.96-4.89 (m, 2H), 4.68 (t, *J =* 5.6 Hz, 1H), 4.63 (t, *J* = 5.1 Hz, 1H), 4.30 (t, *J* = 4.9 Hz, 1H), 4.17-4.08 (m, 1H), 4.01-3.92 (m, 1H), 3.82-3.72 (m, 2H), 3.54 (d, *J =* 5.6 Hz, 2H), 3.40 (t, *J =* 8.5 Hz, 1H), 1.82 (d, *J =* 1.2 Hz, 3H), 0.86-0.80 (m, 2H), 0.80-0.73 (m, 2H).

### Example 44: Synthesis of compound 44

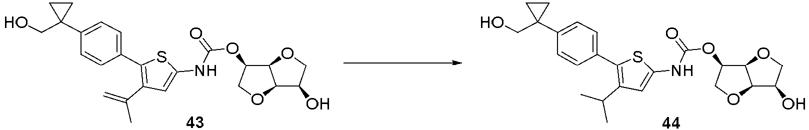

To a 10 mL reaction flask were added methanol (5 mL), compound **43** (50 mg, 0.11 mmol) and 10% palladium on carbon (12 mg, 0.11 mmol). The flask was purged with hydrogen 3 times, and the reaction was carried out at 25°C for 2 hours until completion. Subsequently, the solid was filtered, the filtrate was subjected to distillation under reduced pressure, and the resulting residue was separated and purified by high performance liquid chromatography (mobile phase: A: 0.1% FA/H₂O B: ACN, chromatographic column: Waters-CORTECS-C18-2.7 µm-4.6*30 mm) to afford compound **44.** LC-MS: m/z: 460.0(M+H)⁺. ¹H NMR (400 MHz, DMSO-*d*₆) δ 10.76 (s, 1H), 7.34 (d, *J =* 8.3 Hz, 2H), 7.23 (d, *J =* 8.3 Hz, 2H), 6.54 (s, 1H), 5.05 (q, *J =* 6.1 Hz, 1H), 4.92 (d, *J =* 6.4 Hz, 1H), 4.69 (t, *J =* 5.7 Hz, 1H), 4.62 (t, *J* = 5.1 Hz, 1H), 4.29 (t, *J* = 4.9 Hz, 1H), 4.16-4.08 (m, 1H), 3.99-3.94 (m, 1H), 3.81-3.72 (m, 2H), 3.54 (d, *J =* 5.7 Hz, 2H), 3.40 (t, *J =* 8.5 Hz, 1H), 3.05-2.97 (m, 1H), 1.12 (dd, *J =* 6.8, 1.8 Hz, 6H), 0.86-0.80 (m, 2H), 0.80-0.74 (m, 2H).

### Example 45: Synthesis of compound 45

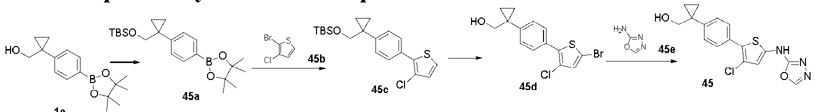

**Step 1:** Dichloromethane (110 mL), compound **1e** (5.0 g, 18.24 mmol) and imidazole (2.48 g, 36.47 mmol) were added to a 250 mL single-necked flask, and tert-butyldimethylchlorosilane (3.83 g, 25.53 mmol) was added under stirring. The reaction was carried out at 25°C for 16 h until completion. The reaction solution was washed with water (50 ml) 3 times, and the organic phase was dried over anhydrous sodium sulphate, and filtered. The filtrate was concentrated under reduced pressure to afford a crude product, and the crude product was purified by normal-phase column chromatography to afford intermediate **45a** as a yellow solid. LC-MS: m/z: 411.2(M+23)⁺.

**Step 2:** Under nitrogen atmosphere, dioxane (10 mL), water (2 mL), intermediate **45a** (1.032 g, 2.66 mmol), compound **45b** (0.5 g, 2.53 mmol), [1,1'-bis(diphenylphosphino)ferrocene]palladium dichloride-dichloromethane complex (0.206 g, 0.25 mmol) and potassium carbonate (0.875 g, 6.33 mmol) were added to a 50 mL single-necked flask. The reaction was carried out at 90°C for 16 h until completion. The reaction solution was filtered, the filtrate was concentrated under reduced pressure to afford a crude product, and the crude product was purified by silica gel column chromatography to afford intermediate **45c.** LC-MS: m/z: 379.2(M+1)⁺.

**Step 3:** To a 50 mL single-necked flask were added acetonitrile (15 mL), intermediate **45c** (790 mg, 2.08 mmol) and N-bromosuccinimide (371 mg, 2.08 mmol). The reaction was carried out at 25°C for 16 hours until completion. The reaction solution was filtered, the filtrate was concentrated under reduced pressure to afford a crude product, and the crude product was separated and purified by silica gel column chromatography to afford intermediate **45d.** LC-MS: m/z: 325.2(M-17)⁺. ¹H NMR (400 MHz, DMSO-d6) δ 7.57-7.47 (m, 2H), 7.40 (d, *J =* 8.3 Hz, 3H), 4.73 (t, *J =* 5.6 Hz, 1H), 3.55 (d, *J =* 5.0 Hz, 2H), 0.91-0.84 (m, 2H), 0.81-0.76 (m, 2H).

**Step 4:** Under nitrogen atmosphere, dioxane (3 mL), intermediate **45d** (50 mg, 0.15 mmol), compound **45e** (24.75 mg, 0.29 mmol), tris(dibenzylideneacetone)dipalladium (13.32 mg, 0.01 mmol), 1,1'-binaphthyl-2,2'-bis(diphenylphosphine) (BINAP) (18.12 mg, 0.03 mmol) and potassium phosphate (92.65 mg, 0.44 mmol) were added to a 10 mL microwave tube. The reaction was carried out at 110°C under microwave conditions for 2.5 hours until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **45.** LC-MS: m/z: 348.2(M+H)⁺. ¹H NMR (400 MHz, DMSO-*d*₆) δ 7.64-7.57 (m, 2H), 7.53 (s, 1H), 7.48-7.38 (m, 4H), 4.75 (t, *J* = 5.6 Hz, 1H), 3.57 (d, *J =* 5.5 Hz, 2H), 0.92-0.86 (m, 2H), 0.85-0.78 (m, 2H).

### Example 46: Synthesis of compound 46

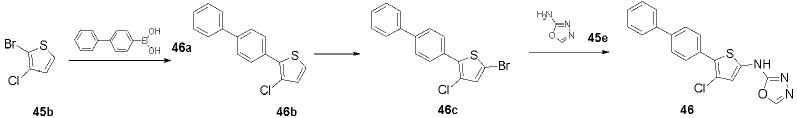

**Step 1:** Under nitrogen atmosphere, dichloromethane (10 mL), water (2 mL), compound **45b** (500 mg, 2.53 mmol), compound **46a** (501.42 mg, 2.53 mmol), [1,1'-bis(diphenylphosphino)ferrocene]palladium dichloride-dichloromethane complex (207.28 mg, 0.25 mmol) and potassium carbonate (874.82 mg, 6.33 mmol) were added to a 50 mL single-necked flask. The reaction was carried out at 90°C for 16 h until completion. The reaction solution was filtered, the filtrate was concentrated under reduced pressure to afford a crude product, and the crude product was separated and purified by silica gel column chromatography to afford intermediate **46b.** LC-MS: m/z: 271.0(M+1)⁺. ¹H NMR (400 MHz, DMSO-d6) δ 7.89-7.78 (m, 2H), 7.77-7.69 (m, 5H), 7.53-7.46 (m, 2H), 7.43-7.35 (m, 1H), 7.22 (dd, J = 12.0, 5.4 Hz, 1H).

**Step 2:** At room temperature, acetonitrile (10 mL), intermediate **46b** (325 mg, 1.05 mmol) and N-bromosuccinimide (NBS) (213.65 mg, 1.20 mmol) were added to a 50 mL single-necked flask. The reaction was carried out at 25°C for 16 hours until completion. The reaction solution was filtered, the filtrate was concentrated under reduced pressure to afford a crude product, and the crude product was purified by silica gel column chromatography to afford intermediate **46c.** ¹H NMR (400 MHz, DMSO-d6) δ 7.84-7.78 (m, 2H), 7.72 (td, *J =* 6.5, 3.4 Hz, 4H), 7.53-7.47 (m, 2H), 7.46-7.37 (m, 2H).

**Step 3:** Under nitrogen atmosphere, dioxane (2.5 mL), intermediate **46c** (50 mg, 0.14 mmol), compound **45e** (24.33 mg, 0.29 mmol), tris(dibenzylideneacetone)dipalladium (13.09 mg, 0.01 mmol), 1,1'-binaphthyl-2,2'-bis(diphenylphosphine) (BINAP) (17.44 mg, 0.03 mmol) and potassium phosphate (89.15 mg, 0.42 mmol) were added to a 10 mL microwave tube. The reaction was carried out at 110°C under microwave conditions for 1 hour until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **46.** LC-MS: m/z: 354.2(M+1)⁺. ¹H NMR (400 MHz, DMSO-*d*₆) δ 7.88-7.79 (m, 4H), 7.78-7.71 (m, 2H), 7.57 (s, 1H), 7.51 (t, *J =* 7.6 Hz, 2H), 7.47-7.38 (m, 3H).

### Example 47: Synthesis of compound 47

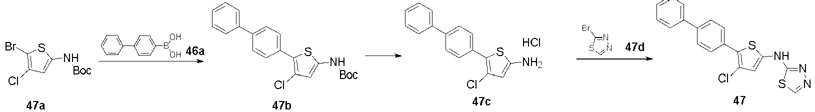

**Step 1:** Under nitrogen atmosphere, dichloromethane (15 mL), water (3 mL), compound **47a** (870 mg, 2.78 mmol),compound **46a** (606.23 mg, 3.06 mmol), [1,1'-bis(diphenylphosphino)ferrocene]palladium dichloride-dichloromethane complex (227.83 mg, 0.28 mmol) and potassium carbonate (961.53 mg, 6.96 mmol) were added to a 50 mL single-necked flask. The reaction was carried out at 100°C for 16 hours until completion. The reaction solution was filtered, the filtrate was concentrated under reduced pressure to afford a crude product, and the crude product was purified by silica gel column chromatography to afford intermediate **47b.** LC-MS: m/z: 408.1(M+23)⁺.

**Step 2:** To a 50 mL single-necked flask were added intermediate **47b** (540 mg, 1.40 mmol) and dichloromethane (5 mL). Hydrogen chloride ethyl acetate solution (5 mL) was added under stirring, and the reaction was carried out at 25°C for 16 hours until completion. The reaction solution was concentrated under reduced pressure to afford hydrochloride of intermediate **47c.** LC-MS: m/z: 286.0(M+1)⁺. ¹H NMR (400 MHz, DMSO-*d*₆) δ 7.73-7.66 (m, 4H), 7.66-7.58 (m, 2H), 7.47 (t, *J* = 7.7 Hz, 2H), 7.40-7.33 (m, 1H), 5.90 (s, 1H), 5.53-5.30 (m, 2H).

**Step 3:** Under nitrogen atmosphere, dioxane (3 mL), hydrochloride of intermediate **47c** (50 mg, 0.17 mmol), compound **47d** (56.10 mg, 0.34 mmol), tris(dibenzylideneacetone)dipalladium (15.57 mg, 0.02 mmol), 1,1'-binaphthyl-2,2'-bis(diphenylphosphine) (BINAP) (21.17 mg, 0.03 mmol) and potassium phosphate (108.25 mg, 0.51 mmol) were added to a 10 mL microwave tube. The reaction was carried out at 110°C under microwave conditions for 1 hour until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **47.** LC-MS: m/z: 370.2(M+1)⁺. ¹H NMR (400 MHz, DMSO- *d*₆) δ 14.21 (s, 1H), 9.12 (d, *J =* 1.0 Hz, 1H), 7.85-7.80 (m, 2H), 7.78-7.73 (m, 4H), 7.71 (s, 1H), 7.51 (dd, *J =* 8.4, 6.9 Hz, 2H), 7.44-7.38 (m, 1H).

### Example 48: Synthesis of compound 48

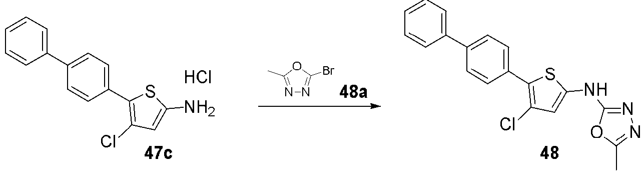

Under nitrogen atmosphere, dioxane (3 mL), hydrochloride of intermediate **47c** (50 mg, 0.17 mmol), compound **48a** (57.02 mg, 0.35 mmol), tris(dibenzylideneacetone)dipalladium (16.02 mg, 0.02 mmol), 1,1'-binaphthyl-2,2'-bis(diphenylphosphine) (BINAP) (21.79 mg, 0.03 mmol) and potassium phosphate (111.41 mg, 0.52 mmol) were added to a 10 mL microwave tube. The reaction was carried out at 110°C under microwave conditions for 1 hour until completion. Subsequently, the solid was filtered, and the filtrate was separated and purified by high performance liquid chromatography (mobile phase: A: 0.1% FA/H₂O B: ACN, chromatographic column: Waters-CORTECS-C18-2.7 µm-4.6*30 mm) to afford compound **48.** LC-MS: m/z: 368.0 (M+1)⁺. ¹H NMR (400 MHz, DMSO-*d*₆) δ 12.15-10.80 (m, 1H), 7.77 (d, *J=* 8.5 Hz, 2H), 7.74-7.69 (m, 4H), 7.49 (t, *J=* 7.7 Hz, 2H), 7.40-7.37 (m, 1H), 6.79 (s, 1H), 2.42 (s, 3H).

### Example 49: Synthesis of compound 49

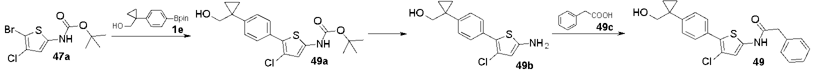

**Step 1:** At room temperature, dioxane (20 mL), water (4 mL), compound **47a** (1.0 g, 3.20 mmol), compound **1e** (1.32 g, 4.80 mmol), 1,1-bis(diphenylphosphino)ferrocene palladium dichloride (0.26 g, 0.32 mmol) and potassium carbonate (1.33 g, 9.60 mmol) were added to a 50 mL single-necked tube. The reaction was carried out at 90°C under nitrogen atmosphere for 16 hours until completion. The reaction solution was poured into water (100 mL), and the mixture was extracted with ethyl acetate (20 mL x 3). The organic phase was washed with saturated sodium chloride solution, dried over anhydrous sodium sulphate, and filtered. The filtrate was concentrated under reduced pressure to afford a crude product, and the crude product was purified by column chromatography to afford intermediate **49a.** LC-MS: m/z: 324.0 (M-55)⁺. ¹H NMR (400 MHz, DMSO-*d*₆) δ 10.75 (s, 1H), 7.48 (d, *J =* 8.2 Hz, 2H), 7.35 (d, *J =* 8.2 Hz, 2H), 6.43 (s, 1H), 4.72 (t, *J =* 5.6 Hz, 1H), 3.54 (d, *J =* 5.6 Hz, 2H), 1.48 (s, 9H), 0.87- 0.81 (m, 2H), 0.81-0.74 (m, 2H).

**Step 2:** To a 100 mL single-necked flask were added intermediate **49a** (200 mg, 0.53 mmol) and ethyl acetate (7 mL), and 4 N hydrogen chloride ethyl acetate solution (7 mL) was added dropwise to the reaction solution. The reaction was carried out at 25°C for 16 hours until completion. Subsequently, the reaction solution was concentrated under reduced pressure to afford intermediate **49b.** LC-MS: m/z: 279.9 (M+H)⁺.

**Step 3:** To a 10 mL single-necked flask were added N,N-dimethylformamide (4 mL), compound **49c** (39 mg, 0.29 mmol), 1-hydroxybenzotriazole (46 mg, 0.34 mmol) and 1-ethyl-(3-dimethylaminopropyl)carbodiimide hydrochloride (66 mg, 0.34 mmol). The mixture was reacted at 25°C for 5 minutes, and then intermediate **49b** (80 mg, 0.29 mmol) and N,N-diisopropylethylamine (148 mg, 1.14 mmol) were added. Subsequently, the reaction was continued for 16 hours until completion. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **49.** LC-MS: m/z: 380.0 (M-17)⁺. ¹H NMR (400 MHz, DMSO-*d*₆) δ 11.63 (s, 1H), 7.52 - 7.48 (m, 2H), 7.36 - 7.24 (m, 7H), 6.63 (s, 1H), 4.18 (s, 2H), 3.70 (s, 2H), 1.00 - 0.96 (m, 2H), 0.95 - 0.91 (m, 2H).

### Example 50: Synthesis of compound 50

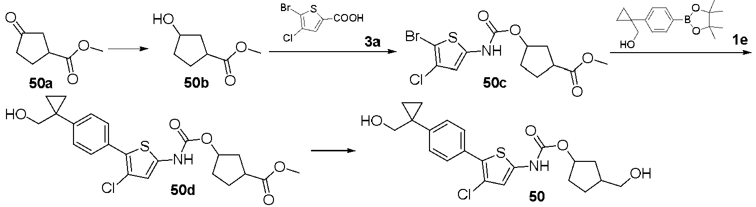

**Step 1:** Compound **50a** (4 g, 28.14 mmol) and dichloromethane (50 mL) were sequentially added to a 250 mL reaction flask, and then sodium borohydride (1.28 g, 33.77 mmol) was added at 0°C, and the mixture was stirred and reacted for 2 hours. After the reaction was completed, a small amount of water was added to the reaction solution to quench the reaction. The mixture was then dried over anhydrous sodium sulphate, and filtered, and the filtrate was concentrated under reduced pressure to afford intermediate **50b.** LC-MS: m/z: 145.2 (M+H)⁺.

**Step 2:** Dioxane (120 mL), intermediate **50b** (4.5 g, 31.07 mmol), compound **3a** (1.5 g, 6.21 mmol), diphenyl phosphorazidate (2.05 g, 7.45 mmol) and triethylamine (1.29 mL, 9.32 mmol) were sequentially added to a 250 mL reaction flask. After purging with nitrogen 3 times, the mixture was stirred and reacted at 90°C for 16 hours. After the reaction was completed, the reaction solution was concentrated under reduced pressure, and the resulting residue was purified by silica gel column chromatography to afford intermediate **50c.** LC-MS: m/z: 382.0 (M+H)⁺.

**Step 3:** Dioxane (8 mL), water (2 mL), intermediate **50c** (1.0 g, 2.61 mmol), compound **1e** (0.79 g, 2.87 mmol), 1,1-bis(diphenylphosphino)ferrocene palladium dichloride (0.21 g, 0.26 mmol) and potassium carbonate (0.90 g, 6.35 mmol) were added to a 50 mL single-necked tube. The reaction was carried out at 90°C under nitrogen atmosphere for 16 hours until completion. The reaction solution was poured into water (50 mL), and the mixture was extracted with ethyl acetate (10 mL x 3). The organic phase was washed with saturated sodium chloride solution, dried over anhydrous sodium sulphate, and filtered. The filtrate was concentrated under reduced pressure to afford a crude product, and the crude product was purified by column chromatography to afford intermediate **50d.** LC-MS: m/z: 450.2 (M+1)⁺.

**Step 4:** Under nitrogen atmosphere, intermediate **50d** (100 mg, 0.22 mmol) and tetrahydrofuran (5 mL) were added to a 10 mL reaction flask. At 0°C, a solution of lithium aluminium hydride in tetrahydrofuran (2.5 N, 0.1 mL) was added dropwise to the reaction flask, and then the reaction was carried out at 25°C for 2 hours until completion. The reaction solution was filtered, and the filtrate was separated and purified by high performance liquid chromatography (mobile phase: A: 10 mM NH₄HCO₃/H₂O B: ACN, column: Waters-Xbridge-C₁₈-10 µm-19*250 mm) to afford compound **50.** LC-MS: m/z: 422.2 (M+H)⁺. ¹H NMR (500 MHz, DMSO-*d*₆) δ 10.90 (s, 1H), 7.48 (d, *J =* 8.2 Hz, 2H), 7.36 (d, *J =* 8.3 Hz, 2H), 6.46 (d, *J =* 3.5 Hz, 1H), 5.09 (d, *J =* 23.2 Hz, 1H), 4.70 (t, *J =* 5.6 Hz, 1H), 4.54 (t, *J =* 5.3 Hz, 1H), 3.55 (d, *J* = 5.6 Hz, 2H), 3.35 (t, *J =* 5.9 Hz, 2H), 2.15 - 1.91 (m, 2H), 1.86 - 1.78 (m, 1H), 1.76 - 1.58 (m, 2H), 1.57 - 1.24 (m, 2H), 0.85 (q, *J =* 4.1 Hz, 2H), 0.76 (q, *J =* 4.6, 4.1 Hz, 2H).

### Example 51: Synthesis of compound 51

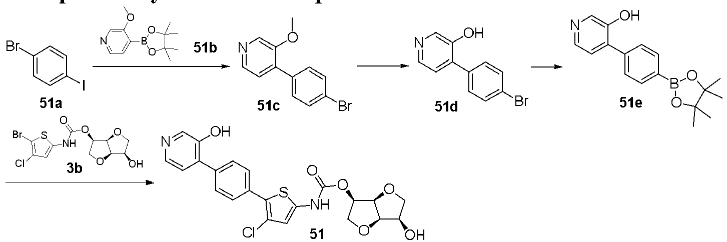

**Step 1:** To a 100 mL single-necked flask were added dioxane (20 mL), water (5 mL), compound **51a** (1 g, 3.53 mmol), compound **51b** (0.75 g, 3.18 mmol), tetrakis(triphenylphosphine)palladium (0.20 g, 0.18 mmol) and potassium carbonate (1.22 g, 8.84 mmol). The reaction was carried out at 85°C under nitrogen atmosphere for 16 hours until completion. The reaction solution was poured into water (100 mL), and the mixture was extracted with ethyl acetate (30 mL x 3). The organic phase was washed with aqueous sodium chloride solution, dried over anhydrous sodium sulphate, and filtered, and the filtrate was concentrated under reduced pressure to afford a crude product. The crude product was separated and purified by silica gel column chromatography to afford intermediate **51c.** LC-MS: m/z: 264.0(M+H)⁺. ¹H NMR (400 MHz, DMSO-*d*₆) δ 8.48 (s, 1H), 8.29 (d, *J =* 4.8 Hz, 1H), 7.70-7.63 (m, 2H), 7.56-7.51 (m, 2H), 7.36 (d, *J* = 4.8 Hz, 1H), 3.90 (s, 3H).

**Step 2:** To a 25 mL single-necked flask was added intermediate **51c** (60 mg, 0.23 mmol) dissolved in dichloromethane (3 mL), and boron tribromide (1 mL, 0.68 mmol) was added at 0°C. The reaction was carried out at 25°C for 16 h until completion. At 0°C, sodium bicarbonate solution (30 mL) was slowly added to the reaction solution to quench the reaction. The mixture was extracted with dichloromethane (10 mL × 3). The organic phase was washed with saturated aqueous sodium chloride solution, dried over anhydrous sodium sulphate, and filtered, and the filtrate was concentrated under reduced pressure to afford intermediate **51d.** LC-MS: m/z: 250.0(M+H)⁺.

**Step 3:** Under nitrogen atmosphere, dioxane (3 mL), intermediate **51d** (50 mg, 0.20 mmol), bis(pinacolato)diboron (60.92 mg, 0.24 mmol), [1,1'-bis(diphenylphosphino)ferrocene]palladium dichloride (14.83 mg, 0.02 mmol) and potassium acetate (58.86 mg, 0.60 mmol) were added to a 25 mL single-necked flask. The reaction was carried out at 85°C for 16 hours until completion. The reaction solution was filtered, and then concentrated under reduced pressure to afford intermediate **51e.** LC-MS: m/z: 298.2(M+H)⁺.

**Step 4:** To a 25 mL single-necked flask were added dioxane (3 mL), intermediate **51e** (50 mg, 0.17 mmol), intermediate **3b** (64.72 mg, 0.17 mmol), [1,1'-bis(diphenylphosphino)ferrocene]palladium dichloride (13.77 mg, 0.02 mmol) and potassium carbonate (69.76 mg, 0.50 mmol). The flask was purged with nitrogen, and the reaction was carried out at 100°C under microwave conditions for 2 hours until completion. The crude product was separated and purified by high performance liquid chromatography (mobile phase: A: 0.1% FA/H₂O B: ACN, chromatographic column: Waters-CORTECS-C18-2.7 µm-4.6*30 mm) to afford compound **51.** LC-MS: m/z: 475.0(M+H)⁺. ¹H NMR (400 MHz, DMSO-*d*₆) δ 11.25 (s, 1H), 10.17 (s, 1H), 8.28 (s, 1H), 8.11 (d, *J =* 4.9 Hz, 1H), 7.79-7.71 (m, 2H), 7.71-7.63 (m, 2H), 7.36 (d, *J* = 4.9 Hz, 1H), 6.54 (s, 1H), 5.10 (q, *J =* 6.0 Hz, 1H), 4.95 (d, *J =* 6.6 Hz, 1H), 4.65 *(t, J=* 5.1 Hz, 1H), 4.30 (t, *J* = 4.9 Hz, 1H), 4.13 (s, 1H), 3.97 (dd, *J* = 9.5, 6.2 Hz, 1H), 3.79 (m, 2H), 3.39 (d, *J =* 8.5 Hz, 1H).

Other compounds of the present invention can be prepared by methods similar to those described in the above examples (with appropriate modifications, if necessary).

### Biological tests:

### Test example 1: In vitro assay for AMPK (α1β1γ1) enzyme agonist activity

Test compounds were dissolved in DMSO to prepare 10 mM stock solution, which was then diluted to the predetermined test concentrations using kinase buffer (50 mM HEPES, 1 mM EDTA, 10 mM MgCl₂, 0.01% Brij-35, pH = 7.5). Into a 384-well plate were added 5 µL of AMPKα1β1γ1 (CARNA, 02-113), 2.5 µL of the diluted test compound solution, and 2.5 µL of a mixture of Ulight-CRBN and ATP, resulting in the following final concentrations: AMPKα1β1γ1 at 1.6 mg/L, Ulight-CRBN at 5 nM, and ATP at 0.1 mM. The components were mixed evenly, and reacted at room temperature for 1 h. Subsequently, 5 µL of LANCE Detection Buffer (CR97-100, PerkinElmer) containing 40 mM EDTA was added. After standing for 5 min, 5 µL of 4× Eu-anti-phospho-CREB Antibody (TRF0200-M, PerkinElmer) was added, and the mixture was allowed to stand at room temperature for 1 h. The values of samples in each well were read using a microplate reader in TR-FRET mode (excitation wavelength: 320 nm; emission wavelengths: 665 nm and 615 nm). By comparing the values with those of the blank control group (1% DMSO) and the positive control group (1 µM A769662), the relative activity of AMPKα1β1γ1 at each test compound concentration (4.57 nM, 13.72 nM, 41.15 nM, 123.46 nM, 370.37 nM, 1111.11 nM, 3333.33 nM, and 10000 nM) was calculated, with the activity of 1 µM A769662 set as 100%. The data were analysed in GraphPad Prism 9 using a Dose-response-Stimulation four-parameter regression model to obtain the Top and EC₅₀ values for the test compounds. Partial test results are shown in Table 2.

A769662 has a structure of

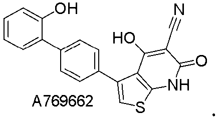

**Table 2**

| Example No. | EC₅₀ (nM) | Top value (%) | Example No. | EC₅₀ (nM) | Top value (%) |
|---|---|---|---|---|---|
| 1 | D | B | 18 | D | C |
| 2 | D | C | 19 | A | C |
| 3 | A | B | 20 | D | A |
| 4 | D | C | 21 | B | C |
| 5 | B | C | 22 | D | C |
| 6 | B | B | 23 | D | A |
| 7 | B | B | 24 | B | C |
| 8 | B | B | 28 | D | E |
| 9 | A | C | 31 | C | C |
| 10 | D | B | 32 | B | D |
| 11 | B | C | 37 | D | E |
| 12 | D | | 38 | C | C |
| 13 | D | B | 39 | D | D |
| 14 | D | E | 40 | D | D |
| 15 | D | E | 41 | D | |
| 16 | D | | 42 | D | C |
| 17 | D | B | | | |

| | | | | | |
|---|---|---|---|---|---|
| Note: in Table 2, A indicates EC₅₀ ≤ 200 nM; B indicates 200 nM < EC₅₀ ≤ 500 nM; C indicates 500 nM < EC₅₀ ≤ 1000 nM; D indicates EC₅₀ > 1000 nM; and with the AMPK (α1β1γ1) enzyme agonist activity of 1 µM A769662 set as 100%, A indicates the Top value of the test compound's AMPK (α1β1γ1) enzyme agonist activity ≥ 300%; B indicates the Top value of the test compound's AMPK (α1β1γ1) enzyme agonist activity ≥ 200% and < 300%; C indicates the Top value of the test compound's AMPK (α1β1γ1) enzyme agonist activity ≥ 150% and < 200%; D indicates the Top value of the test compound's AMPK (α1β1γ1) enzyme agonist activity ≥ 100% and < 150%; E indicates the Top value of the test compound's AMPK (α1β1γ1) enzyme agonist activity ≥ 70% and < 100%. | | | | | |

The test results show that the compounds of the present invention exhibit good AMPK agonist activity, with some compounds demonstrating more excellent AMPK agonist activity.

**Test example 2:** Experimental assay for detecting AMPK phosphorylation at the cellular level

HepG2 cells (ATCC, HB-8065) were cultured in DMEM complete medium (Gibco, 11965-092) containing 10% foetal bovine serum (Gibco, 10099-141C), 100 U penicillin, and 100 µg/mL streptomycin (Gibco, 15140-122). This assay was performed using the Perkin Elmer's HTRF phospho-AMPK (Thr172) detection kits (Perkin Elmer, 64MPKPEG).

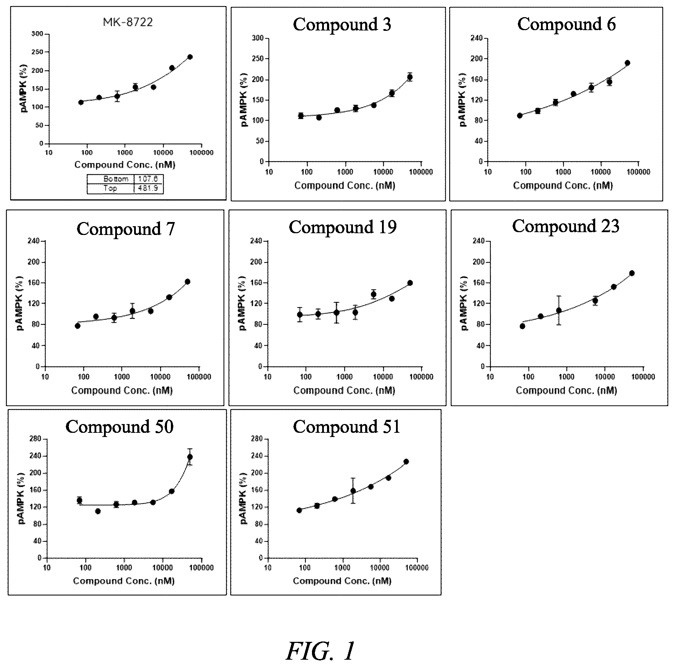

HepG2 cells were cultured in a T75 cell culture flask (Corning, 430641) and placed in an incubator at 37°C and 5% CO₂. When the cell density reached 70%-80%, the cells were passaged approximately twice a week. For the experiment, 20,000 cells/well were seeded into a 384-well plate (Corning, 3765), and the cells were cultured overnight. Subsequently, the test compounds (10 mM stock solution dissolved in DMSO) were added to the 384-well cell assay plate at the predetermined concentrations (69 nM-50 µM) using ECHO. After co-incubating the compounds at different concentrations with HepG2 cells for 40 min, the cell culture medium was removed. Subsequently, 16 µL of cell lysis buffer from the detection kit was added, and the mixture was incubated with shaking at room temperature for 30 min. Thereafter, 4 µL of a mixed solution of diluted pAMPK Eu Cryptate antibody and pAMPK d2 antibody from the kit was added, resulting in a final detection antibody concentration of 1X. Following mixing evenly by centrifugation at 1500 rpm for 2 min, the mixture was incubated at room temperature for 4 hours. The values of samples in each well at 620 nm and 665 nm were read using a microplate reader in TR-FRET mode. By comparing the values with those of the blank control group (medium only) and the positive control group (0.5% DMSO), the pAMPK level of each test compound at different concentrations was calculated, with the activity of 0.5% DMSO set as 100%. The data were analysed in GraphPad Prism 9 using a Dose-response-Stimulation four-parameter regression model to obtain the Top and EC₅₀ values for the test compounds, and the curves showing the changes in intracellular pAMPK (%) with increasing compound concentrations. Partial test results are shown in Figure 1.

MK-8722 has a structure of

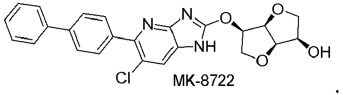

The test results show that the compounds of the present invention exhibit good AMPK agonist activity at the cellular level, with some compounds demonstrating more excellent AMPK agonist activity at the cellular level.

Various modifications of the present invention in addition to those described herein will be apparent to those skilled in the art from the foregoing description. Such modifications are also intended to fall within the scope of the appended claims. Each reference cited in the present application (including all patents, patent applications, journal articles, books and any other disclosures) is incorporated herein by reference in its entirety.

## Claims

1. A compound of formula (I): 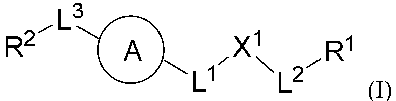
or a stereoisomer, a tautomer, a diastereomer, a racemate, a cis-trans isomer, an isotopically labelled compound (preferably a deuterated compound), an N-oxide, a metabolite, an ester, a prodrug, a crystal form, a hydrate, a solvate or a pharmaceutically acceptable salt thereof, **characterised in that**
X¹ is selected from -C(=O)-, -C(=S)- and -CH₂-;
L¹ is selected from -O-, -S-, -NR^{c}- and -CR^{a}R^{b}-;
L² is selected from -O-, -S-, -NR^{c}-, -CR^{a}R^{b}-, -O-CR^{a}R^{b}- and -S-CR^{a}R^{b}-;
alternatively, -L¹-X¹-L²-, as a whole, is -NH-;
R¹ is selected from C₁₋₆ alkyl, C₃₋₈ cycloalkyl, 4- to 10-membered heterocyclyl, C₆₋₁₀ aryl and 5- to 10-membered heteroaryl, wherein the C₁₋₆ alkyl, C₃₋₈ cycloalkyl, 4- to 10-membered heterocyclyl, C₆₋₁₀ aryl and 5- to 10-membered heteroaryl are optionally substituted with 1, 2 or 3 R^{1a};
R^{1a} is independently selected from halogen, -OH, -CN, an oxo group, -COOR^{c}, -C(=O)NHR^{c}, C₁₋₆ alkyl, C₁₋₆ haloalkyl, C₁₋₆ alkoxy, C₁₋₆ haloalkoxy, -C₁₋₆ alkylene-OH, -OC₁₋₆ alkylene-OH, -C₁₋₆ alkylene-NH₂, -C₁₋₆ alkylene-COOR^{c}, -C₁₋₆ alkylene-C(=O)NHR^{c}, -S=O₂-C₁₋₆ alkyl and 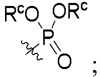
R^{a} and R^{b}, at each occurrence, are each independently selected from H, D, halogen, -OH, -CN, C₁₋₆ alkyl, C₁₋₆ alkoxy, C₁₋₆ haloalkyl, C₁₋₆ haloalkoxy and C₃₋₆ cycloalkyl;
R^{c}, at each occurrence, is independently selected from H, D, C₁₋₆ alkyl, C₁₋₆ haloalkyl and C₃₋₆ cycloalkyl;
ring A 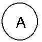 is selected from a benzene ring and a 5- to 6-membered aromatic heterocyclic ring, and the benzene ring and 5- to 6-membered aromatic heterocyclic ring are each optionally substituted with 1, 2 or 3 R^{A}, wherein the R^{A} is independently selected from H, D, halogen, -OH, -NH₂, -CN, C₁₋₆ alkyl, C₂₋₆ alkenyl, C₂₋₆ alkynyl, C₁₋₆ alkoxy, C₁₋₆ haloalkyl, C₂₋₆ haloalkenyl, C₂₋₆ haloalkynyl, C₁₋₆ haloalkoxy, C₃₋₆ cycloalkyl, 3- to 6-membered heterocyclyl, phenyl, 5- to 6-membered heteroaryl, -NH(C₁₋₆ alkyl) and -N(C₁₋₆ alkyl)₂; alternatively, where applicable, two adjacent R^{A}, together with the ring atoms to which they are attached, may form C₄₋₆ carbocyclyl, 4- to 6-membered heterocyclyl or 5- to 6-membered heteroaryl;
L³ is selected from a bond, -O-, -S-, -NR^{L3}-, -C(=O)-NR^{L3}-, -C(=O)-O-, -C₁₋₆ alkylene-O-, -C₁₋₆ alkylene-NR^{L3}-, -C₁₋₆ alkylene-S-, -C₂₋₆ alkenylene- and -C₂₋₆ alkynylene-;
R^{L3} is selected from H, D and C₁₋₆ alkyl;
R² is selected from C₆₋₁₀ aryl, C₃₋₁₀ saturated or partially unsaturated cyclohydrocarbyl, 4- to 10-membered heterocyclyl and 5- to 10-membered heteroaryl, and the C₆₋₁₀ aryl, C₃₋₁₀ saturated or partially unsaturated cyclohydrocarbyl, 4- to 10-membered heterocyclyl and 5- to 10-membered heteroaryl are optionally substituted with 1, 2, 3, 4 or 5 groups each independently selected from R^{2a}, R^{2b}, R^{2c}, R^{2d} and R^{2e};
R^{2a}, R^{2b}, R^{2c}, R^{2d} and R^{2e} are each independently selected from H, halogen, - OH, -CN, C₁₋₆ alkyl, C₁₋₆ haloalkyl, -NH₂, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl, wherein the C₁₋₆ alkyl, C₁₋₆ haloalkyl, -NHC₁₋₆ alkyl, -N(C₁₋₆ alkyl)₂, C₁₋₆ alkoxy, C₁₋₆ haloalkoxy, C₃₋₁₀ saturated or partially unsaturated cyclohydrocarbyl, 4- to 10-membered heterocyclyl, C₆₋₁₀ aryl and 5- to 10-membered heteroaryl are each optionally substituted with 1, 2 or more R; and
R is independently selected from: H, halogen, -OH, -CN, -NH₂, an oxo group, -COOH, C₁₋₆ alkyl, C₁₋₆ haloalkyl, -C₁₋₆ alkyl substituted with 1 or 2 OH, C₁₋₆ alkoxy, -C₁₋₆ alkyleneoxy-C₁₋₆ alkoxy, -C(=O)NH-C₁₋₆ alkyl, -C(=O)NH-C₃₋₁₀ cycloalkyl, - C(=O)-C₁₋₆ alkyl, -NHC(=O)-C₁₋₆ alkyl, -N=S(=O)R^{s1}R^{s2}, -NH-S(=O)₂C₁₋₆ alkyl, - S(=O)₂-C₁₋₆ alkyl, -S(=O)₂-4- to 10-membered heterocyclyl, -S(=O)₂NH-C₁₋₆ alkyl, -S(=O)₂NH-C₃₋₁₀ cycloalkyl, -C₃₋₁₀ cycloalkylene-COOH, -C(=O)-4- to 10-membered heterocyclyl, 4- to 10-membered heterocyclyl, C₆₋₁₀ aryl and 5- to 10-membered heteroaryl, wherein the 4- to 10-membered heterocyclyl is optionally substituted with 1, 2 or more substituents independently selected from C₁₋₆ alkyl, - NH₂, =NH and an oxo group, the C₆₋₁₀ aryl and 5- to 10-membered heteroaryl are each optionally substituted with 1, 2 or more substituents independently selected from halogen, -OH, -CN, -NH₂, -C₁₋₆ alkylene-OH, -C₁₋₆ alkylene-CN and -C₁₋₆ alkylene-NH₂, and R^{s1} and R^{s2} are each C₁₋₆ alkyl, alternatively, R^{s1} and R^{s2}, together with the S atom to which they are attached, form 4- to 6-membered heterocycloalkyl; provided that:
(1) the compound of formula (I) is not the following compound: 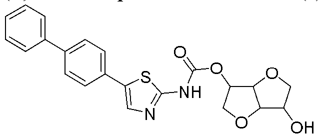 (including 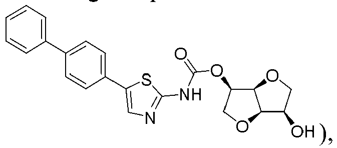 or a stereoisomer, a tautomer, a diastereomer, a racemate, a cis-trans isomer, an isotopically labelled compound, an N-oxide, a metabolite, an ester, a prodrug, a crystal form, a hydrate, a solvate or a pharmaceutically acceptable salt thereof; and
(2) when ring A is a thiazole ring, L³ is -NR^{L3}-, and R² is substituted or unsubstituted phenyl, L¹ is not -CR^{a}R^{b}-;
(3) when -L¹-X¹-L²-, as a whole, is -NH-, 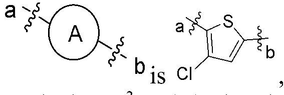 and R² is C₆₋₁₀ aryl, wherein the bond labelled "a" is attached to L³ and the bond labelled "b" is attached to L¹.

2. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to claim 1, **characterised in that** when ring A is a thiazole ring and L³ is absent, R² is not unsubstituted biphenyl.

3. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to claim 1 or 2, **characterised in that**
ring A is selected from a benzene ring and a 5- to 6-membered aromatic heterocyclic ring, and the benzene ring and 5- to 6-membered aromatic heterocyclic ring are each optionally substituted with 1, 2 or 3 R^{A};
preferably, ring A is selected from phenyl, furyl, thienyl, oxazolyl, thiazolyl, pyrrolyl, pyrazolyl, imidazolyl and pyridyl, and the phenyl, furyl, thienyl, oxazolyl, thiazolyl, pyrrolyl, pyrazolyl, imidazolyl and pyridyl are each optionally substituted with 1 or 2 R^{A};
preferably, ring A is selected from phenyl, thienyl, imidazolyl and thiazolyl, and the phenyl, thienyl, imidazolyl and thiazolyl are each optionally substituted with 1 or 2 R^{A};
preferably, ring A is thienyl, and the thienyl is optionally substituted with 1 or 2 R^{A};
preferably, ring A is selected from: 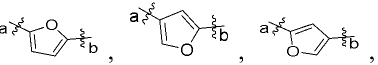 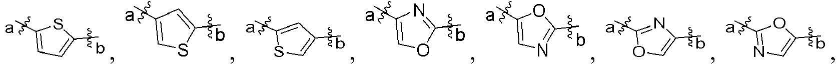 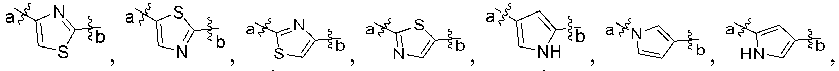 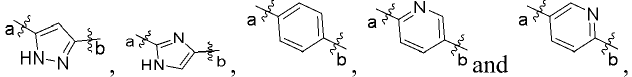 wherein any of the above groups is optionally substituted with 1, 2 or 3 (preferably 1 or 2, more preferably 1) R^{A}, and wherein the bond labelled "a" is attached to L³ and the bond labelled "b" is attached to L¹;
preferably, ring A is selected from: 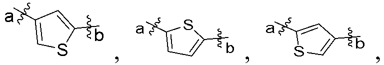 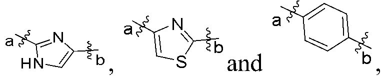 wherein any of the above groups is optionally substituted with 1 or 2 R^{A};
preferably, ring A is selected from: 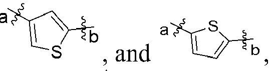 wherein any of the above groups is optionally substituted with 1 R^{A};
preferably, ring A is 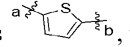 which is optionally substituted with 1 R^{A};
preferably, ring A is selected from: 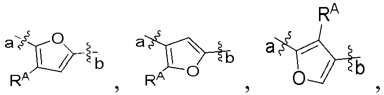 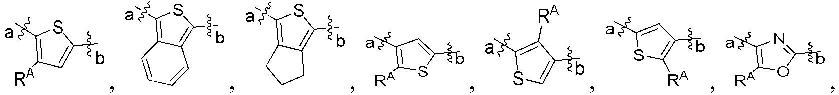 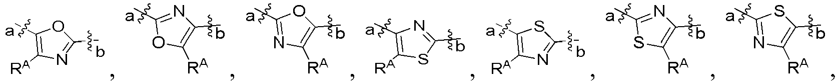 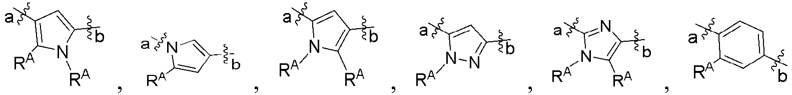 and 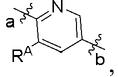 wherein the bond labelled "a" is attached to L³ and the bond labelled "b" is attached to L¹;
preferably, ring A is selected from: 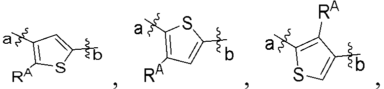 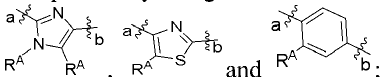
more preferably, ring A is selected from: 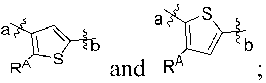
most preferably, ring A is

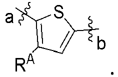

4. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-3, **characterised in that**
the R^{A} is independently selected from H, D, halogen, -CN, C₁₋₆ alkyl, C₂₋₆ alkenyl, C₂₋₆ alkynyl, C₁₋₆ alkoxy, C₁₋₆ haloalkyl, C₂₋₆ haloalkenyl, C₂₋₆ haloalkynyl, C₁₋₆ haloalkoxy, C₃₋₆ cycloalkyl, 3- to 6-membered heterocyclyl, and phenyl; alternatively, where applicable, two adjacent R^{A}, together with the ring atoms to which they are attached, may form C₅₋₆ carbocyclyl, 5- to 6-membered heterocyclyl or 5- to 6-membered heteroaryl;
preferably, the R^{A} is independently selected from H, D, halogen, -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, and phenyl; alternatively, where applicable, two adjacent R^{A}, together with the ring atoms to which they are attached, may form C₅₋₆ carbocyclyl;
preferably, the R^{A} is independently selected from H, D, halogen (such as F and Cl), -CN, C₁₋₄ alkyl, C₂₋₄ alkenyl, C₂₋₄ alkynyl, C₁₋₄ alkoxy, C₁₋₄ haloalkyl, C₂₋₄ haloalkenyl, C₂₋₄ haloalkynyl, C₁₋₄ haloalkoxy, C₃₋₄ cycloalkyl, 3- to 4-membered heterocyclyl, and phenyl;
preferably, the R^{A} is independently selected from H, halogen (such as Cl), C₁₋₄ alkyl, C₃₋₄ cycloalkyl, and phenyl;
preferably, the R^{A} is independently selected from H, halogen (such as Cl), and C₁₋₄ alkyl;
preferably, each of the R^{A} is independently selected from H, D, F, Cl, -CN, methyl, ethyl, isopropyl, sec-butyl, isobutyl, tert-butyl, vinyl, allyl, ethynyl, propargyl, methoxy, ethoxy, isopropoxy, tert-butoxy, -CF₃, -OCF₃, cyclopropyl, cyclobutyl, oxetanyl, azetidinyl, and phenyl;
preferably, each of the R^{A} is independently selected from H, F, Cl, methyl, ethyl, isopropyl, tert-butyl, cyclopropyl, cyclobutyl, and phenyl;
more preferably, each of the R^{A} is independently selected from Cl and methyl.

5. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-4, **characterised in that** the ring A is selected from: 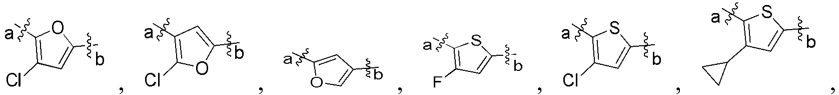 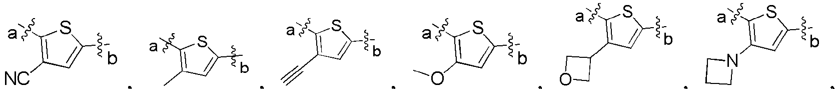 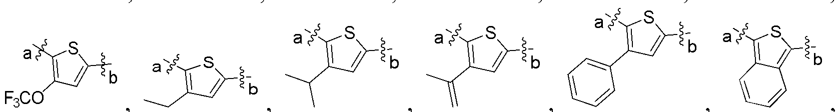 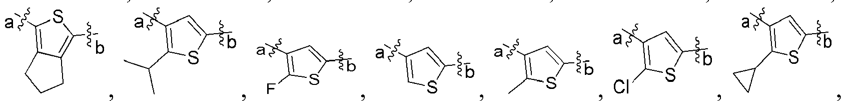 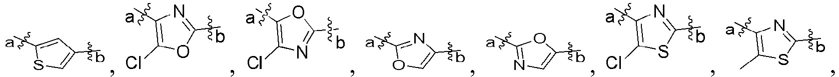 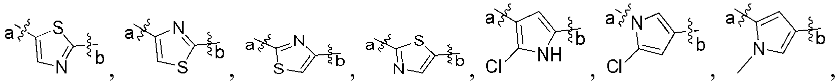 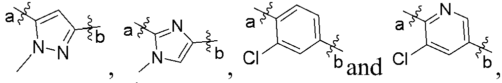 wherein the bond labelled "a" is attached to L³ and the bond labelled "b" is attached to L¹;
preferably the ring A is selected from 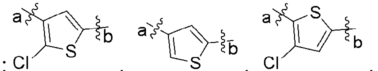 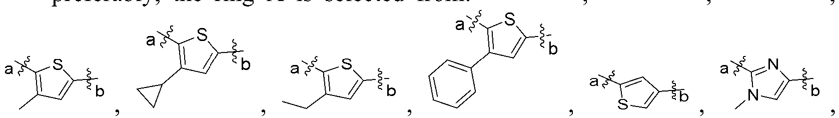 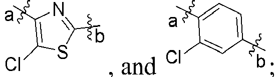
more preferably, the ring A is selected from: 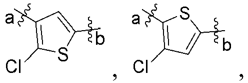 and 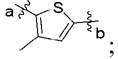
most preferably, the ring A is selected from 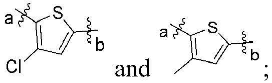
further preferably, the ring A is

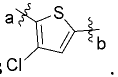

6. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-5, **characterised in that** the compound has a structure shown in one of the following formulas: 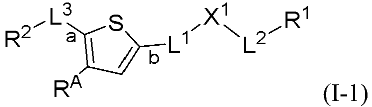
preferably, moiety 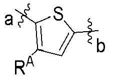 is selected from 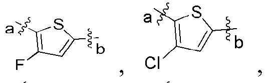 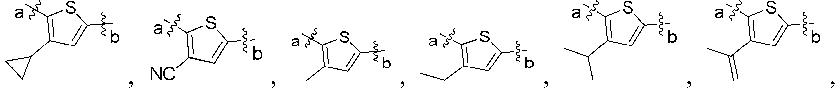 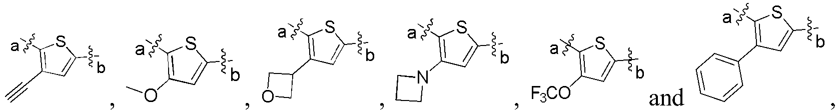 preferably 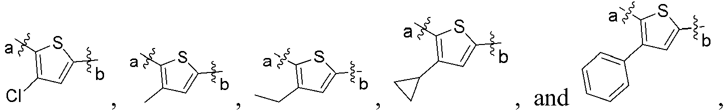 more preferably 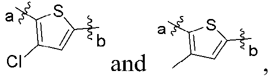 further preferably 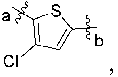 wherein
the bond labelled "a" is attached to L³ and the bond labelled "b" is attached to L¹; 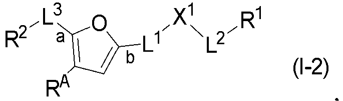
preferably, moiety 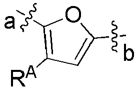
is 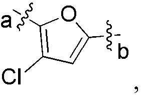
wherein the bond labelled "a" is attached to L³ and the bond labelled "b" is attached to L¹; 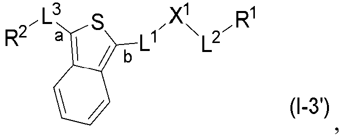 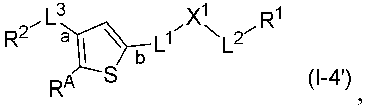
preferably, moiety 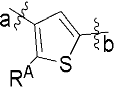
is selected from 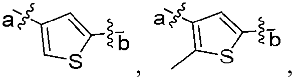 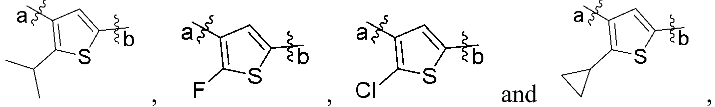 preferably 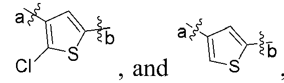 more preferably 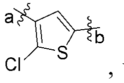 wherein the bond labelled "a" is attached to L³ and the bond labelled "b" is attached to L¹; 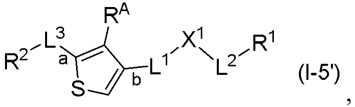
preferably, 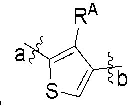 is 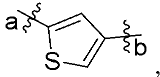 wherein the bond labelled "a" is attached to L³ and the bond labelled "b" is attached to L¹; 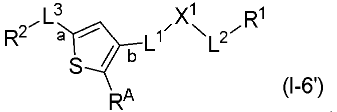 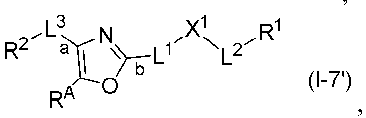
preferably, moiety 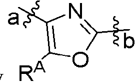 is 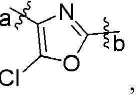 wherein the bond labelled "a" is attached to L³ and the bond labelled "b" is attached to L¹; 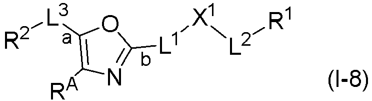
preferably, moiety 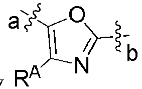 is 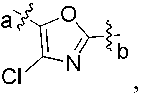 wherein the bond labelled "a" is attached to L³ and the bond labelled "b" is attached to L¹; 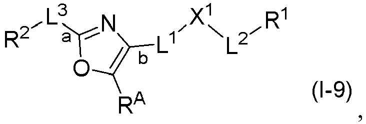
preferably, moiety 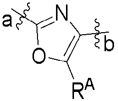 is 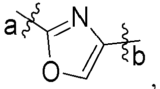 wherein the bond labelled "a" is attached to L³ and the bond labelled "b" is attached to L¹; 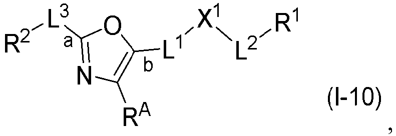
preferably, moiety 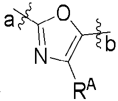 is 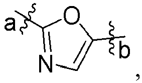 wherein the bond labelled "a" is attached to L³ and the bond labelled "b" is attached to L¹; 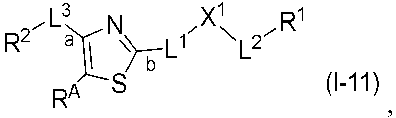
preferably, moiety 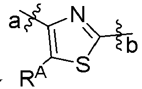 is selected from 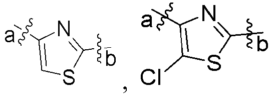 and 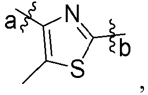 preferably 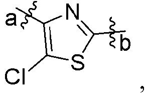 wherein the bond labelled "a" is attached lo L³ and the bond "b" is attached lo L¹; 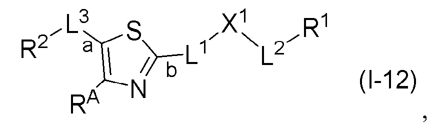
preferably, moiety 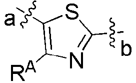 is 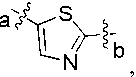 wherein the bond labelled "a" is attached to L³ and the bond labelled "b" is attached to L¹; 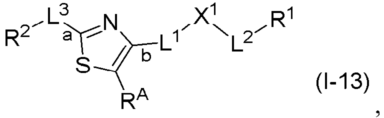
preferably, moiety 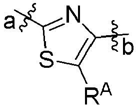 is 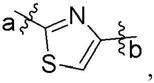 wherein the bond labelled "a" is attached to L³ and the bond labelled "b" is attached to L¹; 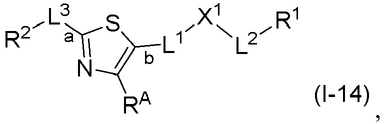
preferably, moiety 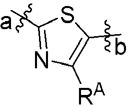 is 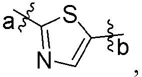 wherein the bond labelled "a"
is attached to L³ and the bond labelled "b" is attached to L¹; 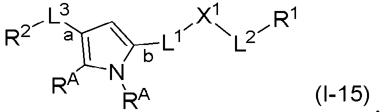
preferably, moiety 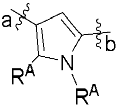 is 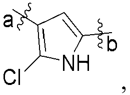 wherein the bond labelled "a" is
attached to L³ and the bond labelled "b" is attached to L¹; 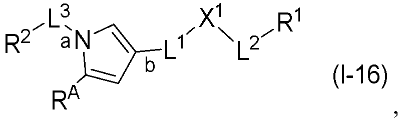
preferably, moiety 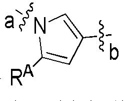 is 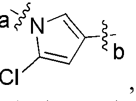 wherein the bond labelled "a"
is attached to L³ and the bond labelled "b" is attached to L¹; 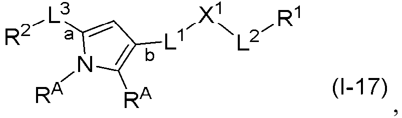
preferably, moiety 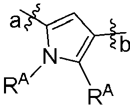 is 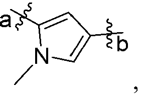 wherein the bond labelled "a" is attached to L³ and the bond labelled "b" is attached to L¹; 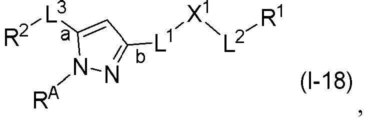
preferably, moiety 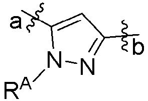 is 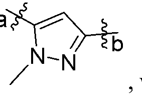 wherein the bond labelled "a" is attached to L³ and the bond labelled "b" is attached to L¹; 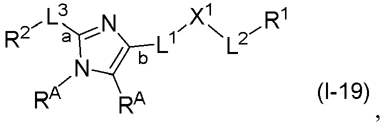
preferably, moiety 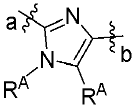 is 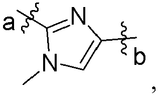 wherein the bond labelled "a" is attached to L³ and the bond labelled "b" is attached to L¹; 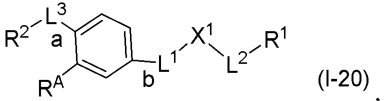
preferably, moiety 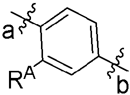 is 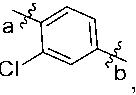 wherein the bond labelled "a"
is attached to L³ and the bond labelled "b" is attached to L¹; 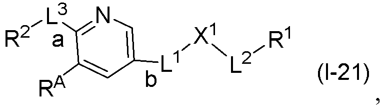
preferably, moiety 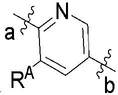 is 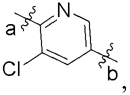 wherein the bond labelled "a" is
attached to L³ and the bond labelled "b" is attached to L¹; 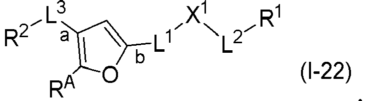
preferably, moiety 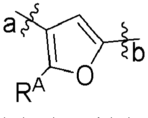
is 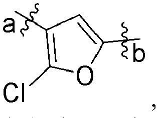 wherein the bond labelled "a" is attached to L³ and the bond labelled "b" is attached to L¹;
or 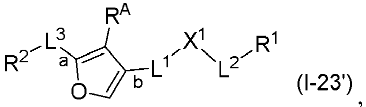
preferably, moiety 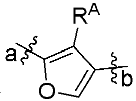 is 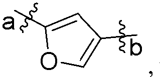 wherein the bond labelled "a" is attached to L³ and the bond labelled "b" is attached to L¹.

7. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-6, **characterised in that**
L³ is selected from a bond, -O-, -S-, -NR^{L3}-, #-C(=O)-NR^{L3}-*, #-C(=O)-O-*, #-C₁₋₆ alkylene-O-*, #-C₁₋₆ alkylene-NR^{L3}-*, #-C₁₋₆ alkylene-S-*, -C₂₋₆ alkenylene- and -C₂₋₆ alkynylene-, wherein the bond labelled "#" is attached to R² and the bond labelled "*" is attached to ring A;
preferably, L³ is selected from a bond, -O-, -S-, -NR^{L3}-, #-C(=O)-NR^{L3}-*, #-C(=O)-O-*, #-C₁₋₄ alkylene-O-*, #-C₁₋₄ alkylene-NR^{L3}-*, #-C₁₋₄ alkylene-S-*, -C₂₋₄ alkenylene- and -C₂₋₄ alkynylene-, wherein the bond labelled "#" is attached to R² and the bond labelled "*" is attached to ring A;
preferably, L³ is selected from a bond, -S-, -O-, -NR^{L3}-, #-C(=O)-NR^{L3}-*, #-C(=O)-O-*, #-(CH₂)ₙ-O-*, #-(CH₂)ₙ-NR^{L3}-*, #-(CH₂)ₙ-S-*, vinylene, propenylene, ethynylene and propynylene, wherein the bond labelled "#" is attached to R² and the bond labelled "*" is attached to ring A, and n is selected from 1 and 2;
preferably, L³ is selected from a bond, -O-, -S-, -NR^{L3}-, #-C(=O)-NR^{L3}-*, #-C(=O)-O-*, #-C₁₋₄ alkylene-NR^{L3}-* and -C₂₋₄ alkynylene-, wherein the bond labelled "#" is attached to R² and the bond labelled "*" is attached to ring A;
preferably, L³ is selected from a bond and -C₂₋₄ alkynylene- (such as ethynylene and propynylene);
preferably, L³ is selected from a bond and ethynylene;
more preferably, L³ is a bond.

8. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-7, **characterised in that**
R^{L3} is selected from H, D and C₁₋₄ alkyl;
preferably, R^{L3} is selected from H, D, -CH₃, -CH₂CH₃ and -CH(CH₃)₂;
more preferably, R^{L3} is H.

9. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-8, **characterised in that**
L³ is selected from a bond, -O-, -S-, -NH-, #-C(=O)-NH-*, #-CH₂-NH-*, #-CH₂-O-*, #-CH₂-S-* and -C≡C-, wherein the bond labelled "#" is attached to R² and the bond labelled "*" is attached to ring A;
preferably, L³ is selected from a bond, -O-, -S-, -NH-, #-C(=O)-NH-*, #-CH₂-NH-* and -C≡C-, wherein the bond labelled "#" is attached to R² and the bond labelled "*" is attached to ring A.

10. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-9, **characterised in that**
R² is selected from C₃₋₆ cycloalkyl, phenyl, 5- to 6-membered heteroaryl, 9-membered heteroaryl, 9- to 10-membered heterocyclyl and C₉₋₁₀ partially unsaturated cyclohydrocarbyl, and the C₃₋₆ cycloalkyl, phenyl, 5- to 6-membered heteroaryl, 9-membered heteroaryl, 9- to 10-membered heterocyclyl and C₉₋₁₀ partially unsaturated cyclohydrocarbyl are optionally substituted with 1, 2, 3, 4, or 5 groups each independently selected from R^{2a}, R^{2b}, R^{2c}, R^{2d} and R^{2e};
preferably, R² is selected from C₃₋₆ cycloalkyl, phenyl, 5- to 6-membered heteroaryl, and 9-membered heteroaryl, and the C₃₋₆ cycloalkyl, phenyl, 5- to 6-membered heteroaryl, and 9-membered heteroaryl are optionally substituted with 1, 2, 3, 4, or 5 groups each independently selected from R^{2a}, R^{2b}, R^{2c}, R^{2d} and R^{2e};
preferably, R² is selected from C₃₋₆ cycloalkyl, phenyl, and 9-membered heteroaryl, and the C₃₋₆ cycloalkyl, phenyl, and 9-membered heteroaryl are optionally substituted with 1, 2, 3, 4, or 5 groups each independently selected from R^{2a}, R^{2b}, R^{2c}, R^{2d} and R^{2e};
preferably, R² is selected from phenyl and 9-membered heteroaryl, and the phenyl and 9-membered heteroaryl are optionally substituted with 1, 2, 3, 4, or 5 groups each independently selected from R^{2a}, R^{2b}, R^{2c}, R^{2d} and R^{2e};
preferably, R² is selected from cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl, and the cyclopropyl, cyclobutyl, cyclopentyl, cyclohexyl, phenyl, thienyl, furyl, pyrrolyl, thiazolyl, oxazolyl, pyridyl, pyrimidyl, pyrazinyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e};
preferably, R² is selected from cyclopropyl, cyclobutyl, phenyl, furyl, thienyl, thiazolyl, oxazolyl, pyridyl, indolyl, indolinyl, benzofuryl, 2,3-dihydrobenzofuryl, 2,3-dihydrobenzodioxanyl, 2,3-dihydroindenyl and 1,2,3,4-tetrahydronaphthyl, and the cyclopropyl, cyclobutyl, phenyl, thienyl, thiazolyl, oxazolyl, pyridyl, indolyl, indolinyl, 2,3-dihydrobenzofuryl, 2,3-dihydrobenzodioxanyl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e};
preferably, R² is selected from cyclopropyl, cyclobutyl, phenyl, thienyl, pyridyl, indolyl, indolinyl, benzofuryl, 2,3-dihydrobenzofuryl, 2,3-dihydroindenyl and 1,2,3,4-tetrahydronaphthyl, and the cyclopropyl, cyclobutyl, phenyl, thienyl, pyridyl, indolyl, indolinyl, benzofuryl, 2,3-dihydrobenzofuryl, 2,3-dihydroindenyl and 1,2,3,4-tetrahydronaphthyl are each optionally substituted with 1, 2, 3, 4, or 5 groups each independently selected from R^{2a}, R^{2b}, R^{2c}, R^{2d} and R^{2e};
preferably, R² is selected from cyclopropyl, cyclobutyl, phenyl, and indolyl, and the cyclopropyl, cyclobutyl, phenyl and indolyl are each optionally substituted with 1, 2, 3, 4, or 5 groups each independently selected from R^{2a}, R^{2b}, R^{2c}, R^{2d} and R^{2e};
preferably, R² is selected from phenyl and indolyl, and the phenyl and indolyl are each optionally substituted with 1, 2, 3, 4, or 5 groups each independently selected from R^{2a}, R^{2b}, R^{2c}, R^{2d} and R^{2e};
preferably, R² is selected from 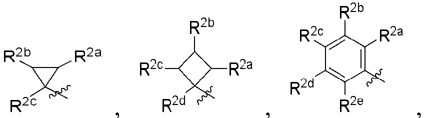 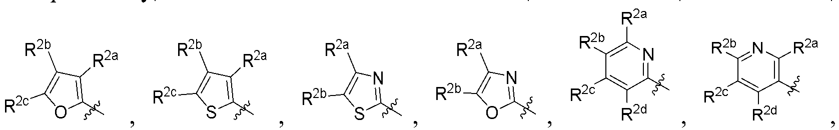 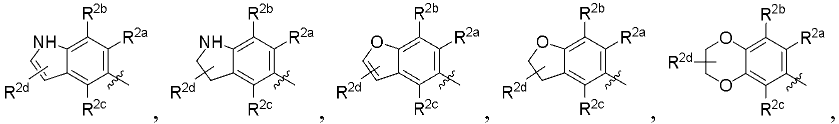 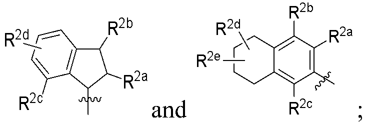
preferably, R² is selected from 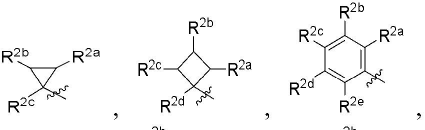 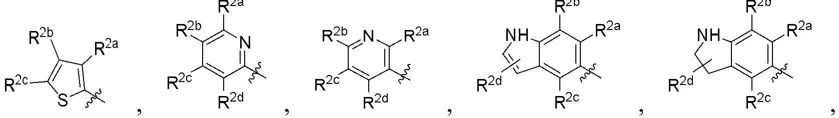 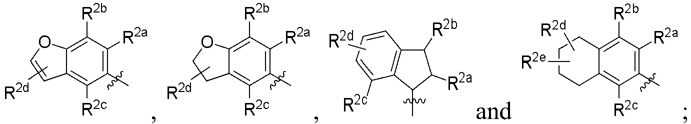
preferably, R² is selected from 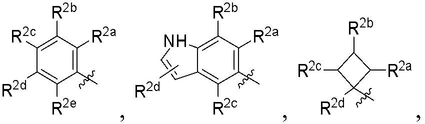 and 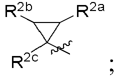
more preferably, R² is selected from

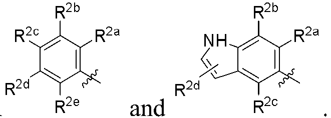

11. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-10, **characterised in that**
R^{2a}, R^{2b}, R^{2c}, R^{2d} and R^{2e} are each independently selected from H, halogen, - OH, -CN, C₁₋₄ alkyl, C₁₋₄ haloalkyl, -NH₂, -NHC₁₋₄ alkyl, -N(C₁₋₄ alkyl)₂, C₁₋₄ alkoxy, C₁₋₄ haloalkoxy, C₃₋₆ cycloalkyl, C₃₋₆ cycloalkenyl, 4- to 6-membered monocyclic heterocyclyl, 8- to 10-membered bicyclic heterocyclyl, phenyl and 5- to 6-membered heteroaryl, wherein the C₁₋₄ alkyl, C₁₋₄ haloalkyl, -NHC₁₋₄ alkyl, -N(C₁₋₄ alkyl)₂, C₁₋₄ alkoxy, C₁₋₄ haloalkoxy, C₃₋₆ cycloalkyl, C₃₋₆ cycloalkenyl, 4- to 6-membered monocyclic heterocyclyl, 8- to 10-membered bicyclic heterocyclyl, phenyl and 5- to 6-membered heteroaryl are each optionally substituted with 1, 2 or 3 R; preferably, R^{2a}, R^{2b}, R^{2c}, R^{2d} and R^{2e} are each independently selected from H, - OH, C₁₋₄ alkyl, C₁₋₄ alkoxy, C₃₋₆ cycloalkyl, 4- to 6-membered monocyclic heterocyclyl, phenyl and 5- to 6-membered heteroaryl, and the C₁₋₄ alkyl, C₁₋₄ alkoxy, C₃₋₆ cycloalkyl, 4- to 6-membered monocyclic heterocyclyl, phenyl and 5- to 6-membered heteroaryl are each optionally substituted with 1, 2 or 3 R;
preferably, R^{2a}, R^{2b}, R^{2c}, R^{2d} and R^{2e} are each independently selected from H, F, Cl, Br, -OH, -CN, methyl, ethyl, propyl, butyl, halomethyl, haloethyl, halopropyl, halobutyl, -NH₂, -NH-methyl, -NH-ethyl, -NH-propyl, -NH-butyl, -N(CH₃)₂, - N(CH₃)(CH₂CH₃), -N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, oxetanyl, azetidinyl, tetrahydrofuryl, pyrrolidyl, tetrahydropyranyl, piperidyl, morpholinyl, dihydrotriazolyl, 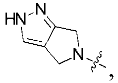 phenyl, imidazolyl, pyrazolyl, thienyl, furyl, pyrrolyl, thiazolyl, thiadiazolyl, oxazolyl, oxadiazolyl, triazolyl, tetrazolyl, pyridyl, pyrimidyl and pyrazinyl, and the methyl, ethyl, propyl, butyl, halomethyl, haloethyl, halopropyl, halobutyl, -NH₂, - NH-methyl, -NH-ethyl, -NH-propyl, -NH-butyl, -N(CH₃)₂, -N(CH₃)(CH₂CH₃), - N(CH₂CH₃)₂, methoxy, ethoxy, propoxy, butoxy, halomethoxy, haloethoxy, halopropoxy, halobutoxy, cyclopropyl, cyclobutyl, cyclopentyl, cyclohexyl, cyclopentenyl, cyclohexenyl, oxetanyl, azetidinyl, tetrahydrofuryl, pyrrolidyl, tetrahydropyranyl, piperidyl, morpholinyl, dihydrotriazolyl, 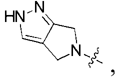 phenyl, imidazolyl, pyrazolyl, thienyl, furyl, pyrrolyl, thiazolyl, thiadiazolyl, oxazolyl, oxadiazolyl, triazolyl, tetrazolyl, pyridyl, pyrimidyl and pyrazinyl are each optionally substituted with 1, 2 or 3 R;
preferably, R^{2a}, R^{2b}, R^{2c}, R^{2d} and R^{2e} are each independently selected from H, F, -OH, -CH₃, -CH₂CH₃, -CH₂CH₂CH₃, -CH₂CH₂CH₂CH₃, -CH(CH₃)₂, -C(CH₃)₃, - N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, piperidyl, morpholinyl, tetrahydrofuryl, pyrrolidyl, tetrahydropyranyl, 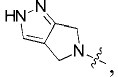 phenyl and pyridyl, and the -CH₃, -CH₂CH₃, -CH₂CH₂CH₃, -CH₂CH₂CH₂CH₃, -CH(CH₃)₂, -C(CH₃)₃, - N(CH₃)₂, -OCH₃, -OCH₂CH₃, -OCH₂CH₂CH₃, -OCH(CH₃)₂, -OCF₃, cyclopropyl, cyclobutyl, cyclohexenyl, azetidinyl, oxetanyl, piperidyl, morpholinyl, tetrahydrofuryl, pyrrolidyl, tetrahydropyranyl, 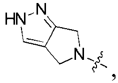 phenyl and pyridyl are each optionally substituted with 1, 2 or 3 R;
preferably, R^{2a}, R^{2b}, R^{2c}, R^{2d} and R^{2e} are each independently selected from H, - OH, -CH₃, -CH₂CH₃, -CH₂CH₂CH₃, -OCH₃, -OCH₂CH₃, -OCH₂CH₂CH₃, cyclopropyl, cyclobutyl, phenyl, pyridyl, pyrrolidyl, morpholinyl, tetrahydropyranyl, and piperidyl, and the -CH₃, -CH₂CH₃, -CH₂CH₂CH₃, -OCH₃, -OCH₂CH₃, - OCH₂CH₂CH₃, cyclopropyl, cyclobutyl, phenyl, pyridyl, pyrrolidyl, morpholinyl, tetrahydropyranyl and piperidyl are each optionally substituted with 1, 2 or 3 R.

12. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-11, **characterised in that**
R is a group selected from the following (1) to (3):
(1) H, halogen, -OH, -CN, -NH₂, an oxo group, -COOH, C₁₋₄ alkyl, C₁₋₄ haloalkyl, -C₁₋₄ alkyl substituted with 1 or 2 OH, C₁₋₄ alkoxy, -C₁₋₄ alkyleneoxy-C₁₋₄ alkoxy, -C(=O)NH-C₁₋₄ alkyl, -C(=O)NH-C₃₋₆ cycloalkyl, -C(=O)-C₁₋₄ alkyl, - NHC(=O)-C₁₋₄ alkyl, -N=S(=O)R^{s1}R^{s2}, -NH-S(=O)₂C₁₋₄ alkyl, -S(=O)₂-C₁₋₄ alkyl, - S(=O)₂-4- to 6-membered heterocyclyl, -S(=O)₂NH-C₁₋₄ alkyl, -S(=O)₂NH-C₃₋₆ cycloalkyl, -C₃₋₆ cycloalkylene-COOH and -C(=O)-4- to 6-membered heterocyclyl, wherein R^{s1} and R^{s2} are each C₁₋₄ alkyl, alternatively, R^{s1} and R^{s2}, together with the S atom to which they are attached, form 4- to 6-membered heterocycloalkyl;
(2) 4- to 6-membered heterocyclyl, wherein the 4- to 6-membered heterocyclyl is optionally substituted with 1, 2 or more substituents independently selected from C₁₋₄ alkyl, -NH₂, =NH and an oxo group; and
(3) phenyl and 5- to 6-membered heteroaryl, wherein the phenyl and 5- to 6-membered heteroaryl are each optionally substituted with 1, 2 or more substituents independently selected from halogen, -OH, -CN, -NH₂, -C₁₋₄ alkylene-OH, -C₁₋₄ alkylene-CN and -C₁₋₄ alkylene-NH₂; preferably, R is selected from: H, -NH₂, -OH, -C₁₋₄ alkyl substituted with one OH, C₁₋₄ alkyl, -S(=O)₂-C₁₋₄ alkyl, and 4- to 6-membered heterocyclyl;
preferably, R is a group selected from the following (1) to (3):
(1) H, halogen, -OH, -CN, -NH₂, an oxo group, -COOH, methyl, ethyl, propyl, butyl, halomethyl, haloethyl, halopropyl, halobutyl, -CH₂OH, -ethylene-OH, 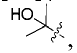 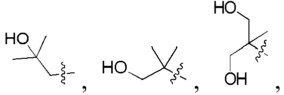 methoxy, ethoxy, propoxy, butoxy, -methyleneoxy-methoxy, -methyleneoxy-ethoxy, -methyleneoxy-propoxy, -ethyleneoxy-methoxy, - ethyleneoxy-ethoxy, -ethyleneoxy-propoxy, -C(=O)-NH-methyl, -C(=O)-NH-ethyl, -C(=O)-NH-propyl, -C(=O)-NH-butyl, -N=S(=O)(methyl)₂, - N=S(=O)(methyl)(ethyl), -N=S(=O)(methyl)(propyl), -N=S(=O)(methyl)(butyl), - N=S(=O)(ethyl)₂, -N=S(=O)(ethyl)(propyl), -(NH)ₚ-W-methyl, -(NH)ₚ-W-ethyl, - (NH)ₚ-W-propyl, -(NH)ₚ-W-butyl, -W-NH-cyclopropyl, -W-oxetanyl, -W-azetidinyl, -W-tetrahydrofuryl, -W-pyrrolidyl, -W-tetrahydropyranyl, -W-piperidyl, -W-morpholinyl, -cyclopropylene-COOH and 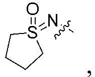 wherein each W is independently C(=O) or S(=O)₂, and each p is independently 0 or 1;
(2) oxetanyl, azetidinyl, tetrahydrofuryl, pyrrolidyl, tetrahydropyranyl, piperidyl, morpholinyl, thiomorpholinyl, dihydropyrazolyl, dihydroimidazolyl and dihydrotriazolyl, wherein the oxetanyl, azetidinyl, tetrahydrofuryl, pyrrolidyl, tetrahydropyranyl, piperidyl, morpholinyl, thiomorpholinyl, dihydropyrazolyl, dihydroimidazolyl and dihydrotriazolyl are each optionally substituted with 1, 2 or more substituents independently selected from methyl, ethyl, propyl, tert-butyl, - NH₂, =NH and an oxo group; and
(3) phenyl, thienyl, furyl, pyrrolyl, pyrazolyl, thiazolyl, thiadiazolyl, oxazolyl, oxadiazolyl, triazolyl, tetrazolyl, pyridyl, pyrimidyl and pyrazinyl, wherein the phenyl, thienyl, furyl, pyrrolyl, pyrazolyl, thiazolyl, thiadiazolyl, oxazolyl, oxadiazolyl, triazolyl, tetrazolyl, pyridyl, pyrimidyl and pyrazinyl are each optionally substituted with 1, 2 or more substituents independently selected from halogen, -OH, -CN, -NH₂, -C₁₋₄ alkylene-OH, -C₁₋₄ alkylene-CN and -C₁₋₄ alkylene-NH₂;
preferably, R is selected from: H, F, -OH, -CN, -NH₂, an oxo group, -COOH, - CH₃, 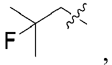 -CH₂OH, 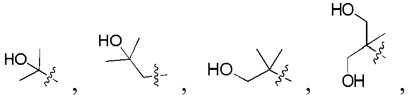 -OCH₃, - OCH₂CH₂OCH₃, -C(=O)CH₃, -C(=O)NHCH₃, -NHC(=O)CH₃, -N=S(=O)(CH₃)₂, 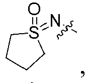 -NH-S(=O)₂CH₃, -S(=O)₂CH₃, 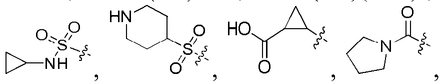 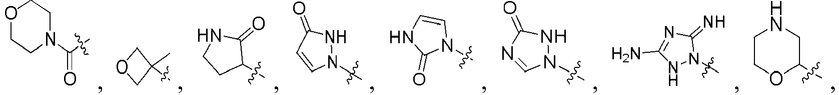 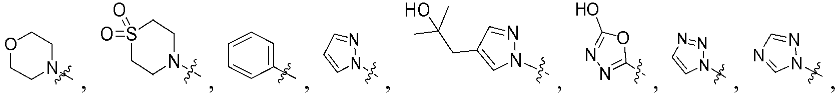 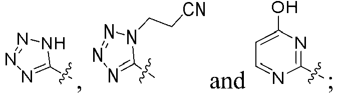
preferably, R is H, -NH₂, -OH, -CH₂OH, -CH₃, -S(=O)₂CH₃, and 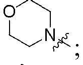
more preferably, R is H, -OH, -CH₂OH, -CH₃, -S(=O)₂CH₃, and

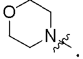

13. The compound according to any of claims 1-12, **characterised in that** R² is selected from: 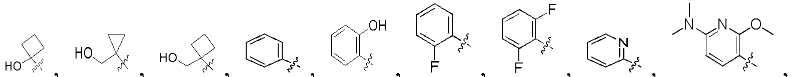 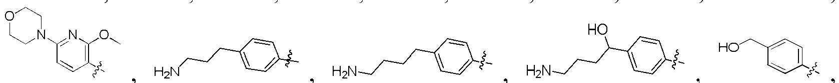 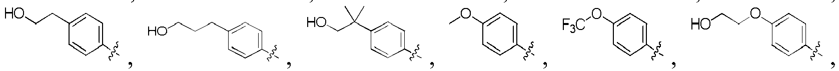 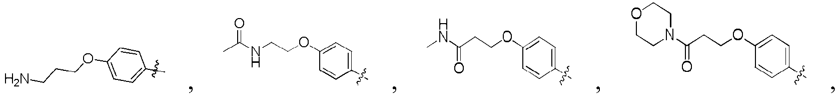 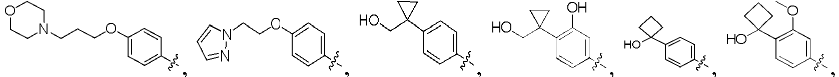 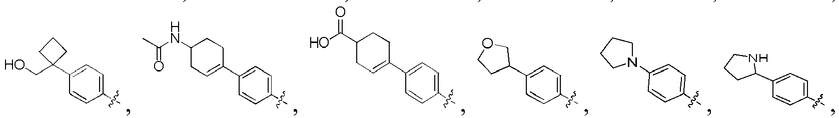 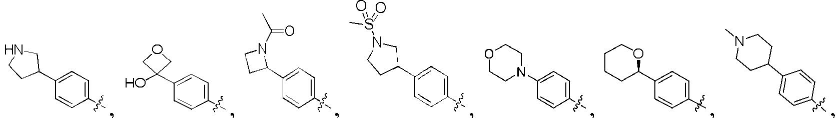 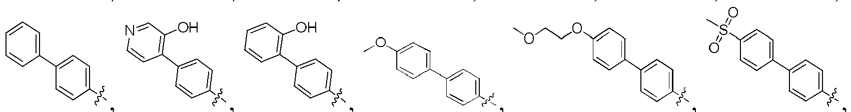 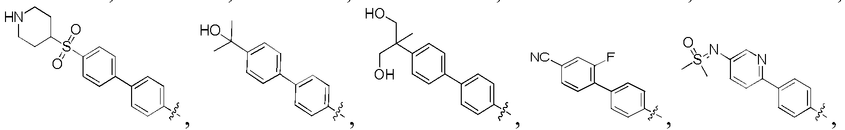 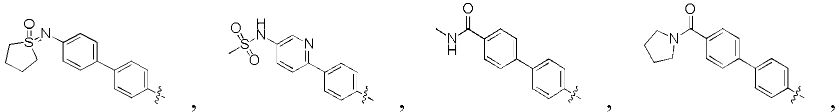 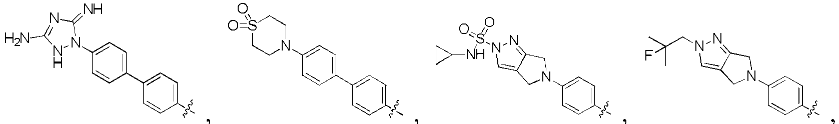 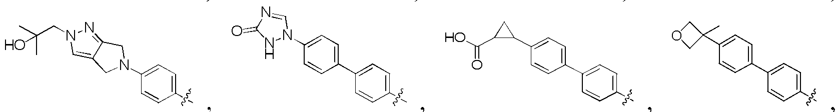 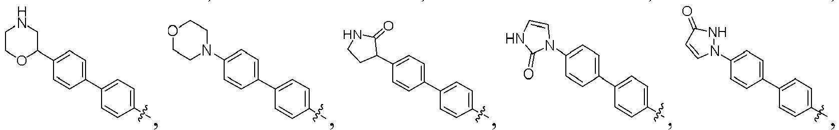 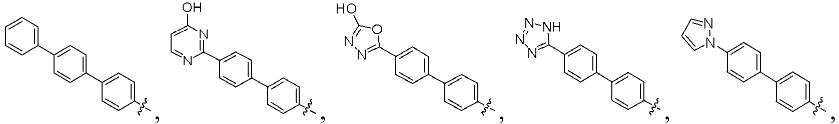 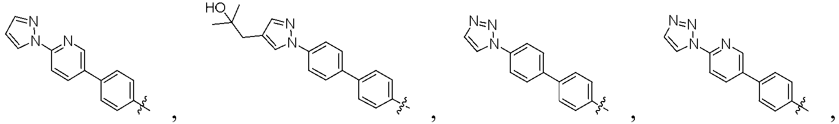 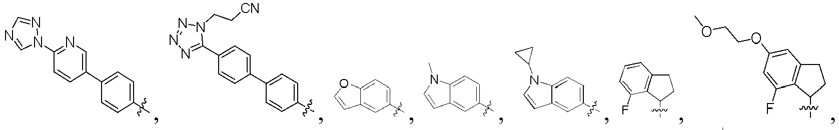 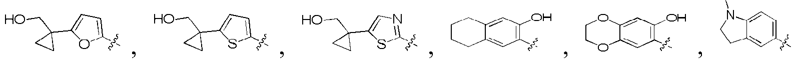 and 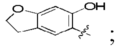
preferably, R² is selected from 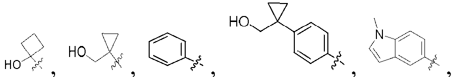 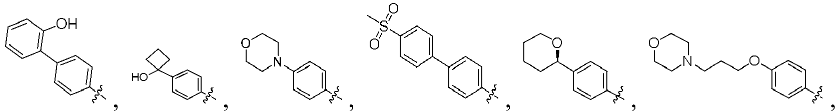 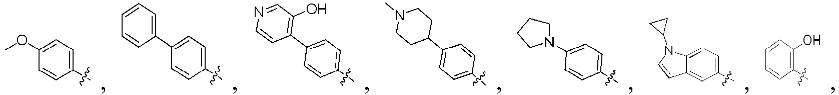 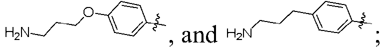
more preferably, R² is selected from

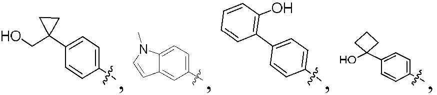

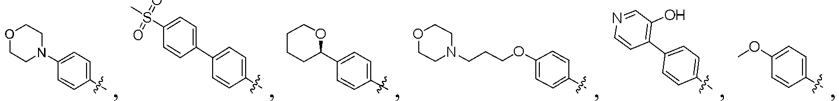

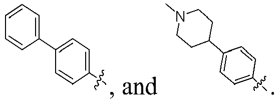

14. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-13, **characterised in that** the compound has a structure shown in any of formulas (II-1) to (II-9): 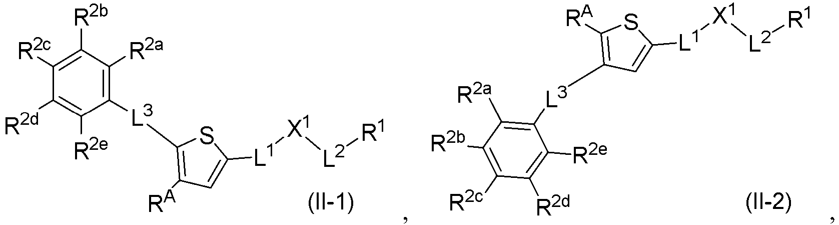 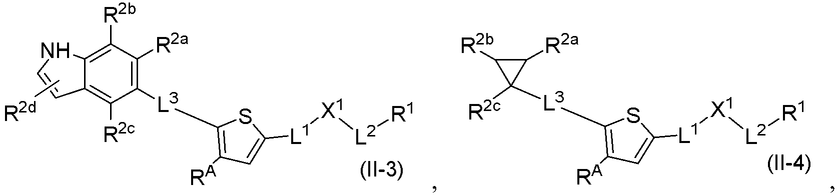 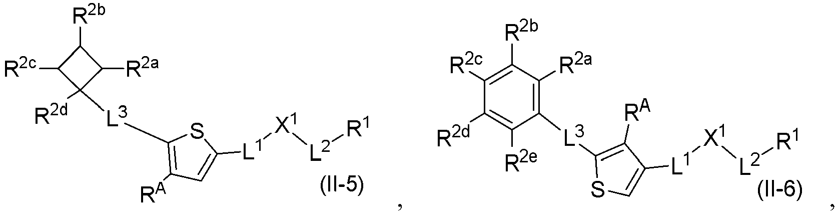 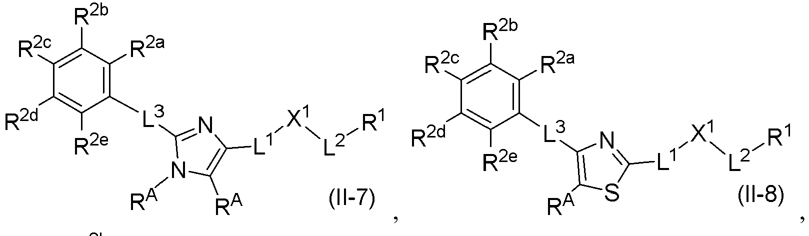 or 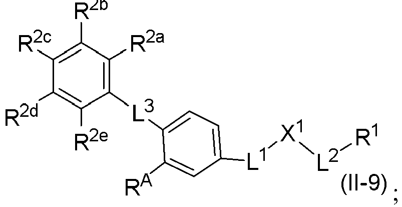 preferably, the compound has a structure shown in any of formulas (I-24) to (I-33):

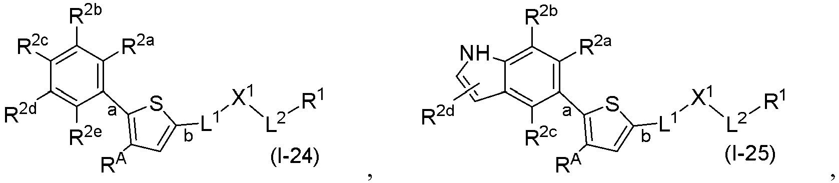

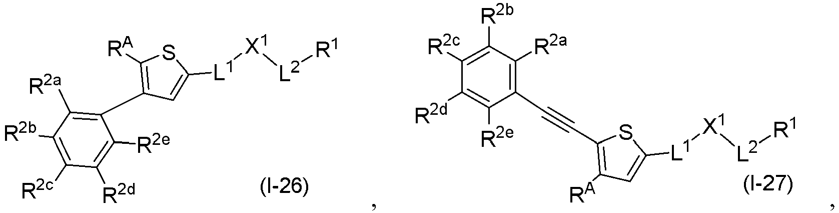

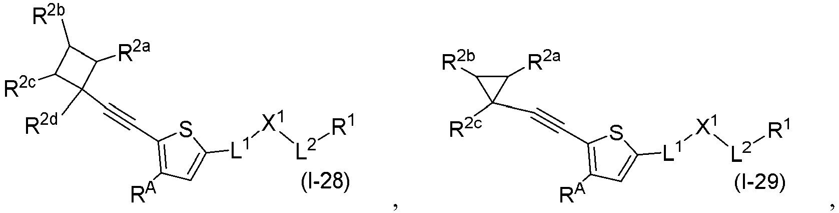

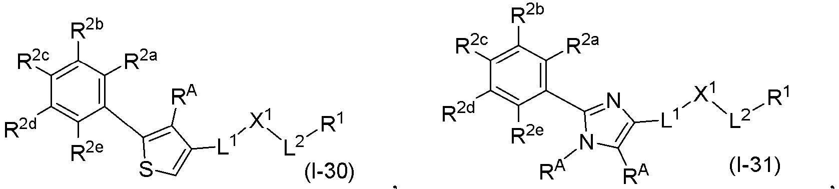

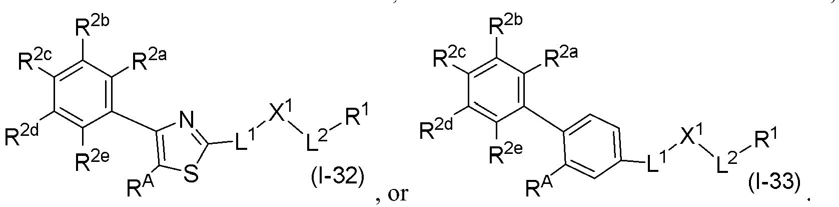

15. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-14, **characterised in that** X¹ is selected from -C(=O)- and -C(=S)-; preferably, X¹ is -C(=O)-.

16. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-15, **characterised in that** R^{a} and R^{b}, at each occurrence, are each independently selected from H, D, F, Cl, Br, -OH, -CN, C₁₋₄ alkyl, C₁₋₄ alkoxy, C₁₋₄ haloalkyl, C₁₋₄ haloalkoxy and C₃₋₄ cycloalkyl;
preferably, R^{a} and R^{b}, at each occurrence, are each independently H.

17. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-16, **characterised in that** R^{c}, at each occurrence, is independently selected from H, D, C₁₋₄ alkyl, C₁₋₄ haloalkyl and C₃₋₄ cycloalkyl; preferably, R^{c}, at each occurrence, is independently H and C₁₋₄ alkyl, preferably H, methyl, ethyl, n-propyl, isopropyl, n-butyl, sec-butyl, isobutyl and tert-butyl, more preferably H and methyl.

18. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-17, **characterised in that** L¹ is selected from -O-, -NR^{c}- and -CR^{a}R^{b}-; preferably, L¹ is -NR^{c}-; preferably, L¹ is selected from -O-, -S-, -NH-, -N(CH₃)- and -CH₂-; more preferably, L¹ is -NH-.

19. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-18, **characterised in that**
L² is selected from -O-, -S-, -NR^{c}-, -CR^{a}R^{b}-, #-O-CR^{a}R^{b}-* and #-S-CR^{a}R^{b}-*, wherein the bond labelled "#" is attached to X¹ and the bond labelled "*" is attached to R¹;
preferably, L² is selected from -O- and -CR^{a}R^{b}-;
preferably, L² is -O-;
preferably, L² is selected from -O-, -S-, -NH-, -N(CH₃)-, -CH₂-, #-O-CH₂-* and #-S-CH₂-*, wherein the bond labelled "#" is attached to X¹ and the bond labelled "*" is attached to R¹;
preferably, L² is selected from -O-, -S-, -NH-, -N(CH₃)-, -CH₂- and #-O-CH₂-*, wherein the bond labelled "#" is attached to X¹ and the bond labelled "*" is attached to R¹;
more preferably, L² is selected from -O- and -CH₂-.

20. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-19, **characterised in that**
moiety -L¹-X¹-L²- is selected from: 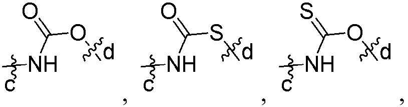 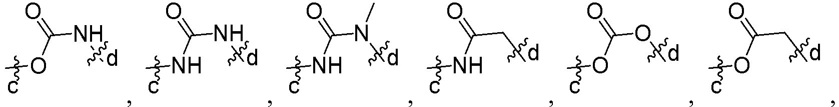 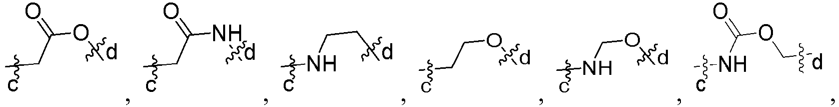 and -NH-, preferably 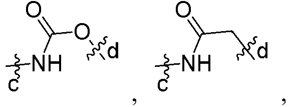 and -NH-, more preferably 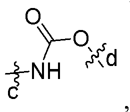 wherein the bond labelled "c" is attached to ring A and the bond labelled "d" is attached to R¹.

21. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-20, **characterised in that** the compound has a structure shown in formula (III'): 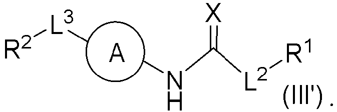
wherein X is selected from O and S; preferably, X is O;
preferably, the compound has a structure shown in formula (III): 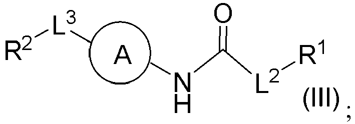
preferably, the compound has a structure shown in formula (III-1): 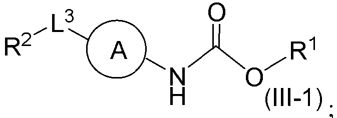
preferably, the compound has a structure shown in any of formulas (III-A) to (III-F):

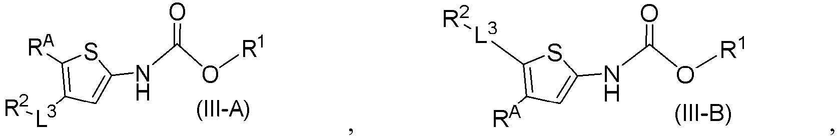

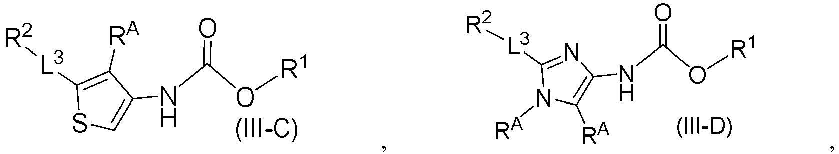

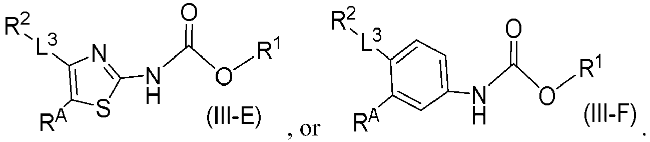

22. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-21, **characterised in that** the compound has a structure shown in formula (III-2) or (III-3): 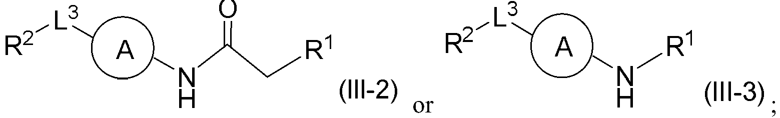
preferably, the compound has a structure shown in formula (III-G) or (III-H): 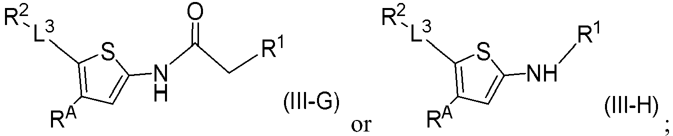
more preferably, the compound has a structure shown in formula (III-I) or (III-J):

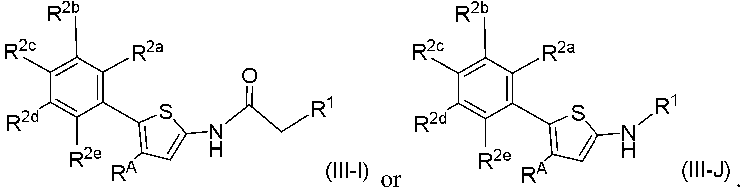

23. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-22, **characterised in that**
R¹ is selected from C₁₋₄ alkyl, C₃₋₆ cycloalkyl, 4- to 6-membered monocyclic heterocyclyl, 8- to 10-membered fused bicyclic heterocyclyl, phenyl and 5- to 6-membered heteroaryl, wherein the C₁₋₄ alkyl, C₃₋₆ cycloalkyl, 4- to 6-membered monocyclic heterocyclyl, 8- to 10-membered fused bicyclic heterocyclyl, phenyl and 5- to 6-membered heteroaryl are optionally substituted with 1, 2 or 3 R^{1a};
preferably, R¹ is selected from C₁₋₄ alkyl, C₃₋₆ cycloalkyl, 4- to 6-membered monocyclic nitrogen-containing heterocyclyl, 4- to 6-membered monocyclic oxygen-containing heterocyclyl, 8- to 10-membered fused bicyclic oxygen-containing heterocyclyl, phenyl and 5- to 6-membered heteroaryl, wherein the C₁₋₄ alkyl, C₃₋₆ cycloalkyl, 4- to 6-membered monocyclic nitrogen-containing heterocyclyl, 4- to 6-membered monocyclic oxygen-containing heterocyclyl, 8- to 10-membered fused bicyclic oxygen-containing heterocyclyl, phenyl and 5- to 6-membered heteroaryl are optionally substituted with 1, 2 or 3 R^{1a};
preferably, R¹ is selected from C₁₋₄ alkyl, C₃₋₆ cycloalkyl, 8- to 10-membered fused bicyclic oxygen-containing heterocyclyl, phenyl and 5- to 6-membered heteroaryl, wherein any of the above groups is optionally substituted with 1, 2 or 3 R^{1a};
preferably, R¹ is selected from C₁₋₄ alkyl, C₃₋₆ cycloalkyl, and 8- to 10-membered fused bicyclic oxygen-containing heterocyclyl, wherein any of the above groups is optionally substituted with 1, 2 or 3 R^{1a};
preferably, R¹ is selected from C₃₋₆ cycloalkyl and 8- to 10-membered fused bicyclic oxygen-containing heterocyclyl, wherein any of the above groups is optionally substituted with 1, 2 or 3 R^{1a};
preferably, R¹ is selected from methyl, ethyl, n-propyl, isopropyl, sec-butyl, isobutyl, tert-butyl, cyclopropyl, cyclobutyl, cyclopentyl, cyclohexyl, oxetanyl, tetrahydrofuryl, tetrahydropyranyl, thietanyl, tetrahydrothienyl, tetrahydrothiopyranyl, azetidinyl, pyrrolidyl, piperidyl, oxadiazolyl, thiadiazolyl, pyrimidyl, phenyl and 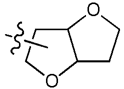 (preferably 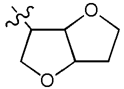 ), wherein any of the above groups is optionally substituted with 1, 2 or 3 R^{1a};
preferably, R¹ is selected from n-propyl, cyclopentyl, cyclohexyl, oxadiazolyl, thiadiazolyl, pyrimidyl, phenyl and 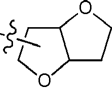
(preferably 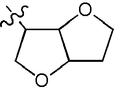 ), wherein any of the above groups is optionally substituted with 1, 2 or 3 R^{1a};
preferably, R¹ is selected from n-propyl, cyclopentyl, cyclohexyl and 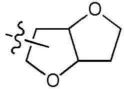
(preferably 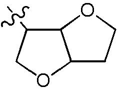 ), wherein any of the above groups is optionally substituted with 1, 2 or 3 R^{1a};
preferably, R¹ is selected from cyclopentyl and 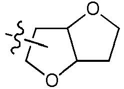
(preferably 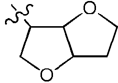 ), wherein any of the above groups is optionally substituted with 1, 2 or 3 R^{1a};
preferably, R¹ is 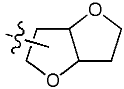 (preferably 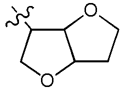 ), which is optionally substituted with 1, 2 or 3 R^{1a};
preferably, R¹ is a group selected from the following (1) to (2):
(1) methyl, ethyl and n-propyl, and the methyl, ethyl and n-propyl are each optionally substituted with 1, 2 or 3 R^{1a};
(2) 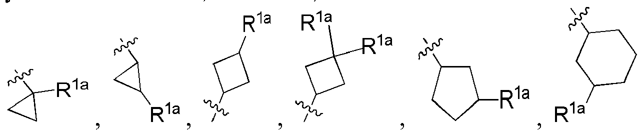 (preferably 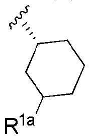 ), 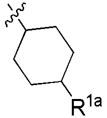 (preferably ), 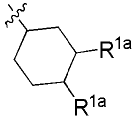 (preferably 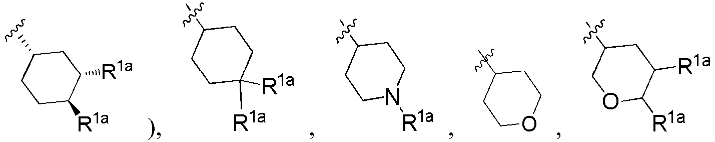 (preferably 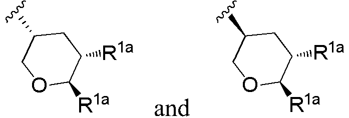 ), 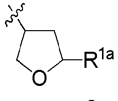 (preferably 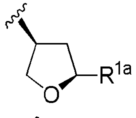 ), 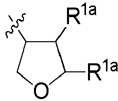 (preferably 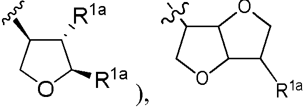 (preferably 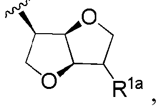 more preferably 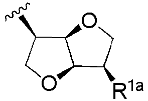 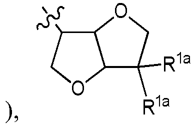 (preferably 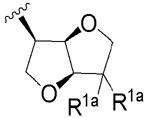 ), 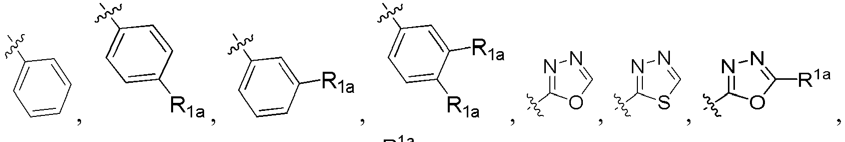 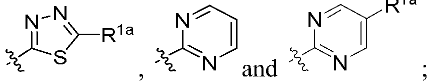
preferably, R¹ is selected from: 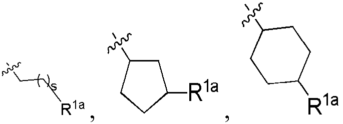 (preferably 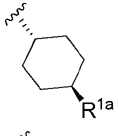 ), and 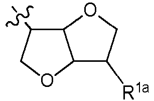 (preferably 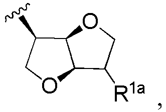 more preferably 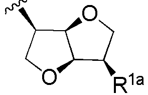 ), wherein s is selected from 0, 1 or 2, preferably, s is 2;
preferably, R¹ is selected from: 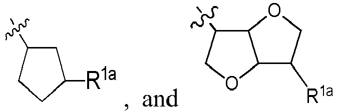 (preferably 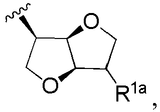 more preferably 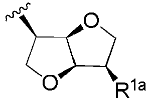 );
more preferably, R¹ is 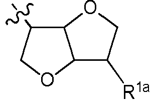 (preferably 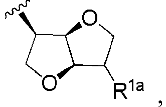 more preferably 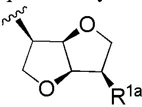 ).

24. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-23, **characterised in that**
each R^{1a} is independently selected from halogen, -OH, -CN, an oxo group, - COOR^{c}, C₁₋₄ alkyl, C₁₋₄ haloalkyl, C₁₋₄ alkoxy, C₁₋₄ haloalkoxy, -C₁₋₄ alkylene-OH, -OC₁₋₄ alkylene-OH, -C₁₋₄ alkylene-COOH, -S(=O)₂-C₁₋₄ alkyl and 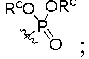
preferably, each R^{1a} is independently selected from -OH, -COOR^{c}, and -C₁₋₄ alkylene-OH;
preferably, each R^{1a} is independently selected from -OH and -C₁₋₂ alkylene-OH;
preferably, each R^{1a} is independently selected from F, Cl, Br, -OH, an oxo group, -COOH, methyl, ethyl, isopropyl, tert-butyl, -CH₂-OH, -CH₂CH₂-OH, - C(CH₃)₂-OH, -CH₂-COOH, -CH₂CH₂-COOH, -C(CH₃)₂-COOH, -S(=O)₂-CH₃ and 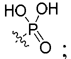
preferably, each R^{1a} is independently selected from Cl, -OH, -CN, an oxo group, -COOH, -COOEt, methyl, ethyl, -CH₂-OH, -CH₂CH₂-OH, -OCH₂CH₂-OH, - C(CH₃)₂-OH, -CH₂-COOH, -CH₂CH₂-COOH, -C(CH₃)₂-COOH, -S(=O)₂-CH₃ and 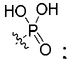
preferably, each R^{1a} is independently selected from -OH, -CH₂-OH, -COOH, and -COOEt;
preferably, each R^{1a} is independently selected from -OH, -CH₂-OH, and - COOH;
preferably, each R^{1a} is independently selected from -OH and -CH₂-OH;
more preferably, each R^{1a} is independently -OH.

25. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-24, **characterised in that** R¹ is selected from: 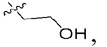 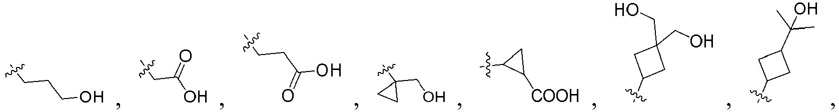 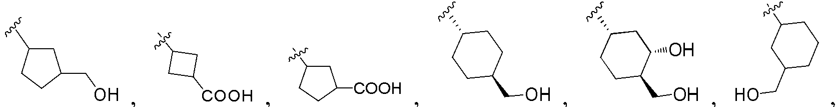 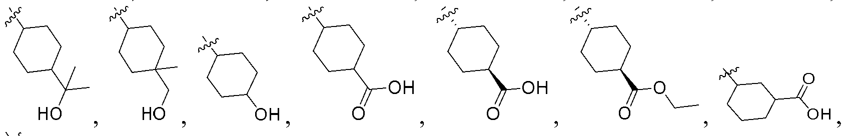 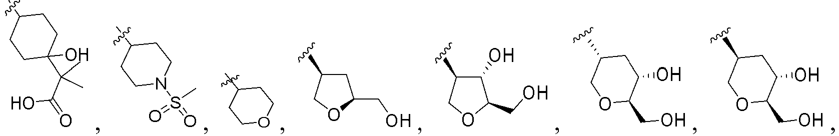 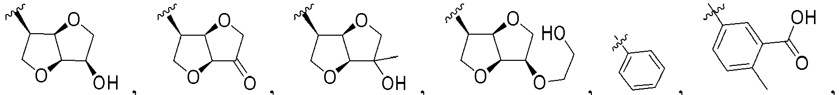 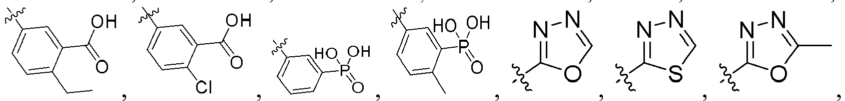 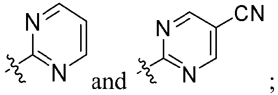
preferably, R¹ is selected from: 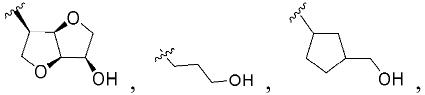 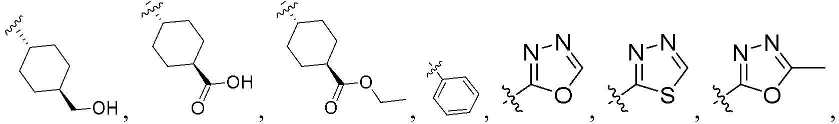 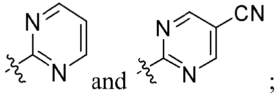
preferably, R¹ is selected from: 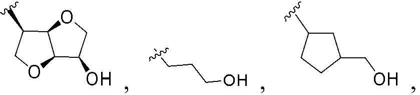 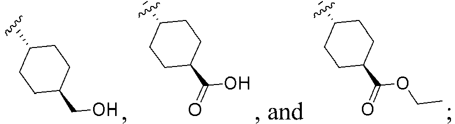
preferably, R¹ is selected from: 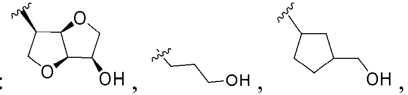 and 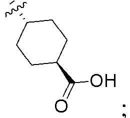
preferably, R¹ is selected from: 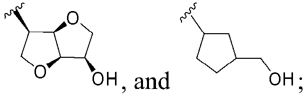
more preferably, R¹ is

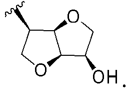

26. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-25, **characterised in that** moiety -L¹-X¹-L²-R¹ is selected from: 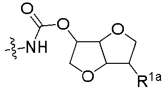 (preferably 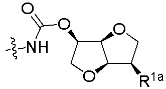 ), 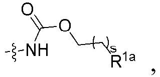 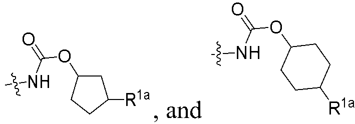 (preferably 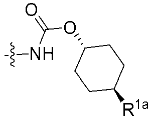 );
preferably, moiety -L¹-X¹-L²-R¹ is selected from: 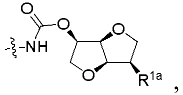 and 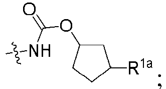
more preferably, moiety -L¹-X¹-L²-R¹ is

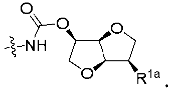

27. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-26 **characterised in that** moiety -L¹-X¹-L²-R¹ is selected from: 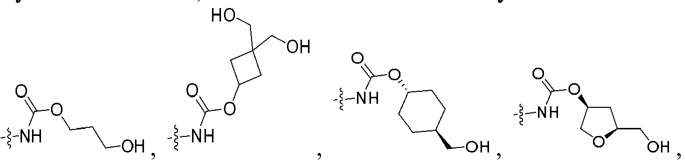 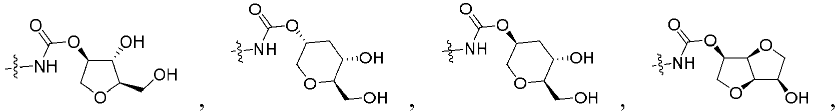 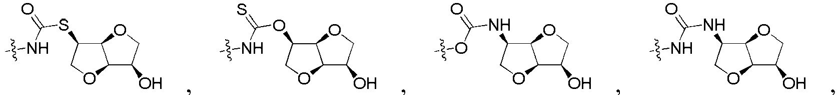 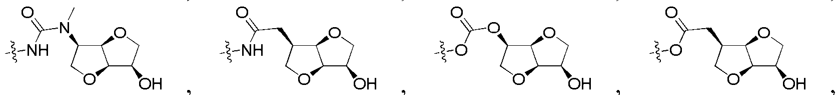 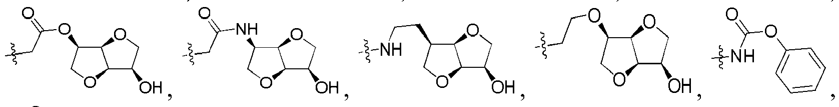 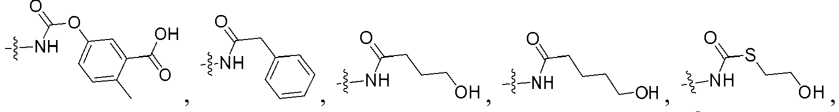 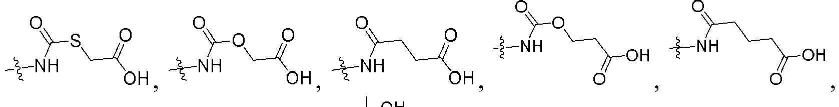 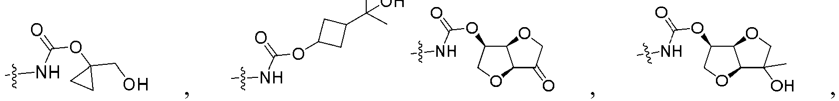 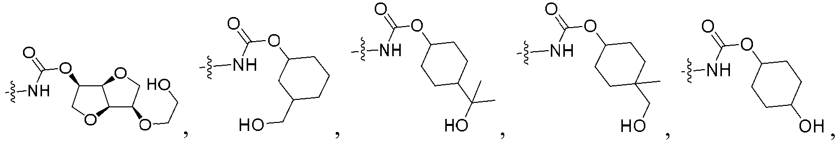 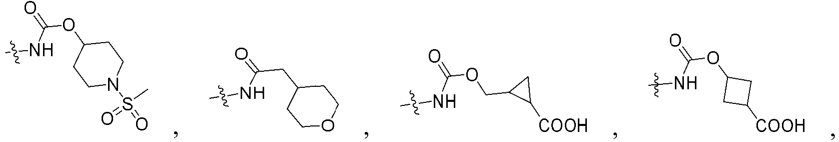 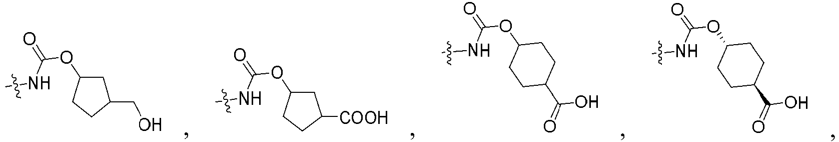 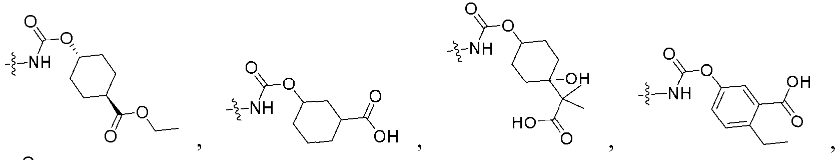 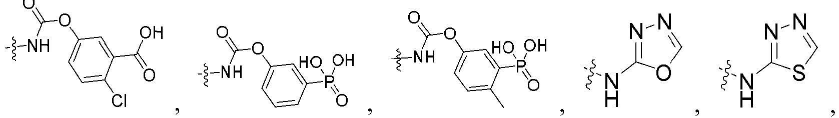 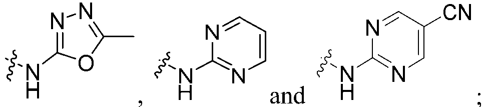
preferably, moiety -L¹-X¹-L²-R¹ is selected from: 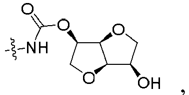 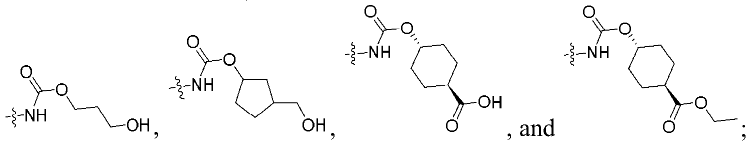
preferably, moiety -L¹-X¹-L²-R¹ is selected from: 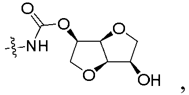 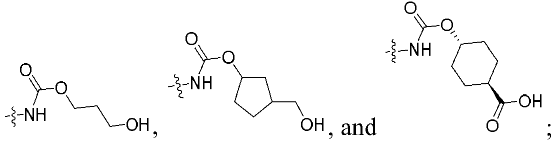
preferably, moiety -L¹-X¹-L²-R¹ is selected from: 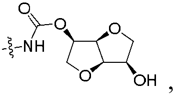 and 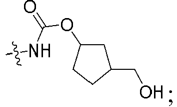
more preferably, moiety -L¹-X¹-L²-R¹ is

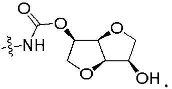

28. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-27, **characterised in that** the compound has a structure shown in any of formulas (IV-1) to (IV-4): 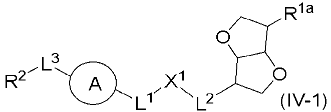 (preferably 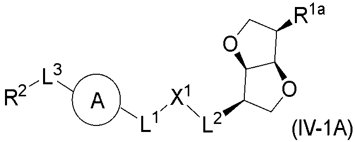 ), 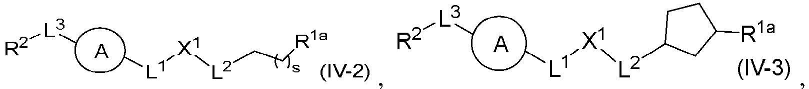 or 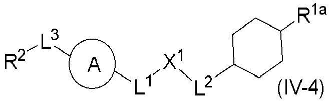 (preferably 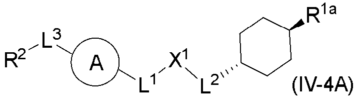 );
preferably, the compound has a structure shown in any of formulas (IV-5) to (IV-8): 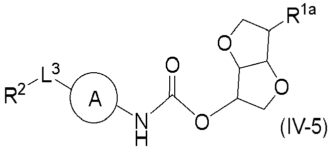 (preferably 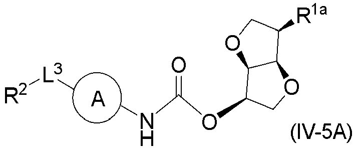 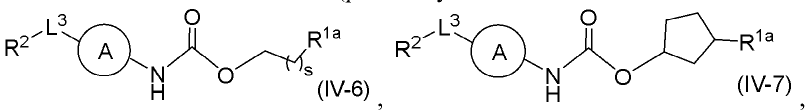 or 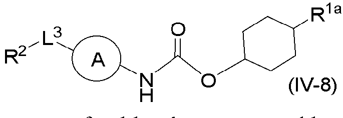 (preferably 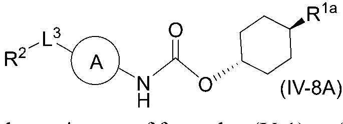 );
preferably, the compound has a structure shown in any of formulas (V-1) to (V-9): 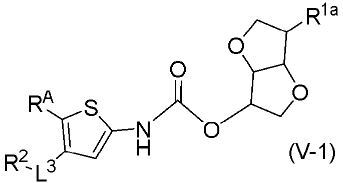 (preferably 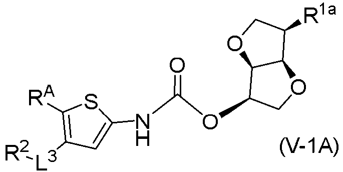 ), 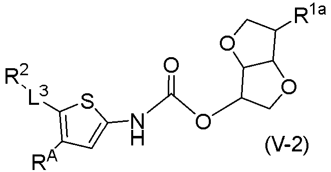 (preferably 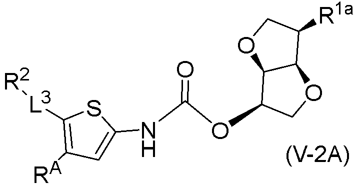 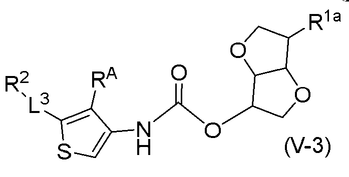 (preferably 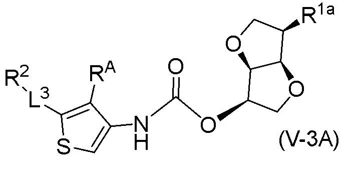 ), 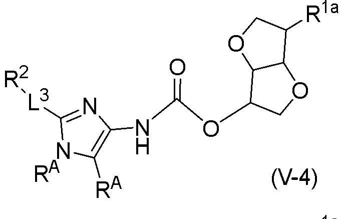 (preferably 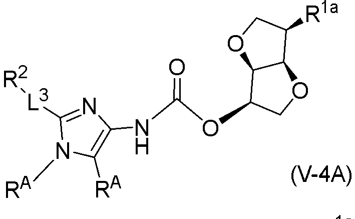 ), 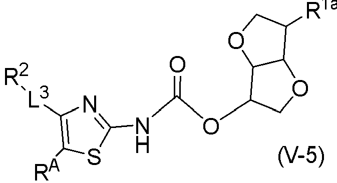 (preferably 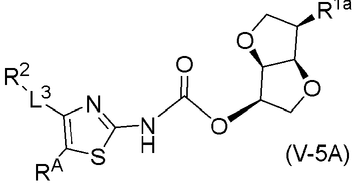 ), 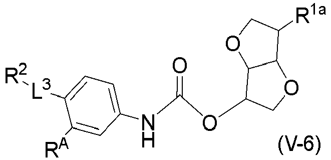 (preferably 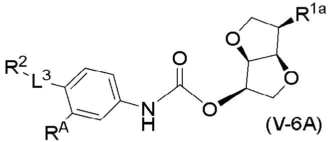 ), 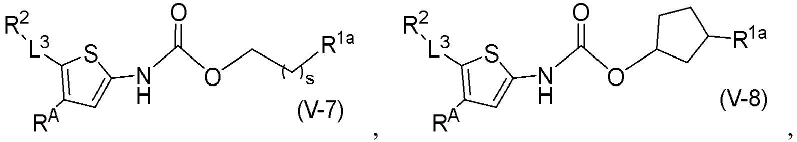 or 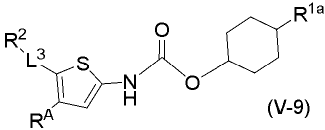 (preferably 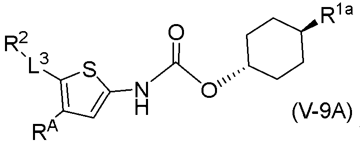
more preferably, the compound has a structure shown in any of formulas (VI-1) to (VI-14): 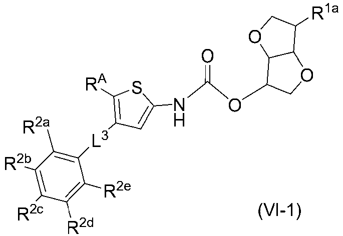 (preferably 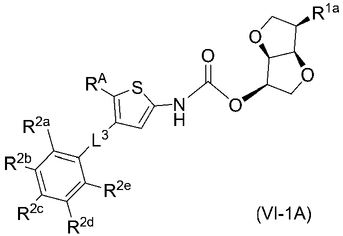 ), 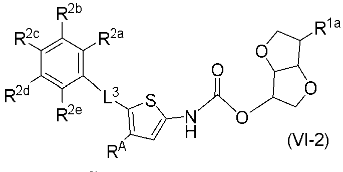 (preferably 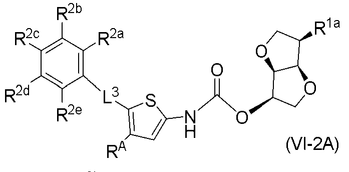 ), 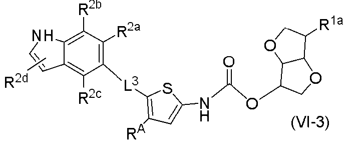 (preferably 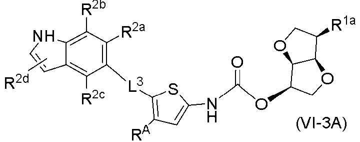 ), 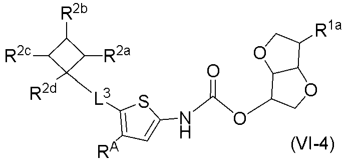 (preferably 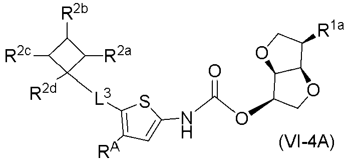 ), 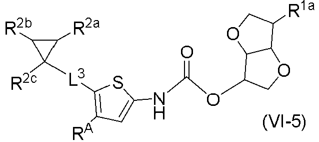 (preferably 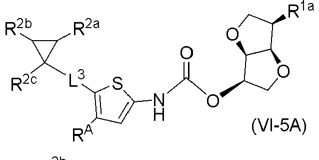 ), 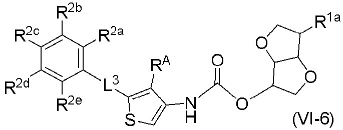 (preferably 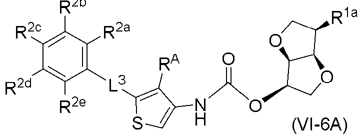 ), 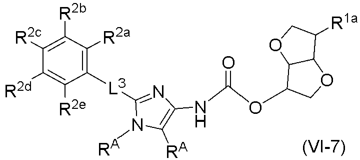 (preferably 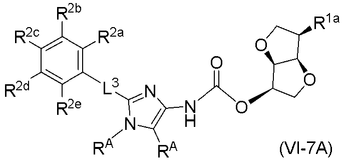 ), 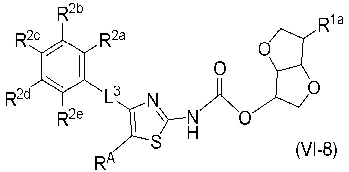 (preferably 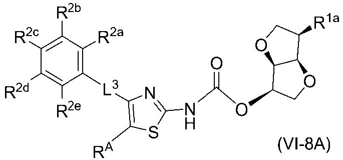 ), 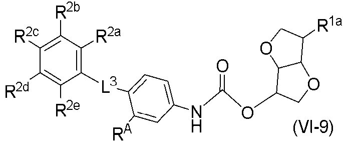 (preferably 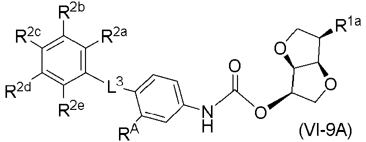 ), 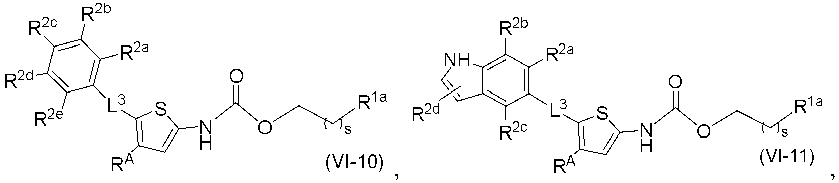 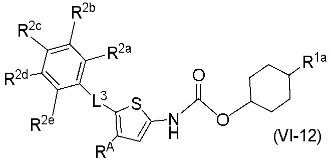 (preferably 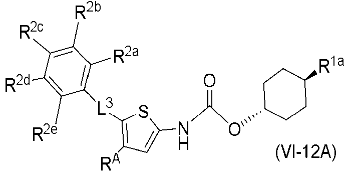 ), 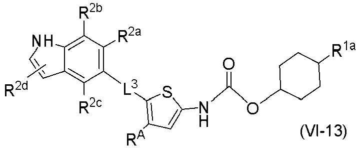 (preferably 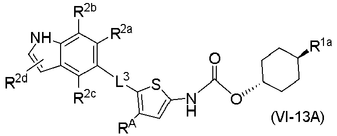 ), or

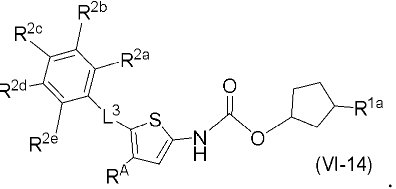

29. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-28, **characterised in that** the compound has a structure shown in any of formulas (I-3) and (X-1) to (X-5): 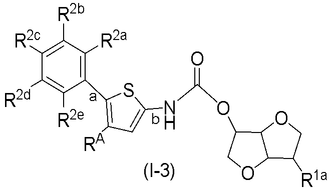 (preferably 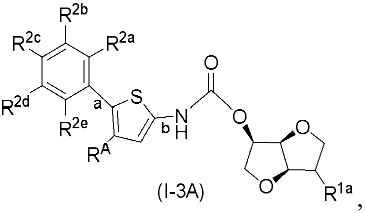 more preferably 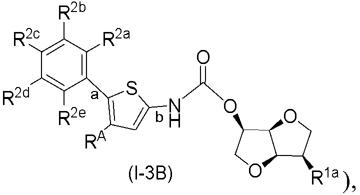 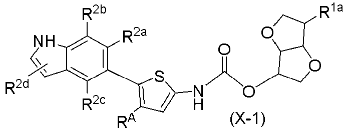 (preferably 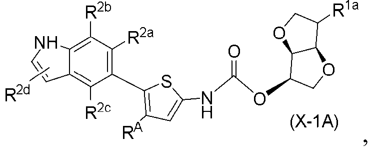 more preferably 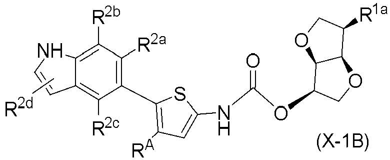 ), 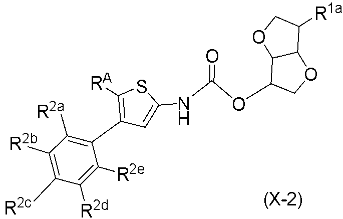 (preferably 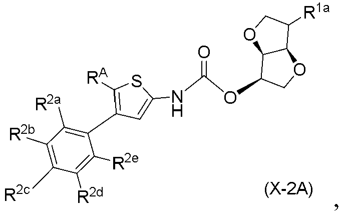 more preferably 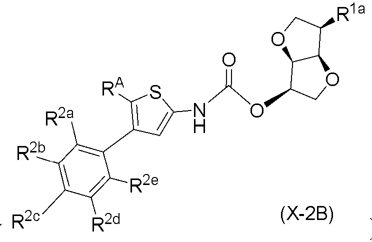 ), 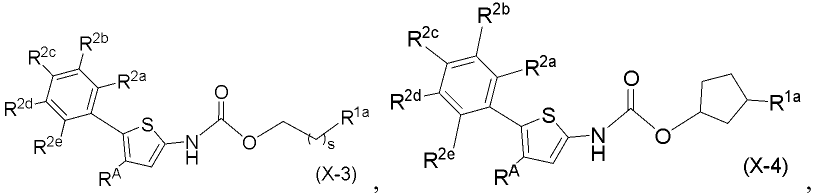 or 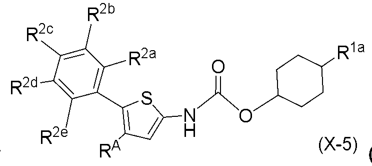 preferably 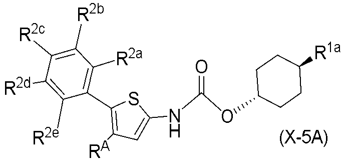 ).

30. The compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-29 **characterised in that** the compound is selected from: 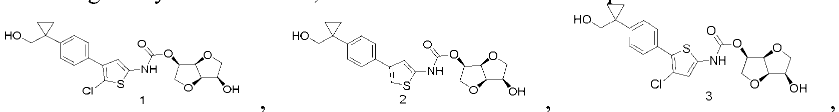 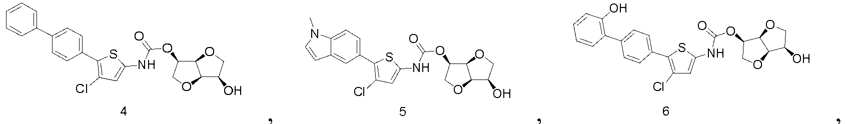 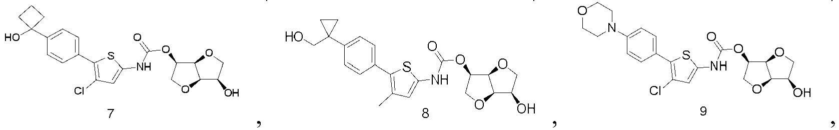 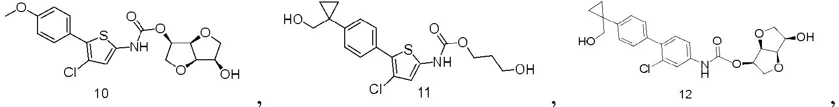 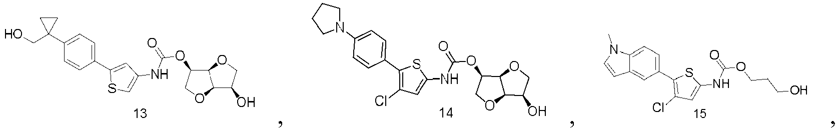 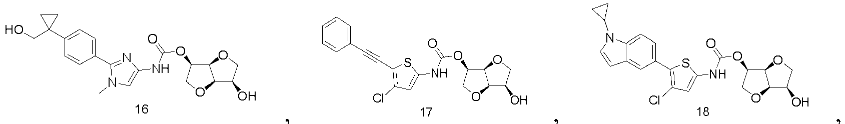 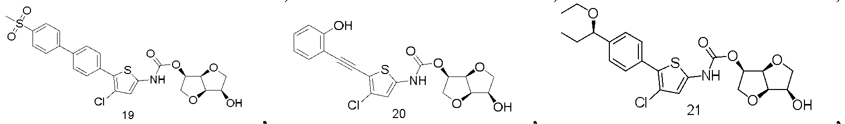 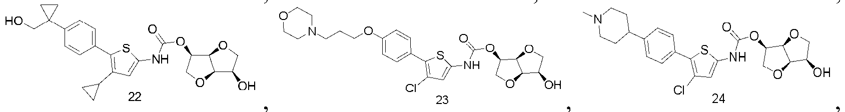 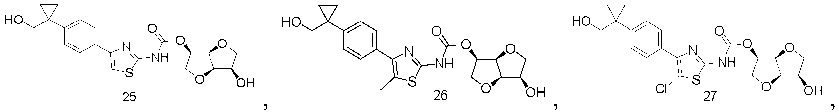 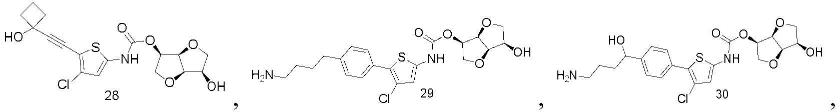 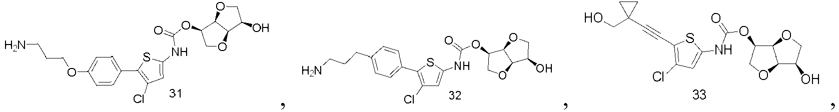 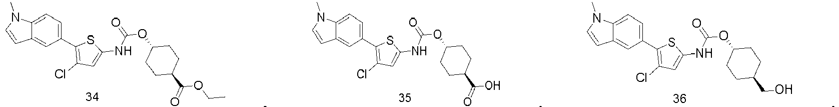 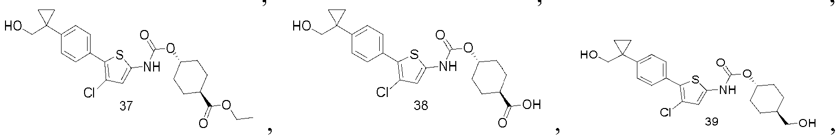 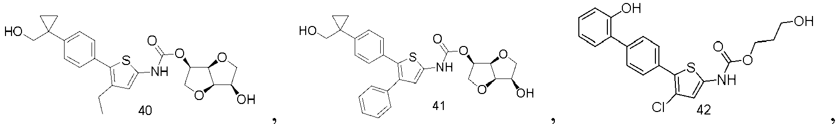 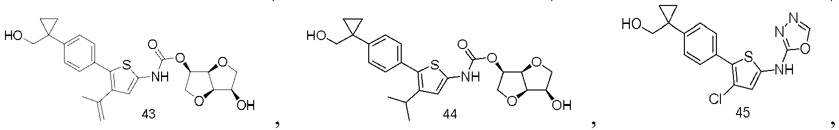 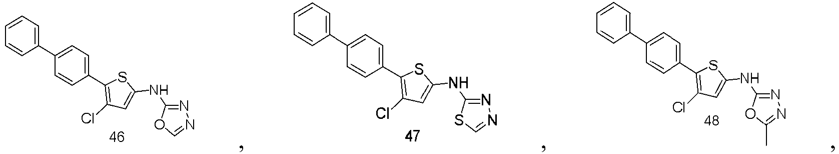 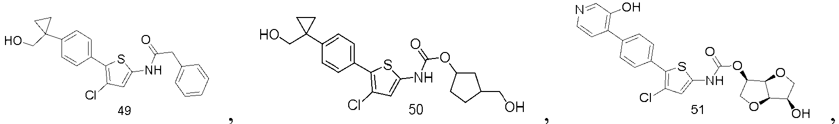 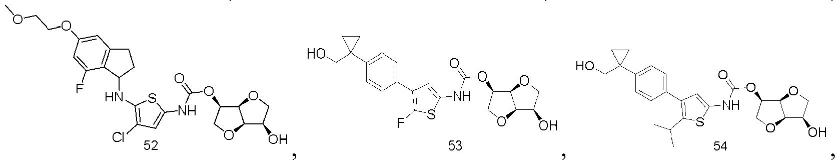 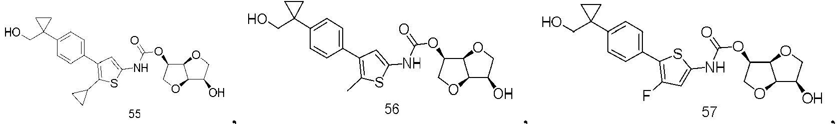 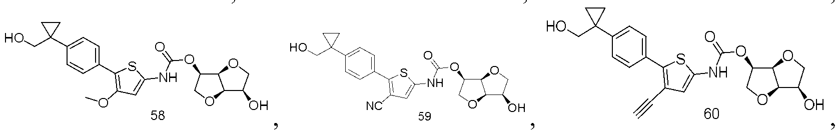 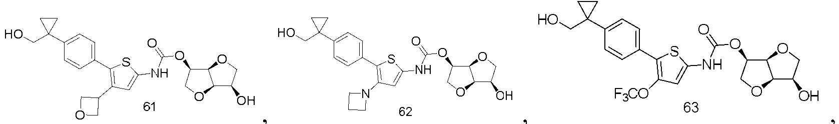 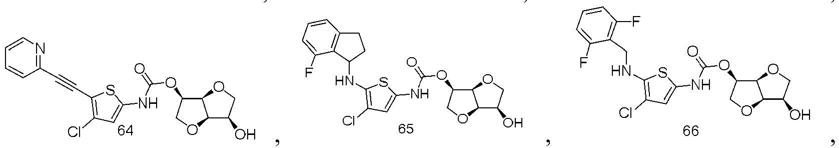 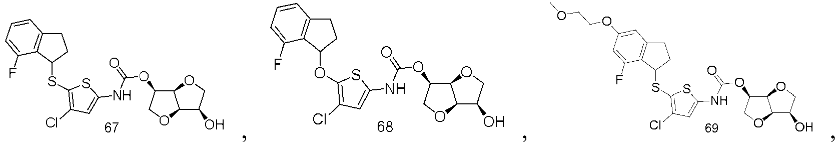 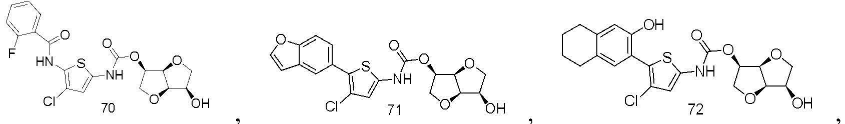 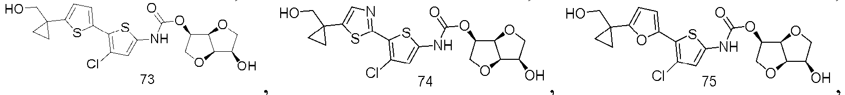 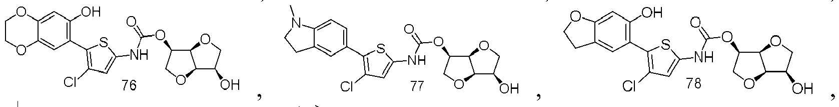 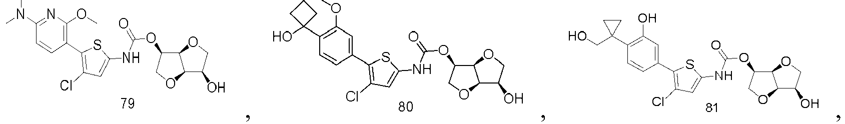 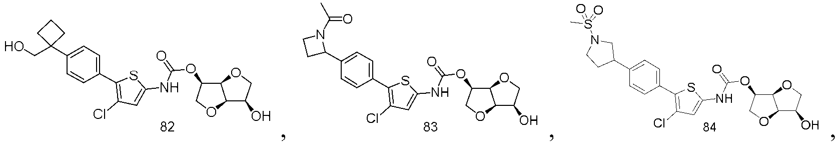 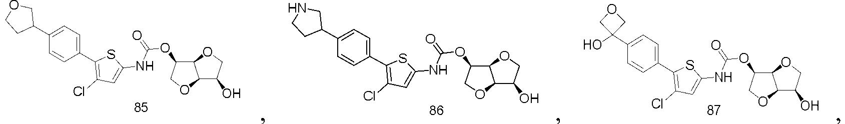 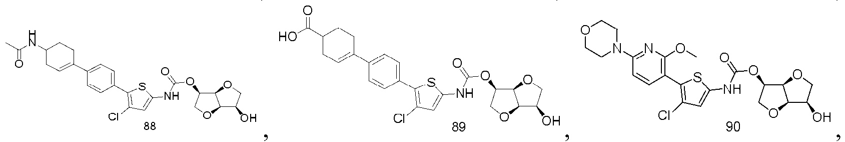 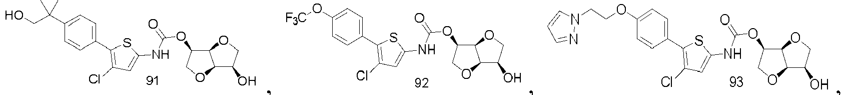 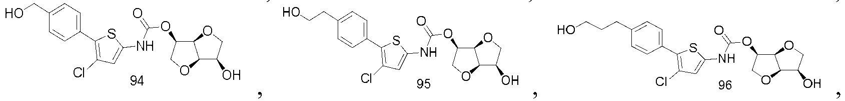 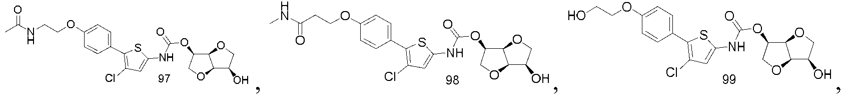 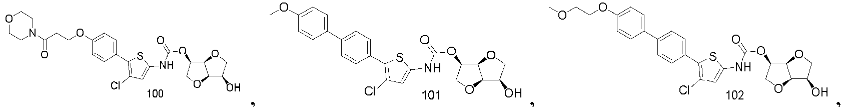 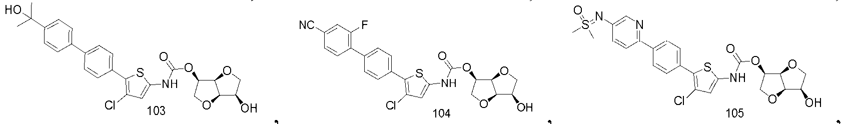 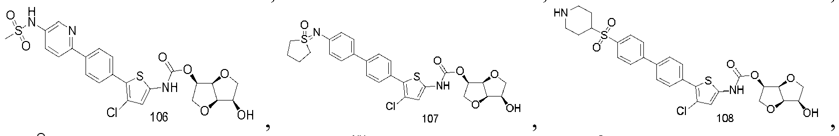 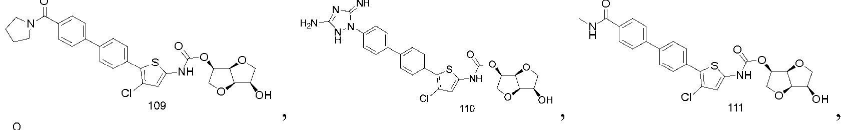 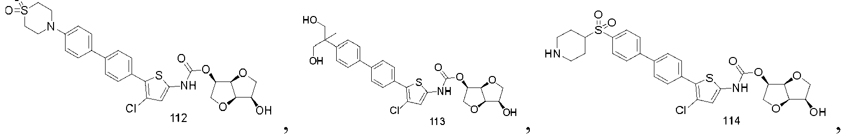 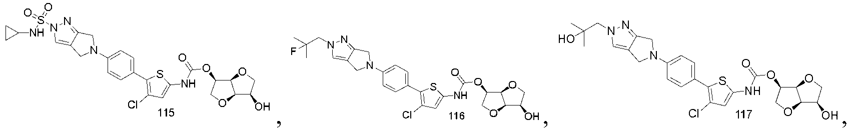 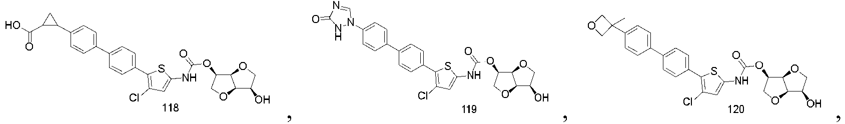 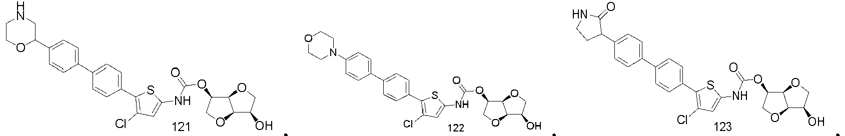 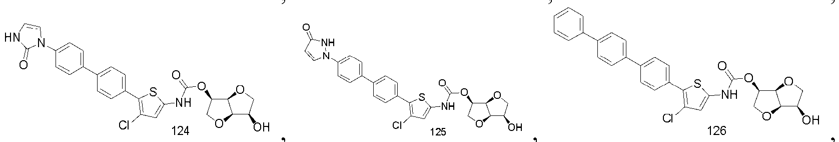 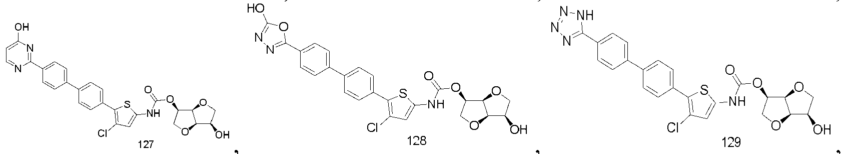 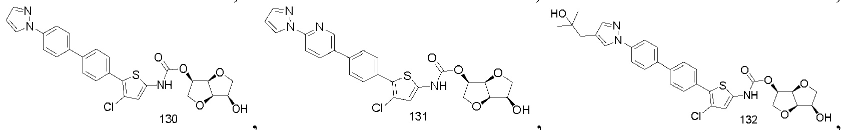 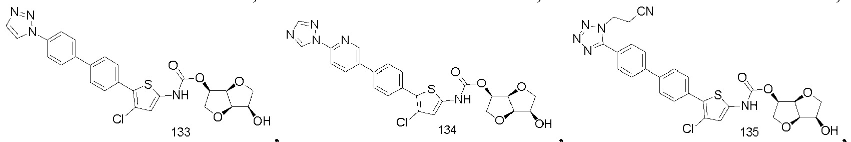 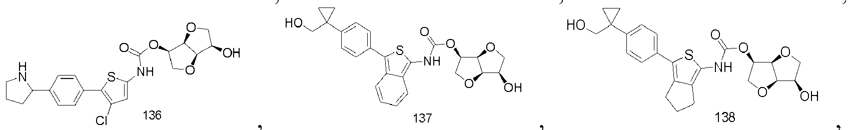 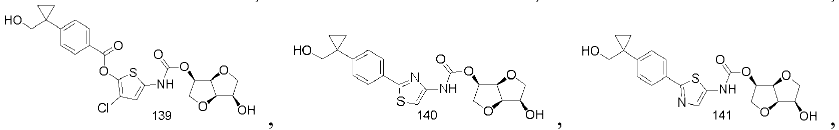 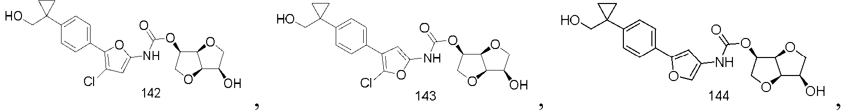 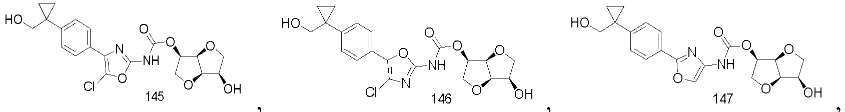 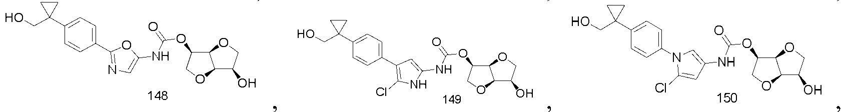 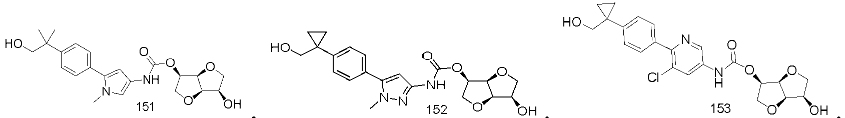 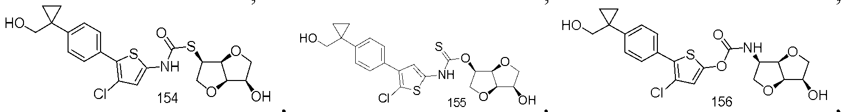 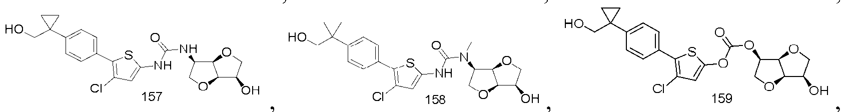 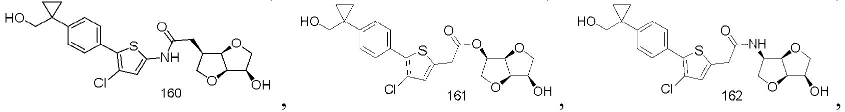 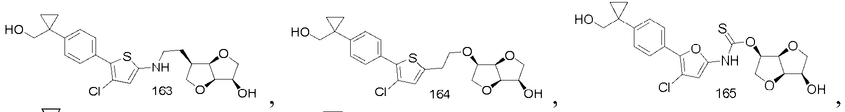 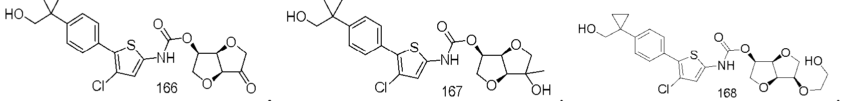 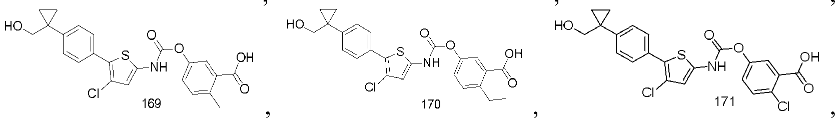 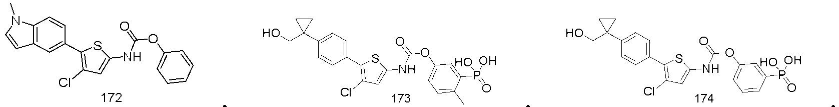 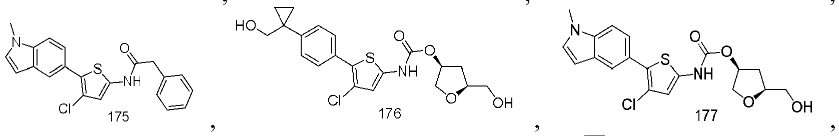 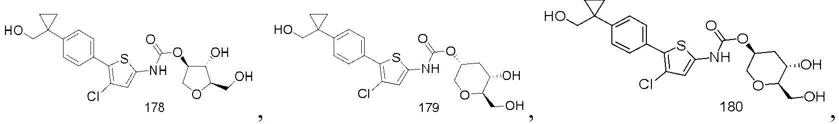 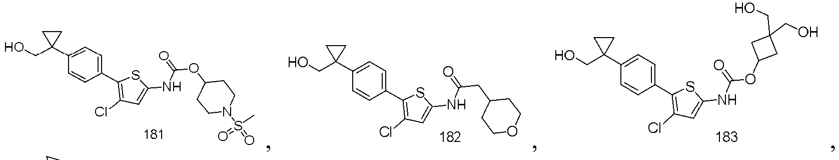 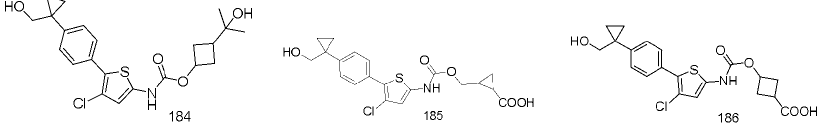 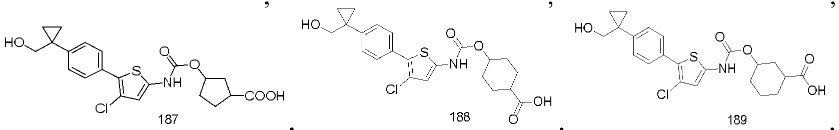 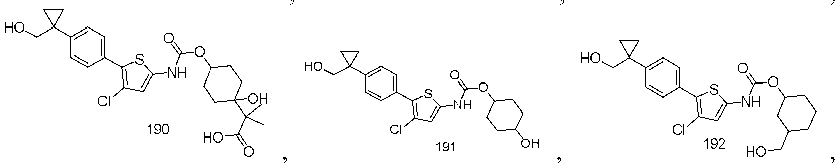 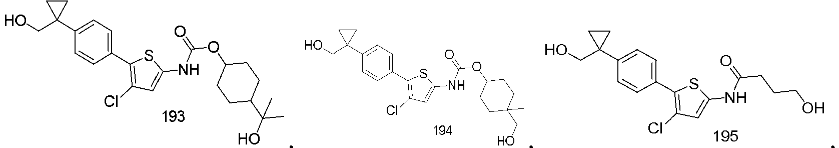 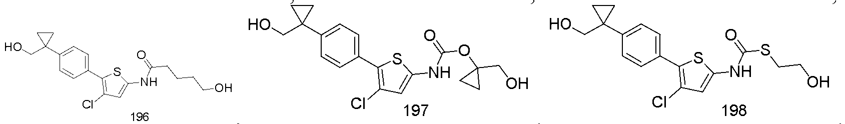 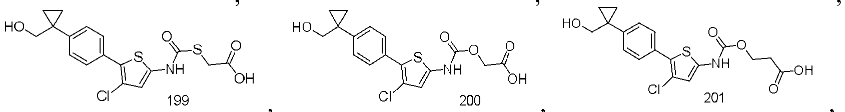 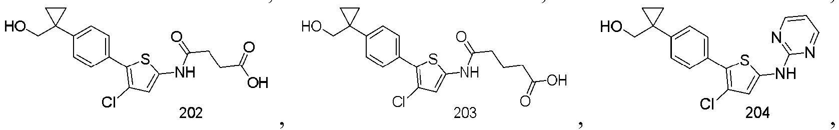 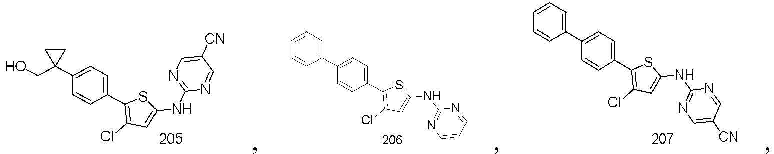 and

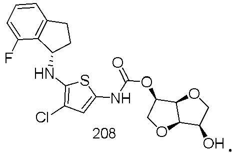

31. A pharmaceutical composition, **characterised by** comprising the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-30, and one or more pharmaceutically acceptable excipients or carriers.

32. Use of the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-30, or the pharmaceutical composition according to claim 31 in the preparation of a medicament as an AMPK agonist.

33. Use of the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-30, or the pharmaceutical composition according to claim 31 in the preparation of a medicament for treating and/or preventing a disease, disorder or condition associated with AMPK kinase, **characterised in that** the disease, disorder or condition is preferably selected from type II diabetes, dyslipidaemia, obesity, chronic kidney disease, diabetic nephropathy, acute kidney injury, polycystic kidney disease and alopecia.

34. Use of the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-30, or the pharmaceutical composition according to claim 31 in the preparation of a medicament for treating and/or preventing a disease, disorder or condition that can be treated and/or prevented by activating AMPK, **characterised in that** the disease, disorder or condition is preferably selected from type II diabetes, dyslipidaemia, obesity, chronic kidney disease, diabetic nephropathy, acute kidney injury, polycystic kidney disease and alopecia.

35. A method for treating and/or preventing a disease, disorder or condition that can be treated and/or prevented by activating AMPK, **characterised in that** the method comprises administering to an individual in need thereof a therapeutically effective amount of the compound, or the stereoisomer, the tautomer, the diastereomer, the racemate, the cis-trans isomer, the isotopically labelled compound (preferably the deuterated compound), the N-oxide, the metabolite, the ester, the prodrug, the crystal form, the hydrate, the solvate or the pharmaceutically acceptable salt thereof according to any of claims 1-30, or the pharmaceutical composition according to claim 31, and the disease, disorder or condition is preferably selected from type II diabetes, dyslipidaemia, obesity, chronic kidney disease, diabetic nephropathy, acute kidney injury, polycystic kidney disease and alopecia.
